# EUROPEAN PATENT APPLICATION

(11) **EP 4 023 230 A1**
(43) Date of publication of application: **06.07.2022**
(21) Application number: 20819246.8
(22) Date of filing: 05.06.2020
(51) Int. Cl.: A61K 35/14, A61K 39/395, A61P 35/00, A61P 35/02, C07K 14/78, C07K 16/00, C07K 19/00

(54) **ANTIBODY CLEAVAGE SITE-BINDING MOLECULE**

(30) Priority: 05.06.2019 JP 2019105761
(71) Applicant: Chugai Seiyaku Kabushiki Kaisha, Kita-ku Tokyo 115-8543 (JP); Yamaguchi University, Yamaguchi 753-8511 (JP)
(72) Inventor: SAKURAI, Mika, Gotemba-shi, Shizuoka 412-8513 (JP); IGAWA, Tomoyuki, Synapse, 138623 (SG); KOMORI, Yasunori, Gotemba-shi, Shizuoka 412-8513 (JP); TAMADA, Koji, Ube-shi, Yamaguchi 755-8505 (JP); SAKODA, Yukimi, Ube-shi, Yamaguchi 755-8505 (JP)
(74) Representative: Vossius & Partner Patentanwälte Rechtsanwälte mbB
(86) International application number: PCT/JP2020/022218
(87) International publication number: WO 2020/246563

(57) **Abstract**

The present disclosure provides a pharmaceutical composition comprising an antibody having ADCC activity, a T cell-redirecting antibody, or a cell expressing a chimeric receptor, for use in combination with the administration of an antigen binding molecule capable of binding to a target antigen, wherein the primary molecule comprises a linker that is cleaved by protease, the antigen binding molecule obtained through the cleavage of the linker has the ability to bind to the target antigen, variable regions of the antibody having ADCC activity or the T cell-redirecting antibody, and an extracellular binding domain of the chimeric receptor bind to a cell expressing the target antigen via binding to the antigen binding molecule resulting from the cleavage of the cleavable linker.

SEQUENCE LISTING

<110>  CHUGAI SEIYAKU KABUSHIKI KAISHA

<120>  Molecular binding to antigen-binding fragment

<130>  FA0001-20073

<150>  JP 2019-105761
<151>  2019-06-05

<160>  749

<170>  PatentIn version 3.5

<210>  1
<211>  12
<212>  PRT
<213>  Artificial sequence

<220>
<223>  an artificially synthesized sequence

<400>  1

Thr Ser Gly Ser Gly Arg Ser Ala Asn Ala Arg Gly
1               5                   10


<210>  2
<211>  12
<212>  PRT
<213>  Artificial sequence

<220>
<223>  an artificially synthesized sequence

<400>  2

Thr Ser Gln Ser Gly Arg Ser Ala Asn Gln Arg Gly
1               5                   10


<210>  3
<211>  12
<212>  PRT
<213>  Artificial sequence

<220>
<223>  an artificially synthesized sequence

<400>  3

Thr Ser Pro Ser Gly Arg Ser Ala Tyr Pro Arg Gly
1               5                   10


<210>  4
<211>  12
<212>  PRT
<213>  Artificial sequence

<220>

<223>  an artificially synthesized sequence

<400>  4

Thr Ser Gly Ser Gly Arg Ser Ala Thr Pro Arg Gly
1               5                   10

<210>  5
<211>  12
<212>  PRT
<213>  Artificial sequence

<220>
<223>  an artificially synthesized sequence

<400>  5

Thr Ser Gln Ser Gly Arg Ser Ala Thr Pro Arg Gly
1               5                   10

<210>  6
<211>  12
<212>  PRT
<213>  Artificial sequence

<220>
<223>  an artificially synthesized sequence

<400>  6

Thr Ser Ala Ser Gly Arg Ser Ala Thr Pro Arg Gly
1               5                   10

<210>  7
<211>  12
<212>  PRT
<213>  Artificial sequence

<220>
<223>  an artificially synthesized sequence

<400>  7

Thr Ser Tyr Ser Gly Arg Ser Ala Val Pro Arg Gly
1               5                   10

<210>  8
<211>  12
<212>  PRT
<213>  Artificial sequence

<220>
<223>  an artificially synthesized sequence

<400>  8

Thr Ser Tyr Ser Gly Arg Ser Ala Asn Phe Arg Gly
1               5                   10

<210>  9
<211>  12
<212>  PRT
<213>  Artificial sequence

<220>
<223>  an artificially synthesized sequence

<400>  9

Thr Ser Ser Ser Gly Arg Ser Ala Thr Pro Arg Gly
1               5                   10


<210>  10
<211>  12
<212>  PRT
<213>  Artificial sequence

<220>
<223>  an artificially synthesized sequence

<400>  10

Thr Ser Thr Thr Gly Arg Ser Ala Ser Pro Arg Gly
1               5                   10


<210>  11
<211>  12
<212>  PRT
<213>  Artificial sequence

<220>
<223>  an artificially synthesized sequence

<400>  11

Thr Ser Thr Ser Gly Arg Ser Ala Asn Pro Arg Gly
1               5                   10


<210>  12
<211>  16
<212>  PRT
<213>  Artificial sequence

<220>
<223>  an artificially synthesized sequence

<400>  12

Gly Gly Gly Gly Ser Pro Leu Gly Leu Ala Gly Gly Gly Gly Gly Ser
1               5                   10                  15


<210>  13
<211>  18
<212>  PRT
<213>  Artificial sequence

<220>

<223>  an artificially synthesized sequence

<400>  13

Gly Gly Gly Gly Ser Gly Pro Leu Gly Ile Ala Gly Gln Gly Gly Gly
1               5                   10                  15

Gly Ser

<210>  14
<211>  23
<212>  PRT
<213>  Artificial sequence

<220>
<223>  an artificially synthesized sequence

<400>  14

Gly Gly Gly Gly Ser Gly Ala Gly Val Pro Leu Ser Leu Tyr Ser Gly
1               5                   10                  15

Ala Gly Gly Gly Gly Gly Ser
            20

<210>  15
<211>  12
<212>  PRT
<213>  Artificial sequence

<220>
<223>  an artificially synthesized sequence

<400>  15

Thr Ser Ala Ser Gly Arg Ser Ala Asn Pro Arg Gly
1               5                   10

<210>  16
<211>  12
<212>  PRT
<213>  Artificial sequence

<220>
<223>  an artificially synthesized sequence

<400>  16

Thr Ser Glu Ser Gly Arg Ser Ala Asn Pro Arg Gly
1               5                   10

<210>  17
<211>  12
<212>  PRT
<213>  Artificial sequence

<220>
<223>  an artificially synthesized sequence

<400>  17

Thr Ser Phe Ser Gly Arg Ser Ala Asn Pro Arg Gly
1               5                   10


<210>  18
<211>  12
<212>  PRT
<213>  Artificial sequence

<220>
<223>  an artificially synthesized sequence

<400>  18

Thr Ser Gly Ser Gly Arg Ser Ala Asn Pro Arg Gly
1               5                   10


<210>  19
<211>  12
<212>  PRT
<213>  Artificial sequence

<220>
<223>  an artificially synthesized sequence

<400>  19

Thr Ser His Ser Gly Arg Ser Ala Asn Pro Arg Gly
1               5                   10


<210>  20
<211>  12
<212>  PRT
<213>  Artificial sequence

<220>
<223>  an artificially synthesized sequence

<400>  20

Thr Ser Lys Ser Gly Arg Ser Ala Asn Pro Arg Gly
1               5                   10


<210>  21
<211>  12
<212>  PRT
<213>  Artificial sequence

<220>
<223>  an artificially synthesized sequence

<400>  21

Thr Ser Met Ser Gly Arg Ser Ala Asn Pro Arg Gly
1               5                   10

<210>  22
<211>  12
<212>  PRT
<213>  Artificial sequence

<220>
<223>  an artificially synthesized sequence

<400>  22

Thr Ser Asn Ser Gly Arg Ser Ala Asn Pro Arg Gly
1               5                   10


<210>  23
<211>  12
<212>  PRT
<213>  Artificial sequence

<220>
<223>  an artificially synthesized sequence

<400>  23

Thr Ser Pro Ser Gly Arg Ser Ala Asn Pro Arg Gly
1               5                   10


<210>  24
<211>  12
<212>  PRT
<213>  Artificial sequence

<220>
<223>  an artificially synthesized sequence

<400>  24

Thr Ser Gln Ser Gly Arg Ser Ala Asn Pro Arg Gly
1               5                   10


<210>  25
<211>  12
<212>  PRT
<213>  Artificial sequence

<220>
<223>  an artificially synthesized sequence

<400>  25

Thr Ser Trp Ser Gly Arg Ser Ala Asn Pro Arg Gly
1               5                   10


<210>  26
<211>  12
<212>  PRT
<213>  Artificial sequence

<220>
<223>  an artificially synthesized sequence

<400>  26

Thr Ser Tyr Ser Gly Arg Ser Ala Asn Pro Arg Gly
1               5                   10


<210>  27
<211>  12
<212>  PRT
<213>  Artificial sequence

<220>
<223>  an artificially synthesized sequence

<400>  27

Thr Ser Thr Ala Gly Arg Ser Ala Asn Pro Arg Gly
1               5                   10


<210>  28
<211>  12
<212>  PRT
<213>  Artificial sequence

<220>
<223>  an artificially synthesized sequence

<400>  28

Thr Ser Thr Asp Gly Arg Ser Ala Asn Pro Arg Gly
1               5                   10


<210>  29
<211>  12
<212>  PRT
<213>  Artificial sequence

<220>
<223>  an artificially synthesized sequence

<400>  29

Thr Ser Thr Glu Gly Arg Ser Ala Asn Pro Arg Gly
1               5                   10


<210>  30
<211>  12
<212>  PRT
<213>  Artificial sequence

<220>
<223>  an artificially synthesized sequence

<400>  30

Thr Ser Thr Phe Gly Arg Ser Ala Asn Pro Arg Gly
1               5                   10

<210>  31
<211>  12
<212>  PRT
<213>  Artificial sequence

<220>
<223>  an artificially synthesized sequence

<400>  31

Thr Ser Thr Leu Gly Arg Ser Ala Asn Pro Arg Gly
1               5                   10


<210>  32
<211>  12
<212>  PRT
<213>  Artificial sequence

<220>
<223>  an artificially synthesized sequence

<400>  32

Thr Ser Thr Met Gly Arg Ser Ala Asn Pro Arg Gly
1               5                   10


<210>  33
<211>  12
<212>  PRT
<213>  Artificial sequence

<220>
<223>  an artificially synthesized sequence

<400>  33

Thr Ser Thr Pro Gly Arg Ser Ala Asn Pro Arg Gly
1               5                   10


<210>  34
<211>  12
<212>  PRT
<213>  Artificial sequence

<220>
<223>  an artificially synthesized sequence

<400>  34

Thr Ser Thr Gln Gly Arg Ser Ala Asn Pro Arg Gly
1               5                   10


<210>  35
<211>  12
<212>  PRT
<213>  Artificial sequence

<220>
<223>  an artificially synthesized sequence

<400>  35

Thr Ser Thr Val Gly Arg Ser Ala Asn Pro Arg Gly
1               5                   10


<210>  36
<211>  12
<212>  PRT
<213>  Artificial sequence

<220>
<223>  an artificially synthesized sequence

<400>  36

Thr Ser Thr Trp Gly Arg Ser Ala Asn Pro Arg Gly
1               5                   10


<210>  37
<211>  12
<212>  PRT
<213>  Artificial sequence

<220>
<223>  an artificially synthesized sequence

<400>  37

Thr Ser Thr Ser Ala Arg Ser Ala Asn Pro Arg Gly
1               5                   10


<210>  38
<211>  12
<212>  PRT
<213>  Artificial sequence

<220>
<223>  an artificially synthesized sequence

<400>  38

Thr Ser Thr Ser Glu Arg Ser Ala Asn Pro Arg Gly
1               5                   10


<210>  39
<211>  12
<212>  PRT
<213>  Artificial sequence

<220>
<223>  an artificially synthesized sequence

<400>  39

Thr Ser Thr Ser Phe Arg Ser Ala Asn Pro Arg Gly
1               5                   10

<210>  40
<211>  12
<212>  PRT
<213>  Artificial sequence

<220>
<223>  an artificially synthesized sequence

<400>  40

Thr Ser Thr Ser His Arg Ser Ala Asn Pro Arg Gly
1               5                   10


<210>  41
<211>  12
<212>  PRT
<213>  Artificial sequence

<220>
<223>  an artificially synthesized sequence

<400>  41

Thr Ser Thr Ser Ile Arg Ser Ala Asn Pro Arg Gly
1               5                   10


<210>  42
<211>  12
<212>  PRT
<213>  Artificial sequence

<220>
<223>  an artificially synthesized sequence

<400>  42

Thr Ser Thr Ser Lys Arg Ser Ala Asn Pro Arg Gly
1               5                   10


<210>  43
<211>  12
<212>  PRT
<213>  Artificial sequence

<220>
<223>  an artificially synthesized sequence

<400>  43

Thr Ser Thr Ser Leu Arg Ser Ala Asn Pro Arg Gly
1               5                   10


<210>  44
<211>  12
<212>  PRT
<213>  Artificial sequence

<220>
<223>  an artificially synthesized sequence

<400>  44

Thr Ser Thr Ser Met Arg Ser Ala Asn Pro Arg Gly
1               5                   10


<210>  45
<211>  12
<212>  PRT
<213>  Artificial sequence

<220>
<223>  an artificially synthesized sequence

<400>  45

Thr Ser Thr Ser Asn Arg Ser Ala Asn Pro Arg Gly
1               5                   10


<210>  46
<211>  12
<212>  PRT
<213>  Artificial sequence

<220>
<223>  an artificially synthesized sequence

<400>  46

Thr Ser Thr Ser Pro Arg Ser Ala Asn Pro Arg Gly
1               5                   10


<210>  47
<211>  12
<212>  PRT
<213>  Artificial sequence

<220>
<223>  an artificially synthesized sequence

<400>  47

Thr Ser Thr Ser Gln Arg Ser Ala Asn Pro Arg Gly
1               5                   10


<210>  48
<211>  12
<212>  PRT
<213>  Artificial sequence

<220>
<223>  an artificially synthesized sequence

<400>  48

Thr Ser Thr Ser Arg Arg Ser Ala Asn Pro Arg Gly
1               5                   10

<210>  49
<211>  12
<212>  PRT
<213>  Artificial sequence

<220>
<223>  an artificially synthesized sequence

<400>  49

Thr Ser Thr Ser Thr Arg Ser Ala Asn Pro Arg Gly
1               5                   10


<210>  50
<211>  12
<212>  PRT
<213>  Artificial sequence

<220>
<223>  an artificially synthesized sequence

<400>  50

Thr Ser Thr Ser Val Arg Ser Ala Asn Pro Arg Gly
1               5                   10


<210>  51
<211>  12
<212>  PRT
<213>  Artificial sequence

<220>
<223>  an artificially synthesized sequence

<400>  51

Thr Ser Thr Ser Trp Arg Ser Ala Asn Pro Arg Gly
1               5                   10


<210>  52
<211>  12
<212>  PRT
<213>  Artificial sequence

<220>
<223>  an artificially synthesized sequence

<400>  52

Thr Ser Thr Ser Tyr Arg Ser Ala Asn Pro Arg Gly
1               5                   10


<210>  53
<211>  12
<212>  PRT
<213>  Artificial sequence

<220>
<223>  an artificially synthesized sequence

<400>  53

Thr Ser Thr Ser Gly Arg Ala Ala Asn Pro Arg Gly
1               5                   10


<210>  54
<211>  12
<212>  PRT
<213>  Artificial sequence

<220>
<223>  an artificially synthesized sequence

<400>  54

Thr Ser Thr Ser Gly Arg Asp Ala Asn Pro Arg Gly
1               5                   10


<210>  55
<211>  12
<212>  PRT
<213>  Artificial sequence

<220>
<223>  an artificially synthesized sequence

<400>  55

Thr Ser Thr Ser Gly Arg Glu Ala Asn Pro Arg Gly
1               5                   10


<210>  56
<211>  12
<212>  PRT
<213>  Artificial sequence

<220>
<223>  an artificially synthesized sequence

<400>  56

Thr Ser Thr Ser Gly Arg Gly Ala Asn Pro Arg Gly
1               5                   10


<210>  57
<211>  12
<212>  PRT
<213>  Artificial sequence

<220>
<223>  an artificially synthesized sequence

<400>  57

Thr Ser Thr Ser Gly Arg His Ala Asn Pro Arg Gly
1               5                   10

<210>  58
<211>  12
<212>  PRT
<213>  Artificial sequence

<220>
<223>  an artificially synthesized sequence

<400>  58

Thr Ser Thr Ser Gly Arg Ile Ala Asn Pro Arg Gly
1               5                   10


<210>  59
<211>  12
<212>  PRT
<213>  Artificial sequence

<220>
<223>  an artificially synthesized sequence

<400>  59

Thr Ser Thr Ser Gly Arg Lys Ala Asn Pro Arg Gly
1               5                   10


<210>  60
<211>  12
<212>  PRT
<213>  Artificial sequence

<220>
<223>  an artificially synthesized sequence

<400>  60

Thr Ser Thr Ser Gly Arg Leu Ala Asn Pro Arg Gly
1               5                   10


<210>  61
<211>  12
<212>  PRT
<213>  Artificial sequence

<220>
<223>  an artificially synthesized sequence

<400>  61

Thr Ser Thr Ser Gly Arg Met Ala Asn Pro Arg Gly
1               5                   10


<210>  62
<211>  12
<212>  PRT
<213>  Artificial sequence

<220>
<223>  an artificially synthesized sequence

<400>  62

Thr Ser Thr Ser Gly Arg Asn Ala Asn Pro Arg Gly
1               5                   10


<210>  63
<211>  12
<212>  PRT
<213>  Artificial sequence

<220>
<223>  an artificially synthesized sequence

<400>  63

Thr Ser Thr Ser Gly Arg Pro Ala Asn Pro Arg Gly
1               5                   10


<210>  64
<211>  12
<212>  PRT
<213>  Artificial sequence

<220>
<223>  an artificially synthesized sequence

<400>  64

Thr Ser Thr Ser Gly Arg Gln Ala Asn Pro Arg Gly
1               5                   10


<210>  65
<211>  12
<212>  PRT
<213>  Artificial sequence

<220>
<223>  an artificially synthesized sequence

<400>  65

Thr Ser Thr Ser Gly Arg Arg Ala Asn Pro Arg Gly
1               5                   10


<210>  66
<211>  12
<212>  PRT
<213>  Artificial sequence

<220>
<223>  an artificially synthesized sequence

<400>  66

Thr Ser Thr Ser Gly Arg Thr Ala Asn Pro Arg Gly
1               5                   10

<210>  67
<211>  12
<212>  PRT
<213>  Artificial sequence

<220>
<223>  an artificially synthesized sequence

<400>  67

Thr Ser Thr Ser Gly Arg Val Ala Asn Pro Arg Gly
1               5                   10


<210>  68
<211>  12
<212>  PRT
<213>  Artificial sequence

<220>
<223>  an artificially synthesized sequence

<400>  68

Thr Ser Thr Ser Gly Arg Trp Ala Asn Pro Arg Gly
1               5                   10


<210>  69
<211>  12
<212>  PRT
<213>  Artificial sequence

<220>
<223>  an artificially synthesized sequence

<400>  69

Thr Ser Thr Ser Gly Arg Tyr Ala Asn Pro Arg Gly
1               5                   10


<210>  70
<211>  12
<212>  PRT
<213>  Artificial sequence

<220>
<223>  an artificially synthesized sequence

<400>  70

Thr Ser Thr Ser Gly Arg Ser Glu Asn Pro Arg Gly
1               5                   10


<210>  71
<211>  12
<212>  PRT
<213>  Artificial sequence

<220>
<223>  an artificially synthesized sequence

<400>  71

Thr Ser Thr Ser Gly Arg Ser Phe Asn Pro Arg Gly
1               5                   10


<210>  72
<211>  12
<212>  PRT
<213>  Artificial sequence

<220>
<223>  an artificially synthesized sequence

<400>  72

Thr Ser Thr Ser Gly Arg Ser Lys Asn Pro Arg Gly
1               5                   10


<210>  73
<211>  12
<212>  PRT
<213>  Artificial sequence

<220>
<223>  an artificially synthesized sequence

<400>  73

Thr Ser Thr Ser Gly Arg Ser Met Asn Pro Arg Gly
1               5                   10


<210>  74
<211>  12
<212>  PRT
<213>  Artificial sequence

<220>
<223>  an artificially synthesized sequence

<400>  74

Thr Ser Thr Ser Gly Arg Ser Asn Asn Pro Arg Gly
1               5                   10


<210>  75
<211>  12
<212>  PRT
<213>  Artificial sequence

<220>
<223>  an artificially synthesized sequence

<400>  75

Thr Ser Thr Ser Gly Arg Ser Pro Asn Pro Arg Gly
1               5                   10

<210>  76
<211>  12
<212>  PRT
<213>  Artificial sequence

<220>
<223>  an artificially synthesized sequence

<400>  76

Thr Ser Thr Ser Gly Arg Ser Gln Asn Pro Arg Gly
1               5                   10


<210>  77
<211>  12
<212>  PRT
<213>  Artificial sequence

<220>
<223>  an artificially synthesized sequence

<400>  77

Thr Ser Thr Ser Gly Arg Ser Arg Asn Pro Arg Gly
1               5                   10


<210>  78
<211>  12
<212>  PRT
<213>  Artificial sequence

<220>
<223>  an artificially synthesized sequence

<400>  78

Thr Ser Thr Ser Gly Arg Ser Ser Asn Pro Arg Gly
1               5                   10


<210>  79
<211>  12
<212>  PRT
<213>  Artificial sequence

<220>
<223>  an artificially synthesized sequence

<400>  79

Thr Ser Thr Ser Gly Arg Ser Trp Asn Pro Arg Gly
1               5                   10


<210>  80
<211>  12
<212>  PRT
<213>  Artificial sequence

<220>
<223>  an artificially synthesized sequence

<400>  80

Thr Ser Thr Ser Gly Arg Ser Tyr Asn Pro Arg Gly
1               5                   10


<210>  81
<211>  12
<212>  PRT
<213>  Artificial sequence

<220>
<223>  an artificially synthesized sequence

<400>  81

Thr Ser Thr Ser Gly Arg Ser Ala Ala Pro Arg Gly
1               5                   10


<210>  82
<211>  12
<212>  PRT
<213>  Artificial sequence

<220>
<223>  an artificially synthesized sequence

<400>  82

Thr Ser Thr Ser Gly Arg Ser Ala Asp Pro Arg Gly
1               5                   10


<210>  83
<211>  12
<212>  PRT
<213>  Artificial sequence

<220>
<223>  an artificially synthesized sequence

<400>  83

Thr Ser Thr Ser Gly Arg Ser Ala Glu Pro Arg Gly
1               5                   10


<210>  84
<211>  12
<212>  PRT
<213>  Artificial sequence

<220>
<223>  an artificially synthesized sequence

<400>  84

Thr Ser Thr Ser Gly Arg Ser Ala Phe Pro Arg Gly
1               5                   10

<210>  85
<211>  12
<212>  PRT
<213>  Artificial sequence

<220>
<223>  an artificially synthesized sequence

<400>  85

Thr Ser Thr Ser Gly Arg Ser Ala Gly Pro Arg Gly
1               5                   10


<210>  86
<211>  12
<212>  PRT
<213>  Artificial sequence

<220>
<223>  an artificially synthesized sequence

<400>  86

Thr Ser Thr Ser Gly Arg Ser Ala Lys Pro Arg Gly
1               5                   10


<210>  87
<211>  12
<212>  PRT
<213>  Artificial sequence

<220>
<223>  an artificially synthesized sequence

<400>  87

Thr Ser Thr Ser Gly Arg Ser Ala Leu Pro Arg Gly
1               5                   10


<210>  88
<211>  12
<212>  PRT
<213>  Artificial sequence

<220>
<223>  an artificially synthesized sequence

<400>  88

Thr Ser Thr Ser Gly Arg Ser Ala Met Pro Arg Gly
1               5                   10


<210>  89
<211>  12
<212>  PRT
<213>  Artificial sequence

<220>
<223>  an artificially synthesized sequence

<400>  89

Thr Ser Thr Ser Gly Arg Ser Ala Pro Pro Arg Gly
1               5                   10


<210>  90
<211>  12
<212>  PRT
<213>  Artificial sequence

<220>
<223>  an artificially synthesized sequence

<400>  90

Thr Ser Thr Ser Gly Arg Ser Ala Gln Pro Arg Gly
1               5                   10


<210>  91
<211>  12
<212>  PRT
<213>  Artificial sequence

<220>
<223>  an artificially synthesized sequence

<400>  91

Thr Ser Thr Ser Gly Arg Ser Ala Val Pro Arg Gly
1               5                   10


<210>  92
<211>  12
<212>  PRT
<213>  Artificial sequence

<220>
<223>  an artificially synthesized sequence

<400>  92

Thr Ser Thr Ser Gly Arg Ser Ala Trp Pro Arg Gly
1               5                   10


<210>  93
<211>  12
<212>  PRT
<213>  Artificial sequence

<220>
<223>  an artificially synthesized sequence

<400>  93

Thr Ser Thr Ser Gly Arg Ser Ala Tyr Pro Arg Gly
1               5                   10

<210>  94
<211>  12
<212>  PRT
<213>  Artificial sequence

<220>
<223>  an artificially synthesized sequence

<400>  94

Thr Ser Thr Ser Gly Arg Ser Ala Asn Ala Arg Gly
1               5                   10


<210>  95
<211>  12
<212>  PRT
<213>  Artificial sequence

<220>
<223>  an artificially synthesized sequence

<400>  95

Thr Ser Thr Ser Gly Arg Ser Ala Asn Asp Arg Gly
1               5                   10


<210>  96
<211>  12
<212>  PRT
<213>  Artificial sequence

<220>
<223>  an artificially synthesized sequence

<400>  96

Thr Ser Thr Ser Gly Arg Ser Ala Asn Glu Arg Gly
1               5                   10


<210>  97
<211>  12
<212>  PRT
<213>  Artificial sequence

<220>
<223>  an artificially synthesized sequence

<400>  97

Thr Ser Thr Ser Gly Arg Ser Ala Asn Phe Arg Gly
1               5                   10


<210>  98
<211>  12
<212>  PRT
<213>  Artificial sequence

<220>
<223>  an artificially synthesized sequence

<400>  98

Thr Ser Thr Ser Gly Arg Ser Ala Asn Gly Arg Gly
1               5                   10


<210>  99
<211>  12
<212>  PRT
<213>  Artificial sequence

<220>
<223>  an artificially synthesized sequence

<400>  99

Thr Ser Thr Ser Gly Arg Ser Ala Asn Ile Arg Gly
1               5                   10


<210>  100
<211>  12
<212>  PRT
<213>  Artificial sequence

<220>
<223>  an artificially synthesized sequence

<400>  100

Thr Ser Thr Ser Gly Arg Ser Ala Asn Lys Arg Gly
1               5                   10


<210>  101
<211>  12
<212>  PRT
<213>  Artificial sequence

<220>
<223>  an artificially synthesized sequence

<400>  101

Thr Ser Thr Ser Gly Arg Ser Ala Asn Asn Arg Gly
1               5                   10


<210>  102
<211>  12
<212>  PRT
<213>  Artificial sequence

<220>
<223>  an artificially synthesized sequence

<400>  102

Thr Ser Thr Ser Gly Arg Ser Ala Asn Gln Arg Gly
1               5                   10

<210>  103
<211>  12
<212>  PRT
<213>  Artificial sequence

<220>
<223>  an artificially synthesized sequence

<400>  103

Thr Ser Thr Ser Gly Arg Ser Ala Asn Ser Arg Gly
1               5                   10


<210>  104
<211>  12
<212>  PRT
<213>  Artificial sequence

<220>
<223>  an artificially synthesized sequence

<400>  104

Thr Ser Thr Ser Gly Arg Ser Ala Asn Thr Arg Gly
1               5                   10


<210>  105
<211>  12
<212>  PRT
<213>  Artificial sequence

<220>
<223>  an artificially synthesized sequence

<400>  105

Thr Ser Thr Ser Gly Arg Ser Ala Asn Trp Arg Gly
1               5                   10


<210>  106
<211>  12
<212>  PRT
<213>  Artificial sequence

<220>
<223>  an artificially synthesized sequence

<400>  106

Thr Ser Asp Ser Gly Arg Ser Ala Asn Pro Arg Gly
1               5                   10


<210>  107
<211>  12
<212>  PRT
<213>  Artificial sequence

<220>
<223>  an artificially synthesized sequence

<400>  107

Thr Ser Ile Ser Gly Arg Ser Ala Asn Pro Arg Gly
1               5                   10


<210>  108
<211>  12
<212>  PRT
<213>  Artificial sequence

<220>
<223>  an artificially synthesized sequence

<400>  108

Thr Ser Ser Ser Gly Arg Ser Ala Asn Pro Arg Gly
1               5                   10


<210>  109
<211>  12
<212>  PRT
<213>  Artificial sequence

<220>
<223>  an artificially synthesized sequence

<400>  109

Thr Ser Thr His Gly Arg Ser Ala Asn Pro Arg Gly
1               5                   10


<210>  110
<211>  12
<212>  PRT
<213>  Artificial sequence

<220>
<223>  an artificially synthesized sequence

<400>  110

Thr Ser Thr Lys Gly Arg Ser Ala Asn Pro Arg Gly
1               5                   10


<210>  111
<211>  12
<212>  PRT
<213>  Artificial sequence

<220>
<223>  an artificially synthesized sequence

<400>  111

Thr Ser Thr Thr Gly Arg Ser Ala Asn Pro Arg Gly
1               5                   10

<210>  112
<211>  12
<212>  PRT
<213>  Artificial sequence

<220>
<223>  an artificially synthesized sequence

<400>  112

Thr Ser Thr Tyr Gly Arg Ser Ala Asn Pro Arg Gly
1               5                   10


<210>  113
<211>  12
<212>  PRT
<213>  Artificial sequence

<220>
<223>  an artificially synthesized sequence

<400>  113

Thr Ser Thr Ser Asp Arg Ser Ala Asn Pro Arg Gly
1               5                   10


<210>  114
<211>  12
<212>  PRT
<213>  Artificial sequence

<220>
<223>  an artificially synthesized sequence

<400>  114

Thr Ser Thr Ser Ser Arg Ser Ala Asn Pro Arg Gly
1               5                   10


<210>  115
<211>  12
<212>  PRT
<213>  Artificial sequence

<220>
<223>  an artificially synthesized sequence

<400>  115

Thr Ser Thr Ser Gly Arg Phe Ala Asn Pro Arg Gly
1               5                   10


<210>  116
<211>  12
<212>  PRT
<213>  Artificial sequence

<220>
<223>  an artificially synthesized sequence

<400>  116

Thr Ser Thr Ser Gly Arg Ser Asp Asn Pro Arg Gly
1               5                   10


<210>  117
<211>  12
<212>  PRT
<213>  Artificial sequence

<220>
<223>  an artificially synthesized sequence

<400>  117

Thr Ser Thr Ser Gly Arg Ser His Asn Pro Arg Gly
1               5                   10


<210>  118
<211>  12
<212>  PRT
<213>  Artificial sequence

<220>
<223>  an artificially synthesized sequence

<400>  118

Thr Ser Thr Ser Gly Arg Ser Ile Asn Pro Arg Gly
1               5                   10


<210>  119
<211>  12
<212>  PRT
<213>  Artificial sequence

<220>
<223>  an artificially synthesized sequence

<400>  119

Thr Ser Thr Ser Gly Arg Ser Leu Asn Pro Arg Gly
1               5                   10


<210>  120
<211>  12
<212>  PRT
<213>  Artificial sequence

<220>
<223>  an artificially synthesized sequence

<400>  120

Thr Ser Thr Ser Gly Arg Ser Thr Asn Pro Arg Gly
1               5                   10

<210>  121
<211>  12
<212>  PRT
<213>  Artificial sequence

<220>
<223>  an artificially synthesized sequence

<400>  121

Thr Ser Thr Ser Gly Arg Ser Val Asn Pro Arg Gly
1               5                   10


<210>  122
<211>  12
<212>  PRT
<213>  Artificial sequence

<220>
<223>  an artificially synthesized sequence

<400>  122

Thr Ser Thr Ser Gly Arg Ser Ala His Pro Arg Gly
1               5                   10


<210>  123
<211>  12
<212>  PRT
<213>  Artificial sequence

<220>
<223>  an artificially synthesized sequence

<400>  123

Thr Ser Thr Ser Gly Arg Ser Ala Ile Pro Arg Gly
1               5                   10


<210>  124
<211>  12
<212>  PRT
<213>  Artificial sequence

<220>
<223>  an artificially synthesized sequence

<400>  124

Thr Ser Thr Ser Gly Arg Ser Ala Arg Pro Arg Gly
1               5                   10


<210>  125
<211>  12
<212>  PRT
<213>  Artificial sequence

<220>
<223>  an artificially synthesized sequence

<400>  125

Thr Ser Thr Ser Gly Arg Ser Ala Ser Pro Arg Gly
1               5                   10


<210>  126
<211>  12
<212>  PRT
<213>  Artificial sequence

<220>
<223>  an artificially synthesized sequence

<400>  126

Thr Ser Thr Ser Gly Arg Ser Ala Thr Pro Arg Gly
1               5                   10


<210>  127
<211>  12
<212>  PRT
<213>  Artificial sequence

<220>
<223>  an artificially synthesized sequence

<400>  127

Thr Ser Thr Ser Gly Arg Ser Ala Asn His Arg Gly
1               5                   10


<210>  128
<211>  12
<212>  PRT
<213>  Artificial sequence

<220>
<223>  an artificially synthesized sequence

<400>  128

Thr Ser Thr Ser Gly Arg Ser Ala Asn Leu Arg Gly
1               5                   10


<210>  129
<211>  12
<212>  PRT
<213>  Artificial sequence

<220>
<223>  an artificially synthesized sequence

<400>  129

Thr Ser Thr Ser Gly Arg Ser Ala Asn Met Arg Gly
1               5                   10

<210>  130
<211>  12
<212>  PRT
<213>  Artificial sequence

<220>
<223>  an artificially synthesized sequence

<400>  130

Thr Ser Thr Ser Gly Arg Ser Ala Asn Arg Arg Gly
1               5                   10


<210>  131
<211>  12
<212>  PRT
<213>  Artificial sequence

<220>
<223>  an artificially synthesized sequence

<400>  131

Thr Ser Thr Ser Gly Arg Ser Ala Asn Val Arg Gly
1               5                   10


<210>  132
<211>  12
<212>  PRT
<213>  Artificial sequence

<220>
<223>  an artificially synthesized sequence

<400>  132

Thr Ser Thr Ser Gly Arg Ser Ala Asn Tyr Arg Gly
1               5                   10


<210>  133
<211>  12
<212>  PRT
<213>  Artificial sequence

<220>
<223>  an artificially synthesized sequence

<400>  133

Thr Ser Gly Ser Gly Arg Ser Ala Val Pro Arg Gly
1               5                   10


<210>  134
<211>  12
<212>  PRT
<213>  Artificial sequence

<220>
<223>  an artificially synthesized sequence

<400>  134

Thr Ser Gly Ser Gly Arg Ser Ala Tyr Pro Arg Gly
1               5                   10


<210>  135
<211>  12
<212>  PRT
<213>  Artificial sequence

<220>
<223>  an artificially synthesized sequence

<400>  135

Thr Ser Gly Ser Gly Arg Ser Ala Asn Gln Arg Gly
1               5                   10


<210>  136
<211>  12
<212>  PRT
<213>  Artificial sequence

<220>
<223>  an artificially synthesized sequence

<400>  136

Thr Ser Gly Ser Gly Arg Ser Ala Asn Ile Arg Gly
1               5                   10


<210>  137
<211>  12
<212>  PRT
<213>  Artificial sequence

<220>
<223>  an artificially synthesized sequence

<400>  137

Thr Ser Gly Ser Gly Arg Ser Ala Asn Phe Arg Gly
1               5                   10


<210>  138
<211>  12
<212>  PRT
<213>  Artificial sequence

<220>
<223>  an artificially synthesized sequence

<400>  138

Thr Ser Gly Ser Gly Arg Ser Ala Asn Ser Arg Gly
1               5                   10

<210>  139
<211>  12
<212>  PRT
<213>  Artificial sequence

<220>
<223>  an artificially synthesized sequence

<400>  139

Thr Ser Gln Ser Gly Arg Ser Ala Val Pro Arg Gly
1               5                   10


<210>  140
<211>  12
<212>  PRT
<213>  Artificial sequence

<220>
<223>  an artificially synthesized sequence

<400>  140

Thr Ser Gln Ser Gly Arg Ser Ala Tyr Pro Arg Gly
1               5                   10


<210>  141
<211>  12
<212>  PRT
<213>  Artificial sequence

<220>
<223>  an artificially synthesized sequence

<400>  141

Thr Ser Gln Ser Gly Arg Ser Ala Asn Ala Arg Gly
1               5                   10


<210>  142
<211>  12
<212>  PRT
<213>  Artificial sequence

<220>
<223>  an artificially synthesized sequence

<400>  142

Thr Ser Gln Ser Gly Arg Ser Ala Asn Ile Arg Gly
1               5                   10


<210>  143
<211>  12
<212>  PRT
<213>  Artificial sequence

<220>
<223>  an artificially synthesized sequence

<400>  143

Thr Ser Gln Ser Gly Arg Ser Ala Asn Phe Arg Gly
1               5                   10


<210>  144
<211>  12
<212>  PRT
<213>  Artificial sequence

<220>
<223>  an artificially synthesized sequence

<400>  144

Thr Ser Gln Ser Gly Arg Ser Ala Asn Ser Arg Gly
1               5                   10


<210>  145
<211>  12
<212>  PRT
<213>  Artificial sequence

<220>
<223>  an artificially synthesized sequence

<400>  145

Thr Ser Pro Ser Gly Arg Ser Ala Val Pro Arg Gly
1               5                   10


<210>  146
<211>  12
<212>  PRT
<213>  Artificial sequence

<220>
<223>  an artificially synthesized sequence

<400>  146

Thr Ser Pro Ser Gly Arg Ser Ala Asn Gln Arg Gly
1               5                   10


<210>  147
<211>  12
<212>  PRT
<213>  Artificial sequence

<220>
<223>  an artificially synthesized sequence

<400>  147

Thr Ser Pro Ser Gly Arg Ser Ala Asn Ala Arg Gly
1               5                   10

<210>  148
<211>  12
<212>  PRT
<213>  Artificial sequence

<220>
<223>  an artificially synthesized sequence

<400>  148

Thr Ser Pro Ser Gly Arg Ser Ala Asn Ile Arg Gly
1               5                   10


<210>  149
<211>  12
<212>  PRT
<213>  Artificial sequence

<220>
<223>  an artificially synthesized sequence

<400>  149

Thr Ser Pro Ser Gly Arg Ser Ala Asn Phe Arg Gly
1               5                   10


<210>  150
<211>  12
<212>  PRT
<213>  Artificial sequence

<220>
<223>  an artificially synthesized sequence

<400>  150

Thr Ser Pro Ser Gly Arg Ser Ala Asn Ser Arg Gly
1               5                   10


<210>  151
<211>  12
<212>  PRT
<213>  Artificial sequence

<220>
<223>  an artificially synthesized sequence

<400>  151

Thr Ser Ala Ser Gly Arg Ser Ala Val Pro Arg Gly
1               5                   10


<210>  152
<211>  12
<212>  PRT
<213>  Artificial sequence

<220>
<223>  an artificially synthesized sequence

<400>  152

Thr Ser Ala Ser Gly Arg Ser Ala Tyr Pro Arg Gly
1               5                   10


<210>  153
<211>  12
<212>  PRT
<213>  Artificial sequence

<220>
<223>  an artificially synthesized sequence

<400>  153

Thr Ser Ala Ser Gly Arg Ser Ala Asn Gln Arg Gly
1               5                   10


<210>  154
<211>  12
<212>  PRT
<213>  Artificial sequence

<220>
<223>  an artificially synthesized sequence

<400>  154

Thr Ser Ala Ser Gly Arg Ser Ala Asn Ala Arg Gly
1               5                   10


<210>  155
<211>  12
<212>  PRT
<213>  Artificial sequence

<220>
<223>  an artificially synthesized sequence

<400>  155

Thr Ser Ala Ser Gly Arg Ser Ala Asn Ile Arg Gly
1               5                   10


<210>  156
<211>  12
<212>  PRT
<213>  Artificial sequence

<220>
<223>  an artificially synthesized sequence

<400>  156

Thr Ser Ala Ser Gly Arg Ser Ala Asn Phe Arg Gly
1               5                   10

<210>  157
<211>  12
<212>  PRT
<213>  Artificial sequence

<220>
<223>  an artificially synthesized sequence

<400>  157

Thr Ser Ala Ser Gly Arg Ser Ala Asn Ser Arg Gly
1               5                   10


<210>  158
<211>  12
<212>  PRT
<213>  Artificial sequence

<220>
<223>  an artificially synthesized sequence

<400>  158

Thr Ser Tyr Ser Gly Arg Ser Glu Asn Pro Arg Gly
1               5                   10


<210>  159
<211>  12
<212>  PRT
<213>  Artificial sequence

<220>
<223>  an artificially synthesized sequence

<400>  159

Thr Ser Gly Ser Gly Arg Ser Glu Asn Pro Arg Gly
1               5                   10


<210>  160
<211>  12
<212>  PRT
<213>  Artificial sequence

<220>
<223>  an artificially synthesized sequence

<400>  160

Thr Ser Gln Ser Gly Arg Ser Glu Asn Pro Arg Gly
1               5                   10


<210>  161
<211>  12
<212>  PRT
<213>  Artificial sequence

<220>
<223>  an artificially synthesized sequence

<400>  161

Thr Ser Pro Ser Gly Arg Ser Glu Asn Pro Arg Gly
1               5                   10


<210>  162
<211>  12
<212>  PRT
<213>  Artificial sequence

<220>
<223>  an artificially synthesized sequence

<400>  162

Thr Ser Ala Ser Gly Arg Ser Glu Asn Pro Arg Gly
1               5                   10


<210>  163
<211>  12
<212>  PRT
<213>  Artificial sequence

<220>
<223>  an artificially synthesized sequence

<400>  163

Thr Ser His Ser Gly Arg Ser Glu Asn Pro Arg Gly
1               5                   10


<210>  164
<211>  12
<212>  PRT
<213>  Artificial sequence

<220>
<223>  an artificially synthesized sequence

<400>  164

Thr Ser Thr Ser Gly Arg Ser Glu Asn Gln Arg Gly
1               5                   10


<210>  165
<211>  12
<212>  PRT
<213>  Artificial sequence

<220>
<223>  an artificially synthesized sequence

<400>  165

Thr Ser Thr Ser Gly Arg Ser Glu Asn Ala Arg Gly
1               5                   10

<210>  166
<211>  12
<212>  PRT
<213>  Artificial sequence

<220>
<223>  an artificially synthesized sequence

<400>  166

Thr Ser Thr Ser Gly Arg Ser Glu Asn Ile Arg Gly
1               5                   10


<210>  167
<211>  12
<212>  PRT
<213>  Artificial sequence

<220>
<223>  an artificially synthesized sequence

<400>  167

Thr Ser Thr Ser Gly Arg Ser Glu Asn Phe Arg Gly
1               5                   10


<210>  168
<211>  12
<212>  PRT
<213>  Artificial sequence

<220>
<223>  an artificially synthesized sequence

<400>  168

Thr Ser Thr Ser Gly Arg Ser Glu Asn Ser Arg Gly
1               5                   10


<210>  169
<211>  12
<212>  PRT
<213>  Artificial sequence

<220>
<223>  an artificially synthesized sequence

<400>  169

Thr Ser Tyr Ser Gly Arg Ser Ala Glu Pro Arg Gly
1               5                   10


<210>  170
<211>  12
<212>  PRT
<213>  Artificial sequence

<220>
<223>  an artificially synthesized sequence

<400>  170

Thr Ser Gly Ser Gly Arg Ser Ala Glu Pro Arg Gly
1               5                   10


<210>  171
<211>  12
<212>  PRT
<213>  Artificial sequence

<220>
<223>  an artificially synthesized sequence

<400>  171

Thr Ser Gln Ser Gly Arg Ser Ala Glu Pro Arg Gly
1               5                   10


<210>  172
<211>  12
<212>  PRT
<213>  Artificial sequence

<220>
<223>  an artificially synthesized sequence

<400>  172

Thr Ser Pro Ser Gly Arg Ser Ala Glu Pro Arg Gly
1               5                   10


<210>  173
<211>  12
<212>  PRT
<213>  Artificial sequence

<220>
<223>  an artificially synthesized sequence

<400>  173

Thr Ser Ala Ser Gly Arg Ser Ala Glu Pro Arg Gly
1               5                   10


<210>  174
<211>  12
<212>  PRT
<213>  Artificial sequence

<220>
<223>  an artificially synthesized sequence

<400>  174

Thr Ser His Ser Gly Arg Ser Ala Glu Pro Arg Gly
1               5                   10

<210>  175
<211>  12
<212>  PRT
<213>  Artificial sequence

<220>
<223>  an artificially synthesized sequence

<400>  175

Thr Ser Thr Ser Gly Arg Ser Ala Glu Gln Arg Gly
1               5                   10


<210>  176
<211>  12
<212>  PRT
<213>  Artificial sequence

<220>
<223>  an artificially synthesized sequence

<400>  176

Thr Ser Thr Ser Gly Arg Ser Ala Glu Ala Arg Gly
1               5                   10


<210>  177
<211>  12
<212>  PRT
<213>  Artificial sequence

<220>
<223>  an artificially synthesized sequence

<400>  177

Thr Ser Thr Ser Gly Arg Ser Ala Glu Ile Arg Gly
1               5                   10


<210>  178
<211>  12
<212>  PRT
<213>  Artificial sequence

<220>
<223>  an artificially synthesized sequence

<400>  178

Thr Ser Thr Ser Gly Arg Ser Ala Glu Phe Arg Gly
1               5                   10


<210>  179
<211>  12
<212>  PRT
<213>  Artificial sequence

<220>
<223>  an artificially synthesized sequence

<400>  179

Thr Ser Thr Ser Gly Arg Ser Ala Glu Ser Arg Gly
1               5                   10


<210>  180
<211>  12
<212>  PRT
<213>  Artificial sequence

<220>
<223>  an artificially synthesized sequence

<400>  180

Thr Ser Gly Thr Gly Arg Ser Ala Asn Pro Arg Gly
1               5                   10


<210>  181
<211>  12
<212>  PRT
<213>  Artificial sequence

<220>
<223>  an artificially synthesized sequence

<400>  181

Thr Ser Gly Lys Gly Arg Ser Ala Asn Pro Arg Gly
1               5                   10


<210>  182
<211>  12
<212>  PRT
<213>  Artificial sequence

<220>
<223>  an artificially synthesized sequence

<400>  182

Thr Ser Gly Ser Gly Arg Ser Ala Ile Pro Arg Gly
1               5                   10


<210>  183
<211>  12
<212>  PRT
<213>  Artificial sequence

<220>
<223>  an artificially synthesized sequence

<400>  183

Thr Ser Gly Ser Gly Arg Ser Ala Ser Pro Arg Gly
1               5                   10

<210>  184
<211>  12
<212>  PRT
<213>  Artificial sequence

<220>
<223>  an artificially synthesized sequence

<400>  184

Thr Ser Gly Ser Gly Arg Ser Ala His Pro Arg Gly
1               5                   10


<210>  185
<211>  12
<212>  PRT
<213>  Artificial sequence

<220>
<223>  an artificially synthesized sequence

<400>  185

Thr Ser Gly Ser Gly Arg Ser Ala Asn Tyr Arg Gly
1               5                   10


<210>  186
<211>  12
<212>  PRT
<213>  Artificial sequence

<220>
<223>  an artificially synthesized sequence

<400>  186

Thr Ser Gly Ser Gly Arg Ser Ala Asn Val Arg Gly
1               5                   10


<210>  187
<211>  12
<212>  PRT
<213>  Artificial sequence

<220>
<223>  an artificially synthesized sequence

<400>  187

Thr Ser Gly Ser Gly Arg Ser Ala Asn His Arg Gly
1               5                   10


<210>  188
<211>  12
<212>  PRT
<213>  Artificial sequence

<220>
<223>  an artificially synthesized sequence

<400>  188

Thr Ser Gln Thr Gly Arg Ser Ala Asn Pro Arg Gly
1               5                   10


<210>  189
<211>  12
<212>  PRT
<213>  Artificial sequence

<220>
<223>  an artificially synthesized sequence

<400>  189

Thr Ser Gln Lys Gly Arg Ser Ala Asn Pro Arg Gly
1               5                   10


<210>  190
<211>  12
<212>  PRT
<213>  Artificial sequence

<220>
<223>  an artificially synthesized sequence

<400>  190

Thr Ser Gln Ser Gly Arg Ser Ala Ile Pro Arg Gly
1               5                   10


<210>  191
<211>  12
<212>  PRT
<213>  Artificial sequence

<220>
<223>  an artificially synthesized sequence

<400>  191

Thr Ser Gln Ser Gly Arg Ser Ala Ser Pro Arg Gly
1               5                   10


<210>  192
<211>  12
<212>  PRT
<213>  Artificial sequence

<220>
<223>  an artificially synthesized sequence

<400>  192

Thr Ser Gln Ser Gly Arg Ser Ala His Pro Arg Gly
1               5                   10

<210>  193
<211>  12
<212>  PRT
<213>  Artificial sequence

<220>
<223>  an artificially synthesized sequence

<400>  193

Thr Ser Gln Ser Gly Arg Ser Ala Asn Tyr Arg Gly
1               5                   10


<210>  194
<211>  12
<212>  PRT
<213>  Artificial sequence

<220>
<223>  an artificially synthesized sequence

<400>  194

Thr Ser Gln Ser Gly Arg Ser Ala Asn Val Arg Gly
1               5                   10


<210>  195
<211>  12
<212>  PRT
<213>  Artificial sequence

<220>
<223>  an artificially synthesized sequence

<400>  195

Thr Ser Gln Ser Gly Arg Ser Ala Asn His Arg Gly
1               5                   10


<210>  196
<211>  12
<212>  PRT
<213>  Artificial sequence

<220>
<223>  an artificially synthesized sequence

<400>  196

Thr Ser Pro Thr Gly Arg Ser Ala Asn Pro Arg Gly
1               5                   10


<210>  197
<211>  12
<212>  PRT
<213>  Artificial sequence

<220>
<223>  an artificially synthesized sequence

<400>  197

Thr Ser Pro Lys Gly Arg Ser Ala Asn Pro Arg Gly
1               5                   10


<210>  198
<211>  12
<212>  PRT
<213>  Artificial sequence

<220>
<223>  an artificially synthesized sequence

<400>  198

Thr Ser Pro Ser Gly Arg Ser Ala Ile Pro Arg Gly
1               5                   10


<210>  199
<211>  12
<212>  PRT
<213>  Artificial sequence

<220>
<223>  an artificially synthesized sequence

<400>  199

Thr Ser Pro Ser Gly Arg Ser Ala Thr Pro Arg Gly
1               5                   10


<210>  200
<211>  12
<212>  PRT
<213>  Artificial sequence

<220>
<223>  an artificially synthesized sequence

<400>  200

Thr Ser Pro Ser Gly Arg Ser Ala Ser Pro Arg Gly
1               5                   10


<210>  201
<211>  12
<212>  PRT
<213>  Artificial sequence

<220>
<223>  an artificially synthesized sequence

<400>  201

Thr Ser Pro Ser Gly Arg Ser Ala His Pro Arg Gly
1               5                   10

<210>  202
<211>  12
<212>  PRT
<213>  Artificial sequence

<220>
<223>  an artificially synthesized sequence

<400>  202

Thr Ser Pro Ser Gly Arg Ser Ala Asn Tyr Arg Gly
1               5                   10


<210>  203
<211>  12
<212>  PRT
<213>  Artificial sequence

<220>
<223>  an artificially synthesized sequence

<400>  203

Thr Ser Pro Ser Gly Arg Ser Ala Asn Val Arg Gly
1               5                   10


<210>  204
<211>  12
<212>  PRT
<213>  Artificial sequence

<220>
<223>  an artificially synthesized sequence

<400>  204

Thr Ser Pro Ser Gly Arg Ser Ala Asn His Arg Gly
1               5                   10


<210>  205
<211>  12
<212>  PRT
<213>  Artificial sequence

<220>
<223>  an artificially synthesized sequence

<400>  205

Thr Ser Ala Thr Gly Arg Ser Ala Asn Pro Arg Gly
1               5                   10


<210>  206
<211>  12
<212>  PRT
<213>  Artificial sequence

<220>
<223>  an artificially synthesized sequence

<400>  206

Thr Ser Ala Lys Gly Arg Ser Ala Asn Pro Arg Gly
1               5                   10


<210>  207
<211>  12
<212>  PRT
<213>  Artificial sequence

<220>
<223>  an artificially synthesized sequence

<400>  207

Thr Ser Ala Ser Gly Arg Ser Ala Ile Pro Arg Gly
1               5                   10


<210>  208
<211>  12
<212>  PRT
<213>  Artificial sequence

<220>
<223>  an artificially synthesized sequence

<400>  208

Thr Ser Ala Ser Gly Arg Ser Ala Ser Pro Arg Gly
1               5                   10


<210>  209
<211>  12
<212>  PRT
<213>  Artificial sequence

<220>
<223>  an artificially synthesized sequence

<400>  209

Thr Ser Ala Ser Gly Arg Ser Ala His Pro Arg Gly
1               5                   10


<210>  210
<211>  12
<212>  PRT
<213>  Artificial sequence

<220>
<223>  an artificially synthesized sequence

<400>  210

Thr Ser Ala Ser Gly Arg Ser Ala Asn Tyr Arg Gly
1               5                   10

<210>  211
<211>  12
<212>  PRT
<213>  Artificial sequence

<220>
<223>  an artificially synthesized sequence

<400>  211

Thr Ser Ala Ser Gly Arg Ser Ala Asn Val Arg Gly
1               5                   10


<210>  212
<211>  12
<212>  PRT
<213>  Artificial sequence

<220>
<223>  an artificially synthesized sequence

<400>  212

Thr Ser Ala Ser Gly Arg Ser Ala Asn His Arg Gly
1               5                   10


<210>  213
<211>  12
<212>  PRT
<213>  Artificial sequence

<220>
<223>  an artificially synthesized sequence

<400>  213

Thr Ser Tyr Thr Gly Arg Ser Ala Asn Pro Arg Gly
1               5                   10


<210>  214
<211>  12
<212>  PRT
<213>  Artificial sequence

<220>
<223>  an artificially synthesized sequence

<400>  214

Thr Ser Tyr Lys Gly Arg Ser Ala Asn Pro Arg Gly
1               5                   10


<210>  215
<211>  12
<212>  PRT
<213>  Artificial sequence

<220>
<223>  an artificially synthesized sequence

<400>  215

Thr Ser Tyr Ser Gly Arg Ser Ala Ile Pro Arg Gly
1               5                   10


<210>  216
<211>  12
<212>  PRT
<213>  Artificial sequence

<220>
<223>  an artificially synthesized sequence

<400>  216

Thr Ser Tyr Ser Gly Arg Ser Ala Thr Pro Arg Gly
1               5                   10


<210>  217
<211>  12
<212>  PRT
<213>  Artificial sequence

<220>
<223>  an artificially synthesized sequence

<400>  217

Thr Ser Tyr Ser Gly Arg Ser Ala Ser Pro Arg Gly
1               5                   10


<210>  218
<211>  12
<212>  PRT
<213>  Artificial sequence

<220>
<223>  an artificially synthesized sequence

<400>  218

Thr Ser Tyr Ser Gly Arg Ser Ala His Pro Arg Gly
1               5                   10


<210>  219
<211>  12
<212>  PRT
<213>  Artificial sequence

<220>
<223>  an artificially synthesized sequence

<400>  219

Thr Ser Tyr Ser Gly Arg Ser Ala Asn Ala Arg Gly
1               5                   10

<210>  220
<211>  12
<212>  PRT
<213>  Artificial sequence

<220>
<223>  an artificially synthesized sequence

<400>  220

Thr Ser Tyr Ser Gly Arg Ser Ala Asn Tyr Arg Gly
1               5                   10


<210>  221
<211>  12
<212>  PRT
<213>  Artificial sequence

<220>
<223>  an artificially synthesized sequence

<400>  221

Thr Ser Tyr Ser Gly Arg Ser Ala Asn Val Arg Gly
1               5                   10


<210>  222
<211>  12
<212>  PRT
<213>  Artificial sequence

<220>
<223>  an artificially synthesized sequence

<400>  222

Thr Ser Tyr Ser Gly Arg Ser Ala Asn His Arg Gly
1               5                   10


<210>  223
<211>  12
<212>  PRT
<213>  Artificial sequence

<220>
<223>  an artificially synthesized sequence

<400>  223

Thr Ser Ser Thr Gly Arg Ser Ala Asn Pro Arg Gly
1               5                   10


<210>  224
<211>  12
<212>  PRT
<213>  Artificial sequence

<220>
<223>  an artificially synthesized sequence

<400>  224

Thr Ser Ser Lys Gly Arg Ser Ala Asn Pro Arg Gly
1               5                   10


<210>  225
<211>  12
<212>  PRT
<213>  Artificial sequence

<220>
<223>  an artificially synthesized sequence

<400>  225

Thr Ser Ser Ser Gly Arg Ser Ala Val Pro Arg Gly
1               5                   10


<210>  226
<211>  12
<212>  PRT
<213>  Artificial sequence

<220>
<223>  an artificially synthesized sequence

<400>  226

Thr Ser Ser Ser Gly Arg Ser Ala Ile Pro Arg Gly
1               5                   10


<210>  227
<211>  12
<212>  PRT
<213>  Artificial sequence

<220>
<223>  an artificially synthesized sequence

<400>  227

Thr Ser Ser Ser Gly Arg Ser Ala Ser Pro Arg Gly
1               5                   10


<210>  228
<211>  12
<212>  PRT
<213>  Artificial sequence

<220>
<223>  an artificially synthesized sequence

<400>  228

Thr Ser Ser Ser Gly Arg Ser Ala His Pro Arg Gly
1               5                   10

<210>  229
<211>  12
<212>  PRT
<213>  Artificial sequence

<220>
<223>  an artificially synthesized sequence

<400>  229

Thr Ser Ser Ser Gly Arg Ser Ala Asn Ala Arg Gly
1               5                   10


<210>  230
<211>  12
<212>  PRT
<213>  Artificial sequence

<220>
<223>  an artificially synthesized sequence

<400>  230

Thr Ser Ser Ser Gly Arg Ser Ala Asn Phe Arg Gly
1               5                   10


<210>  231
<211>  12
<212>  PRT
<213>  Artificial sequence

<220>
<223>  an artificially synthesized sequence

<400>  231

Thr Ser Ser Ser Gly Arg Ser Ala Asn Tyr Arg Gly
1               5                   10


<210>  232
<211>  12
<212>  PRT
<213>  Artificial sequence

<220>
<223>  an artificially synthesized sequence

<400>  232

Thr Ser Ser Ser Gly Arg Ser Ala Asn Val Arg Gly
1               5                   10


<210>  233
<211>  12
<212>  PRT
<213>  Artificial sequence

<220>
<223>  an artificially synthesized sequence

<400>  233

Thr Ser Ser Ser Gly Arg Ser Ala Asn His Arg Gly
1               5                   10


<210>  234
<211>  12
<212>  PRT
<213>  Artificial sequence

<220>
<223>  an artificially synthesized sequence

<400>  234

Thr Ser Ile Thr Gly Arg Ser Ala Asn Pro Arg Gly
1               5                   10


<210>  235
<211>  12
<212>  PRT
<213>  Artificial sequence

<220>
<223>  an artificially synthesized sequence

<400>  235

Thr Ser Ile Lys Gly Arg Ser Ala Asn Pro Arg Gly
1               5                   10


<210>  236
<211>  12
<212>  PRT
<213>  Artificial sequence

<220>
<223>  an artificially synthesized sequence

<400>  236

Thr Ser Ile Ser Gly Arg Ser Ala Val Pro Arg Gly
1               5                   10


<210>  237
<211>  12
<212>  PRT
<213>  Artificial sequence

<220>
<223>  an artificially synthesized sequence

<400>  237

Thr Ser Ile Ser Gly Arg Ser Ala Ile Pro Arg Gly
1               5                   10

<210>  238
<211>  12
<212>  PRT
<213>  Artificial sequence

<220>
<223>  an artificially synthesized sequence

<400>  238

Thr Ser Ile Ser Gly Arg Ser Ala Thr Pro Arg Gly
1               5                   10


<210>  239
<211>  12
<212>  PRT
<213>  Artificial sequence

<220>
<223>  an artificially synthesized sequence

<400>  239

Thr Ser Ile Ser Gly Arg Ser Ala Ser Pro Arg Gly
1               5                   10


<210>  240
<211>  12
<212>  PRT
<213>  Artificial sequence

<220>
<223>  an artificially synthesized sequence

<400>  240

Thr Ser Ile Ser Gly Arg Ser Ala His Pro Arg Gly
1               5                   10


<210>  241
<211>  12
<212>  PRT
<213>  Artificial sequence

<220>
<223>  an artificially synthesized sequence

<400>  241

Thr Ser Ile Ser Gly Arg Ser Ala Asn Ala Arg Gly
1               5                   10


<210>  242
<211>  12
<212>  PRT
<213>  Artificial sequence

<220>
<223>  an artificially synthesized sequence

<400>  242

Thr Ser Ile Ser Gly Arg Ser Ala Asn Phe Arg Gly
1               5                   10


<210>  243
<211>  12
<212>  PRT
<213>  Artificial sequence

<220>
<223>  an artificially synthesized sequence

<400>  243

Thr Ser Ile Ser Gly Arg Ser Ala Asn Tyr Arg Gly
1               5                   10


<210>  244
<211>  12
<212>  PRT
<213>  Artificial sequence

<220>
<223>  an artificially synthesized sequence

<400>  244

Thr Ser Ile Ser Gly Arg Ser Ala Asn Val Arg Gly
1               5                   10


<210>  245
<211>  12
<212>  PRT
<213>  Artificial sequence

<220>
<223>  an artificially synthesized sequence

<400>  245

Thr Ser Ile Ser Gly Arg Ser Ala Asn His Arg Gly
1               5                   10


<210>  246
<211>  12
<212>  PRT
<213>  Artificial sequence

<220>
<223>  an artificially synthesized sequence

<400>  246

Thr Ser Thr Thr Gly Arg Ser Ala Val Pro Arg Gly
1               5                   10

<210>  247
<211>  12
<212>  PRT
<213>  Artificial sequence

<220>
<223>  an artificially synthesized sequence

<400>  247

Thr Ser Thr Thr Gly Arg Ser Ala Ile Pro Arg Gly
1               5                   10


<210>  248
<211>  12
<212>  PRT
<213>  Artificial sequence

<220>
<223>  an artificially synthesized sequence

<400>  248

Thr Ser Thr Thr Gly Arg Ser Ala Thr Pro Arg Gly
1               5                   10


<210>  249
<211>  12
<212>  PRT
<213>  Artificial sequence

<220>
<223>  an artificially synthesized sequence

<400>  249

Thr Ser Thr Thr Gly Arg Ser Ala His Pro Arg Gly
1               5                   10


<210>  250
<211>  12
<212>  PRT
<213>  Artificial sequence

<220>
<223>  an artificially synthesized sequence

<400>  250

Thr Ser Thr Thr Gly Arg Ser Ala Asn Ala Arg Gly
1               5                   10


<210>  251
<211>  12
<212>  PRT
<213>  Artificial sequence

<220>
<223>  an artificially synthesized sequence

<400>  251

Thr Ser Thr Thr Gly Arg Ser Ala Asn Phe Arg Gly
1               5                   10


<210>  252
<211>  12
<212>  PRT
<213>  Artificial sequence

<220>
<223>  an artificially synthesized sequence

<400>  252

Thr Ser Thr Thr Gly Arg Ser Ala Asn Tyr Arg Gly
1               5                   10


<210>  253
<211>  12
<212>  PRT
<213>  Artificial sequence

<220>
<223>  an artificially synthesized sequence

<400>  253

Thr Ser Thr Thr Gly Arg Ser Ala Asn Val Arg Gly
1               5                   10


<210>  254
<211>  12
<212>  PRT
<213>  Artificial sequence

<220>
<223>  an artificially synthesized sequence

<400>  254

Thr Ser Thr Thr Gly Arg Ser Ala Asn His Arg Gly
1               5                   10


<210>  255
<211>  12
<212>  PRT
<213>  Artificial sequence

<220>
<223>  an artificially synthesized sequence

<400>  255

Thr Ser Thr Lys Gly Arg Ser Ala Val Pro Arg Gly
1               5                   10

<210>  256
<211>  12
<212>  PRT
<213>  Artificial sequence

<220>
<223>  an artificially synthesized sequence

<400>  256

Thr Ser Thr Lys Gly Arg Ser Ala Ile Pro Arg Gly
1               5                   10


<210>  257
<211>  12
<212>  PRT
<213>  Artificial sequence

<220>
<223>  an artificially synthesized sequence

<400>  257

Thr Ser Thr Lys Gly Arg Ser Ala Thr Pro Arg Gly
1               5                   10


<210>  258
<211>  12
<212>  PRT
<213>  Artificial sequence

<220>
<223>  an artificially synthesized sequence

<400>  258

Thr Ser Thr Lys Gly Arg Ser Ala Ser Pro Arg Gly
1               5                   10


<210>  259
<211>  12
<212>  PRT
<213>  Artificial sequence

<220>
<223>  an artificially synthesized sequence

<400>  259

Thr Ser Thr Lys Gly Arg Ser Ala His Pro Arg Gly
1               5                   10


<210>  260
<211>  12
<212>  PRT
<213>  Artificial sequence

<220>
<223>  an artificially synthesized sequence

<400>  260

Thr Ser Thr Lys Gly Arg Ser Ala Asn Ala Arg Gly
1               5                   10


<210>  261
<211>  12
<212>  PRT
<213>  Artificial sequence

<220>
<223>  an artificially synthesized sequence

<400>  261

Thr Ser Thr Lys Gly Arg Ser Ala Asn Phe Arg Gly
1               5                   10


<210>  262
<211>  12
<212>  PRT
<213>  Artificial sequence

<220>
<223>  an artificially synthesized sequence

<400>  262

Thr Ser Thr Lys Gly Arg Ser Ala Asn Tyr Arg Gly
1               5                   10


<210>  263
<211>  12
<212>  PRT
<213>  Artificial sequence

<220>
<223>  an artificially synthesized sequence

<400>  263

Thr Ser Thr Lys Gly Arg Ser Ala Asn Val Arg Gly
1               5                   10


<210>  264
<211>  12
<212>  PRT
<213>  Artificial sequence

<220>
<223>  an artificially synthesized sequence

<400>  264

Thr Ser Thr Lys Gly Arg Ser Ala Asn His Arg Gly
1               5                   10

<210>  265
<211>  12
<212>  PRT
<213>  Artificial sequence

<220>
<223>  an artificially synthesized sequence

<400>  265

Thr Ser Thr Ser Gly Arg Ser Ala Val Tyr Arg Gly
1               5                   10


<210>  266
<211>  12
<212>  PRT
<213>  Artificial sequence

<220>
<223>  an artificially synthesized sequence

<400>  266

Thr Ser Thr Ser Gly Arg Ser Ala Val Val Arg Gly
1               5                   10


<210>  267
<211>  12
<212>  PRT
<213>  Artificial sequence

<220>
<223>  an artificially synthesized sequence

<400>  267

Thr Ser Thr Ser Gly Arg Ser Ala Val His Arg Gly
1               5                   10


<210>  268
<211>  12
<212>  PRT
<213>  Artificial sequence

<220>
<223>  an artificially synthesized sequence

<400>  268

Thr Ser Thr Ser Gly Arg Ser Ala Ile Tyr Arg Gly
1               5                   10


<210>  269
<211>  12
<212>  PRT
<213>  Artificial sequence

<220>
<223>  an artificially synthesized sequence

<400>  269

Thr Ser Thr Ser Gly Arg Ser Ala Ile Val Arg Gly
1               5                   10


<210>  270
<211>  12
<212>  PRT
<213>  Artificial sequence

<220>
<223>  an artificially synthesized sequence

<400>  270

Thr Ser Thr Ser Gly Arg Ser Ala Ile His Arg Gly
1               5                   10


<210>  271
<211>  12
<212>  PRT
<213>  Artificial sequence

<220>
<223>  an artificially synthesized sequence

<400>  271

Thr Ser Thr Ser Gly Arg Ser Ala Ser Tyr Arg Gly
1               5                   10


<210>  272
<211>  12
<212>  PRT
<213>  Artificial sequence

<220>
<223>  an artificially synthesized sequence

<400>  272

Thr Ser Thr Ser Gly Arg Ser Ala Ser Val Arg Gly
1               5                   10


<210>  273
<211>  12
<212>  PRT
<213>  Artificial sequence

<220>
<223>  an artificially synthesized sequence

<400>  273

Thr Ser Thr Ser Gly Arg Ser Ala Ser His Arg Gly
1               5                   10

<210>  274
<211>  12
<212>  PRT
<213>  Artificial sequence

<220>
<223>  an artificially synthesized sequence

<400>  274

Thr Ser Thr Ser Gly Arg Ser Ala His Tyr Arg Gly
1               5                   10


<210>  275
<211>  12
<212>  PRT
<213>  Artificial sequence

<220>
<223>  an artificially synthesized sequence

<400>  275

Thr Ser Thr Ser Gly Arg Ser Ala His Val Arg Gly
1               5                   10


<210>  276
<211>  12
<212>  PRT
<213>  Artificial sequence

<220>
<223>  an artificially synthesized sequence

<400>  276

Thr Ser Thr Ser Gly Arg Ser Ala His His Arg Gly
1               5                   10


<210>  277
<211>  12
<212>  PRT
<213>  Artificial sequence

<220>
<223>  an artificially synthesized sequence

<400>  277

Thr Ser Pro Ser Gly Arg Ser Glu Val Pro Arg Gly
1               5                   10


<210>  278
<211>  12
<212>  PRT
<213>  Artificial sequence

<220>
<223>  an artificially synthesized sequence

<400>  278

Thr Ser Pro Ser Gly Arg Ser Ala Glu Pro Arg Gly
1               5                   10


<210>  279
<211>  12
<212>  PRT
<213>  Artificial sequence

<220>
<223>  an artificially synthesized sequence

<400>  279

Thr Ser Pro Ser Gly Arg Ser Ala Gly Pro Arg Gly
1               5                   10


<210>  280
<211>  12
<212>  PRT
<213>  Artificial sequence

<220>
<223>  an artificially synthesized sequence

<400>  280

Thr Ser Ala Ser Gly Arg Ser Glu Asn Ala Arg Gly
1               5                   10


<210>  281
<211>  12
<212>  PRT
<213>  Artificial sequence

<220>
<223>  an artificially synthesized sequence

<400>  281

Thr Ser Ala Ser Gly Arg Ser Ala Glu Ala Arg Gly
1               5                   10


<210>  282
<211>  12
<212>  PRT
<213>  Artificial sequence

<220>
<223>  an artificially synthesized sequence

<400>  282

Thr Ser Ala Ser Gly Arg Ser Ala Gly Ala Arg Gly
1               5                   10

<210>  283
<211>  12
<212>  PRT
<213>  Artificial sequence

<220>
<223>  an artificially synthesized sequence

<400>  283

Thr Ser Gly Thr Gly Arg Ser Ala Thr Pro Arg Gly
1               5                   10


<210>  284
<211>  12
<212>  PRT
<213>  Artificial sequence

<220>
<223>  an artificially synthesized sequence

<400>  284

Thr Ser Gly Ser Gly Arg Ser Ala Thr Tyr Arg Gly
1               5                   10


<210>  285
<211>  12
<212>  PRT
<213>  Artificial sequence

<220>
<223>  an artificially synthesized sequence

<400>  285

Thr Ser Gly Ser Gly Arg Ser Ala Thr Val Arg Gly
1               5                   10


<210>  286
<211>  12
<212>  PRT
<213>  Artificial sequence

<220>
<223>  an artificially synthesized sequence

<400>  286

Thr Ser Gly Ser Gly Arg Ser Ala Thr His Arg Gly
1               5                   10


<210>  287
<211>  12
<212>  PRT
<213>  Artificial sequence

<220>
<223>  an artificially synthesized sequence

<400>  287

Thr Ser Gly Thr Gly Arg Ser Ala Thr Tyr Arg Gly
1               5                   10


<210>  288
<211>  12
<212>  PRT
<213>  Artificial sequence

<220>
<223>  an artificially synthesized sequence

<400>  288

Thr Ser Gly Thr Gly Arg Ser Ala Thr Val Arg Gly
1               5                   10


<210>  289
<211>  12
<212>  PRT
<213>  Artificial sequence

<220>
<223>  an artificially synthesized sequence

<400>  289

Thr Ser Gly Thr Gly Arg Ser Ala Thr His Arg Gly
1               5                   10


<210>  290
<211>  12
<212>  PRT
<213>  Artificial sequence

<220>
<223>  an artificially synthesized sequence

<400>  290

Thr Ser Gly Ser Gly Arg Ser Glu Thr Pro Arg Gly
1               5                   10


<210>  291
<211>  12
<212>  PRT
<213>  Artificial sequence

<220>
<223>  an artificially synthesized sequence

<400>  291

Thr Ser Gly Thr Gly Arg Ser Glu Thr Pro Arg Gly
1               5                   10

<210>  292
<211>  12
<212>  PRT
<213>  Artificial sequence

<220>
<223>  an artificially synthesized sequence

<400>  292

Thr Ser Gly Ser Gly Arg Ser Glu Thr Tyr Arg Gly
1               5                   10


<210>  293
<211>  12
<212>  PRT
<213>  Artificial sequence

<220>
<223>  an artificially synthesized sequence

<400>  293

Thr Ser Gly Ser Gly Arg Ser Glu Thr Val Arg Gly
1               5                   10


<210>  294
<211>  12
<212>  PRT
<213>  Artificial sequence

<220>
<223>  an artificially synthesized sequence

<400>  294

Thr Ser Gly Ser Gly Arg Ser Glu Thr His Arg Gly
1               5                   10


<210>  295
<211>  12
<212>  PRT
<213>  Artificial sequence

<220>
<223>  an artificially synthesized sequence

<400>  295

Thr Ser Tyr Thr Gly Arg Ser Ala Val Pro Arg Gly
1               5                   10


<210>  296
<211>  12
<212>  PRT
<213>  Artificial sequence

<220>
<223>  an artificially synthesized sequence

<400>  296

Thr Ser Tyr Ser Gly Arg Ser Ala Val Tyr Arg Gly
1               5                   10


<210>  297
<211>  12
<212>  PRT
<213>  Artificial sequence

<220>
<223>  an artificially synthesized sequence

<400>  297

Thr Ser Tyr Ser Gly Arg Ser Ala Val Val Arg Gly
1               5                   10


<210>  298
<211>  12
<212>  PRT
<213>  Artificial sequence

<220>
<223>  an artificially synthesized sequence

<400>  298

Thr Ser Tyr Ser Gly Arg Ser Ala Val His Arg Gly
1               5                   10


<210>  299
<211>  12
<212>  PRT
<213>  Artificial sequence

<220>
<223>  an artificially synthesized sequence

<400>  299

Thr Ser Tyr Thr Gly Arg Ser Ala Val Tyr Arg Gly
1               5                   10


<210>  300
<211>  12
<212>  PRT
<213>  Artificial sequence

<220>
<223>  an artificially synthesized sequence

<400>  300

Thr Ser Tyr Thr Gly Arg Ser Ala Val Val Arg Gly
1               5                   10

<210>  301
<211>  12
<212>  PRT
<213>  Artificial sequence

<220>
<223>  an artificially synthesized sequence

<400>  301

Thr Ser Tyr Thr Gly Arg Ser Ala Val His Arg Gly
1               5                   10


<210>  302
<211>  12
<212>  PRT
<213>  Artificial sequence

<220>
<223>  an artificially synthesized sequence

<400>  302

Thr Ser Tyr Ser Gly Arg Ser Glu Val Pro Arg Gly
1               5                   10


<210>  303
<211>  12
<212>  PRT
<213>  Artificial sequence

<220>
<223>  an artificially synthesized sequence

<400>  303

Thr Ser Tyr Thr Gly Arg Ser Glu Val Pro Arg Gly
1               5                   10


<210>  304
<211>  12
<212>  PRT
<213>  Artificial sequence

<220>
<223>  an artificially synthesized sequence

<400>  304

Thr Ser Tyr Ser Gly Arg Ser Glu Val Tyr Arg Gly
1               5                   10


<210>  305
<211>  12
<212>  PRT
<213>  Artificial sequence

<220>
<223>  an artificially synthesized sequence

<400>  305

Thr Ser Tyr Ser Gly Arg Ser Glu Val Val Arg Gly
1               5                   10


<210>  306
<211>  12
<212>  PRT
<213>  Artificial sequence

<220>
<223>  an artificially synthesized sequence

<400>  306

Thr Ser Tyr Ser Gly Arg Ser Glu Val His Arg Gly
1               5                   10


<210>  307
<211>  12
<212>  PRT
<213>  Artificial sequence

<220>
<223>  an artificially synthesized sequence

<400>  307

Thr Ser Tyr Thr Gly Arg Ser Ala Val Pro Gly Gly
1               5                   10


<210>  308
<211>  12
<212>  PRT
<213>  Artificial sequence

<220>
<223>  an artificially synthesized sequence

<400>  308

Thr Ser Tyr Ser Gly Arg Ser Ala Val Tyr Gly Gly
1               5                   10


<210>  309
<211>  12
<212>  PRT
<213>  Artificial sequence

<220>
<223>  an artificially synthesized sequence

<400>  309

Thr Ser Tyr Ser Gly Arg Ser Ala Val Val Gly Gly
1               5                   10

<210>  310
<211>  12
<212>  PRT
<213>  Artificial sequence

<220>
<223>  an artificially synthesized sequence

<400>  310

Thr Ser Tyr Ser Gly Arg Ser Ala Val His Gly Gly
1               5                   10


<210>  311
<211>  12
<212>  PRT
<213>  Artificial sequence

<220>
<223>  an artificially synthesized sequence

<400>  311

Thr Ser Tyr Thr Gly Arg Ser Ala Val Tyr Gly Gly
1               5                   10


<210>  312
<211>  12
<212>  PRT
<213>  Artificial sequence

<220>
<223>  an artificially synthesized sequence

<400>  312

Thr Ser Tyr Thr Gly Arg Ser Ala Val Val Gly Gly
1               5                   10


<210>  313
<211>  12
<212>  PRT
<213>  Artificial sequence

<220>
<223>  an artificially synthesized sequence

<400>  313

Thr Ser Tyr Thr Gly Arg Ser Ala Val His Gly Gly
1               5                   10


<210>  314
<211>  8
<212>  PRT
<213>  Artificial sequence

<220>
<223>  an artificially synthesized sequence

<400>  314

Ala Ser Gly Arg Ser Ala Asn Pro
1               5


<210>  315
<211>  8
<212>  PRT
<213>  Artificial sequence

<220>
<223>  an artificially synthesized sequence

<400>  315

Glu Ser Gly Arg Ser Ala Asn Pro
1               5


<210>  316
<211>  8
<212>  PRT
<213>  Artificial sequence

<220>
<223>  an artificially synthesized sequence

<400>  316

Phe Ser Gly Arg Ser Ala Asn Pro
1               5


<210>  317
<211>  8
<212>  PRT
<213>  Artificial sequence

<220>
<223>  an artificially synthesized sequence

<400>  317

Gly Ser Gly Arg Ser Ala Asn Pro
1               5


<210>  318
<211>  8
<212>  PRT
<213>  Artificial sequence

<220>
<223>  an artificially synthesized sequence

<400>  318

His Ser Gly Arg Ser Ala Asn Pro
1               5

<210>  319
<211>  8
<212>  PRT
<213>  Artificial sequence

<220>
<223>  an artificially synthesized sequence

<400>  319

Lys Ser Gly Arg Ser Ala Asn Pro
1               5


<210>  320
<211>  8
<212>  PRT
<213>  Artificial sequence

<220>
<223>  an artificially synthesized sequence

<400>  320

Met Ser Gly Arg Ser Ala Asn Pro
1               5


<210>  321
<211>  8
<212>  PRT
<213>  Artificial sequence

<220>
<223>  an artificially synthesized sequence

<400>  321

Asn Ser Gly Arg Ser Ala Asn Pro
1               5


<210>  322
<211>  8
<212>  PRT
<213>  Artificial sequence

<220>
<223>  an artificially synthesized sequence

<400>  322

Pro Ser Gly Arg Ser Ala Asn Pro
1               5


<210>  323
<211>  8
<212>  PRT
<213>  Artificial sequence

<220>
<223>  an artificially synthesized sequence

<400>  323

Gln Ser Gly Arg Ser Ala Asn Pro
1               5


<210>  324
<211>  8
<212>  PRT
<213>  Artificial sequence

<220>
<223>  an artificially synthesized sequence

<400>  324

Trp Ser Gly Arg Ser Ala Asn Pro
1               5


<210>  325
<211>  8
<212>  PRT
<213>  Artificial sequence

<220>
<223>  an artificially synthesized sequence

<400>  325

Tyr Ser Gly Arg Ser Ala Asn Pro
1               5


<210>  326
<211>  8
<212>  PRT
<213>  Artificial sequence

<220>
<223>  an artificially synthesized sequence

<400>  326

Thr Ala Gly Arg Ser Ala Asn Pro
1               5


<210>  327
<211>  8
<212>  PRT
<213>  Artificial sequence

<220>
<223>  an artificially synthesized sequence

<400>  327

Thr Asp Gly Arg Ser Ala Asn Pro
1               5

<210>  328
<211>  8
<212>  PRT
<213>  Artificial sequence

<220>
<223>  an artificially synthesized sequence

<400>  328

Thr Glu Gly Arg Ser Ala Asn Pro
1               5


<210>  329
<211>  8
<212>  PRT
<213>  Artificial sequence

<220>
<223>  an artificially synthesized sequence

<400>  329

Thr Phe Gly Arg Ser Ala Asn Pro
1               5


<210>  330
<211>  8
<212>  PRT
<213>  Artificial sequence

<220>
<223>  an artificially synthesized sequence

<400>  330

Thr Leu Gly Arg Ser Ala Asn Pro
1               5


<210>  331
<211>  8
<212>  PRT
<213>  Artificial sequence

<220>
<223>  an artificially synthesized sequence

<400>  331

Thr Met Gly Arg Ser Ala Asn Pro
1               5


<210>  332
<211>  8
<212>  PRT
<213>  Artificial sequence

<220>
<223>  an artificially synthesized sequence

<400>  332

Thr Pro Gly Arg Ser Ala Asn Pro
1               5


<210>  333
<211>  8
<212>  PRT
<213>  Artificial sequence

<220>
<223>  an artificially synthesized sequence

<400>  333

Thr Gln Gly Arg Ser Ala Asn Pro
1               5


<210>  334
<211>  8
<212>  PRT
<213>  Artificial sequence

<220>
<223>  an artificially synthesized sequence

<400>  334

Thr Val Gly Arg Ser Ala Asn Pro
1               5


<210>  335
<211>  8
<212>  PRT
<213>  Artificial sequence

<220>
<223>  an artificially synthesized sequence

<400>  335

Thr Trp Gly Arg Ser Ala Asn Pro
1               5


<210>  336
<211>  8
<212>  PRT
<213>  Artificial sequence

<220>
<223>  an artificially synthesized sequence

<400>  336

Thr Ser Ala Arg Ser Ala Asn Pro
1               5

<210>  337
<211>  8
<212>  PRT
<213>  Artificial sequence

<220>
<223>  an artificially synthesized sequence

<400>  337

Thr Ser Glu Arg Ser Ala Asn Pro
1               5


<210>  338
<211>  8
<212>  PRT
<213>  Artificial sequence

<220>
<223>  an artificially synthesized sequence

<400>  338

Thr Ser Phe Arg Ser Ala Asn Pro
1               5


<210>  339
<211>  8
<212>  PRT
<213>  Artificial sequence

<220>
<223>  an artificially synthesized sequence

<400>  339

Thr Ser His Arg Ser Ala Asn Pro
1               5


<210>  340
<211>  8
<212>  PRT
<213>  Artificial sequence

<220>
<223>  an artificially synthesized sequence

<400>  340

Thr Ser Ile Arg Ser Ala Asn Pro
1               5


<210>  341
<211>  8
<212>  PRT
<213>  Artificial sequence

<220>
<223>  an artificially synthesized sequence

<400>  341

Thr Ser Lys Arg Ser Ala Asn Pro
1               5


<210>  342
<211>  8
<212>  PRT
<213>  Artificial sequence

<220>
<223>  an artificially synthesized sequence

<400>  342

Thr Ser Leu Arg Ser Ala Asn Pro
1               5


<210>  343
<211>  8
<212>  PRT
<213>  Artificial sequence

<220>
<223>  an artificially synthesized sequence

<400>  343

Thr Ser Met Arg Ser Ala Asn Pro
1               5


<210>  344
<211>  8
<212>  PRT
<213>  Artificial sequence

<220>
<223>  an artificially synthesized sequence

<400>  344

Thr Ser Asn Arg Ser Ala Asn Pro
1               5


<210>  345
<211>  8
<212>  PRT
<213>  Artificial sequence

<220>
<223>  an artificially synthesized sequence

<400>  345

Thr Ser Pro Arg Ser Ala Asn Pro
1               5

<210>  346
<211>  8
<212>  PRT
<213>  Artificial sequence

<220>
<223>  an artificially synthesized sequence

<400>  346

Thr Ser Gln Arg Ser Ala Asn Pro
1               5


<210>  347
<211>  8
<212>  PRT
<213>  Artificial sequence

<220>
<223>  an artificially synthesized sequence

<400>  347

Thr Ser Arg Arg Ser Ala Asn Pro
1               5


<210>  348
<211>  8
<212>  PRT
<213>  Artificial sequence

<220>
<223>  an artificially synthesized sequence

<400>  348

Thr Ser Thr Arg Ser Ala Asn Pro
1               5


<210>  349
<211>  8
<212>  PRT
<213>  Artificial sequence

<220>
<223>  an artificially synthesized sequence

<400>  349

Thr Ser Val Arg Ser Ala Asn Pro
1               5


<210>  350
<211>  8
<212>  PRT
<213>  Artificial sequence

<220>
<223>  an artificially synthesized sequence

<400>  350

Thr Ser Trp Arg Ser Ala Asn Pro
1               5


<210>  351
<211>  8
<212>  PRT
<213>  Artificial sequence

<220>
<223>  an artificially synthesized sequence

<400>  351

Thr Ser Tyr Arg Ser Ala Asn Pro
1               5


<210>  352
<211>  8
<212>  PRT
<213>  Artificial sequence

<220>
<223>  an artificially synthesized sequence

<400>  352

Thr Ser Gly Arg Ala Ala Asn Pro
1               5


<210>  353
<211>  8
<212>  PRT
<213>  Artificial sequence

<220>
<223>  an artificially synthesized sequence

<400>  353

Thr Ser Gly Arg Asp Ala Asn Pro
1               5


<210>  354
<211>  8
<212>  PRT
<213>  Artificial sequence

<220>
<223>  an artificially synthesized sequence

<400>  354

Thr Ser Gly Arg Glu Ala Asn Pro
1               5

<210>  355
<211>  8
<212>  PRT
<213>  Artificial sequence

<220>
<223>  an artificially synthesized sequence

<400>  355

Thr Ser Gly Arg Gly Ala Asn Pro
1               5


<210>  356
<211>  8
<212>  PRT
<213>  Artificial sequence

<220>
<223>  an artificially synthesized sequence

<400>  356

Thr Ser Gly Arg His Ala Asn Pro
1               5


<210>  357
<211>  8
<212>  PRT
<213>  Artificial sequence

<220>
<223>  an artificially synthesized sequence

<400>  357

Thr Ser Gly Arg Ile Ala Asn Pro
1               5


<210>  358
<211>  8
<212>  PRT
<213>  Artificial sequence

<220>
<223>  an artificially synthesized sequence

<400>  358

Thr Ser Gly Arg Lys Ala Asn Pro
1               5


<210>  359
<211>  8
<212>  PRT
<213>  Artificial sequence

<220>
<223>  an artificially synthesized sequence

<400>  359

Thr Ser Gly Arg Leu Ala Asn Pro
1               5


<210>  360
<211>  8
<212>  PRT
<213>  Artificial sequence

<220>
<223>  an artificially synthesized sequence

<400>  360

Thr Ser Gly Arg Met Ala Asn Pro
1               5


<210>  361
<211>  8
<212>  PRT
<213>  Artificial sequence

<220>
<223>  an artificially synthesized sequence

<400>  361

Thr Ser Gly Arg Asn Ala Asn Pro
1               5


<210>  362
<211>  8
<212>  PRT
<213>  Artificial sequence

<220>
<223>  an artificially synthesized sequence

<400>  362

Thr Ser Gly Arg Pro Ala Asn Pro
1               5


<210>  363
<211>  8
<212>  PRT
<213>  Artificial sequence

<220>
<223>  an artificially synthesized sequence

<400>  363

Thr Ser Gly Arg Gln Ala Asn Pro
1               5

<210>  364
<211>  8
<212>  PRT
<213>  Artificial sequence

<220>
<223>  an artificially synthesized sequence

<400>  364

Thr Ser Gly Arg Arg Ala Asn Pro
1               5


<210>  365
<211>  8
<212>  PRT
<213>  Artificial sequence

<220>
<223>  an artificially synthesized sequence

<400>  365

Thr Ser Gly Arg Thr Ala Asn Pro
1               5


<210>  366
<211>  8
<212>  PRT
<213>  Artificial sequence

<220>
<223>  an artificially synthesized sequence

<400>  366

Thr Ser Gly Arg Val Ala Asn Pro
1               5


<210>  367
<211>  8
<212>  PRT
<213>  Artificial sequence

<220>
<223>  an artificially synthesized sequence

<400>  367

Thr Ser Gly Arg Trp Ala Asn Pro
1               5


<210>  368
<211>  8
<212>  PRT
<213>  Artificial sequence

<220>
<223>  an artificially synthesized sequence

<400>  368

Thr Ser Gly Arg Tyr Ala Asn Pro
1               5


<210>  369
<211>  8
<212>  PRT
<213>  Artificial sequence

<220>
<223>  an artificially synthesized sequence

<400>  369

Thr Ser Gly Arg Ser Glu Asn Pro
1               5


<210>  370
<211>  8
<212>  PRT
<213>  Artificial sequence

<220>
<223>  an artificially synthesized sequence

<400>  370

Thr Ser Gly Arg Ser Phe Asn Pro
1               5


<210>  371
<211>  8
<212>  PRT
<213>  Artificial sequence

<220>
<223>  an artificially synthesized sequence

<400>  371

Thr Ser Gly Arg Ser Lys Asn Pro
1               5


<210>  372
<211>  8
<212>  PRT
<213>  Artificial sequence

<220>
<223>  an artificially synthesized sequence

<400>  372

Thr Ser Gly Arg Ser Met Asn Pro
1               5

<210>  373
<211>  8
<212>  PRT
<213>  Artificial sequence

<220>
<223>  an artificially synthesized sequence

<400>  373

Thr Ser Gly Arg Ser Asn Asn Pro
1               5


<210>  374
<211>  8
<212>  PRT
<213>  Artificial sequence

<220>
<223>  an artificially synthesized sequence

<400>  374

Thr Ser Gly Arg Ser Pro Asn Pro
1               5


<210>  375
<211>  8
<212>  PRT
<213>  Artificial sequence

<220>
<223>  an artificially synthesized sequence

<400>  375

Thr Ser Gly Arg Ser Gln Asn Pro
1               5


<210>  376
<211>  8
<212>  PRT
<213>  Artificial sequence

<220>
<223>  an artificially synthesized sequence

<400>  376

Thr Ser Gly Arg Ser Arg Asn Pro
1               5


<210>  377
<211>  8
<212>  PRT
<213>  Artificial sequence

<220>
<223>  an artificially synthesized sequence

<400>  377

Thr Ser Gly Arg Ser Ser Asn Pro
1               5


<210>  378
<211>  8
<212>  PRT
<213>  Artificial sequence

<220>
<223>  an artificially synthesized sequence

<400>  378

Thr Ser Gly Arg Ser Trp Asn Pro
1               5


<210>  379
<211>  8
<212>  PRT
<213>  Artificial sequence

<220>
<223>  an artificially synthesized sequence

<400>  379

Thr Ser Gly Arg Ser Tyr Asn Pro
1               5


<210>  380
<211>  8
<212>  PRT
<213>  Artificial sequence

<220>
<223>  an artificially synthesized sequence

<400>  380

Thr Ser Gly Arg Ser Ala Ala Pro
1               5


<210>  381
<211>  8
<212>  PRT
<213>  Artificial sequence

<220>
<223>  an artificially synthesized sequence

<400>  381

Thr Ser Gly Arg Ser Ala Asp Pro
1               5

<210>  382
<211>  8
<212>  PRT
<213>  Artificial sequence

<220>
<223>  an artificially synthesized sequence

<400>  382

Thr Ser Gly Arg Ser Ala Glu Pro
1               5


<210>  383
<211>  8
<212>  PRT
<213>  Artificial sequence

<220>
<223>  an artificially synthesized sequence

<400>  383

Thr Ser Gly Arg Ser Ala Phe Pro
1               5


<210>  384
<211>  8
<212>  PRT
<213>  Artificial sequence

<220>
<223>  an artificially synthesized sequence

<400>  384

Thr Ser Gly Arg Ser Ala Gly Pro
1               5


<210>  385
<211>  8
<212>  PRT
<213>  Artificial sequence

<220>
<223>  an artificially synthesized sequence

<400>  385

Thr Ser Gly Arg Ser Ala Lys Pro
1               5


<210>  386
<211>  8
<212>  PRT
<213>  Artificial sequence

<220>
<223>  an artificially synthesized sequence

<400>  386

Thr Ser Gly Arg Ser Ala Leu Pro
1               5


<210>  387
<211>  8
<212>  PRT
<213>  Artificial sequence

<220>
<223>  an artificially synthesized sequence

<400>  387

Thr Ser Gly Arg Ser Ala Met Pro
1               5


<210>  388
<211>  8
<212>  PRT
<213>  Artificial sequence

<220>
<223>  an artificially synthesized sequence

<400>  388

Thr Ser Gly Arg Ser Ala Pro Pro
1               5


<210>  389
<211>  8
<212>  PRT
<213>  Artificial sequence

<220>
<223>  an artificially synthesized sequence

<400>  389

Thr Ser Gly Arg Ser Ala Gln Pro
1               5


<210>  390
<211>  8
<212>  PRT
<213>  Artificial sequence

<220>
<223>  an artificially synthesized sequence

<400>  390

Thr Ser Gly Arg Ser Ala Val Pro
1               5

<210>  391
<211>  8
<212>  PRT
<213>  Artificial sequence

<220>
<223>  an artificially synthesized sequence

<400>  391

Thr Ser Gly Arg Ser Ala Trp Pro
1               5


<210>  392
<211>  8
<212>  PRT
<213>  Artificial sequence

<220>
<223>  an artificially synthesized sequence

<400>  392

Thr Ser Gly Arg Ser Ala Tyr Pro
1               5


<210>  393
<211>  8
<212>  PRT
<213>  Artificial sequence

<220>
<223>  an artificially synthesized sequence

<400>  393

Thr Ser Gly Arg Ser Ala Asn Ala
1               5


<210>  394
<211>  8
<212>  PRT
<213>  Artificial sequence

<220>
<223>  an artificially synthesized sequence

<400>  394

Thr Ser Gly Arg Ser Ala Asn Asp
1               5


<210>  395
<211>  8
<212>  PRT
<213>  Artificial sequence

<220>
<223>  an artificially synthesized sequence

<400>  395

Thr Ser Gly Arg Ser Ala Asn Glu
1               5


<210>  396
<211>  8
<212>  PRT
<213>  Artificial sequence

<220>
<223>  an artificially synthesized sequence

<400>  396

Thr Ser Gly Arg Ser Ala Asn Phe
1               5


<210>  397
<211>  8
<212>  PRT
<213>  Artificial sequence

<220>
<223>  an artificially synthesized sequence

<400>  397

Thr Ser Gly Arg Ser Ala Asn Gly
1               5


<210>  398
<211>  8
<212>  PRT
<213>  Artificial sequence

<220>
<223>  an artificially synthesized sequence

<400>  398

Thr Ser Gly Arg Ser Ala Asn Ile
1               5


<210>  399
<211>  8
<212>  PRT
<213>  Artificial sequence

<220>
<223>  an artificially synthesized sequence

<400>  399

Thr Ser Gly Arg Ser Ala Asn Lys
1               5

<210>  400
<211>  8
<212>  PRT
<213>  Artificial sequence

<220>
<223>  an artificially synthesized sequence

<400>  400

Thr Ser Gly Arg Ser Ala Asn Asn
1               5


<210>  401
<211>  8
<212>  PRT
<213>  Artificial sequence

<220>
<223>  an artificially synthesized sequence

<400>  401

Thr Ser Gly Arg Ser Ala Asn Gln
1               5


<210>  402
<211>  8
<212>  PRT
<213>  Artificial sequence

<220>
<223>  an artificially synthesized sequence

<400>  402

Thr Ser Gly Arg Ser Ala Asn Ser
1               5


<210>  403
<211>  8
<212>  PRT
<213>  Artificial sequence

<220>
<223>  an artificially synthesized sequence

<400>  403

Thr Ser Gly Arg Ser Ala Asn Thr
1               5


<210>  404
<211>  8
<212>  PRT
<213>  Artificial sequence

<220>
<223>  an artificially synthesized sequence

<400>  404

Thr Ser Gly Arg Ser Ala Asn Trp
1               5


<210>  405
<211>  8
<212>  PRT
<213>  Artificial sequence

<220>
<223>  an artificially synthesized sequence

<400>  405

Asp Ser Gly Arg Ser Ala Asn Pro
1               5


<210>  406
<211>  8
<212>  PRT
<213>  Artificial sequence

<220>
<223>  an artificially synthesized sequence

<400>  406

Ile Ser Gly Arg Ser Ala Asn Pro
1               5


<210>  407
<211>  8
<212>  PRT
<213>  Artificial sequence

<220>
<223>  an artificially synthesized sequence

<400>  407

Ser Ser Gly Arg Ser Ala Asn Pro
1               5


<210>  408
<211>  8
<212>  PRT
<213>  Artificial sequence

<220>
<223>  an artificially synthesized sequence

<400>  408

Thr His Gly Arg Ser Ala Asn Pro
1               5

<210>  409
<211>  8
<212>  PRT
<213>  Artificial sequence

<220>
<223>  an artificially synthesized sequence

<400>  409

Thr Lys Gly Arg Ser Ala Asn Pro
1               5


<210>  410
<211>  8
<212>  PRT
<213>  Artificial sequence

<220>
<223>  an artificially synthesized sequence

<400>  410

Thr Thr Gly Arg Ser Ala Asn Pro
1               5


<210>  411
<211>  8
<212>  PRT
<213>  Artificial sequence

<220>
<223>  an artificially synthesized sequence

<400>  411

Thr Tyr Gly Arg Ser Ala Asn Pro
1               5


<210>  412
<211>  8
<212>  PRT
<213>  Artificial sequence

<220>
<223>  an artificially synthesized sequence

<400>  412

Thr Ser Asp Arg Ser Ala Asn Pro
1               5


<210>  413
<211>  8
<212>  PRT
<213>  Artificial sequence

<220>
<223>  an artificially synthesized sequence

<400>  413

Thr Ser Ser Arg Ser Ala Asn Pro
1               5


<210>  414
<211>  8
<212>  PRT
<213>  Artificial sequence

<220>
<223>  an artificially synthesized sequence

<400>  414

Thr Ser Gly Arg Phe Ala Asn Pro
1               5


<210>  415
<211>  8
<212>  PRT
<213>  Artificial sequence

<220>
<223>  an artificially synthesized sequence

<400>  415

Thr Ser Gly Arg Ser Asp Asn Pro
1               5


<210>  416
<211>  8
<212>  PRT
<213>  Artificial sequence

<220>
<223>  an artificially synthesized sequence

<400>  416

Thr Ser Gly Arg Ser His Asn Pro
1               5


<210>  417
<211>  8
<212>  PRT
<213>  Artificial sequence

<220>
<223>  an artificially synthesized sequence

<400>  417

Thr Ser Gly Arg Ser Ile Asn Pro
1               5

<210>  418
<211>  8
<212>  PRT
<213>  Artificial sequence

<220>
<223>  an artificially synthesized sequence

<400>  418

Thr Ser Gly Arg Ser Leu Asn Pro
1               5


<210>  419
<211>  8
<212>  PRT
<213>  Artificial sequence

<220>
<223>  an artificially synthesized sequence

<400>  419

Thr Ser Gly Arg Ser Thr Asn Pro
1               5


<210>  420
<211>  8
<212>  PRT
<213>  Artificial sequence

<220>
<223>  an artificially synthesized sequence

<400>  420

Thr Ser Gly Arg Ser Val Asn Pro
1               5


<210>  421
<211>  8
<212>  PRT
<213>  Artificial sequence

<220>
<223>  an artificially synthesized sequence

<400>  421

Thr Ser Gly Arg Ser Ala His Pro
1               5


<210>  422
<211>  8
<212>  PRT
<213>  Artificial sequence

<220>
<223>  an artificially synthesized sequence

<400>  422

Thr Ser Gly Arg Ser Ala Ile Pro
1               5


<210>  423
<211>  8
<212>  PRT
<213>  Artificial sequence

<220>
<223>  an artificially synthesized sequence

<400>  423

Thr Ser Gly Arg Ser Ala Arg Pro
1               5


<210>  424
<211>  8
<212>  PRT
<213>  Artificial sequence

<220>
<223>  an artificially synthesized sequence

<400>  424

Thr Ser Gly Arg Ser Ala Ser Pro
1               5


<210>  425
<211>  8
<212>  PRT
<213>  Artificial sequence

<220>
<223>  an artificially synthesized sequence

<400>  425

Thr Ser Gly Arg Ser Ala Thr Pro
1               5


<210>  426
<211>  8
<212>  PRT
<213>  Artificial sequence

<220>
<223>  an artificially synthesized sequence

<400>  426

Thr Ser Gly Arg Ser Ala Asn His
1               5

<210>  427
<211>  8
<212>  PRT
<213>  Artificial sequence

<220>
<223>  an artificially synthesized sequence

<400>  427

Thr Ser Gly Arg Ser Ala Asn Leu
1               5


<210>  428
<211>  8
<212>  PRT
<213>  Artificial sequence

<220>
<223>  an artificially synthesized sequence

<400>  428

Thr Ser Gly Arg Ser Ala Asn Met
1               5


<210>  429
<211>  8
<212>  PRT
<213>  Artificial sequence

<220>
<223>  an artificially synthesized sequence

<400>  429

Thr Ser Gly Arg Ser Ala Asn Arg
1               5


<210>  430
<211>  8
<212>  PRT
<213>  Artificial sequence

<220>
<223>  an artificially synthesized sequence

<400>  430

Thr Ser Gly Arg Ser Ala Asn Val
1               5


<210>  431
<211>  8
<212>  PRT
<213>  Artificial sequence

<220>
<223>  an artificially synthesized sequence

<400>  431

Thr Ser Gly Arg Ser Ala Asn Tyr
1               5


<210>  432
<211>  8
<212>  PRT
<213>  Artificial sequence

<220>
<223>  an artificially synthesized sequence

<400>  432

Gly Ser Gly Arg Ser Ala Val Pro
1               5


<210>  433
<211>  8
<212>  PRT
<213>  Artificial sequence

<220>
<223>  an artificially synthesized sequence

<400>  433

Gly Ser Gly Arg Ser Ala Tyr Pro
1               5


<210>  434
<211>  8
<212>  PRT
<213>  Artificial sequence

<220>
<223>  an artificially synthesized sequence

<400>  434

Gly Ser Gly Arg Ser Ala Asn Gln
1               5


<210>  435
<211>  8
<212>  PRT
<213>  Artificial sequence

<220>
<223>  an artificially synthesized sequence

<400>  435

Gly Ser Gly Arg Ser Ala Asn Ala
1               5

<210>  436
<211>  8
<212>  PRT
<213>  Artificial sequence

<220>
<223>  an artificially synthesized sequence

<400>  436

Gly Ser Gly Arg Ser Ala Asn Ile
1               5


<210>  437
<211>  8
<212>  PRT
<213>  Artificial sequence

<220>
<223>  an artificially synthesized sequence

<400>  437

Gly Ser Gly Arg Ser Ala Asn Phe
1               5


<210>  438
<211>  8
<212>  PRT
<213>  Artificial sequence

<220>
<223>  an artificially synthesized sequence

<400>  438

Gly Ser Gly Arg Ser Ala Asn Ser
1               5


<210>  439
<211>  8
<212>  PRT
<213>  Artificial sequence

<220>
<223>  an artificially synthesized sequence

<400>  439

Gln Ser Gly Arg Ser Ala Val Pro
1               5


<210>  440
<211>  8
<212>  PRT
<213>  Artificial sequence

<220>
<223>  an artificially synthesized sequence

<400>  440

Gln Ser Gly Arg Ser Ala Tyr Pro
1               5


<210>  441
<211>  8
<212>  PRT
<213>  Artificial sequence

<220>
<223>  an artificially synthesized sequence

<400>  441

Gln Ser Gly Arg Ser Ala Asn Gln
1               5


<210>  442
<211>  8
<212>  PRT
<213>  Artificial sequence

<220>
<223>  an artificially synthesized sequence

<400>  442

Gln Ser Gly Arg Ser Ala Asn Ala
1               5


<210>  443
<211>  8
<212>  PRT
<213>  Artificial sequence

<220>
<223>  an artificially synthesized sequence

<400>  443

Gln Ser Gly Arg Ser Ala Asn Ile
1               5


<210>  444
<211>  8
<212>  PRT
<213>  Artificial sequence

<220>
<223>  an artificially synthesized sequence

<400>  444

Gln Ser Gly Arg Ser Ala Asn Phe
1               5

<210>  445
<211>  8
<212>  PRT
<213>  Artificial sequence

<220>
<223>  an artificially synthesized sequence

<400>  445

Gln Ser Gly Arg Ser Ala Asn Ser
1               5


<210>  446
<211>  8
<212>  PRT
<213>  Artificial sequence

<220>
<223>  an artificially synthesized sequence

<400>  446

Pro Ser Gly Arg Ser Ala Val Pro
1               5


<210>  447
<211>  8
<212>  PRT
<213>  Artificial sequence

<220>
<223>  an artificially synthesized sequence

<400>  447

Pro Ser Gly Arg Ser Ala Tyr Pro
1               5


<210>  448
<211>  8
<212>  PRT
<213>  Artificial sequence

<220>
<223>  an artificially synthesized sequence

<400>  448

Pro Ser Gly Arg Ser Ala Asn Gln
1               5


<210>  449
<211>  8
<212>  PRT
<213>  Artificial sequence

<220>
<223>  an artificially synthesized sequence

<400>  449

Pro Ser Gly Arg Ser Ala Asn Ala
1               5


<210>  450
<211>  8
<212>  PRT
<213>  Artificial sequence

<220>
<223>  an artificially synthesized sequence

<400>  450

Pro Ser Gly Arg Ser Ala Asn Ile
1               5


<210>  451
<211>  8
<212>  PRT
<213>  Artificial sequence

<220>
<223>  an artificially synthesized sequence

<400>  451

Pro Ser Gly Arg Ser Ala Asn Phe
1               5


<210>  452
<211>  8
<212>  PRT
<213>  Artificial sequence

<220>
<223>  an artificially synthesized sequence

<400>  452

Pro Ser Gly Arg Ser Ala Asn Ser
1               5


<210>  453
<211>  8
<212>  PRT
<213>  Artificial sequence

<220>
<223>  an artificially synthesized sequence

<400>  453

Ala Ser Gly Arg Ser Ala Val Pro
1               5

<210>  454
<211>  8
<212>  PRT
<213>  Artificial sequence

<220>
<223>  an artificially synthesized sequence

<400>  454

Ala Ser Gly Arg Ser Ala Tyr Pro
1               5


<210>  455
<211>  8
<212>  PRT
<213>  Artificial sequence

<220>
<223>  an artificially synthesized sequence

<400>  455

Ala Ser Gly Arg Ser Ala Asn Gln
1               5


<210>  456
<211>  8
<212>  PRT
<213>  Artificial sequence

<220>
<223>  an artificially synthesized sequence

<400>  456

Ala Ser Gly Arg Ser Ala Asn Ala
1               5


<210>  457
<211>  8
<212>  PRT
<213>  Artificial sequence

<220>
<223>  an artificially synthesized sequence

<400>  457

Ala Ser Gly Arg Ser Ala Asn Ile
1               5


<210>  458
<211>  8
<212>  PRT
<213>  Artificial sequence

<220>
<223>  an artificially synthesized sequence

<400>  458

Ala Ser Gly Arg Ser Ala Asn Phe
1               5


<210>  459
<211>  8
<212>  PRT
<213>  Artificial sequence

<220>
<223>  an artificially synthesized sequence

<400>  459

Ala Ser Gly Arg Ser Ala Asn Ser
1               5


<210>  460
<211>  8
<212>  PRT
<213>  Artificial sequence

<220>
<223>  an artificially synthesized sequence

<400>  460

Tyr Ser Gly Arg Ser Glu Asn Pro
1               5


<210>  461
<211>  8
<212>  PRT
<213>  Artificial sequence

<220>
<223>  an artificially synthesized sequence

<400>  461

Gly Ser Gly Arg Ser Glu Asn Pro
1               5


<210>  462
<211>  8
<212>  PRT
<213>  Artificial sequence

<220>
<223>  an artificially synthesized sequence

<400>  462

Gln Ser Gly Arg Ser Glu Asn Pro
1               5

<210>  463
<211>  8
<212>  PRT
<213>  Artificial sequence

<220>
<223>  an artificially synthesized sequence

<400>  463

Pro Ser Gly Arg Ser Glu Asn Pro
1               5


<210>  464
<211>  8
<212>  PRT
<213>  Artificial sequence

<220>
<223>  an artificially synthesized sequence

<400>  464

Ala Ser Gly Arg Ser Glu Asn Pro
1               5


<210>  465
<211>  8
<212>  PRT
<213>  Artificial sequence

<220>
<223>  an artificially synthesized sequence

<400>  465

His Ser Gly Arg Ser Glu Asn Pro
1               5


<210>  466
<211>  8
<212>  PRT
<213>  Artificial sequence

<220>
<223>  an artificially synthesized sequence

<400>  466

Thr Ser Gly Arg Ser Glu Asn Gln
1               5


<210>  467
<211>  8
<212>  PRT
<213>  Artificial sequence

<220>
<223>  an artificially synthesized sequence

<400>  467

Thr Ser Gly Arg Ser Glu Asn Ala
1               5


<210>  468
<211>  8
<212>  PRT
<213>  Artificial sequence

<220>
<223>  an artificially synthesized sequence

<400>  468

Thr Ser Gly Arg Ser Glu Asn Ile
1               5


<210>  469
<211>  8
<212>  PRT
<213>  Artificial sequence

<220>
<223>  an artificially synthesized sequence

<400>  469

Thr Ser Gly Arg Ser Glu Asn Phe
1               5


<210>  470
<211>  8
<212>  PRT
<213>  Artificial sequence

<220>
<223>  an artificially synthesized sequence

<400>  470

Thr Ser Gly Arg Ser Glu Asn Ser
1               5


<210>  471
<211>  8
<212>  PRT
<213>  Artificial sequence

<220>
<223>  an artificially synthesized sequence

<400>  471

Tyr Ser Gly Arg Ser Ala Glu Pro
1               5

<210>  472
<211>  8
<212>  PRT
<213>  Artificial sequence

<220>
<223>  an artificially synthesized sequence

<400>  472

Gly Ser Gly Arg Ser Ala Glu Pro
1               5


<210>  473
<211>  8
<212>  PRT
<213>  Artificial sequence

<220>
<223>  an artificially synthesized sequence

<400>  473

Gln Ser Gly Arg Ser Ala Glu Pro
1               5


<210>  474
<211>  8
<212>  PRT
<213>  Artificial sequence

<220>
<223>  an artificially synthesized sequence

<400>  474

Pro Ser Gly Arg Ser Ala Glu Pro
1               5


<210>  475
<211>  8
<212>  PRT
<213>  Artificial sequence

<220>
<223>  an artificially synthesized sequence

<400>  475

Ala Ser Gly Arg Ser Ala Glu Pro
1               5


<210>  476
<211>  8
<212>  PRT
<213>  Artificial sequence

<220>
<223>  an artificially synthesized sequence

<400>  476

His Ser Gly Arg Ser Ala Glu Pro
1               5


<210>  477
<211>  8
<212>  PRT
<213>  Artificial sequence

<220>
<223>  an artificially synthesized sequence

<400>  477

Thr Ser Gly Arg Ser Ala Glu Gln
1               5


<210>  478
<211>  8
<212>  PRT
<213>  Artificial sequence

<220>
<223>  an artificially synthesized sequence

<400>  478

Thr Ser Gly Arg Ser Ala Glu Ala
1               5


<210>  479
<211>  8
<212>  PRT
<213>  Artificial sequence

<220>
<223>  an artificially synthesized sequence

<400>  479

Thr Ser Gly Arg Ser Ala Glu Ile
1               5


<210>  480
<211>  8
<212>  PRT
<213>  Artificial sequence

<220>
<223>  an artificially synthesized sequence

<400>  480

Thr Ser Gly Arg Ser Ala Glu Phe
1               5

<210>  481
<211>  8
<212>  PRT
<213>  Artificial sequence

<220>
<223>  an artificially synthesized sequence

<400>  481

Thr Ser Gly Arg Ser Ala Glu Ser
1               5


<210>  482
<211>  8
<212>  PRT
<213>  Artificial sequence

<220>
<223>  an artificially synthesized sequence

<400>  482

Gly Thr Gly Arg Ser Ala Asn Pro
1               5


<210>  483
<211>  8
<212>  PRT
<213>  Artificial sequence

<220>
<223>  an artificially synthesized sequence

<400>  483

Gly Lys Gly Arg Ser Ala Asn Pro
1               5


<210>  484
<211>  8
<212>  PRT
<213>  Artificial sequence

<220>
<223>  an artificially synthesized sequence

<400>  484

Gly Ser Gly Arg Ser Ala Ile Pro
1               5


<210>  485
<211>  8
<212>  PRT
<213>  Artificial sequence

<220>
<223>  an artificially synthesized sequence

<400>  485

Gly Ser Gly Arg Ser Ala Thr Pro
1               5


<210>  486
<211>  8
<212>  PRT
<213>  Artificial sequence

<220>
<223>  an artificially synthesized sequence

<400>  486

Gly Ser Gly Arg Ser Ala Ser Pro
1               5


<210>  487
<211>  8
<212>  PRT
<213>  Artificial sequence

<220>
<223>  an artificially synthesized sequence

<400>  487

Gly Ser Gly Arg Ser Ala His Pro
1               5


<210>  488
<211>  8
<212>  PRT
<213>  Artificial sequence

<220>
<223>  an artificially synthesized sequence

<400>  488

Gly Ser Gly Arg Ser Ala Asn Tyr
1               5


<210>  489
<211>  8
<212>  PRT
<213>  Artificial sequence

<220>
<223>  an artificially synthesized sequence

<400>  489

Gly Ser Gly Arg Ser Ala Asn Val
1               5

<210>  490
<211>  8
<212>  PRT
<213>  Artificial sequence

<220>
<223>  an artificially synthesized sequence

<400>  490

Gly Ser Gly Arg Ser Ala Asn His
1               5


<210>  491
<211>  8
<212>  PRT
<213>  Artificial sequence

<220>
<223>  an artificially synthesized sequence

<400>  491

Gln Thr Gly Arg Ser Ala Asn Pro
1               5


<210>  492
<211>  8
<212>  PRT
<213>  Artificial sequence

<220>
<223>  an artificially synthesized sequence

<400>  492

Gln Lys Gly Arg Ser Ala Asn Pro
1               5


<210>  493
<211>  8
<212>  PRT
<213>  Artificial sequence

<220>
<223>  an artificially synthesized sequence

<400>  493

Gln Ser Gly Arg Ser Ala Ile Pro
1               5


<210>  494
<211>  8
<212>  PRT
<213>  Artificial sequence

<220>
<223>  an artificially synthesized sequence

<400>  494

Gln Ser Gly Arg Ser Ala Thr Pro
1               5


<210>  495
<211>  8
<212>  PRT
<213>  Artificial sequence

<220>
<223>  an artificially synthesized sequence

<400>  495

Gln Ser Gly Arg Ser Ala Ser Pro
1               5


<210>  496
<211>  8
<212>  PRT
<213>  Artificial sequence

<220>
<223>  an artificially synthesized sequence

<400>  496

Gln Ser Gly Arg Ser Ala His Pro
1               5


<210>  497
<211>  8
<212>  PRT
<213>  Artificial sequence

<220>
<223>  an artificially synthesized sequence

<400>  497

Gln Ser Gly Arg Ser Ala Asn Tyr
1               5


<210>  498
<211>  8
<212>  PRT
<213>  Artificial sequence

<220>
<223>  an artificially synthesized sequence

<400>  498

Gln Ser Gly Arg Ser Ala Asn Val
1               5

<210>  499
<211>  8
<212>  PRT
<213>  Artificial sequence

<220>
<223>  an artificially synthesized sequence

<400>  499

Gln Ser Gly Arg Ser Ala Asn His
1               5


<210>  500
<211>  8
<212>  PRT
<213>  Artificial sequence

<220>
<223>  an artificially synthesized sequence

<400>  500

Pro Thr Gly Arg Ser Ala Asn Pro
1               5


<210>  501
<211>  8
<212>  PRT
<213>  Artificial sequence

<220>
<223>  an artificially synthesized sequence

<400>  501

Pro Lys Gly Arg Ser Ala Asn Pro
1               5


<210>  502
<211>  8
<212>  PRT
<213>  Artificial sequence

<220>
<223>  an artificially synthesized sequence

<400>  502

Pro Ser Gly Arg Ser Ala Ile Pro
1               5


<210>  503
<211>  8
<212>  PRT
<213>  Artificial sequence

<220>
<223>  an artificially synthesized sequence

<400>  503

Pro Ser Gly Arg Ser Ala Thr Pro
1               5


<210>  504
<211>  8
<212>  PRT
<213>  Artificial sequence

<220>
<223>  an artificially synthesized sequence

<400>  504

Pro Ser Gly Arg Ser Ala Ser Pro
1               5


<210>  505
<211>  8
<212>  PRT
<213>  Artificial sequence

<220>
<223>  an artificially synthesized sequence

<400>  505

Pro Ser Gly Arg Ser Ala His Pro
1               5


<210>  506
<211>  8
<212>  PRT
<213>  Artificial sequence

<220>
<223>  an artificially synthesized sequence

<400>  506

Pro Ser Gly Arg Ser Ala Asn Tyr
1               5


<210>  507
<211>  8
<212>  PRT
<213>  Artificial sequence

<220>
<223>  an artificially synthesized sequence

<400>  507

Pro Ser Gly Arg Ser Ala Asn Val
1               5

<210>  508
<211>  8
<212>  PRT
<213>  Artificial sequence

<220>
<223>  an artificially synthesized sequence

<400>  508

Pro Ser Gly Arg Ser Ala Asn His
1               5


<210>  509
<211>  8
<212>  PRT
<213>  Artificial sequence

<220>
<223>  an artificially synthesized sequence

<400>  509

Ala Thr Gly Arg Ser Ala Asn Pro
1               5


<210>  510
<211>  8
<212>  PRT
<213>  Artificial sequence

<220>
<223>  an artificially synthesized sequence

<400>  510

Ala Lys Gly Arg Ser Ala Asn Pro
1               5


<210>  511
<211>  8
<212>  PRT
<213>  Artificial sequence

<220>
<223>  an artificially synthesized sequence

<400>  511

Ala Ser Gly Arg Ser Ala Ile Pro
1               5


<210>  512
<211>  8
<212>  PRT
<213>  Artificial sequence

<220>
<223>  an artificially synthesized sequence

<400>  512

Ala Ser Gly Arg Ser Ala Thr Pro
1               5


<210>  513
<211>  8
<212>  PRT
<213>  Artificial sequence

<220>
<223>  an artificially synthesized sequence

<400>  513

Ala Ser Gly Arg Ser Ala Ser Pro
1               5


<210>  514
<211>  8
<212>  PRT
<213>  Artificial sequence

<220>
<223>  an artificially synthesized sequence

<400>  514

Ala Ser Gly Arg Ser Ala His Pro
1               5


<210>  515
<211>  8
<212>  PRT
<213>  Artificial sequence

<220>
<223>  an artificially synthesized sequence

<400>  515

Ala Ser Gly Arg Ser Ala Asn Tyr
1               5


<210>  516
<211>  8
<212>  PRT
<213>  Artificial sequence

<220>
<223>  an artificially synthesized sequence

<400>  516

Ala Ser Gly Arg Ser Ala Asn Val
1               5

<210>  517
<211>  8
<212>  PRT
<213>  Artificial sequence

<220>
<223>  an artificially synthesized sequence

<400>  517

Ala Ser Gly Arg Ser Ala Asn His
1               5


<210>  518
<211>  8
<212>  PRT
<213>  Artificial sequence

<220>
<223>  an artificially synthesized sequence

<400>  518

Tyr Thr Gly Arg Ser Ala Asn Pro
1               5


<210>  519
<211>  8
<212>  PRT
<213>  Artificial sequence

<220>
<223>  an artificially synthesized sequence

<400>  519

Tyr Lys Gly Arg Ser Ala Asn Pro
1               5


<210>  520
<211>  8
<212>  PRT
<213>  Artificial sequence

<220>
<223>  an artificially synthesized sequence

<400>  520

Tyr Ser Gly Arg Ser Ala Val Pro
1               5


<210>  521
<211>  8
<212>  PRT
<213>  Artificial sequence

<220>
<223>  an artificially synthesized sequence

<400>  521

Tyr Ser Gly Arg Ser Ala Ile Pro
1               5


<210>  522
<211>  8
<212>  PRT
<213>  Artificial sequence

<220>
<223>  an artificially synthesized sequence

<400>  522

Tyr Ser Gly Arg Ser Ala Thr Pro
1               5


<210>  523
<211>  8
<212>  PRT
<213>  Artificial sequence

<220>
<223>  an artificially synthesized sequence

<400>  523

Tyr Ser Gly Arg Ser Ala Ser Pro
1               5


<210>  524
<211>  8
<212>  PRT
<213>  Artificial sequence

<220>
<223>  an artificially synthesized sequence

<400>  524

Tyr Ser Gly Arg Ser Ala His Pro
1               5


<210>  525
<211>  8
<212>  PRT
<213>  Artificial sequence

<220>
<223>  an artificially synthesized sequence

<400>  525

Tyr Ser Gly Arg Ser Ala Asn Ala
1               5

<210>  526
<211>  8
<212>  PRT
<213>  Artificial sequence

<220>
<223>  an artificially synthesized sequence

<400>  526

Tyr Ser Gly Arg Ser Ala Asn Phe
1               5


<210>  527
<211>  8
<212>  PRT
<213>  Artificial sequence

<220>
<223>  an artificially synthesized sequence

<400>  527

Tyr Ser Gly Arg Ser Ala Asn Tyr
1               5


<210>  528
<211>  8
<212>  PRT
<213>  Artificial sequence

<220>
<223>  an artificially synthesized sequence

<400>  528

Tyr Ser Gly Arg Ser Ala Asn Val
1               5


<210>  529
<211>  8
<212>  PRT
<213>  Artificial sequence

<220>
<223>  an artificially synthesized sequence

<400>  529

Tyr Ser Gly Arg Ser Ala Asn His
1               5


<210>  530
<211>  8
<212>  PRT
<213>  Artificial sequence

<220>
<223>  an artificially synthesized sequence

<400>  530

Ser Thr Gly Arg Ser Ala Asn Pro
1               5


<210>  531
<211>  8
<212>  PRT
<213>  Artificial sequence

<220>
<223>  an artificially synthesized sequence

<400>  531

Ser Lys Gly Arg Ser Ala Asn Pro
1               5


<210>  532
<211>  8
<212>  PRT
<213>  Artificial sequence

<220>
<223>  an artificially synthesized sequence

<400>  532

Ser Ser Gly Arg Ser Ala Val Pro
1               5


<210>  533
<211>  8
<212>  PRT
<213>  Artificial sequence

<220>
<223>  an artificially synthesized sequence

<400>  533

Ser Ser Gly Arg Ser Ala Ile Pro
1               5


<210>  534
<211>  8
<212>  PRT
<213>  Artificial sequence

<220>
<223>  an artificially synthesized sequence

<400>  534

Ser Ser Gly Arg Ser Ala Thr Pro
1               5

<210>  535
<211>  8
<212>  PRT
<213>  Artificial sequence

<220>
<223>  an artificially synthesized sequence

<400>  535

Ser Ser Gly Arg Ser Ala Ser Pro
1               5


<210>  536
<211>  8
<212>  PRT
<213>  Artificial sequence

<220>
<223>  an artificially synthesized sequence

<400>  536

Ser Ser Gly Arg Ser Ala His Pro
1               5


<210>  537
<211>  8
<212>  PRT
<213>  Artificial sequence

<220>
<223>  an artificially synthesized sequence

<400>  537

Ser Ser Gly Arg Ser Ala Asn Ala
1               5


<210>  538
<211>  8
<212>  PRT
<213>  Artificial sequence

<220>
<223>  an artificially synthesized sequence

<400>  538

Ser Ser Gly Arg Ser Ala Asn Phe
1               5


<210>  539
<211>  8
<212>  PRT
<213>  Artificial sequence

<220>
<223>  an artificially synthesized sequence

<400>  539

Ser Ser Gly Arg Ser Ala Asn Tyr
1               5


<210>  540
<211>  8
<212>  PRT
<213>  Artificial sequence

<220>
<223>  an artificially synthesized sequence

<400>  540

Ser Ser Gly Arg Ser Ala Asn Val
1               5


<210>  541
<211>  8
<212>  PRT
<213>  Artificial sequence

<220>
<223>  an artificially synthesized sequence

<400>  541

Ser Ser Gly Arg Ser Ala Asn His
1               5


<210>  542
<211>  8
<212>  PRT
<213>  Artificial sequence

<220>
<223>  an artificially synthesized sequence

<400>  542

Ile Thr Gly Arg Ser Ala Asn Pro
1               5


<210>  543
<211>  8
<212>  PRT
<213>  Artificial sequence

<220>
<223>  an artificially synthesized sequence

<400>  543

Ile Lys Gly Arg Ser Ala Asn Pro
1               5

<210>  544
<211>  8
<212>  PRT
<213>  Artificial sequence

<220>
<223>  an artificially synthesized sequence

<400>  544

Ile Ser Gly Arg Ser Ala Val Pro
1               5


<210>  545
<211>  8
<212>  PRT
<213>  Artificial sequence

<220>
<223>  an artificially synthesized sequence

<400>  545

Ile Ser Gly Arg Ser Ala Ile Pro
1               5


<210>  546
<211>  8
<212>  PRT
<213>  Artificial sequence

<220>
<223>  an artificially synthesized sequence

<400>  546

Ile Ser Gly Arg Ser Ala Thr Pro
1               5


<210>  547
<211>  8
<212>  PRT
<213>  Artificial sequence

<220>
<223>  an artificially synthesized sequence

<400>  547

Ile Ser Gly Arg Ser Ala Ser Pro
1               5


<210>  548
<211>  8
<212>  PRT
<213>  Artificial sequence

<220>
<223>  an artificially synthesized sequence

<400>  548

Ile Ser Gly Arg Ser Ala His Pro
1               5


<210>  549
<211>  8
<212>  PRT
<213>  Artificial sequence

<220>
<223>  an artificially synthesized sequence

<400>  549

Ile Ser Gly Arg Ser Ala Asn Ala
1               5


<210>  550
<211>  8
<212>  PRT
<213>  Artificial sequence

<220>
<223>  an artificially synthesized sequence

<400>  550

Ile Ser Gly Arg Ser Ala Asn Phe
1               5


<210>  551
<211>  8
<212>  PRT
<213>  Artificial sequence

<220>
<223>  an artificially synthesized sequence

<400>  551

Ile Ser Gly Arg Ser Ala Asn Tyr
1               5


<210>  552
<211>  8
<212>  PRT
<213>  Artificial sequence

<220>
<223>  an artificially synthesized sequence

<400>  552

Ile Ser Gly Arg Ser Ala Asn Val
1               5

<210>  553
<211>  8
<212>  PRT
<213>  Artificial sequence

<220>
<223>  an artificially synthesized sequence

<400>  553

Ile Ser Gly Arg Ser Ala Asn His
1               5


<210>  554
<211>  8
<212>  PRT
<213>  Artificial sequence

<220>
<223>  an artificially synthesized sequence

<400>  554

Thr Thr Gly Arg Ser Ala Val Pro
1               5


<210>  555
<211>  8
<212>  PRT
<213>  Artificial sequence

<220>
<223>  an artificially synthesized sequence

<400>  555

Thr Thr Gly Arg Ser Ala Ile Pro
1               5


<210>  556
<211>  8
<212>  PRT
<213>  Artificial sequence

<220>
<223>  an artificially synthesized sequence

<400>  556

Thr Thr Gly Arg Ser Ala Thr Pro
1               5


<210>  557
<211>  8
<212>  PRT
<213>  Artificial sequence

<220>
<223>  an artificially synthesized sequence

<400>  557

Thr Thr Gly Arg Ser Ala Ser Pro
1               5


<210>  558
<211>  8
<212>  PRT
<213>  Artificial sequence

<220>
<223>  an artificially synthesized sequence

<400>  558

Thr Thr Gly Arg Ser Ala His Pro
1               5


<210>  559
<211>  8
<212>  PRT
<213>  Artificial sequence

<220>
<223>  an artificially synthesized sequence

<400>  559

Thr Thr Gly Arg Ser Ala Asn Ala
1               5


<210>  560
<211>  8
<212>  PRT
<213>  Artificial sequence

<220>
<223>  an artificially synthesized sequence

<400>  560

Thr Thr Gly Arg Ser Ala Asn Phe
1               5


<210>  561
<211>  8
<212>  PRT
<213>  Artificial sequence

<220>
<223>  an artificially synthesized sequence

<400>  561

Thr Thr Gly Arg Ser Ala Asn Tyr
1               5

<210>  562
<211>  8
<212>  PRT
<213>  Artificial sequence

<220>
<223>  an artificially synthesized sequence

<400>  562

Thr Thr Gly Arg Ser Ala Asn Val
1               5


<210>  563
<211>  8
<212>  PRT
<213>  Artificial sequence

<220>
<223>  an artificially synthesized sequence

<400>  563

Thr Thr Gly Arg Ser Ala Asn His
1               5


<210>  564
<211>  8
<212>  PRT
<213>  Artificial sequence

<220>
<223>  an artificially synthesized sequence

<400>  564

Thr Lys Gly Arg Ser Ala Val Pro
1               5


<210>  565
<211>  8
<212>  PRT
<213>  Artificial sequence

<220>
<223>  an artificially synthesized sequence

<400>  565

Thr Lys Gly Arg Ser Ala Ile Pro
1               5


<210>  566
<211>  8
<212>  PRT
<213>  Artificial sequence

<220>
<223>  an artificially synthesized sequence

<400>  566

Thr Lys Gly Arg Ser Ala Thr Pro
1               5


<210>  567
<211>  8
<212>  PRT
<213>  Artificial sequence

<220>
<223>  an artificially synthesized sequence

<400>  567

Thr Lys Gly Arg Ser Ala Ser Pro
1               5


<210>  568
<211>  8
<212>  PRT
<213>  Artificial sequence

<220>
<223>  an artificially synthesized sequence

<400>  568

Thr Lys Gly Arg Ser Ala His Pro
1               5


<210>  569
<211>  8
<212>  PRT
<213>  Artificial sequence

<220>
<223>  an artificially synthesized sequence

<400>  569

Thr Lys Gly Arg Ser Ala Asn Ala
1               5


<210>  570
<211>  8
<212>  PRT
<213>  Artificial sequence

<220>
<223>  an artificially synthesized sequence

<400>  570

Thr Lys Gly Arg Ser Ala Asn Phe
1               5

<210>  571
<211>  8
<212>  PRT
<213>  Artificial sequence

<220>
<223>  an artificially synthesized sequence

<400>  571

Thr Lys Gly Arg Ser Ala Asn Tyr
1               5


<210>  572
<211>  8
<212>  PRT
<213>  Artificial sequence

<220>
<223>  an artificially synthesized sequence

<400>  572

Thr Lys Gly Arg Ser Ala Asn Val
1               5


<210>  573
<211>  8
<212>  PRT
<213>  Artificial sequence

<220>
<223>  an artificially synthesized sequence

<400>  573

Thr Lys Gly Arg Ser Ala Asn His
1               5


<210>  574
<211>  8
<212>  PRT
<213>  Artificial sequence

<220>
<223>  an artificially synthesized sequence

<400>  574

Thr Ser Gly Arg Ser Ala Val Tyr
1               5


<210>  575
<211>  8
<212>  PRT
<213>  Artificial sequence

<220>
<223>  an artificially synthesized sequence

<400>  575

Thr Ser Gly Arg Ser Ala Val Val
1               5


<210>  576
<211>  8
<212>  PRT
<213>  Artificial sequence

<220>
<223>  an artificially synthesized sequence

<400>  576

Thr Ser Gly Arg Ser Ala Val His
1               5


<210>  577
<211>  8
<212>  PRT
<213>  Artificial sequence

<220>
<223>  an artificially synthesized sequence

<400>  577

Thr Ser Gly Arg Ser Ala Ile Tyr
1               5


<210>  578
<211>  8
<212>  PRT
<213>  Artificial sequence

<220>
<223>  an artificially synthesized sequence

<400>  578

Thr Ser Gly Arg Ser Ala Ile Val
1               5


<210>  579
<211>  8
<212>  PRT
<213>  Artificial sequence

<220>
<223>  an artificially synthesized sequence

<400>  579

Thr Ser Gly Arg Ser Ala Ile His
1               5

<210>  580
<211>  8
<212>  PRT
<213>  Artificial sequence

<220>
<223>  an artificially synthesized sequence

<400>  580

Thr Ser Gly Arg Ser Ala Ser Tyr
1               5


<210>  581
<211>  8
<212>  PRT
<213>  Artificial sequence

<220>
<223>  an artificially synthesized sequence

<400>  581

Thr Ser Gly Arg Ser Ala Ser Val
1               5


<210>  582
<211>  8
<212>  PRT
<213>  Artificial sequence

<220>
<223>  an artificially synthesized sequence

<400>  582

Thr Ser Gly Arg Ser Ala Ser His
1               5


<210>  583
<211>  8
<212>  PRT
<213>  Artificial sequence

<220>
<223>  an artificially synthesized sequence

<400>  583

Thr Ser Gly Arg Ser Ala His Tyr
1               5


<210>  584
<211>  8
<212>  PRT
<213>  Artificial sequence

<220>
<223>  an artificially synthesized sequence

<400>  584

Thr Ser Gly Arg Ser Ala His Val
1               5


<210>  585
<211>  8
<212>  PRT
<213>  Artificial sequence

<220>
<223>  an artificially synthesized sequence

<400>  585

Thr Ser Gly Arg Ser Ala His His
1               5


<210>  586
<211>  8
<212>  PRT
<213>  Artificial sequence

<220>
<223>  an artificially synthesized sequence

<400>  586

Pro Ser Gly Arg Ser Glu Val Pro
1               5


<210>  587
<211>  8
<212>  PRT
<213>  Artificial sequence

<220>
<223>  an artificially synthesized sequence

<400>  587

Pro Ser Gly Arg Ser Ala Glu Pro
1               5


<210>  588
<211>  8
<212>  PRT
<213>  Artificial sequence

<220>
<223>  an artificially synthesized sequence

<400>  588

Pro Ser Gly Arg Ser Ala Gly Pro
1               5

<210>  589
<211>  8
<212>  PRT
<213>  Artificial sequence

<220>
<223>  an artificially synthesized sequence

<400>  589

Ala Ser Gly Arg Ser Glu Asn Ala
1               5


<210>  590
<211>  8
<212>  PRT
<213>  Artificial sequence

<220>
<223>  an artificially synthesized sequence

<400>  590

Ala Ser Gly Arg Ser Ala Glu Ala
1               5


<210>  591
<211>  8
<212>  PRT
<213>  Artificial sequence

<220>
<223>  an artificially synthesized sequence

<400>  591

Ala Ser Gly Arg Ser Ala Gly Ala
1               5


<210>  592
<211>  8
<212>  PRT
<213>  Artificial sequence

<220>
<223>  an artificially synthesized sequence

<400>  592

Gly Thr Gly Arg Ser Ala Thr Pro
1               5


<210>  593
<211>  8
<212>  PRT
<213>  Artificial sequence

<220>
<223>  an artificially synthesized sequence

<400>  593

Gly Ser Gly Arg Ser Ala Thr Tyr
1               5


<210>  594
<211>  8
<212>  PRT
<213>  Artificial sequence

<220>
<223>  an artificially synthesized sequence

<400>  594

Gly Ser Gly Arg Ser Ala Thr Val
1               5


<210>  595
<211>  8
<212>  PRT
<213>  Artificial sequence

<220>
<223>  an artificially synthesized sequence

<400>  595

Gly Ser Gly Arg Ser Ala Thr His
1               5


<210>  596
<211>  8
<212>  PRT
<213>  Artificial sequence

<220>
<223>  an artificially synthesized sequence

<400>  596

Gly Thr Gly Arg Ser Ala Thr Tyr
1               5


<210>  597
<211>  8
<212>  PRT
<213>  Artificial sequence

<220>
<223>  an artificially synthesized sequence

<400>  597

Gly Thr Gly Arg Ser Ala Thr Val
1               5

<210>  598
<211>  8
<212>  PRT
<213>  Artificial sequence

<220>
<223>  an artificially synthesized sequence

<400>  598

Gly Thr Gly Arg Ser Ala Thr His
1               5


<210>  599
<211>  8
<212>  PRT
<213>  Artificial sequence

<220>
<223>  an artificially synthesized sequence

<400>  599

Gly Ser Gly Arg Ser Glu Thr Pro
1               5


<210>  600
<211>  8
<212>  PRT
<213>  Artificial sequence

<220>
<223>  an artificially synthesized sequence

<400>  600

Gly Thr Gly Arg Ser Glu Thr Pro
1               5


<210>  601
<211>  8
<212>  PRT
<213>  Artificial sequence

<220>
<223>  an artificially synthesized sequence

<400>  601

Gly Ser Gly Arg Ser Glu Thr Tyr
1               5


<210>  602
<211>  8
<212>  PRT
<213>  Artificial sequence

<220>
<223>  an artificially synthesized sequence

<400>  602

Gly Ser Gly Arg Ser Glu Thr Val
1               5


<210>  603
<211>  8
<212>  PRT
<213>  Artificial sequence

<220>
<223>  an artificially synthesized sequence

<400>  603

Gly Ser Gly Arg Ser Glu Thr His
1               5


<210>  604
<211>  8
<212>  PRT
<213>  Artificial sequence

<220>
<223>  an artificially synthesized sequence

<400>  604

Tyr Thr Gly Arg Ser Ala Val Pro
1               5


<210>  605
<211>  8
<212>  PRT
<213>  Artificial sequence

<220>
<223>  an artificially synthesized sequence

<400>  605

Tyr Ser Gly Arg Ser Ala Val Tyr
1               5


<210>  606
<211>  8
<212>  PRT
<213>  Artificial sequence

<220>
<223>  an artificially synthesized sequence

<400>  606

Tyr Ser Gly Arg Ser Ala Val Val
1               5

<210>  607
<211>  8
<212>  PRT
<213>  Artificial sequence

<220>
<223>  an artificially synthesized sequence

<400>  607

Tyr Ser Gly Arg Ser Ala Val His
1               5


<210>  608
<211>  8
<212>  PRT
<213>  Artificial sequence

<220>
<223>  an artificially synthesized sequence

<400>  608

Tyr Thr Gly Arg Ser Ala Val Tyr
1               5


<210>  609
<211>  8
<212>  PRT
<213>  Artificial sequence

<220>
<223>  an artificially synthesized sequence

<400>  609

Tyr Thr Gly Arg Ser Ala Val Val
1               5


<210>  610
<211>  8
<212>  PRT
<213>  Artificial sequence

<220>
<223>  an artificially synthesized sequence

<400>  610

Tyr Thr Gly Arg Ser Ala Val His
1               5


<210>  611
<211>  8
<212>  PRT
<213>  Artificial sequence

<220>
<223>  an artificially synthesized sequence

<400>  611

Tyr Ser Gly Arg Ser Glu Val Pro
1               5


<210>  612
<211>  8
<212>  PRT
<213>  Artificial sequence

<220>
<223>  an artificially synthesized sequence

<400>  612

Tyr Thr Gly Arg Ser Glu Val Pro
1               5


<210>  613
<211>  8
<212>  PRT
<213>  Artificial sequence

<220>
<223>  an artificially synthesized sequence

<400>  613

Tyr Ser Gly Arg Ser Glu Val Tyr
1               5


<210>  614
<211>  8
<212>  PRT
<213>  Artificial sequence

<220>
<223>  an artificially synthesized sequence

<400>  614

Tyr Ser Gly Arg Ser Glu Val Val
1               5


<210>  615
<211>  8
<212>  PRT
<213>  Artificial sequence

<220>
<223>  an artificially synthesized sequence

<400>  615

Tyr Ser Gly Arg Ser Glu Val His
1               5

<210>  616
<211>  8
<212>  PRT
<213>  Artificial sequence

<220>
<223>  an artificially synthesized sequence

<400>  616

Tyr Thr Gly Arg Ser Ala Val Pro
1               5


<210>  617
<211>  8
<212>  PRT
<213>  Artificial sequence

<220>
<223>  an artificially synthesized sequence

<400>  617

Tyr Ser Gly Arg Ser Ala Val Tyr
1               5


<210>  618
<211>  8
<212>  PRT
<213>  Artificial sequence

<220>
<223>  an artificially synthesized sequence

<400>  618

Tyr Ser Gly Arg Ser Ala Val Val
1               5


<210>  619
<211>  8
<212>  PRT
<213>  Artificial sequence

<220>
<223>  an artificially synthesized sequence

<400>  619

Tyr Ser Gly Arg Ser Ala Val His
1               5


<210>  620
<211>  8
<212>  PRT
<213>  Artificial sequence

<220>
<223>  an artificially synthesized sequence

<400>  620

Tyr Thr Gly Arg Ser Ala Val Tyr
1               5


<210>  621
<211>  8
<212>  PRT
<213>  Artificial sequence

<220>
<223>  an artificially synthesized sequence

<400>  621

Tyr Thr Gly Arg Ser Ala Val Val
1               5


<210>  622
<211>  8
<212>  PRT
<213>  Artificial sequence

<220>
<223>  an artificially synthesized sequence

<400>  622

Tyr Thr Gly Arg Ser Ala Val His
1               5


<210>  623
<211>  12
<212>  PRT
<213>  Artificial sequence

<220>
<223>  an artificially synthesized sequence

<400>  623

Thr Ser Thr Ser Gly Arg Ser Ala Asn Pro Arg Gly
1               5                   10


<210>  624
<211>  12
<212>  PRT
<213>  Artificial sequence

<220>
<223>  an artificially synthesized sequence

<400>  624

Thr Ser Thr Ser Gly Arg Ser Ala Asn Pro Ala Gly
1               5                   10

<210>  625
<211>  12
<212>  PRT
<213>  Artificial sequence

<220>
<223>  an artificially synthesized sequence

<400>  625

Thr Ser Thr Ser Gly Arg Ser Ala Asn Pro His Gly
1               5                   10


<210>  626
<211>  12
<212>  PRT
<213>  Artificial sequence

<220>
<223>  an artificially synthesized sequence

<400>  626

Thr Ser Thr Ser Gly Arg Ser Ala Asn Pro Ile Gly
1               5                   10


<210>  627
<211>  12
<212>  PRT
<213>  Artificial sequence

<220>
<223>  an artificially synthesized sequence

<400>  627

Thr Ser Thr Ser Gly Arg Ser Ala Asn Pro Leu Gly
1               5                   10


<210>  628
<211>  12
<212>  PRT
<213>  Artificial sequence

<220>
<223>  an artificially synthesized sequence

<400>  628

Thr Ser Thr Ser Gly Arg Ser Ala Asn Pro Ser Gly
1               5                   10


<210>  629
<211>  12
<212>  PRT
<213>  Artificial sequence

<220>
<223>  an artificially synthesized sequence

<400>  629

Ile Ser Thr Ser Gly Arg Ser Ala Asn Pro Ile Gly
1               5                   10


<210>  630
<211>  12
<212>  PRT
<213>  Artificial sequence

<220>
<223>  an artificially synthesized sequence

<400>  630

Tyr Ser Thr Ser Gly Arg Ser Ala Asn Pro Ile Gly
1               5                   10


<210>  631
<211>  12
<212>  PRT
<213>  Artificial sequence

<220>
<223>  an artificially synthesized sequence

<400>  631

Thr Ser Tyr Ser Gly Arg Ser Ala Val Pro Ala Gly
1               5                   10


<210>  632
<211>  12
<212>  PRT
<213>  Artificial sequence

<220>
<223>  an artificially synthesized sequence

<400>  632

Thr Ser Pro Ser Gly Arg Ser Ala Asn Ile Ala Gly
1               5                   10


<210>  633
<211>  12
<212>  PRT
<213>  Artificial sequence

<220>
<223>  an artificially synthesized sequence

<400>  633

Thr Ser Pro Ser Gly Arg Ser Ala Asn Phe Ala Gly
1               5                   10

<210>  634
<211>  12
<212>  PRT
<213>  Artificial sequence

<220>
<223>  an artificially synthesized sequence

<400>  634

Thr Ser Pro Thr Gly Arg Ser Ala Asn Pro Ala Gly
1               5                   10


<210>  635
<211>  12
<212>  PRT
<213>  Artificial sequence

<220>
<223>  an artificially synthesized sequence

<400>  635

Thr Ser Pro Ser Gly Arg Ser Ala Ile Pro Ala Gly
1               5                   10


<210>  636
<211>  12
<212>  PRT
<213>  Artificial sequence

<220>
<223>  an artificially synthesized sequence

<400>  636

Thr Ser Tyr Thr Gly Arg Ser Ala Asn Pro Ala Gly
1               5                   10


<210>  637
<211>  12
<212>  PRT
<213>  Artificial sequence

<220>
<223>  an artificially synthesized sequence

<400>  637

Thr Ser Tyr Ser Gly Arg Ser Ala Ile Pro Ala Gly
1               5                   10


<210>  638
<211>  12
<212>  PRT
<213>  Artificial sequence

<220>
<223>  an artificially synthesized sequence

<400>  638

Thr Ser Ile Ser Gly Arg Ser Ala Asn Tyr Ala Gly
1               5                   10


<210>  639
<211>  12
<212>  PRT
<213>  Artificial sequence

<220>
<223>  an artificially synthesized sequence

<400>  639

Thr Ser Pro Ser Gly Arg Ser Ala Gly Pro Ala Gly
1               5                   10


<210>  640
<211>  12
<212>  PRT
<213>  Artificial sequence

<220>
<223>  an artificially synthesized sequence

<400>  640

Thr Ser Tyr Thr Gly Arg Ser Ala Val Pro Ala Gly
1               5                   10


<210>  641
<211>  12
<212>  PRT
<213>  Artificial sequence

<220>
<223>  an artificially synthesized sequence

<400>  641

Thr Ser Tyr Thr Gly Arg Ser Ala Val Tyr Ala Gly
1               5                   10


<210>  642
<211>  12
<212>  PRT
<213>  Artificial sequence

<220>
<223>  an artificially synthesized sequence

<400>  642

Thr Ser Tyr Thr Gly Arg Ser Ala Val Val Ala Gly
1               5                   10

<210>  643
<211>  12
<212>  PRT
<213>  Artificial sequence

<220>
<223>  an artificially synthesized sequence

<400>  643

Thr Ser Tyr Thr Gly Arg Ser Ala Val His Ala Gly
1               5                   10


<210>  644
<211>  12
<212>  PRT
<213>  Artificial sequence

<220>
<223>  an artificially synthesized sequence

<400>  644

Thr Ser Tyr Ser Gly Arg Ser Ala Val Pro His Gly
1               5                   10


<210>  645
<211>  12
<212>  PRT
<213>  Artificial sequence

<220>
<223>  an artificially synthesized sequence

<400>  645

Thr Ser Pro Ser Gly Arg Ser Ala Asn Ile His Gly
1               5                   10


<210>  646
<211>  12
<212>  PRT
<213>  Artificial sequence

<220>
<223>  an artificially synthesized sequence

<400>  646

Thr Ser Pro Ser Gly Arg Ser Ala Asn Phe His Gly
1               5                   10


<210>  647
<211>  12
<212>  PRT
<213>  Artificial sequence

<220>
<223>  an artificially synthesized sequence

<400>  647

Thr Ser Pro Thr Gly Arg Ser Ala Asn Pro His Gly
1               5                   10


<210>  648
<211>  12
<212>  PRT
<213>  Artificial sequence

<220>
<223>  an artificially synthesized sequence

<400>  648

Thr Ser Pro Ser Gly Arg Ser Ala Ile Pro His Gly
1               5                   10


<210>  649
<211>  12
<212>  PRT
<213>  Artificial sequence

<220>
<223>  an artificially synthesized sequence

<400>  649

Thr Ser Tyr Thr Gly Arg Ser Ala Asn Pro His Gly
1               5                   10


<210>  650
<211>  12
<212>  PRT
<213>  Artificial sequence

<220>
<223>  an artificially synthesized sequence

<400>  650

Thr Ser Tyr Ser Gly Arg Ser Ala Ile Pro His Gly
1               5                   10


<210>  651
<211>  12
<212>  PRT
<213>  Artificial sequence

<220>
<223>  an artificially synthesized sequence

<400>  651

Thr Ser Ile Ser Gly Arg Ser Ala Asn Tyr His Gly
1               5                   10

<210>  652
<211>  12
<212>  PRT
<213>  Artificial sequence

<220>
<223>  an artificially synthesized sequence

<400>  652

Thr Ser Pro Ser Gly Arg Ser Ala Gly Pro His Gly
1               5                   10


<210>  653
<211>  12
<212>  PRT
<213>  Artificial sequence

<220>
<223>  an artificially synthesized sequence

<400>  653

Thr Ser Tyr Thr Gly Arg Ser Ala Val Pro His Gly
1               5                   10


<210>  654
<211>  12
<212>  PRT
<213>  Artificial sequence

<220>
<223>  an artificially synthesized sequence

<400>  654

Thr Ser Tyr Thr Gly Arg Ser Ala Val Tyr His Gly
1               5                   10


<210>  655
<211>  12
<212>  PRT
<213>  Artificial sequence

<220>
<223>  an artificially synthesized sequence

<400>  655

Thr Ser Tyr Thr Gly Arg Ser Ala Val Val His Gly
1               5                   10


<210>  656
<211>  12
<212>  PRT
<213>  Artificial sequence

<220>
<223>  an artificially synthesized sequence

<400>  656

Thr Ser Tyr Thr Gly Arg Ser Ala Val His His Gly
1               5                   10


<210>  657
<211>  12
<212>  PRT
<213>  Artificial sequence

<220>
<223>  an artificially synthesized sequence

<400>  657

Thr Ser Tyr Ser Gly Arg Ser Ala Val Pro Ile Gly
1               5                   10


<210>  658
<211>  12
<212>  PRT
<213>  Artificial sequence

<220>
<223>  an artificially synthesized sequence

<400>  658

Thr Ser Pro Ser Gly Arg Ser Ala Asn Ile Ile Gly
1               5                   10


<210>  659
<211>  12
<212>  PRT
<213>  Artificial sequence

<220>
<223>  an artificially synthesized sequence

<400>  659

Thr Ser Pro Ser Gly Arg Ser Ala Asn Phe Ile Gly
1               5                   10


<210>  660
<211>  12
<212>  PRT
<213>  Artificial sequence

<220>
<223>  an artificially synthesized sequence

<400>  660

Thr Ser Pro Thr Gly Arg Ser Ala Asn Pro Ile Gly
1               5                   10

<210>  661
<211>  12
<212>  PRT
<213>  Artificial sequence

<220>
<223>  an artificially synthesized sequence

<400>  661

Thr Ser Pro Ser Gly Arg Ser Ala Ile Pro Ile Gly
1               5                   10


<210>  662
<211>  12
<212>  PRT
<213>  Artificial sequence

<220>
<223>  an artificially synthesized sequence

<400>  662

Thr Ser Tyr Thr Gly Arg Ser Ala Asn Pro Ile Gly
1               5                   10


<210>  663
<211>  12
<212>  PRT
<213>  Artificial sequence

<220>
<223>  an artificially synthesized sequence

<400>  663

Thr Ser Tyr Ser Gly Arg Ser Ala Ile Pro Ile Gly
1               5                   10


<210>  664
<211>  12
<212>  PRT
<213>  Artificial sequence

<220>
<223>  an artificially synthesized sequence

<400>  664

Thr Ser Ile Ser Gly Arg Ser Ala Asn Tyr Ile Gly
1               5                   10


<210>  665
<211>  12
<212>  PRT
<213>  Artificial sequence

<220>
<223>  an artificially synthesized sequence

<400>  665

Thr Ser Pro Ser Gly Arg Ser Ala Gly Pro Ile Gly
1               5                   10


<210>  666
<211>  12
<212>  PRT
<213>  Artificial sequence

<220>
<223>  an artificially synthesized sequence

<400>  666

Thr Ser Tyr Thr Gly Arg Ser Ala Val Pro Ile Gly
1               5                   10


<210>  667
<211>  12
<212>  PRT
<213>  Artificial sequence

<220>
<223>  an artificially synthesized sequence

<400>  667

Thr Ser Tyr Thr Gly Arg Ser Ala Val Tyr Ile Gly
1               5                   10


<210>  668
<211>  12
<212>  PRT
<213>  Artificial sequence

<220>
<223>  an artificially synthesized sequence

<400>  668

Thr Ser Tyr Thr Gly Arg Ser Ala Val Val Ile Gly
1               5                   10


<210>  669
<211>  12
<212>  PRT
<213>  Artificial sequence

<220>
<223>  an artificially synthesized sequence

<400>  669

Thr Ser Tyr Thr Gly Arg Ser Ala Val His Ile Gly
1               5                   10

<210>  670
<211>  12
<212>  PRT
<213>  Artificial sequence

<220>
<223>  an artificially synthesized sequence

<400>  670

Thr Ser Tyr Ser Gly Arg Ser Ala Val Pro Leu Gly
1               5                   10


<210>  671
<211>  12
<212>  PRT
<213>  Artificial sequence

<220>
<223>  an artificially synthesized sequence

<400>  671

Thr Ser Pro Ser Gly Arg Ser Ala Asn Ile Leu Gly
1               5                   10


<210>  672
<211>  12
<212>  PRT
<213>  Artificial sequence

<220>
<223>  an artificially synthesized sequence

<400>  672

Thr Ser Pro Ser Gly Arg Ser Ala Asn Phe Leu Gly
1               5                   10


<210>  673
<211>  12
<212>  PRT
<213>  Artificial sequence

<220>
<223>  an artificially synthesized sequence

<400>  673

Thr Ser Pro Thr Gly Arg Ser Ala Asn Pro Leu Gly
1               5                   10


<210>  674
<211>  12
<212>  PRT
<213>  Artificial sequence

<220>
<223>  an artificially synthesized sequence

<400>  674

Thr Ser Pro Ser Gly Arg Ser Ala Ile Pro Leu Gly
1               5                   10


<210>  675
<211>  12
<212>  PRT
<213>  Artificial sequence

<220>
<223>  an artificially synthesized sequence

<400>  675

Thr Ser Tyr Thr Gly Arg Ser Ala Asn Pro Leu Gly
1               5                   10


<210>  676
<211>  12
<212>  PRT
<213>  Artificial sequence

<220>
<223>  an artificially synthesized sequence

<400>  676

Thr Ser Tyr Ser Gly Arg Ser Ala Ile Pro Leu Gly
1               5                   10


<210>  677
<211>  12
<212>  PRT
<213>  Artificial sequence

<220>
<223>  an artificially synthesized sequence

<400>  677

Thr Ser Ile Ser Gly Arg Ser Ala Asn Tyr Leu Gly
1               5                   10


<210>  678
<211>  12
<212>  PRT
<213>  Artificial sequence

<220>
<223>  an artificially synthesized sequence

<400>  678

Thr Ser Pro Ser Gly Arg Ser Ala Gly Pro Leu Gly
1               5                   10

<210>  679
<211>  12
<212>  PRT
<213>  Artificial sequence

<220>
<223>  an artificially synthesized sequence

<400>  679

Thr Ser Tyr Thr Gly Arg Ser Ala Val Pro Leu Gly
1               5                   10


<210>  680
<211>  12
<212>  PRT
<213>  Artificial sequence

<220>
<223>  an artificially synthesized sequence

<400>  680

Thr Ser Tyr Thr Gly Arg Ser Ala Val Tyr Leu Gly
1               5                   10


<210>  681
<211>  12
<212>  PRT
<213>  Artificial sequence

<220>
<223>  an artificially synthesized sequence

<400>  681

Thr Ser Tyr Thr Gly Arg Ser Ala Val Val Leu Gly
1               5                   10


<210>  682
<211>  12
<212>  PRT
<213>  Artificial sequence

<220>
<223>  an artificially synthesized sequence

<400>  682

Thr Ser Tyr Thr Gly Arg Ser Ala Val His Leu Gly
1               5                   10


<210>  683
<211>  12
<212>  PRT
<213>  Artificial sequence

<220>
<223>  an artificially synthesized sequence

<400>  683

Thr Ser Tyr Ser Gly Arg Ser Ala Val Pro Ser Gly
1               5                   10


<210>  684
<211>  12
<212>  PRT
<213>  Artificial sequence

<220>
<223>  an artificially synthesized sequence

<400>  684

Thr Ser Pro Ser Gly Arg Ser Ala Asn Ile Ser Gly
1               5                   10


<210>  685
<211>  12
<212>  PRT
<213>  Artificial sequence

<220>
<223>  an artificially synthesized sequence

<400>  685

Thr Ser Pro Ser Gly Arg Ser Ala Asn Phe Ser Gly
1               5                   10


<210>  686
<211>  12
<212>  PRT
<213>  Artificial sequence

<220>
<223>  an artificially synthesized sequence

<400>  686

Thr Ser Pro Thr Gly Arg Ser Ala Asn Pro Ser Gly
1               5                   10


<210>  687
<211>  12
<212>  PRT
<213>  Artificial sequence

<220>
<223>  an artificially synthesized sequence

<400>  687

Thr Ser Pro Ser Gly Arg Ser Ala Ile Pro Ser Gly
1               5                   10

<210>  688
<211>  12
<212>  PRT
<213>  Artificial sequence

<220>
<223>  an artificially synthesized sequence

<400>  688

Thr Ser Tyr Thr Gly Arg Ser Ala Asn Pro Ser Gly
1               5                   10


<210>  689
<211>  12
<212>  PRT
<213>  Artificial sequence

<220>
<223>  an artificially synthesized sequence

<400>  689

Thr Ser Tyr Ser Gly Arg Ser Ala Ile Pro Ser Gly
1               5                   10


<210>  690
<211>  12
<212>  PRT
<213>  Artificial sequence

<220>
<223>  an artificially synthesized sequence

<400>  690

Thr Ser Ile Ser Gly Arg Ser Ala Asn Tyr Ser Gly
1               5                   10


<210>  691
<211>  12
<212>  PRT
<213>  Artificial sequence

<220>
<223>  an artificially synthesized sequence

<400>  691

Thr Ser Pro Ser Gly Arg Ser Ala Gly Pro Ser Gly
1               5                   10


<210>  692
<211>  12
<212>  PRT
<213>  Artificial sequence

<220>
<223>  an artificially synthesized sequence

<400>  692

Thr Ser Tyr Thr Gly Arg Ser Ala Val Pro Ser Gly
1               5                   10


<210>  693
<211>  12
<212>  PRT
<213>  Artificial sequence

<220>
<223>  an artificially synthesized sequence

<400>  693

Thr Ser Tyr Thr Gly Arg Ser Ala Val Tyr Ser Gly
1               5                   10


<210>  694
<211>  12
<212>  PRT
<213>  Artificial sequence

<220>
<223>  an artificially synthesized sequence

<400>  694

Thr Ser Tyr Thr Gly Arg Ser Ala Val Val Ser Gly
1               5                   10


<210>  695
<211>  12
<212>  PRT
<213>  Artificial sequence

<220>
<223>  an artificially synthesized sequence

<400>  695

Thr Ser Tyr Thr Gly Arg Ser Ala Val His Ser Gly
1               5                   10


<210>  696
<211>  12
<212>  PRT
<213>  Artificial sequence

<220>
<223>  an artificially synthesized sequence

<400>  696

Ile Ser Tyr Ser Gly Arg Ser Ala Val Pro Ile Gly
1               5                   10

<210>  697
<211>  12
<212>  PRT
<213>  Artificial sequence

<220>
<223>  an artificially synthesized sequence

<400>  697

Ile Ser Pro Ser Gly Arg Ser Ala Asn Ile Ile Gly
1               5                   10


<210>  698
<211>  12
<212>  PRT
<213>  Artificial sequence

<220>
<223>  an artificially synthesized sequence

<400>  698

Ile Ser Pro Ser Gly Arg Ser Ala Asn Phe Ile Gly
1               5                   10


<210>  699
<211>  12
<212>  PRT
<213>  Artificial sequence

<220>
<223>  an artificially synthesized sequence

<400>  699

Ile Ser Pro Thr Gly Arg Ser Ala Asn Pro Ile Gly
1               5                   10


<210>  700
<211>  12
<212>  PRT
<213>  Artificial sequence

<220>
<223>  an artificially synthesized sequence

<400>  700

Ile Ser Pro Ser Gly Arg Ser Ala Ile Pro Ile Gly
1               5                   10


<210>  701
<211>  12
<212>  PRT
<213>  Artificial sequence

<220>
<223>  an artificially synthesized sequence

<400>  701

Ile Ser Tyr Thr Gly Arg Ser Ala Asn Pro Ile Gly
1               5                   10


<210>  702
<211>  12
<212>  PRT
<213>  Artificial sequence

<220>
<223>  an artificially synthesized sequence

<400>  702

Ile Ser Tyr Ser Gly Arg Ser Ala Ile Pro Ile Gly
1               5                   10


<210>  703
<211>  12
<212>  PRT
<213>  Artificial sequence

<220>
<223>  an artificially synthesized sequence

<400>  703

Ile Ser Ile Ser Gly Arg Ser Ala Asn Tyr Ile Gly
1               5                   10


<210>  704
<211>  12
<212>  PRT
<213>  Artificial sequence

<220>
<223>  an artificially synthesized sequence

<400>  704

Ile Ser Pro Ser Gly Arg Ser Ala Gly Pro Ile Gly
1               5                   10


<210>  705
<211>  12
<212>  PRT
<213>  Artificial sequence

<220>
<223>  an artificially synthesized sequence

<400>  705

Ile Ser Tyr Thr Gly Arg Ser Ala Val Pro Ile Gly
1               5                   10

<210>  706
<211>  12
<212>  PRT
<213>  Artificial sequence

<220>
<223>  an artificially synthesized sequence

<400>  706

Ile Ser Tyr Thr Gly Arg Ser Ala Val Tyr Ile Gly
1               5                   10


<210>  707
<211>  12
<212>  PRT
<213>  Artificial sequence

<220>
<223>  an artificially synthesized sequence

<400>  707

Ile Ser Tyr Thr Gly Arg Ser Ala Val Val Ile Gly
1               5                   10


<210>  708
<211>  12
<212>  PRT
<213>  Artificial sequence

<220>
<223>  an artificially synthesized sequence

<400>  708

Ile Ser Tyr Thr Gly Arg Ser Ala Val His Ile Gly
1               5                   10


<210>  709
<211>  12
<212>  PRT
<213>  Artificial sequence

<220>
<223>  an artificially synthesized sequence

<400>  709

Tyr Ser Tyr Ser Gly Arg Ser Ala Val Pro Ile Gly
1               5                   10


<210>  710
<211>  12
<212>  PRT
<213>  Artificial sequence

<220>
<223>  an artificially synthesized sequence

<400>  710

Tyr Ser Pro Ser Gly Arg Ser Ala Asn Ile Ile Gly
1               5                   10


<210>  711
<211>  12
<212>  PRT
<213>  Artificial sequence

<220>
<223>  an artificially synthesized sequence

<400>  711

Tyr Ser Pro Ser Gly Arg Ser Ala Asn Phe Ile Gly
1               5                   10


<210>  712
<211>  12
<212>  PRT
<213>  Artificial sequence

<220>
<223>  an artificially synthesized sequence

<400>  712

Tyr Ser Pro Thr Gly Arg Ser Ala Asn Pro Ile Gly
1               5                   10


<210>  713
<211>  12
<212>  PRT
<213>  Artificial sequence

<220>
<223>  an artificially synthesized sequence

<400>  713

Tyr Ser Pro Ser Gly Arg Ser Ala Ile Pro Ile Gly
1               5                   10


<210>  714
<211>  12
<212>  PRT
<213>  Artificial sequence

<220>
<223>  an artificially synthesized sequence

<400>  714

Tyr Ser Tyr Thr Gly Arg Ser Ala Asn Pro Ile Gly
1               5                   10

<210>  715
<211>  12
<212>  PRT
<213>  Artificial sequence

<220>
<223>  an artificially synthesized sequence

<400>  715

Tyr Ser Tyr Ser Gly Arg Ser Ala Ile Pro Ile Gly
1               5                   10


<210>  716
<211>  12
<212>  PRT
<213>  Artificial sequence

<220>
<223>  an artificially synthesized sequence

<400>  716

Tyr Ser Ile Ser Gly Arg Ser Ala Asn Tyr Ile Gly
1               5                   10


<210>  717
<211>  12
<212>  PRT
<213>  Artificial sequence

<220>
<223>  an artificially synthesized sequence

<400>  717

Tyr Ser Pro Ser Gly Arg Ser Ala Gly Pro Ile Gly
1               5                   10


<210>  718
<211>  12
<212>  PRT
<213>  Artificial sequence

<220>
<223>  an artificially synthesized sequence

<400>  718

Tyr Ser Tyr Thr Gly Arg Ser Ala Val Pro Ile Gly
1               5                   10


<210>  719
<211>  12
<212>  PRT
<213>  Artificial sequence

<220>
<223>  an artificially synthesized sequence

<400>  719

Tyr Ser Tyr Thr Gly Arg Ser Ala Val Tyr Ile Gly
1               5                   10


<210>  720
<211>  12
<212>  PRT
<213>  Artificial sequence

<220>
<223>  an artificially synthesized sequence

<400>  720

Tyr Ser Tyr Thr Gly Arg Ser Ala Val Val Ile Gly
1               5                   10


<210>  721
<211>  12
<212>  PRT
<213>  Artificial sequence

<220>
<223>  an artificially synthesized sequence

<400>  721

Tyr Ser Tyr Thr Gly Arg Ser Ala Val His Ile Gly
1               5                   10


<210>  722
<211>  12
<212>  PRT
<213>  Artificial sequence

<220>
<223>  an artificially synthesized sequence

<400>  722

Thr Ser Tyr Thr Gly Arg Ser Ala Val Pro Arg Gly
1               5                   10


<210>  723
<211>  12
<212>  PRT
<213>  Artificial sequence

<220>
<223>  an artificially synthesized sequence

<400>  723

Thr Ser Tyr Ser Gly Arg Ser Ala Val Val Arg Gly
1               5                   10

<210>  724
<211>  12
<212>  PRT
<213>  Artificial sequence

<220>
<223>  an artificially synthesized sequence

<400>  724

Thr Ser Tyr Thr Gly Arg Ser Ala Val Tyr Arg Gly
1               5                   10


<210>  725
<211>  12
<212>  PRT
<213>  Artificial sequence

<220>
<223>  an artificially synthesized sequence

<400>  725

Thr Ser Tyr Thr Gly Arg Ser Ala Val His Arg Gly
1               5                   10


<210>  726
<211>  120
<212>  PRT
<213>  Artificial sequence

<220>
<223>  an artificially synthesized sequence

<400>  726

Gln Ile Gln Leu Val Gln Ser Gly Pro Glu Leu Lys Glu Pro Gly Glu
1               5                   10                  15

Thr Val Arg Ile Ser Cys Lys Ala Ser Gly Tyr Thr Phe Thr Lys Tyr
            20                  25                  30

Gly Ile Asn Trp Val Lys Gln Ala Pro Gly Lys Gly Leu Glu Trp Met
        35                  40                  45

Ala Trp Ile Asn Thr Tyr Ser Gly Met Thr Thr Tyr Ala Asp Asp Phe
    50                  55                  60

Lys Gly Arg Phe Ala Phe Ser Leu Glu Thr Ser Ala Asn Thr Ala Tyr
65                  70                  75                  80

Leu Gln Ile Asn His Leu Lys Asn Asp Asp Thr Ala Thr Tyr Phe Cys
                85                  90                  95

Ala Arg Ser Leu Gly Tyr Asp Tyr Gly Gly Phe Ala Tyr Trp Gly Gln
            100                 105                 110

Gly Thr Leu Val Thr Val Ser Ala
        115                 120

<210>  727
<211>  328
<212>  PRT
<213>  Artificial sequence

<220>
<223>  an artificially synthesized sequence

<400>  727

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Tyr Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Glu Ser Leu Ser Leu Ser Pro
                325

<210>  728
<211>  120
<212>  PRT
<213>  Artificial sequence

<220>
<223>  an artificially synthesized sequence

<400>  728

Asp Val Leu Leu Thr Gln Thr Pro Leu Ser Leu Pro Val Ser Leu Gly
1               5                   10                  15

Asp Gln Ala Phe Ile Ser Cys Arg Ser Gly Gln Thr Leu Val His Asp
            20                  25                  30

Asn Glu Asn Thr Tyr Phe His Trp Tyr Leu Gln Lys Pro Gly Gln Ser
        35                  40                  45

Pro Lys Leu Leu Ile Tyr Lys Ile Ser Asn Arg Phe Ser Gly Val Pro

50                  55                  60

Asp Arg Phe Ser Gly Ser Gly Ser Gly Thr Asp Phe Thr Leu Lys Ile
65                  70                  75                  80

Ser Arg Val Glu Pro Glu Asp Leu Gly Ile Tyr Phe Cys Ser Gln Asn
                85                  90                  95

Thr His Val Pro Phe Thr Phe Gly Ser Gly Thr Lys Leu Glu Ile Lys
            100                 105                 110

Arg Thr Val Ala Ala Pro Ser Val
        115                 120

<210>  729
<211>  107
<212>  PRT
<213>  Artificial sequence

<220>
<223>  an artificially synthesized sequence

<400>  729

<210>  730
<211>  114
<212>  PRT
<213>  Artificial sequence

<220>

<223>  an artificially synthesized sequence

<400>  730

Gln Ser Val Glu Glu Ser Gly Gly Arg Leu Val Thr Pro Gly Thr Pro
1               5                   10                  15

Leu Thr Leu Thr Cys Thr Val Ser Gly Phe Ser Leu Ser Ser Tyr Pro
            20                  25                  30

Met Asn Trp Val Arg Gln Ala Pro Gly Lys Gly Leu Glu Trp Ile Gly
        35                  40                  45

Gly Ile Gly Thr Ser Gly Asn Ile Trp Tyr Ala Ser Trp Ala Lys Gly
    50                  55                  60

Arg Phe Ile Ile Ser Arg Ala Ser Ser Thr Thr Val Asp Leu Lys Val
65                  70                  75                  80

Thr Ser Pro Thr Thr Glu Asp Thr Ala Thr Tyr Phe Cys Ala Arg Gly
                85                  90                  95

Leu Tyr Asn Asp Tyr Thr Val Trp Gly Pro Gly Thr Leu Val Thr Val
            100                 105                 110

Ser Ser

<210>  731
<211>  122
<212>  PRT
<213>  Artificial sequence

<220>
<223>  an artificially synthesized sequence

<400>  731

Ala Gln Val Leu Thr Gln Thr Pro Ser Ser Val Ser Ala Ala Val Gly
1               5                   10                  15

Gly Thr Val Thr Ile Asn Cys Gln Ala Ser Gln Ser Val Tyr Asn Lys
            20                  25                  30

Asn Tyr Leu Ala Trp Tyr Gln Gln Lys Pro Gly Gln Pro Pro Lys Arg
        35                  40                  45

Leu Ile Tyr Ser Ala Ser Thr Leu Ala Ser Gly Val Ser Ser Arg Phe
    50                  55                  60

Lys Gly Ser Gly Ser Gly Thr Gln Phe Thr Leu Thr Ile Ser Asp Val

65                  70                  75                  80

Gln Cys Asp Asp Val Ala Thr Tyr Tyr Cys Leu Gly Ser Tyr Asp Cys
                85                  90                  95

Asn Arg Ala Glu Cys His Ala Phe Gly Gly Gly Thr Lys Val Val Val
            100                 105                 110

Glu Val Arg Thr Val Ala Ala Pro Ser Val
        115                 120

<210>  732
<211>  122
<212>  PRT
<213>  Artificial sequence

<220>
<223>  an artificially synthesized sequence

<400>  732

Gln Val Gln Leu Val Glu Ser Gly Gly Gly Val Val Gln Pro Gly Gly
1               5                   10                  15

Ser Leu Arg Leu Ser Cys Ala Ala Ser Gly Phe Thr Phe Ser Asn Ala
            20                  25                  30

Trp Met His Trp Val Arg Gln Ala Pro Gly Lys Gly Leu Glu Trp Val
        35                  40                  45

Ala Gln Ile Lys Asp Lys Ser Gln Asn Tyr Ala Thr Tyr Val Ala Glu
    50                  55                  60

Ser Val Lys Gly Arg Phe Thr Ile Ser Arg Ala Asp Ser Lys Asn Ser
65                  70                  75                  80

Ile Tyr Leu Gln Met Asn Ser Leu Lys Thr Glu Asp Thr Ala Val Tyr
                85                  90                  95

Tyr Cys Arg Tyr Val His Tyr Ala Ala Gly Tyr Gly Val Asp Ile Trp
            100                 105                 110

Gly Gln Gly Thr Thr Val Thr Val Ser Ser
        115                 120

<210>  733
<211>  328
<212>  PRT
<213>  Artificial sequence

<220>

<223>  an artificially synthesized sequence

<400>  733

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Lys Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Tyr Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210> 734
<211> 112
<212> PRT
<213> Artificial sequence

<220>
<223> an artificially synthesized sequence

<400> 734

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Pro Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Gly
                85                  90                  95

Thr Gln Val Pro Tyr Thr Phe Gly Gln Gly Thr Lys Leu Glu Ile Lys
            100                 105                 110

<210>  735
<211>  328
<212>  PRT
<213>  Artificial sequence

<220>
<223>  an artificially synthesized sequence

<400>  735

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Tyr Gln Trp Gly Pro Met Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser Asp Glu Glu Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ala Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly

210                 215                 220

Gln Pro Arg Glu Pro Gln Val Cys Thr Leu Pro Pro Ser Arg Asp Glu
225                 230                 235                 240

Leu Thr Lys Asn Gln Val Ser Leu Trp Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  736
<211>  328
<212>  PRT
<213>  Artificial sequence

<220>
<223>  an artificially synthesized sequence

<400>  736

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
        100                 105                 110

Pro Ala Pro Glu Leu Leu Gly Gly Pro Ser Val Phe Leu Phe Pro Pro
    115                 120                 125

Lys Pro Lys Asp Thr Leu Met Ile Ser Arg Thr Pro Glu Val Thr Cys
    130                 135                 140

Val Val Val Asp Val Ser His Glu Glu Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
        180                 185                 190

His Gln Asp Trp Leu Asn Gly Lys Glu Tyr Lys Cys Lys Val Ser Asn
    195                 200                 205

Asp Ala Leu Pro Met Pro Ile Glu Glu Thr Ile Ser Lys Ala Lys Gly
    210                 215                 220

Gln Pro Arg Glu Pro Gln Val Tyr Thr Leu Pro Pro Ser Arg Cys Glu
225                 230                 235                 240

Leu Thr Lys Asn Gln Val Ser Leu Ser Cys Ala Val Lys Gly Phe Tyr
                245                 250                 255

Pro Ser Asp Ile Ala Val Glu Trp Glu Ser Asn Gly Gln Pro Glu Asn
        260                 265                 270

Asn Tyr Lys Thr Thr Pro Pro Val Leu Asp Ser Asp Gly Ser Phe Phe
    275                 280                 285

Leu Val Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  737

<211>  328
<212>  PRT
<213>  Artificial sequence

<220>
<223>  an artificially synthesized sequence

<400>  737

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Glu Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Glu Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Asp Ala Leu Pro Met Pro Ile Glu Glu Thr Ile Ser Lys Ala Lys Gly
    210                 215                 220

Gln Pro Arg Glu Pro Gln Val Tyr Thr Leu Pro Pro Ser Arg Cys Glu
225                 230                 235                 240

Leu Thr Lys Asn Gln Val Ser Leu Ser Cys Ala Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Val Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  738
<211>  115
<212>  PRT
<213>  Artificial sequence

<220>
<223>  an artificially synthesized sequence

<400>  738

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Val Arg Gln Ala Pro Gly Gln Gly Leu Glu Trp Met
        35                  40                  45

Gly Ala Leu Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115

<210> 739
<211> 352
<212> PRT
<213> Artificial sequence

<220>
<223> an artificially synthesized sequence

<400> 739

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Gly Gly Ser Gly Gly Pro Pro Gly
            100                 105                 110

Pro Gln Gly Leu Ala Gly Gln Arg Gly Ile Val Gly Leu Gly Ser Ser
        115                 120                 125

Gly Thr His Thr Cys Pro Pro Cys Pro Ala Pro Glu Leu Arg Arg Gly
    130                 135                 140

Pro Ser Val Phe Leu Phe Pro Pro Lys Pro Lys Asp Thr Leu Met Ile
145                 150                 155                 160

Ser Arg Thr Pro Glu Val Thr Cys Val Val Val Asp Val Ser His Glu
                165                 170                 175

Asp Pro Glu Val Lys Phe Asn Trp Tyr Val Asp Gly Val Glu Val His
            180                 185                 190

Asn Ala Lys Thr Lys Pro Arg Glu Glu Gln Tyr Asn Ser Thr Tyr Arg
        195                 200                 205

Val Val Ser Val Leu Thr Val Leu His Gln Asp Trp Leu Asn Gly Lys
    210                 215                 220

Glu Tyr Lys Cys Lys Val Ser Asn Lys Gly Leu Pro Ser Ser Ile Glu
225                 230                 235                 240

Lys Thr Ile Ser Lys Ala Lys Gly Gln Pro Arg Glu Pro Gln Val Tyr
                245                 250                 255

Thr Leu Pro Pro Ser Arg Glu Glu Met Thr Lys Asn Gln Val Ser Leu
            260                 265                 270

Thr Cys Leu Val Lys Gly Phe Tyr Pro Ser Asp Ile Ala Val Glu Trp
        275                 280                 285

Glu Ser Asn Gly Gln Pro Glu Asn Asn Tyr Lys Thr Thr Pro Pro Val
    290                 295                 300

Leu Asp Ser Asp Gly Ser Phe Phe Leu Tyr Ser Lys Leu Thr Val Asp
305                 310                 315                 320

Lys Ser Arg Trp Gln Glu Gly Asn Val Phe Ser Cys Ser Val Met His
                325                 330                 335

Glu Ala Leu His Asn His Tyr Thr Gln Lys Ser Leu Ser Leu Ser Pro
            340                 345                 350

<210>  740
<211>  112
<212>  PRT
<213>  Artificial sequence

<220>
<223>  an artificially synthesized sequence

<400>  740

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Gln Gln Lys Pro Gly Gln Ala

35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210>  741
<211>  328
<212>  PRT
<213>  Artificial sequence

<220>
<223>  an artificially synthesized sequence

<400>  741

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Arg Arg Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Gly Leu Pro Ser Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  742
<211>  328
<212>  PRT
<213>  Artificial sequence

<220>
<223>  an artificially synthesized sequence

<400>  742

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  743
<211>  328
<212>  PRT
<213>  Artificial sequence

<220>
<223>  an artificially synthesized sequence

<400>  743

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210>  744
<211>  327
<212>  PRT
<213>  Artificial sequence

<220>
<223>  an artificially synthesized sequence

<400>  744

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Pro Ser Val Phe Leu Phe Pro Pro Lys
        115                 120                 125

Pro Lys Asp Thr Leu Met Ile Ser Arg Thr Pro Glu Val Thr Cys Val
    130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                165                 170                 175

Gln Tyr Asn Ser Thr Tyr Arg Val Val Ser Val Leu Thr Val Leu His
            180                 185                 190

Gln Asp Trp Leu Asn Gly Lys Glu Tyr Lys Cys Lys Val Ser Asn Lys
        195                 200                 205

Ala Leu Pro Ala Pro Ile Glu Lys Thr Ile Ser Lys Ala Lys Gly Gln
    210                 215                 220

Pro Arg Glu Pro Gln Val Tyr Thr Leu Pro Pro Ser Arg Glu Glu Met
225                 230                 235                 240

Thr Lys Asn Gln Val Ser Leu Thr Cys Leu Val Lys Gly Phe Tyr Pro
                245                 250                 255

Ser Asp Ile Ala Val Glu Trp Glu Ser Asn Gly Gln Pro Glu Asn Asn
            260                 265                 270

Tyr Lys Thr Thr Pro Pro Val Leu Asp Ser Asp Gly Ser Phe Phe Leu
        275                 280                 285

Tyr Ser Lys Leu Thr Val Asp Lys Ser Arg Trp Gln Glu Gly Asn Val
    290                 295                 300

Phe Ser Cys Ser Val Met His Glu Ala Leu His Asn His Tyr Thr Gln
305                 310                 315                 320

Lys Ser Leu Ser Leu Ser Pro
                325

<210>  745
<211>  352
<212>  PRT
<213>  Artificial sequence

<220>
<223>  an artificially synthesized sequence

<400>  745

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Gly Gly Ser Gly Gly Pro Pro Gly
            100                 105                 110

Pro Gln Gly Leu Ala Gly Gln Arg Gly Ile Val Gly Leu Gly Ser Ser
        115                 120                 125

Gly Thr His Thr Cys Pro Pro Cys Pro Ala Pro Glu Leu Leu Gly Gly
    130                 135                 140

Pro Ser Val Phe Leu Phe Pro Pro Lys Pro Lys Asp Thr Leu Met Ile
145                 150                 155                 160

Ser Arg Thr Pro Glu Val Thr Cys Val Val Val Asp Val Ser His Glu
                165                 170                 175

Asp Pro Glu Val Lys Phe Asn Trp Tyr Val Asp Gly Val Glu Val His
            180                 185                 190

Asn Ala Lys Thr Lys Pro Arg Glu Glu Gln Tyr Asn Ser Thr Tyr Arg
        195                 200                 205

Val Val Ser Val Leu Thr Val Leu His Gln Asp Trp Leu Asn Gly Lys
    210                 215                 220

Glu Tyr Lys Cys Lys Val Ser Asn Lys Ala Leu Pro Ala Pro Ile Glu
225                 230                 235                 240

Lys Thr Ile Ser Lys Ala Lys Gly Gln Pro Arg Glu Pro Gln Val Tyr
                245                 250                 255

Thr Leu Pro Pro Ser Arg Glu Glu Met Thr Lys Asn Gln Val Ser Leu
            260                 265                 270

Thr Cys Leu Val Lys Gly Phe Tyr Pro Ser Asp Ile Ala Val Glu Trp
        275                 280                 285

Glu Ser Asn Gly Gln Pro Glu Asn Asn Tyr Lys Thr Thr Pro Pro Val
    290                 295                 300

Leu Asp Ser Asp Gly Ser Phe Phe Leu Tyr Ser Lys Leu Thr Val Asp
305                 310                 315                 320

Lys Ser Arg Trp Gln Glu Gly Asn Val Phe Ser Cys Ser Val Met His
                325                 330                 335

Glu Ala Leu His Asn His Tyr Thr Gln Lys Ser Leu Ser Leu Ser Pro
            340                 345                 350

<210>  746
<211>  107
<212>  PRT
<213>  Artificial Sequence

<220>
<223>  an artificially synthesized sequence

<400>  746

Arg Thr Val Ala Ala Pro Ser Val Phe Ile Phe Pro Pro Ser Asp Glu

1                   5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Cys
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105

<210>  747
<211>  276
<212>  PRT
<213>  Artificial Sequence

<220>
<223>  an artificially synthesized sequence

<400>  747

Met Gly Trp Ser Cys Ile Ile Leu Phe Leu Val Ala Thr Ala Thr Gly
1               5                   10                  15

Val His Ser Gln Ile Gln Leu Val Gln Ser Gly Pro Glu Leu Lys Glu
            20                  25                  30

Pro Gly Glu Thr Val Arg Ile Ser Cys Lys Ala Ser Gly Tyr Thr Phe
        35                  40                  45

Thr Lys Tyr Gly Ile Asn Trp Val Lys Gln Ala Pro Gly Lys Gly Leu
    50                  55                  60

Glu Trp Met Ala Trp Ile Asn Thr Tyr Ser Gly Met Thr Thr Tyr Ala
65                  70                  75                  80

Asp Asp Phe Lys Gly Arg Phe Ala Phe Ser Leu Glu Thr Ser Ala Asn
                85                  90                  95

Thr Ala Tyr Leu Gln Ile Asn His Leu Lys Asn Asp Asp Thr Ala Thr
            100                 105                 110

Tyr Phe Cys Ala Arg Ser Leu Gly Tyr Asp Tyr Gly Gly Phe Ala Tyr
        115                 120                 125

Trp Gly Gln Gly Thr Leu Val Thr Val Ser Ala Ser Ser Ala Asp Asp
    130                 135                 140

Ala Lys Lys Asp Ala Ala Lys Lys Asp Asp Ala Lys Lys Asp Asp Ala
145                 150                 155                 160

Lys Lys Asp Gly Asp Val Leu Leu Thr Gln Thr Pro Leu Ser Leu Pro
                165                 170                 175

Val Ser Leu Gly Asp Gln Ala Phe Ile Ser Cys Arg Ser Gly Gln Thr
            180                 185                 190

Leu Val His Asp Asn Glu Asn Thr Tyr Phe His Trp Tyr Leu Gln Lys
        195                 200                 205

Pro Gly Gln Ser Pro Lys Leu Leu Ile Tyr Lys Ile Ser Asn Arg Phe
    210                 215                 220

Ser Gly Val Pro Asp Arg Phe Ser Gly Ser Gly Ser Gly Thr Asp Phe
225                 230                 235                 240

Thr Leu Lys Ile Ser Arg Val Glu Pro Glu Asp Leu Gly Ile Tyr Phe
                245                 250                 255

Cys Ser Gln Asn Thr His Val Pro Phe Thr Phe Gly Ser Gly Thr Lys
            260                 265                 270

Leu Glu Ile Lys
        275

<210>  748
<211>  829
<212>  PRT
<213>  Artificial Sequence

<220>
<223>  an artificially synthesized sequence

<400>  748

Met Gly Trp Ser Cys Ile Ile Leu Phe Leu Val Ala Thr Ala Thr Gly
1               5                   10                  15

Val His Ser Gln Ile Gln Leu Val Gln Ser Gly Pro Glu Leu Lys Glu
            20                  25                  30

Pro Gly Glu Thr Val Arg Ile Ser Cys Lys Ala Ser Gly Tyr Thr Phe
        35                  40                  45

Thr Lys Tyr Gly Ile Asn Trp Val Lys Gln Ala Pro Gly Lys Gly Leu
    50                  55                  60

Glu Trp Met Ala Trp Ile Asn Thr Tyr Ser Gly Met Thr Thr Tyr Ala
65                  70                  75                  80

Asp Asp Phe Lys Gly Arg Phe Ala Phe Ser Leu Glu Thr Ser Ala Asn
                85                  90                  95

Thr Ala Tyr Leu Gln Ile Asn His Leu Lys Asn Asp Asp Thr Ala Thr
            100                 105                 110

Tyr Phe Cys Ala Arg Ser Leu Gly Tyr Asp Tyr Gly Gly Phe Ala Tyr
        115                 120                 125

Trp Gly Gln Gly Thr Leu Val Thr Val Ser Ala Ser Ser Ala Asp Asp
    130                 135                 140

Ala Lys Lys Asp Ala Ala Lys Lys Asp Asp Ala Lys Lys Asp Asp Ala
145                 150                 155                 160

Lys Lys Asp Gly Asp Val Leu Leu Thr Gln Thr Pro Leu Ser Leu Pro
                165                 170                 175

Val Ser Leu Gly Asp Gln Ala Phe Ile Ser Cys Arg Ser Gly Gln Thr
            180                 185                 190

Leu Val His Asp Asn Glu Asn Thr Tyr Phe His Trp Tyr Leu Gln Lys
        195                 200                 205

Pro Gly Gln Ser Pro Lys Leu Leu Ile Tyr Lys Ile Ser Asn Arg Phe
    210                 215                 220

Ser Gly Val Pro Asp Arg Phe Ser Gly Ser Gly Ser Gly Thr Asp Phe
225                 230                 235                 240

Thr Leu Lys Ile Ser Arg Val Glu Pro Glu Asp Leu Gly Ile Tyr Phe
                245                 250                 255

Cys Ser Gln Asn Thr His Val Pro Phe Thr Phe Gly Ser Gly Thr Lys
            260                 265                 270

Leu Glu Ile Lys Ala Ala Ala Phe Val Pro Val Phe Leu Pro Ala Lys
        275                 280                 285

Pro Thr Thr Thr Pro Ala Pro Arg Pro Pro Thr Pro Ala Pro Thr Ile
    290                 295                 300

Ala Ser Gln Pro Leu Ser Leu Arg Pro Glu Ala Cys Arg Pro Ala Ala
305                 310                 315                 320

Gly Gly Ala Val His Thr Arg Gly Leu Asp Phe Ala Cys Asp Ile Tyr
                325                 330                 335

Ile Trp Ala Pro Leu Ala Gly Thr Cys Gly Val Leu Leu Leu Ser Leu
            340                 345                 350

Val Ile Thr Leu Tyr Cys Asn His Arg Asn Arg Ser Lys Arg Ser Arg
        355                 360                 365

Leu Leu His Ser Asp Tyr Met Asn Met Thr Pro Arg Arg Pro Gly Pro
    370                 375                 380

Thr Arg Lys His Tyr Gln Pro Tyr Ala Pro Pro Arg Asp Phe Ala Ala
385                 390                 395                 400

Tyr Arg Ser Arg Phe Ser Val Val Lys Arg Gly Arg Lys Lys Leu Leu
                405                 410                 415

Tyr Ile Phe Lys Gln Pro Phe Met Arg Pro Val Gln Thr Thr Gln Glu
            420                 425                 430

Glu Asp Gly Cys Ser Cys Arg Phe Pro Glu Glu Glu Glu Gly Gly Cys
        435                 440                 445

Glu Leu Arg Val Lys Phe Ser Arg Ser Ala Asp Ala Pro Ala Tyr Gln
    450                 455                 460

Gln Gly Gln Asn Gln Leu Tyr Asn Glu Leu Asn Leu Gly Arg Arg Glu
465                 470                 475                 480

Glu Tyr Asp Val Leu Asp Lys Arg Arg Gly Arg Asp Pro Glu Met Gly
                485                 490                 495

Gly Lys Pro Arg Arg Lys Asn Pro Gln Glu Gly Leu Tyr Asn Glu Leu
            500                 505                 510

Gln Lys Asp Lys Met Ala Glu Ala Tyr Ser Glu Ile Gly Met Lys Gly
        515                 520                 525

Glu Arg Arg Arg Gly Lys Gly His Asp Gly Leu Tyr Gln Gly Leu Ser
    530                 535                 540

Thr Ala Thr Lys Asp Thr Tyr Asp Ala Leu His Met Gln Ala Leu Pro
545                 550                 555                 560

Pro Arg Glu Phe Gly Ser Gly Val Lys Gln Thr Leu Asn Phe Asp Leu
                565                 570                 575

Leu Lys Leu Ala Gly Asp Val Glu Ser Asn Pro Gly Pro Cys Met Val
            580                 585                 590

Ser Lys Gly Glu Glu Leu Phe Thr Gly Val Val Pro Ile Leu Val Glu
        595                 600                 605

Leu Asp Gly Asp Val Asn Gly His Lys Phe Ser Val Ser Gly Glu Gly
    610                 615                 620

Glu Gly Asp Ala Thr Tyr Gly Lys Leu Thr Leu Lys Phe Ile Cys Thr
625                 630                 635                 640

Thr Gly Lys Leu Pro Val Pro Trp Pro Thr Leu Val Thr Thr Leu Thr
                645                 650                 655

Tyr Gly Val Gln Cys Phe Ser Arg Tyr Pro Asp His Met Lys Gln His
            660                 665                 670

Asp Phe Phe Lys Ser Ala Met Pro Glu Gly Tyr Val Gln Glu Arg Thr
        675                 680                 685

Ile Phe Phe Lys Asp Asp Gly Asn Tyr Lys Thr Arg Ala Glu Val Lys
    690                 695                 700

Phe Glu Gly Asp Thr Leu Val Asn Arg Ile Glu Leu Lys Gly Ile Asp
705                 710                 715                 720

Phe Lys Glu Asp Gly Asn Ile Leu Gly His Lys Leu Glu Tyr Asn Tyr
                725                 730                 735

Asn Ser His Asn Val Tyr Ile Met Ala Asp Lys Gln Lys Asn Gly Ile
            740                 745                 750

Lys Val Asn Phe Lys Ile Arg His Asn Ile Glu Asp Gly Ser Val Gln
        755                 760                 765

Leu Ala Asp His Tyr Gln Gln Asn Thr Pro Ile Gly Asp Gly Pro Val
    770                 775                 780

Leu Leu Pro Asp Asn His Tyr Leu Ser Thr Gln Ser Ala Leu Ser Lys

785                 790                 795                 800

Asp Pro Asn Glu Lys Arg Asp His Met Val Leu Leu Glu Phe Val Thr
                805                 810                 815

Ala Ala Gly Ile Thr Leu Gly Met Asp Glu Leu Tyr Lys
            820                 825

<210>  749
<211>  20
<212>  PRT
<213>  Artificial Sequence

<220>
<223>  an artificially synthesized sequence

<400>  3

Lys Ser Cys Asp Lys Thr Asp Gly Pro Ser Gly Ala Glu Gly Pro Pro
1               5                   10                  15

Gly Pro Gln Gly
            20

## Description

### TECHNICAL FIELD

The present disclosure relates to an antibody having ADCC activity, a T cell-redirecting antibody, a chimeric receptor, a cell expressing a chimeric receptor, and a procedure method for a disease using the cell or the antibody, particularly, therapy using the ADCC activity of the antibody, CAR-T therapy using the cell, and a T cell-redirecting antibody therapy.

### BACKGROUND ART

Antibody drugs are drugs composed mainly of immunoglobulins which partly constitute the immune system of living bodies, or analogs thereof (Non Patent Literatures 1 and 2). The antibody drugs compared with conventional low-molecular compounds have a large molecular weight and are capable of complicated molecular recognition. Therefore, the antibody drugs have high specificity of target recognition and cause few unexpected adverse reactions. Foreign matter in blood is generally taken up into cells by endocytosis and degraded. Features of antibodies, which contain an antibody recovery mechanism mediated by a specific receptor FcRn and an antibody Fc region, are to have a long circulation time in blood and to produce drug efficacy for a long period by single administration. Moreover, the antibody drugs are prepared as recombinant proteins and can therefore be functionally altered by the application of genetic engineering.

It is known that, for example, antibody-dependent cellular cytotoxicity (ADCC) which is induced by the binding of antibody constant regions to FcyR of NK cells or macrophages induces stronger cytotoxic activity by using an antibody having constant regions altered to enhance binding to FcyR (Non Patent Literature 3).

Although a normal antibody recognizes and binds to only one epitope of an antigen, an antibody binding to two or more types of antigens by one molecule (referred to as a bispecific antibody) has been developed by improving a natural IgG antibody (Non Patent Literature 11). Such a bispecific antibody is capable of binding to a protein expressed in T cells (CD3 epsilon or TCR) and a protein expressed in cancer cells (cancer antigen). T cell-redirecting antibodies, which are antibodies having an antitumor effect based on a cytotoxic mechanism through which T cells are recruited as effector cells, have been known as one of the bispecific antibodies since 1980s (Non Patent Literatures 12, 13 and 14). Unlike antibodies having an antitumor effect based on an ADCC mechanism through which NK cells or macrophages are recruited as effector cells, the T cell-redirecting antibodies have a binding domain for any constituent subunit of a T cell receptor (TCR) complex, particularly, a domain binding to a CD3 epsilon chain, and are capable of binding to an antigen on targeted cancer cells so that the T cells and the cancer antigen-expressing cells are bridged to induce strong cytotoxicity (T-cell dependent cellular cytotoxicity; TDCC) to the cancer antigen-expressing cells with the T cells as effector cells (Non Patent Literatures 4, 12, 13 and 14).

Antitumor therapy that utilizes the high specificity of antibodies for the antigen recognition of effector cells has been developed in recent years, and has exhibited considerable drug efficacy on some carcinomas. In this therapy, called chimeric antigen receptor (hereinafter, also referred to as "CAR") adoptive immunotherapy, an intracellular signal transduction domain is artificially fused to an extracellular domain having the ability to bind to an antigen, such as antibody-derived scFv, and this fusion product is expressed as CAR in effector cells such as T cells. The T cells expressing this CAR (hereinafter, also referred to as "CAR-T cells") are transferred to cancer patients. When CAR recognizes a tumor antigen, the intracellular domain is activated so that the cytotoxic activity of the effector cells is induced and damages tumor cells, exerting drug efficacy (Non Patent Literature 5).

Clinical trials have been conducted on cancer immunotherapy by the administration of CAR-T cells (Non Patent Literature 10), and the obtained results indicate that the cancer immunotherapy by the administration of CAR-T cells is effective for, for example, malignant hematopoietic tumor such as leukemia or lymphoma. Kymriah(R) (Novartis AG, tisagenlecleucel, CTL-019, CD3 zeta-CD137) and Yescarta(R) (KiTE/Gilead Sciences, Inc., axicabtagene ciloleucel, CD3 zeta-CD28), which are CAR-T against CD19 as an antigen, were approved as drugs in the USA in 2017 and in Japan in 2019.

In addition, a method that utilizes *Camelidae* animal-derived single domain antibodies to target diverse antigens and further simplify antibody preparation has also been proposed. The antibody drugs generally have many advantages and as such, are applied to a wide range of diseases such as tumor, autoimmune diseases, and infections (Non Patent Literature 6).

On the other hand, the limits of the antibody drugs have also been pointed out.

One of these limits is lesion site specificity for an antigen. A surface antigen targeted by an antibody may be expressed in normal tissues in addition to lesion sites, and the antibody may act on the normal tissues expressing the antigen, albeit at a lower expression level than that at the lesion sites, causing adverse reactions.

A possible solution to this problem is the identification of lesion sites targeting lesion site-specific protease activity. It is disclosed that, for example, a protease substrate supplemented with a fluorescent dye is administered to a synthetic peptide chain comprising a cleavage site for protease that is activated at a lesion site, and change in fluorescence associated with cleavage can be measured to identify the lesion site through protease activity (Non Patent Literature 7).

Research has been reported to allow an antibody to recognize a protease cleavage product formed at a lesion site to detect the lesion site (Non Patent Literature 8). In this research, it has been reported that a cleavage product by IdeS protease expressed by *Actinomycetes* can be specifically recognized *in vivo* using an antibody specifically binding to the cleavage product (Non Patent Literature 8).

For chronic disease regions as well, an antibody recognizing collagen II cleaved by MMP (matrix metalloprotease) activated at a knee osteoarthritis lesion site has been developed for the purpose of developing a reagent detecting a lesion of knee osteoarthritis (Non Patent Literature 9).

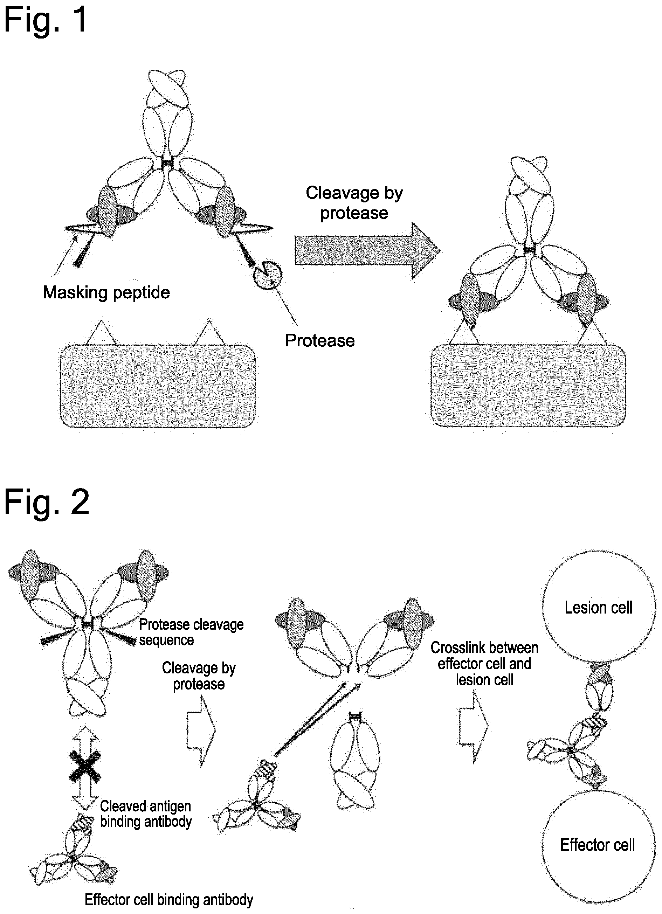

Examples of the application of existing protease activated antibody techniques to treatment include "Probody(R) technology" of expanding tissue specificity and therapeutic window by imparting sensitivity to protease having the degree of expression or activation increased at a lesion site such as a cancer tissue or an inflammatory tissue to an antibody (Figure 1).

The "Probody(R)" is a molecule in which an antibody is connected to a masking peptide that masks the antigen binding site of the antibody via a cleavage peptide sequence that is cleavable by protease expressed at a lesion site (Non Patent Literature 15). The antigen binding site of the antibody is masked by the masking peptide and cannot thereby bind to the antigen, in an uncleaved state of the peptide sequence. The cleavage peptide sequence of the Probody^{®} is cleaved by protease expressed at a target pathological site so that the masking peptide is dissociated to produce an antibody molecule having antigen binding activity, which is in turn capable of binding to the antigen specific for the target pathological tissue. The Probody(R) can be administered in a larger amount than that of normal antibodies and is expected to be able to expand the therapeutic window, because antigen-antibody binding is inhibited at a non-lesion site where protease is absent.

### CITATION LIST

### PATENT LITERATURE

[Patent Literature 1] WO2009/025846
[Patent Literature 2] WO2017/143094
[Patent Literature 3] WO2018/097307

### NON PATENT LITERATURE

[Non Patent Literature 1] Janice M Reichert, Clark J Rosensweig, Laura B Faden & Matthew C Dewitz, Monoclonal antibody successes in the clinic., Nat. Biotechnol. (2005) 23, 1073-1078
[Non Patent Literature 2] Pavlou AK, Belsey MJ., The therapeutic antibodies market to 2008., Eur J Pharm Biopharm. (2005) 59 (3), 389-396
[Non Patent Literature 3] The impact of Fc engineering on an anti-CD19 antibody: increased Fcgamma receptor affinity enhances B-cell clearing in nonhuman primates. Zalevsky J, Leung IW, Karki S, Chu SY, Zhukovsky EA, Desjarlais JR, Carmichael DF, Lawrence CE. Blood. 2009 Apr 16; 113(16): 3735-43.
[Non Patent Literature 4] Advances in bispecific biotherapeutics for the treatment of cancer Biochem Pharmacol. 2012 Nov 1; 84(9): 1105-12
[Non Patent Literature 5] Chimeric Antigen Receptor Therapy N Engl J Med 2018; 379: 64-73
[Non Patent Literature 6] Single-domain antibodies for biomedical applications. Immunopharmacol Immunotoxicol. 2016; 38(1): 21-8
[Non Patent Literature 7] Shedding light onto live molecular targets Nat Med. 2003 Jan; 9(1): 123-8
[Non Patent Literature 8] Structure and specificity of an antibody targeting a proteolytically cleaved IgG hinge Malia TJ1, Teplyakov A, Brezski RJ, Luo J, Kinder M, Sweet RW, Almagro JC, Jordan RE, Gilliland GL. Proteins. 2014 Aug; 82(8): 1656-67
[Non Patent Literature 9] Development of a novel immunoassay for the measurement of type II collagen neoepitope generated by collagenase cleavage Clin Chim Acta. 2012 Oct 9; 413(19-20): 1591-9.
[Non Patent Literature 10] Grupp et al. 2013 N Engl J Med 368(16): 1509-18.
[Non Patent Literature 11] Kontermann, mAbs 2012; 4: 182-197.
[Non Patent Literature 12] Mezzanzanica et al., International journal of cancer 1988; 41: 609-615.
[Non Patent Literature 13] Staerz and Bevan, Proceedings of the National Academy of Sciences of the United States of America 1986; 83: 1453-1457.
[Non Patent Literature 14] Staerz et al., Nature 1985; 314: 628-631.
[Non Patent Literature 15] Desnoyers LR et al., Sci Transl Med. 2013 Oct 16; 5(207): 207ra144.

### SUMMARY OF INVENTION

### TECHNICAL PROBLEM

Probody(R) exerts cytotoxic activity even in normal tissues due to the long circulation time in blood of activated Probody(R) and antigen binding activity possessed in a nonactivated state without protease cleavage. Although high cytotoxic activity is obtained in T cell-redirecting antibody therapy or CAR-T therapy, it is known that the activity takes place even in normal cells, causing severe adverse reactions. Thus, improvement related to the safety of such therapy has been desired.

A single tumor antigen is not universally expressed in all cancers. Therefore, an antigen recognition site in the therapy needs to be constructed on a targeted tumor antigen basis, and economical cost and labor associated with the operation are major problems. Furthermore, targeted tumor antigens may cause immune escape, causing reduction or disappearance of therapeutic effect, in such a way that treatment decreases their expression levels or mutates the tumor antigens.

Although research has been made on CAR-T cells and T cell-redirecting antibodies recognizing tumor antigens that can be changed according to the phase of treatment, and treatment methods using them, there has been a demand for more safe and inexpensive treatment by the development of treatment methods having sufficient therapeutic effects upon administration to patients and having high safety and techniques that can be used for general purposes.

### SOLUTION TO PROBLEM

To solve these problems, the inventors have conducted studies and completed the present invention by finding that an antibody having ADCC activity, a T cell-redirecting antibody or a CAR-T cell binding to an antigen binding domain newly produced through cleavage by protease acts selectively on a therapeutic target cell and is thus useful for treatment. One aspect of the present disclosure discloses a group of versatile therapeutic molecules which are molecules comprising an antigen binding domain that is produced by protease and having a short half-life in blood, and do not exert a pharmacological effect without cleavage by the protease.

The present disclosure provides, for example, a pharmaceutical composition comprising a molecule having the ability to bind to an antigen and a pharmaceutical composition comprising a molecule having the ability to activate an effector, wherein the pharmaceutical compositions are used for activating an effector cell by associating these two molecules through cleavage by protease expressed in a target tissue-specific manner, and bridging a target cell expressing the antigen to the effector cell, a pharmaceutical composition for use in the treatment of a disease caused by a target tissue, and a molecule having the ability to bind to an antigen and a molecule having the ability to activate an effector for use as active ingredients in the pharmaceutical compositions. The present disclosure further provides methods for producing the pharmaceutical compositions, and the molecule having the ability to bind to an antigen and the molecule having the ability to activate an effector for use as active ingredients.

The present disclosure, by using a molecule binding to a tumor antigen in common, further enables the optimum therapy to be selected according to patient's compatibility with treatment from a plurality of therapies, CAR-T therapy, bispecific antibody therapy and antibody therapy having ADCC activity, and also enables therapy to be changed or added according to a treatment status.

The present disclosure, by using a plurality of molecules binding to a tumor antigen in common, further enables the optimum therapy to be selected according to patient's compatibility with treatment from a plurality of therapies, CAR-T therapy, bispecific antibody therapy and antibody therapy having ADCC activity, and also enables therapy to be changed or added according to a treatment status.

One aspect of the present disclosure is an antibody having ADCC activity, a bispecific antibody or a CAR-T cell against a target cell expressing a target antigen, wherein the CAR-T cell, the bispecific antibody or the antibody having ADCC activity binds to the target cell via binding to an antigen binding molecule. The antigen binding molecule comprises a linker that is cleavable by protease, and has the ability to bind to the target antigen after cleavage of the linker by the protease. The present disclosure relates to the CAR-T cell, the bispecific antibody or the antibody having ADCC activity.

A further aspect of the present disclosure relates to an isolated nucleic acid molecule encoding an antibody having ADCC activity, a bispecific antibody and/or CAR, wherein the isolated nucleic acid molecule is usable for targeting a cell expressing the target antigen of interest.

A further aspect of the present disclosure relates to a vector comprising an isolated nucleic acid molecule encoding an antibody having ADCC activity, a bispecific antibody and/or CAR, wherein the vector is usable for targeting a cell expressing the target antigen of interest.

A further aspect of the present disclosure relates to a cell expressing the CAR of the present disclosure, or a cell transfected or transduced with the nucleic acid molecule or the vector of the present disclosure.

More specifically, one aspect of the present disclosure provides the following invention.

[1] A pharmaceutical composition comprising a cell expressing a chimeric receptor, for use in combination with the administration of an antigen binding molecule, wherein
   the antigen binding molecule comprises a linker that is cleavable by protease, and has the ability to bind to a target antigen after cleavage of the linker, and
   the chimeric receptor comprises an extracellular binding domain, a transmembrane domain and an intracellular signal transduction domain, and the extracellular binding domain has the ability to bind to the antigen binding molecule after cleavage of the linker, and is capable of binding to a cell expressing the target antigen via binding to the antigen binding molecule after cleavage of the linker.
[2] A pharmaceutical composition comprising an antigen binding molecule, for use in combination with the administration of a cell expressing a chimeric receptor, wherein
   the antigen binding molecule comprises a linker that is cleavable by protease, and has the ability to bind to a target antigen after cleavage of the linker,
   the chimeric receptor comprises an extracellular binding domain, a transmembrane domain and an intracellular signal transduction domain, and the extracellular binding domain has the ability to bind to the antigen binding molecule after cleavage of the linker, and is capable of binding to a cell expressing the target antigen via binding to the antigen binding molecule after cleavage of the linker.
[3] A pharmaceutical composition comprising a bispecific antibody, for use in combination with the administration of an antigen binding molecule, wherein
   the antigen binding molecule comprises a linker that is cleavable by protease, and has the ability to bind to a target antigen after cleavage of the linker,
   the bispecific antibody comprises antibody variable regions having binding activity against the antigen binding molecule after cleavage of the linker by the protease, and antibody variable regions having binding activity against a molecule expressed on T cell surface, and
   the bispecific antibody is capable of binding to a cell expressing the target antigen via binding to the antigen binding molecule after cleavage of the linker.
[4] A pharmaceutical composition comprising an antigen binding molecule, for use in combination with the administration of a bispecific antibody, wherein
   the antigen binding molecule comprises a linker that is cleavable by protease, and has the ability to bind to a target antigen after cleavage of the linker,
   the bispecific antibody comprises antibody variable regions having binding activity against the antigen binding molecule after cleavage of the linker by the protease, and antibody variable regions having binding activity against a molecule expressed on T cell surface, and
   the bispecific antibody is capable of binding to a cell expressing the target antigen via binding to the antigen binding molecule after cleavage of the linker.
[5] A pharmaceutical composition comprising an IgG antibody having enhanced antibody-dependent cellular cytotoxicity, for use in combination with the administration of an antigen binding molecule, wherein
   the antigen binding molecule comprises a linker that is cleavable cleaved by protease, and has binding activity against an antigen expressed on target cell surface after cleavage of the linker by the protease,
   the IgG antibody comprises antibody variable regions having binding activity against the antigen binding molecule after cleavage of the linker by the protease, and
   the IgG antibody is capable of binding to the target cell via binding to the antigen binding molecule after cleavage of the linker.
[6] A pharmaceutical composition comprising an antigen binding molecule, for use in combination with the administration of an IgG antibody having enhanced antibody-dependent cellular cytotoxicity, wherein
   the antigen binding molecule comprises a linker that is cleavable by protease, and has binding activity against an antigen expressed on target cell surface after cleavage of the linker by the protease, and
   the IgG comprises antibody variable regions having binding activity against the antigen binding molecule after cleavage of the linker by the protease, and
   the IgG antibody is capable of binding to the target cell via binding to the antigen binding molecule after cleavage of the linker.
[7] The pharmaceutical composition according to any of [1] to [6], wherein a ratio of a K_{D} value of the antigen binding molecule after cleavage of the linker for the antigen to a K_{D} value of the antigen binding molecule before cleavage of the linker for the antigen (K_{D} (after cleavage)/K_{D} (before cleavage)) is 0.1 or less or 0.01 or less.
[8] The pharmaceutical composition according to any of [1] to [7], wherein the antigen binding molecule is an IgG antibody, an IgG antibody-like molecule, a heavy chain antibody or a single domain antibody.
[9] The pharmaceutical composition according to any of [1] to [8], wherein the antigen binding molecule comprises variable and constant regions of an antibody, and a linker that is cleavable by protease, wherein the antibody is selected from an IgG antibody, an IgG antibody-like molecule and a heavy chain antibody, and the antigen binding molecule obtained through the cleavage of the linker by the protease comprises an antigen binding domain and a portion of the cleaved linker.
[10] The pharmaceutical composition according to any of [1] to [9], wherein in the antigen binding molecule, the linker that is cleavable by protease is located near the boundary between the variable region and the constant region or near the boundary between CH1 and CH2 in the constant region.
[11] The pharmaceutical composition according to any of [1] to [10], wherein the antigen binding molecule is an antibody or an IgG antibody-like molecule comprising a linker that is cleavable by protease, and the antigen binding molecule after cleavage of the linker is VL, VH, or VHH of the antibody or an antigen binding fragment thereof.
[12] The pharmaceutical composition according to any of [1] to [11], wherein the antigen binding molecule is a single domain antibody comprising a linker that is cleavable by protease, and the antigen binding molecule after cleavage of the linker is an antigen binding domain of the single domain antibody and a portion of the linker.
[13] The pharmaceutical composition according to any of [1] to [12], wherein the linker that is cleavable by protease comprises a protease cleavage sequence.
[14] The pharmaceutical composition according to any of [1] to [12], wherein the linker that is cleavable by protease comprises a peptide having any of the protease cleavage sequences of SEQ ID NOs: 1 to 725.
[15] The pharmaceutical composition according to any of [1] to [14] for use in the treatment or prevention of a cancer.

[A1-1] A pharmaceutical composition comprising a cell expressing a chimeric receptor, for use in combination with the administration of an antigen binding molecule, wherein
   the antigen binding molecule comprises a linker that is cleavable by protease, and has the ability to bind to a target antigen after cleavage of the linker, and
   the chimeric receptor comprises an extracellular binding domain, a transmembrane domain and an intracellular signal transduction domain, and the extracellular binding domain has the ability to bind to the antigen binding molecule after cleavage of the linker, and is capable of binding to a cell expressing the target antigen via binding to the antigen binding molecule after cleavage of the linker.
[A1-2] A pharmaceutical composition comprising an antigen binding molecule, for use in combination with the administration of a cell expressing a chimeric receptor, wherein
   the antigen binding molecule comprises a linker that is cleavable by protease, and has the ability to bind to a target antigen after cleavage of the linker,
   the chimeric receptor comprises an extracellular binding domain, a transmembrane domain and an intracellular signal transduction domain, and the extracellular binding domain has the ability to bind to the antigen binding molecule after cleavage of the linker, and is capable of binding to a cell expressing the target antigen via binding to the antigen binding molecule after cleavage of the linker.
[A1-3] The pharmaceutical composition according to [A1-1] or [A1-2], wherein a ratio of a K_{D} value of the antigen binding molecule after cleavage of the linker for the antigen to a K_{D} value of the antigen binding molecule before cleavage of the linker for the antigen (K_{D} (after cleavage)/K_{D} (before cleavage)) is 0.1 or less or 0.01 or less.
[A1-4] The pharmaceutical composition according to any of [A1-1] to [A1-3], wherein the antigen binding molecule is an IgG antibody, an IgG antibody-like molecule, a heavy chain antibody or a single domain antibody.
[A1-5] The pharmaceutical composition according to any of [A1-1] to [A1-4], wherein the antigen binding molecule comprises variable and constant regions of an antibody, and a linker that is cleavable by protease, wherein the antibody is selected from an IgG antibody, an IgG antibody-like molecule and a heavy chain antibody, and the antigen binding molecule obtained through the cleavage of the linker by the protease comprises an antigen binding domain and a portion of the cleaved linker.
[A1-6] The pharmaceutical composition according to any of [A1-1] to [A1-4], wherein the antigen binding molecule comprises VHH of a single domain antibody and a linker that is cleavable by protease, and the antigen binding molecule obtained through the cleavage of the linker by the protease comprises an antigen binding domain and a portion of the cleaved linker.
[A1-7] The pharmaceutical composition according to any of [A1-1] to [A1-6], wherein in the antigen binding molecule, the linker that is cleavable by protease is located near the boundary between the variable region and the constant region or near the boundary between CH1 and CH2 in the constant region.
[A1-8] The pharmaceutical composition according to any of [A1-1] to [A1-7], wherein in the antigen binding molecule, the linker that is cleavable by protease is located near a hinge region.
[A1-9] The pharmaceutical composition according to any of [A1-1] to [A1-8], wherein the antigen binding molecule is an antibody or an IgG antibody-like molecule comprising a linker that is cleavable by protease, and the antigen binding molecule after cleavage of the linker is VL, VH, or VHH of the antibody or an antigen binding fragment thereof.
[A1-10] The pharmaceutical composition according to any of [A1-1] to [A1-9], wherein the antigen binding molecule is a heavy chain antibody or a single domain antibody comprising a linker that is cleavable by protease, and the antigen binding molecule after cleavage of the linker is VHH of the antigen binding molecule or a portion thereof comprising an antigen binding domain.
[A1-11] The pharmaceutical composition according to any of [A1-1] to [A1-10], wherein the antigen binding molecule after cleavage of the linker is scFv, Fv, Fab, Fab', F(ab')₂, VH or VHH
[A1-12] The pharmaceutical composition according to any of [A1-1] to [A1-11], wherein the extracellular binding domain of the chimeric receptor recognizes the cleaved linker, a portion of the linker, or a moiety comprising the linker.
[A1-13] The pharmaceutical composition according to any of [A1-1] to [A1-12], wherein a ratio of a K_{D} value of the extracellular binding domain of the chimeric receptor for the antigen binding molecule after cleavage of the linker to a K_{D} value of the extracellular binding domain of the chimeric receptor for the antigen binding molecule before cleavage of the linker (K_{D} (after cleavage)/K_{D} (before cleavage)) is 0.1 or less or 0.01 or less.
[A1-14] The pharmaceutical composition according to any of [A1-1] to [A1-13], wherein the linker that is cleavable by protease comprises a protease cleavage sequence.
[A1-15] The pharmaceutical composition according to any of [A1-1] to [A1-14], wherein the linker that is cleavable by protease comprises a peptide having any of the protease cleavage sequences of SEQ ID NOs: 1 to 725.
[A1-16] The pharmaceutical composition according to any of [A1-1] to [A1-15], wherein the linker that is cleavable by protease further comprises a flexible linker.
[A1-17] The pharmaceutical composition according to any of [A1-1] to [A1-16], wherein the protease is protease specifically expressed in a target tissue.
[A1-18] The pharmaceutical composition according to any of [A1-1] to [A1-17], wherein the target cell is a tumor cell, and the protease is tumor protease.
[A1-19] The pharmaceutical composition according to any of [A1-1] to [A1-18] for use in the treatment or prevention of a cancer.
[A1-20] The pharmaceutical composition according to [A1-19], wherein the cancer is selected from the group consisting of carcinoma, lymphoma, sarcoma, blastoma and leukemia.
[A1-21] The pharmaceutical composition according to [A1-19], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.
[A1-22] The pharmaceutical composition according to any of [A1-1] to [A1-21] for use in CAR-T therapy.
[A2-1] A chimeric receptor comprising an extracellular binding domain, a transmembrane domain and an intracellular signal transduction domain, wherein the extracellular binding domain binds to an antigen binding molecule obtained from an antigen binding molecule comprising a linker that is cleavable by protease, through the cleavage of the linker by the protease, and the extracellular binding domain binds to a cell expressing an antigen via binding to the antigen binding molecule after cleavage of the linker.
[A2-2] The chimeric receptor according to [A2-1], wherein the extracellular binding domain recognizes the linker cleaved by the protease, a portion of the linker, or a moiety comprising the linker.
[A2-3] The chimeric receptor according to [A2-1] or [A2-2], wherein the transmembrane domain comprises CD28.
[A2-4] The chimeric receptor according to any of [A2-1] to [A2-3], further comprising one or more costimulatory molecules located between the transmembrane domain and the intracellular signal transduction domain.
[A2-5] The chimeric receptor according to [A2-4], wherein the costimulatory molecule is CD3 zeta, CD28, 4-1BB, 4-1BBL, ICOS or OX40.
[A2-6] The chimeric receptor according to any of [A2-1] to [A2-5], wherein the intracellular signal transduction domain comprises CD3 zeta.
[A2-7] The chimeric receptor according to any of [A2-1] to [A2-6], wherein the linker that is cleavable by protease comprises a peptide having any of the protease cleavage sequences of SEQ ID NOs: 1 to 725.
[A2-8] A nucleic acid encoding a chimeric receptor according to any of [A2-1] to [A2-7].
[A2-9] A vector comprising a nucleic acid according to [A2-8].
[A2-10] A cell comprising a vector according to [A2-8].
[A2-11] The cell according to [A2-9], wherein the cell is a T cell.
[A2-12] The cell according to [A2-11], wherein the T cell is a CD4⁺ or CD8⁺ T cell.
[A2-13] The cell according to [A2-11], wherein the T cell is a regulatory T cell (Treg) or a follicular regulatory T cell (TFR).
[A3-1] An antigen binding molecule comprising a linker that is cleavable by protease, wherein
   the antigen binding molecule after cleavage of the linker has the ability to bind to an antigen, and an extracellular binding domain of a chimeric receptor is capable of binding to a target cell expressing the antigen via binding to the antigen binding molecule after cleavage of the linker.
[A3-2] The antigen binding molecule according to [A3-1], wherein the linker that is cleavable by protease comprises a peptide having any of the protease cleavage sequences of SEQ ID NOs: 1 to 725.

[B1-1] A pharmaceutical composition comprising a bispecific antibody, for use in combination with the administration of an antigen binding molecule, wherein
   the antigen binding molecule comprises a linker that is cleavable by protease, and has the ability to bind to a target antigen after cleavage of the linker,
   the bispecific antibody comprises antibody variable regions having binding activity against the antigen binding molecule after cleavage of the linker by the protease, and antibody variable regions having binding activity against a molecule expressed on T cell surface, and
   the bispecific antibody is capable of binding to a cell expressing the target antigen via binding to the antigen binding molecule after cleavage of the linker.
[B1-2] A pharmaceutical composition comprising an antigen binding molecule, for use in combination with the administration of a bispecific antibody, wherein
   the antigen binding molecule comprises a linker that is cleavable by protease, and has the ability to bind to a target antigen after cleavage of the linker,
   the bispecific antibody comprises antibody variable regions having binding activity against the antigen binding molecule after cleavage of the linker by the protease, and antibody variable regions having binding activity against a molecule expressed on T cell surface, and
   the bispecific antibody is capable of binding to a cell expressing the target antigen via binding to the antigen binding molecule after cleavage of the linker.
[B1-3] The pharmaceutical composition according to [B1-1] or [B1-2], wherein a ratio of a K_{D} value of the antigen binding molecule after cleavage of the linker for the antigen to a K_{D} value of the antigen binding molecule before cleavage of the linker for the antigen (K_{D} (after cleavage)/K_{D} (before cleavage)) is 0.1 or less or 0.01 or less.
[B1-4] The pharmaceutical composition according to any of [B1-1] to [B1-3], wherein the antigen binding molecule is an IgG antibody or a heavy chain antibody.
[B1-5] The pharmaceutical composition according to any of [B1-1] to [B1-4], wherein the antigen binding molecule comprises variable and constant regions of an antibody, and a linker that is cleavable by protease, and the antigen binding molecule obtained through the cleavage of the linker by the protease comprises the variable region or an antigen binding fragment thereof.
[B1-6] The pharmaceutical composition according to any of [B1-1] to [B1-4], wherein the antigen binding molecule comprises variable and constant regions of an antibody, and a linker that is cleavable by protease, and the linker is located near the boundary between the variable region and the constant region or near the boundary between CH1 and CH2 in the constant region.
[B1-7] The pharmaceutical composition according to any of [B1-1] to [B1-6], wherein the antigen binding molecule is an antibody comprising a linker that is cleavable by protease, and the antigen binding molecule after cleavage of the linker is VL or VH of the antibody, or an antigen binding fragment thereof.
[B1-8] The pharmaceutical composition according to any of [B1-1] to [B1-6], wherein the antigen binding molecule is a heavy chain antibody comprising a linker that is cleavable by protease, and the antigen binding molecule after cleavage of the linker is VHH of the heavy chain antibody.
[B1-9] The pharmaceutical composition according to any of [B1-1] to [B1-8], wherein the antigen binding molecule after cleavage of the linker is scFv, Fv, Fab, Fab', F(ab')₂, VH or VHH
[B1-10] The pharmaceutical composition according to any of [B1-1] to [B1-9], wherein the bispecific antibody recognizes the cleaved linker, a portion of the linker, or a moiety comprising the linker.
[B1-11] The pharmaceutical composition according to any of [B1-1] to [B1-10], wherein a ratio of a K_{D} value of the bispecific antibody for the antigen binding molecule after cleavage of the linker to a K_{D} value of the bispecific antibody for the antigen binding molecule before cleavage of the linker (K_{D} (after cleavage)/K_{D} (before cleavage)) is 0.1 or less or 0.01 or less.
[B1-12] The pharmaceutical composition according to any of [B1-1] to [B1-11], wherein the linker that is cleavable by protease comprises a protease cleavage sequence.
[B1-13] The pharmaceutical composition according to any of [B1-1] to [B1-12], wherein the linker that is cleavable by protease comprises a peptide having any of the protease cleavage sequences of SEQ ID NOs: 1 to 725.
[B1-14] The pharmaceutical composition according to any of [B1-1] to [B1-13], wherein the linker that is cleavable by protease further comprises a flexible linker.
[B1-15] The pharmaceutical composition according to any of [B1-1] to [B1-14], wherein the protease is protease specifically expressed in a target tissue.
[B1-16] The pharmaceutical composition according to any of [B1-1] to [B1-15], wherein the target cell is a tumor cell, and the protease is tumor protease.
[B1-17] The pharmaceutical composition according to any of [B1-1] to [B1-16] for use in the treatment or prevention of a cancer.
[B1-18] The pharmaceutical composition according to [B1-17], wherein the cancer is selected from the group consisting of carcinoma, lymphoma, sarcoma, blastoma and leukemia.
[B1-19] The pharmaceutical composition according to [B1-17], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.
[B1-20] The pharmaceutical composition according to any of [B1-1] to [B1-19] for use in bispecific antibody therapy.

[B2-1] A bispecific antibody comprising: 1) first antibody variable regions having binding activity against a molecule expressed on T cell surface; and 2) second antibody variable regions having binding activity against an antigen binding molecule obtained from an antigen binding molecule comprising a linker that is cleavable by protease, after cleavage of the linker by the protease, wherein
   the antigen binding molecule has binding activity against an antigen expressed on target cell surface after cleavage of the linker by the protease, and the bispecific antibody is capable of binding to the target cell via binding to the antigen binding molecule after cleavage of the linker.
[B2-2] The bispecific antibody according to [B2-1], wherein the molecule expressed on T cell surface is CD3.
[B2-3] The bispecific antibody according to [B2-1], wherein the antibody variable regions having binding activity against a molecule expressed on T cell surface binds to CD3 epsilon.
[B2-4] The bispecific antibody according to any of [B2-1] to [B2-3], wherein the bispecific antibody recognizes the linker cleaved by the protease, a portion of the linker, or a moiety comprising the linker.
[B2-5] The bispecific antibody according to any of [B2-1] to [B2-4], wherein the bispecific antibody comprises an Fc region having reduced binding activity against an Fc gamma receptor.
[B2-6] The bispecific antibody according to any of [B1-1] to [B1-5], wherein the linker that is cleavable by protease comprises a peptide having any protease cleavage sequence.
[B2-7] The bispecific antibody according to any of [B1-1] to [Bl-5], wherein the linker that is cleavable by protease comprises a peptide having any of the protease cleavage sequences of SEQ ID NOs: 1 to 725.
[B2-8] The bispecific antibody according to any of [B1-1] to [Bl-7], wherein the linker that is cleavable by protease further comprises a flexible linker.
[B2-9] The bispecific antibody according to any of [B2-1] to [B2-8], wherein the bispecific antibody is an IgG antibody.

[B3-1] A nucleic acid encoding a bispecific antibody according to any of [B2-1] to [B2-9] .
[B3-2] A vector comprising a nucleic acid according to [B3-1].
[B3-3] A cell comprising a vector according to [B3-2].
[B3-4] A method for producing a bispecific antibody, comprising culturing a cell according to [B3-3] and recovering a bispecific antibody from the culture supernatant.

[C1-1] A pharmaceutical composition comprising an IgG antibody having enhanced antibody-dependent cellular cytotoxicity (ADCC), for use in combination with the administration of an antigen binding molecule, wherein
   the antigen binding molecule comprises a linker that is cleavable by protease, and has binding activity against an antigen expressed on target cell surface after cleavage of the linker by the protease,
   the IgG antibody comprises antibody variable regions having binding activity against the antigen binding molecule after cleavage of the linker by the protease, and
   the IgG antibody is capable of binding to the target cell via binding to the antigen binding molecule after cleavage of the linker.
[C1-2] A pharmaceutical composition comprising an antigen binding molecule, for use in combination with the administration of an IgG antibody having enhanced antibody-dependent cellular cytotoxicity (ADCC), wherein
   the antigen binding molecule comprises a linker that is cleavable by protease, and has binding activity against an antigen expressed on target cell surface after cleavage of the linker by the protease,
   the IgG comprises antibody variable regions having binding activity against the antigen binding molecule after cleavage of the linker by the protease, and
   the IgG antibody is capable of binding to the target cell via binding to the antigen binding molecule after cleavage of the linker.
[C1-3] The pharmaceutical composition according to [C1-1] or [C1-2], wherein a ratio of a K_{D} value of the antigen binding molecule after cleavage of the linker for the antigen to a K_{D} value of the antigen binding molecule before cleavage of the linker for the antigen (K_{D} (after cleavage)/K_{D} (before cleavage)) is 0.1 or less or 0.01 or less.
[C1-4] The pharmaceutical composition according to any of [C1-1] to [C1-3], wherein the antigen binding molecule is an IgG antibody, an IgG antibody-like molecule, a heavy chain antibody or a single domain antibody.
[Cl-5] The pharmaceutical composition according to any of [C1-1] to [Cl-4], wherein the antigen binding molecule comprises variable and constant regions of an antibody, and a linker that is cleavable by protease, and the antigen binding molecule obtained through the cleavage of the linker by the protease comprises the variable region or an antigen binding fragment thereof.
[CI-6] The pharmaceutical composition according to any of [C1-1] to [C1-4], wherein the antigen binding molecule comprises variable and constant regions of an antibody, and a linker that is cleavable by protease, and the linker is located near the boundary between CH1 and CH2 in the constant region.
[C1-7] The pharmaceutical composition according to any of [C1-1] to [C1-6], wherein the antigen binding molecule is an antibody comprising a linker that is cleavable by protease, and the antigen binding molecule after cleavage of the linker is VL or VH of the antibody, or an antigen binding fragment thereof.
[C1-8] The pharmaceutical composition according to any of [C1-1] to [C1-6], wherein the antigen binding molecule is a heavy chain antibody comprising a linker that is cleavable by protease, and the antigen binding molecule after cleavage of the linker is VHH of the heavy chain antibody.
[C1-9] The pharmaceutical composition according to any of [C1-1] to [C1-8], wherein the antigen binding molecule after cleavage of the linker is scFv, Fv, Fab, Fab', F(ab')₂, VH or VHH
[C1-10] The pharmaceutical composition according to any of [C1-1] to [C1-9], wherein the IgG antibody recognizes the cleaved linker, a portion of the linker, or a moiety comprising the linker.
[C1-11] The pharmaceutical composition according to any of [C1-1] to [C1-10], wherein a ratio of a K_{D} value of the IgG antibody for the antigen binding molecule after cleavage of the linker to a K_{D} value of the IgG antibody for the antigen binding molecule before cleavage of the linker (K_{D} (after cleavage)/K_{D} (before cleavage)) is 0.1 or less or 0.01 or less.
[C1-12] The pharmaceutical composition according to any of [C1-1] to [C1-11], wherein the linker that is cleavable by protease comprises a protease cleavage sequence.
[C1-13] The pharmaceutical composition according to any of [C1-1] to [C1-12], wherein the linker that is cleavable by protease comprises a peptide having any of the protease cleavage sequences of SEQ ID NOs: 1 to 725.
[C1-14] The pharmaceutical composition according to any of [C1-1] to [C1-13], wherein the linker that is cleavable by protease further comprises a flexible linker.
[Cl-14] The pharmaceutical composition according to any of [C1-1] to [C1-13], wherein the protease is protease specifically expressed in a target tissue.
[C1-15] The pharmaceutical composition according to any of [C1-1] to [C1-14], wherein the target cell is a tumor cell, and the protease is tumor protease.
[C1-16] The pharmaceutical composition according to any of [C1-1] to [C1-15] for use in treatment or prevention using antibody-dependent cellular cytotoxicity (ADCC) or antibody-dependent cellular phagocytosis (ADCP).
[Cl-17] The pharmaceutical composition according to any of [C1-1] to [Cl-16] for use in the treatment or prevention of a cancer.
[Cl-18] The pharmaceutical composition according to [CI-17], wherein the cancer is selected from the group consisting of carcinoma, lymphoma, sarcoma, blastoma and leukemia.
[C1-19] The pharmaceutical composition according to [CI-17], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.
   [Cl-20] The pharmaceutical composition according to any of [C1-1] to [CI-19] for use in IgG antibody therapy.

[C2-1] An IgG antibody comprising antibody variable regions having binding activity against an antigen binding molecule obtained from an antigen binding molecule comprising a linker that is cleavable by protease, after cleavage of the linker by the protease, wherein
   the antigen binding molecule has binding activity against an antigen expressed on target cell surface after cleavage of the linker by the protease, and the IgG antibody is capable of binding to the target cell via binding to the antigen binding molecule after cleavage of the linker.
[C2-2] The IgG antibody according to [C2-1], wherein the IgG antibody has enhanced antibody-dependent cellular cytotoxicity (ADCC).
[C2-3] The IgG antibody according to [C2-1] or [C2-2], wherein the IgG antibody comprises an Fc region having increased binding activity against an Fc gamma receptor.
[C2-4] The pharmaceutical composition according to any of [C1-1] to [CI-3], wherein the linker that is cleavable by protease comprises a protease cleavage sequence.
[C2-5] The pharmaceutical composition according to any of [C1-1] to [C1-4], wherein the linker that is cleavable by protease comprises a peptide having any of the protease cleavage sequences of SEQ ID NOs: 1 to 725.
[C2-6] The pharmaceutical composition according to any of [C1-1] to [Cl-5], wherein the linker that is cleavable by protease further comprises a flexible linker.

[C3-1] A nucleic acid encoding an IgG antibody according to any of [C2-1] to [C2-6].
[C3-2] A vector comprising a nucleic acid according to [C3-1].
[C3-3] A cell comprising a vector according to [C3-2].
[C3-4] A method for producing an IgG antibody, comprising culturing a cell according to [C3-3] and recovering an IgG antibody from the culture supernatant.

[D1-1] A pharmaceutical composition comprising a secondary molecule, for use in combination with the administration of a primary molecule, wherein
   the primary molecule comprises a linker that is cleavable by protease, and has the ability to bind to a target antigen after cleavage of the linker, and
   the secondary molecule has the ability to bind to the primary molecule after cleavage of the linker, and is capable of binding to a cell expressing the target antigen via binding to the primary molecule after cleavage of the linker.
[Dl-2] A pharmaceutical composition comprising a primary molecule, for use in combination with the administration of a secondary molecule, wherein
   the primary molecule comprises a linker that is cleavable by protease, and has the ability to bind to a target antigen after cleavage of the linker, and
   the secondary molecule has the ability to bind to the primary molecule after cleavage of the linker, and is capable of binding to a cell expressing the target antigen via binding to the primary molecule after cleavage of the linker.
[Dl-3] The pharmaceutical composition according to [D1-1] or [Dl-2], wherein the primary molecule is an antigen binding molecule, and the secondary molecule is a bispecific antibody, a chimeric receptor, or an IgG antibody having enhanced antibody-dependent cellular cytotoxicity.
[Dl-4] The pharmaceutical composition according to any of [D1-1] to [Dl-3], wherein the pharmaceutical composition is a pharmaceutical composition according to any of [A1-1] to [Al-21], [B1-1] to [B1-20], and [C1-1] to [C1-20].

### BRIEF DESCRIPTION OF DRAWINGS

Figure 1 shows the concept of antibody technology (Probody(R)) of expanding tissue specificity and therapeutic window by imparting sensitivity to protease having an expression level elevated at a lesion site such as a cancer tissue or an inflammatory tissue to an antibody.
Figure 2 is a schematic view showing the induction of TDCC activity by an antibody specifically recognizing an antigen resulting from the cleavage of a linker by protease.
Figure 3 is a schematic view showing the induction of ADCC activity by an antibody specifically recognizing an antigen resulting from the cleavage of a linker by protease.
Figure 4 is a schematic view showing the induction of TDCC activity by an antibody specifically recognizing an antigen resulting from the cleavage of a linker by protease.
Figure 5 is a schematic view showing the induction of ADCC activity by an antibody specifically recognizing an antigen resulting from the cleavage of a linker by protease.

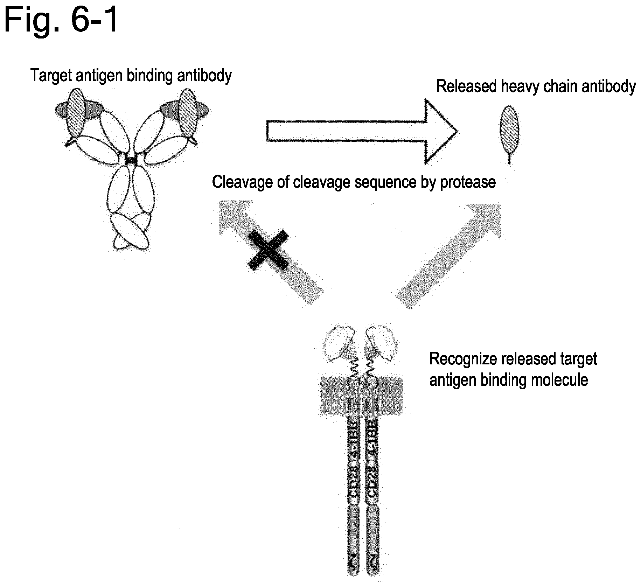

Figure 6-1 is a schematic view showing the induction of cytotoxic activity by CAR-T specifically recognizing an antigen binding molecule cleaved by protease.

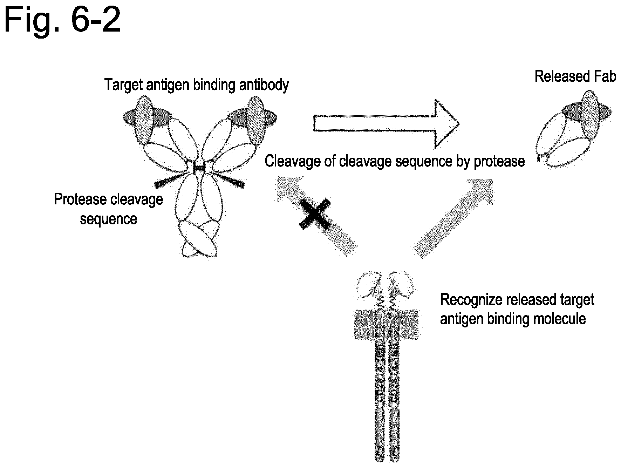

Figure 6-2 is a schematic view showing the induction of cytotoxic activity by CAR-T specifically recognizing an antigen exposed by protease cleavage.
Figure 7 shows results of the *in vitro* cleavage of an antibody harboring a protease cleavage sequence (partial collagen II sequence) by protease (MMP13). The leftmost to rightmost lanes correspond to wells 1 to 5. Well 1 depicts results about MWM. Wells 2 and 3 depict results about an antigen binding molecule containing no cleavage sequence before and after reaction, respectively. Wells 4 and 5 depict results about the antigen binding molecule comprising the cleavage sequence before and after reaction, respectively, when MMP13 was added.
Figure 8 shows results of the *in vitro* cleavage of an antibody recognizing a tumor antigen by protease (IdeS). The leftmost to rightmost lanes correspond to wells 6 to 10. Well 6 depicts results about MWM. Wells 7 and 8 depict results about an antigen binding molecule containing no cleavage sequence before and after reaction, respectively. Wells 9 and 10 depict results about the antigen binding molecule comprising the cleavage sequence before and after reaction, respectively, when IdeS was added.
Figure 9 (left) shows results of the treatment with a tumor cell line of an antigen binding molecule (antibody) harboring a sequence (partial collagen II sequence) that is cleavable by protease. Figure 9 (right) shows results of the treatment with a tumor cell line of an antigen binding molecule harboring no sequence (partial collagen II sequence) that is cleavable by protease.
Figure 10 shows Biacore measurement results indicating that an anti-cleaved linker anti-CD3 bispecific antibody binds to an antigen binding molecule obtained through the cleavage of a linker (partial collagen II sequence) by protease.
Figure 11 shows Biacore measurement results indicating that an anti-cleaved linker anti-CD3 bispecific antibody binds to an antigen binding molecule (IgG1) cleaved by protease.
Figure 12 shows Biacore measurement results indicating that an antibody with enhanced ADCC activity binds to an antigen binding molecule obtained through the cleavage of a linker (partial collagen II sequence) by protease.
Figure 13 shows Biacore measurement results indicating that an antibody with enhanced ADCC activity binds to an antigen binding molecule (IgG1) cleaved by protease.
Figure 14 shows results of Jurkat reporter gene assay using an antigen binding molecule (anti-GPC3 antibody, IgG1) obtained through the cleavage of a linker (partial collagen II sequence) by protease and an anti-linker anti-CD3 bispecific antibody.
Figure 15 shows results of Jurkat reporter gene assay using an antigen binding molecule (anti-GPC3 antibody, IgG1) obtained through the cleavage of a linker (partial collagen II sequence) by protease and an anti-linker anti-CD3 bispecific antibody.
Figure 16 shows results of Jurkat reporter gene assay using an antigen binding molecule obtained through the cleavage of a linker (partial collagen II sequence) by protease and an antibody with enhanced ADCC activity.
Figure 17 shows results of Jurkat reporter gene assay using an antigen binding molecule obtained through the cleavage of a linker (partial collagen II sequence) by protease and an antibody with enhanced ADCC activity.
Figure 18 shows results of cytotoxicity assay on human PBMC using an anti-linker anti-CD3 bispecific antibody recognizing an antigen binding molecule obtained through the cleavage of a linker (partial collagen II sequence) by protease, and an anti-GPC3 antibody harboring a protease cleavage sequence.
Figure 19 is a schematic view showing a vector construct and the order of arrangement of components in frame units from the 5' end to the 3' end.
Figure 20 shows results of evaluating cytotoxic activity against PC-10 in which an MMP-cleavable linker can be cleaved. The percentage of residual cancer cells was calculated as the ratio of CD45" fraction cells to live cells. The abscissa depicts the concentration of an added antigen binding molecule.
Figure 21 shows results of evaluating cytotoxic activity against KYSE70 in which an MMP-cleavable linker can rarely be cleaved. The percentage of residual cancer cells was calculated as the ratio of CD45" fraction cells to live cells. The abscissa depicts the concentration of an added antigen binding molecule.

### DESCRIPTION OF EMBODIMENTS

Other features and advantages of the present disclosure will be evident from the detailed description given below. However, it is obvious to those skilled in the art from this detailed description that various changes or modifications can be made in the present disclosure without departing from the spirit and scope of the present disclosure. Therefore, the detailed description and specific examples showing preferred embodiments of the present disclosure should be construed as being given merely for illustrative purposes.

Hereinafter, embodiments of the present disclosure will be described with reference to the drawings.

The terms "substantially" and "approximately" or "about" mean a reasonable amount of deviation of the modified term such that end results are not significantly changed, i.e., an acceptable error range of a particular value determined by those skilled in the art. For example, the term "approximately" may mean acceptable standard deviation for practice in the art. Alternatively, the term "approximately" may mean up to ±20%, preferably up to ±10%, more preferably up to ±5%, further preferably up to ±1%, of a certain value. Alternatively, this term, particularly, in a biological system or process, may mean within a single digit, preferably within twice, from a certain value. When a particular value is described in the present specification and the appended claims, the term "approximately" is implicated therein and means an acceptable error range for the particular value in the context, unless otherwise specified.

As used in the English translated sentences of the present specification and the appended claims, the singular forms "a", "an" and "the" include a plurality of references unless the content clearly dictates otherwise. It should also be noted that the term "or" is generally employed in its sense including "and/or" unless the content clearly dictates otherwise.

The recitation of numerical ranges by endpoints in the present disclosure includes all numbers and fractions subsumed within that range (e.g., 1 to 5 includes 1, 1.5, 2, 2.75, 3, 3.90, 4, and 5). It should also be understood that all numbers and fractions are presumed to be modified by the term "approximately". However, when it is evident that numerical values indicated by a numerical range are integers, the numerical range is construed as reciting integers included in this range in a limited manner. In such a case, for example, 1 to 5, is construed as reciting 1, 2, 3, 4, and 5 in a limited manner.

Further, the definitions and embodiments described in particular sections are intended to be applicable to other embodiments herein described for which they are suitable as would be understood by those skilled in the art. For example, in the following passages, different aspects of the present disclosure are defined in more detail. Each aspect thus defined may be combined with any other aspect or aspects unless clearly indicated to the contrary. In particular, any feature indicated as being preferred or advantageous may be combined with any other feature or features indicated as being preferred or advantageous.

In the present disclosure, an antigen binding molecule comprising a region binding to an antigen expressed in target cells ("antigen binding domain"), and a linker that is cleavable by protease is also referred to as a "primary molecule". The primary molecule releases an antigen binding fragment binding to an antigen ("target antigen") expressed in target cells or lesion cells through the cleavage of the linker by the protease. An antigen binding molecule resulting from the cleavage of the linker by the protease is referred to as an "antigen binding molecule obtained through the cleavage of the linker" or an "antigen binding molecule after cleavage of the linker", and comprises an antigen binding domain and a portion of the cleaved linker. A polypeptide that induces cytotoxic activity by bridging target cells to effector cells is also referred to as a "secondary molecule". Examples of the secondary molecule include an antibody having ADCC activity and having antibody variable regions capable of binding to the antigen binding molecule obtained through the cleavage of the linker, a T cell-redirecting antibody having antibody variable regions capable of binding to the antigen binding molecule obtained through the cleavage of the linker and antibody variable regions capable of binding to a T cell receptor complex, and a chimeric receptor having an extracellular domain capable of binding to the antigen binding molecule obtained through the cleavage of the linker. The linker contained in the antigen binding molecule comprises a protease cleavage sequence and has a cleavage site that is cleavable by protease. A linker consisting of a peptide having a protease cleavage sequence is also referred to as a protease-cleavable linker.

In one aspect, the antigen binding molecule (primary molecule) comprising a linker that is cleavable by protease is, for example, an antibody, more specifically an IgG antibody or a heavy chain antibody comprising a linker that is cleavable by protease, and is more preferably an IgG1 antibody, a *Camelidae* heavy chain antibody (hcIgG) or a shark heavy chain antibody (IgNAR).

In one aspect, the antigen binding molecule resulting from the cleavage of the linker by the protease is Fv, Fab, Fab', Fab'-SH, F(ab')₂, a minibody, a single chain antibody molecule (e.g., scFv), VHH, or VH, more specifically, Fab, scFv, VHH, or VH

The polypeptide according to the present invention usually refers to a peptide having a length on the order of 4 amino acids or longer, and a protein. Also, the polypeptide according to the present invention is usually a polypeptide consisting of an artificially designed sequence, but is not limited thereto. For example, an organism-derived polypeptide may be used. Alternatively, the polypeptide according to the present invention may be any of a natural polypeptide, a synthetic polypeptide, a recombinant polypeptide, and the like. Furthermore, fragments of these polypeptides are also included in the polypeptide of the present invention.

In the present specification, each amino acid is indicated by one-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V. For expressing an amino acid located at a particular position, an expression using a number representing the particular position in combination with the one-letter code or the three-letter code of the amino acid can be appropriately used. For example, an amino acid 37V, which is an amino acid contained in a single-domain antibody, represents Val located at position 37 defined by the Kabat numbering.

For the alteration of an amino acid in the amino acid sequence of a polypeptide such as an antibody, a method known in the art such as site-directed mutagenesis (Kunkel et al. (Proc. Natl. Acad. Sci. USA (1985) 82, 488-492)) or overlap extension PCR can be appropriately adopted. A plurality of methods known in the art can also be adopted as alteration methods for substituting an amino acid by an amino acid other than a natural amino acid (Annu. Rev. Biophys. Biomol. Struct. (2006) 35, 225-249; and Proc. Natl. Acad. Sci. U.S.A. (2003) 100 (11), 6353-6357). For example, a tRNA-containing cell-free translation system (Clover Direct (Protein Express)) having a non-natural amino acid bound with amber suppressor tRNA complementary to UAG codon (amber codon), which is a stop codon, is also preferably used. In the present specification, examples of the alteration include, but are not limited to, substitution.

In the present specification, the term "and/or" that is used in representing an amino acid alteration position is meant to include every combination appropriately represented by "and" and "or". Specifically, for example, the phrase "amino acids at positions 37, 45, and/or 47 are substituted" includes the following variations of amino acid alteration position: (a) position 37, (b) position 45, (c) position 47, (d) positions 37 and 45, (e) positions 37 and 47, (f) positions 45 and 47, and (g) positions 37, 45 and 47.

In the present specification, expression in which the one-letter codes or three-letter-codes of amino acids before and after alteration are used previous and next to a number representing a particular position can be appropriately used for representing amino acid alteration. For example, an alteration F37V or Phe37Val used for substituting an amino acid contained in an antibody variable region or a single-domain antibody represents the substitution of Phe at position 37 defined by the Kabat numbering by Val. Specifically, the number represents an amino acid position defined by the Kabat numbering; the one-letter code or three-letter code of the amino acid previous to the number represents the amino acid before the substitution; and the one-letter code or three-letter code of the amino acid next to the number represents the amino acid after the substitution. Likewise, an alteration P238A or Pro238Ala used for substituting an amino acid in a Fc region contained in an antibody constant region represents the substitution of Pro at position 238 defined by the EU numbering by Ala. Specifically, the number represents an amino acid position defined by the EU numbering; the one-letter code or three-letter code of the amino acid previous to the number represents the amino acid before the substitution; and the one-letter code or three-letter code of the amino acid next to the number represents the amino acid after the substitution.

In the present specification, the term "antibody" is used in the broadest sense and encompasses various antibody structures including, but are not limited to, a monoclonal antibody, a polyclonal antibody, a multispecific antibody (e.g., a bispecific antibody), a single-domain antibody, and an antibody fragment as long as the antibody exhibits the desired antigen binding activity.

The "antibody fragment" refers to a molecule, other than a complete antibody, containing a portion of the complete antibody and binding to an antigen to which the complete antibody binds. Examples of the antibody fragment include, but are not limited to, Fv, Fab, Fab', Fab'-SH, F(ab')₂, diabody, linear antibodies, single-chain antibody molecules (e.g., scFv), and multispecific antibodies formed from antibody fragments.

The terms "full-length antibody", "complete antibody", and "whole antibody" are used interchangeably with each other in the present specification and refer to an antibody having a structure substantially similar to a natural antibody structure, or having heavy chains containing a Fc region defined in the present specification.

The term "variable region" or "variable domain" refers to a region or a domain of an antibody heavy chain or light chain involved in the binding of the antibody to its antigen. Usually, antibody heavy chain and light chain variable domains (VH and VL, respectively) are structurally similar and each contain 4 conserved framework regions (FRs) and 3 complementarity determining regions (CDRs) (see e.g., Kindt et al., Kuby Immunology, 6th ed., W.H. Freeman and Co., page 91 (2007)). One VH or VL domain may suffice for conferring antigen binding specificity.

The term "complementarity determining region" or "CDR" used in the present specification is hypervariable in the sequence, and/or forms a structurally determined loop ("hypervariable loop"), and/or refers to antigen contact residues ("antigen contacts") or each region of an antibody variable domain. Usually, an antibody contains 6 CDRs: three in VH (H1, H2, and H3), and three in VL (L1, L2, and L3). In the present specification, exemplary CDRs include the following:
(a) hypervariable loops formed at amino acid residues 26 to 32 (L1), 50 to 52 (L2), 91 to 96 (L3), 26 to 32 (HI), 53 to 55 (H2), and 96 to 101 (H3) (Chothia and Lesk, J. Mol. Biol. 196: 901-917 (1987));
(b) CDRs formed at amino acid residues 24 to 34 (L1), 50 to 56 (L2), 89 to 97 (L3), 31 to 35b (HI), 50 to 65 (H2), and 95 to 102 (H3) (Kabat et al., Sequences of Proteins of Immunological Interest, 5th Ed. Public Health Service, National Institutes of Health, Bethesda, MD (1991));
(c) antigen contacts formed at amino acid residues 27c to 36 (L1), 46 to 55 (L2), 89 to 96 (L3), 30 to 35b (HI), 47 to 58 (H2), and 93 to 101 (H3) (MacCallum et al., J. Mol. Biol. 262: 732-745 (1996)); and
(d) a combination of (a), (b), and/or (c) containing HVR amino acid residues 46 to 56 (L2), 47 to 56 (L2), 48 to 56 (L2), 49 to 56 (L2), 26 to 35 (HI), 26 to 35b (HI), 49 to 65 (H2), 93 to 102 (H3), and 94 to 102 (H3).

In the present specification, CDR residues and other residues (e.g., FR residues) in a variable domain are numbered according to Kabat et al. (supra), unless otherwise specified.

The term "framework" or "FR" refers to variable domain residues other than complementarity determining region (CDR) residues. FRs in a variable domain consist of 4 FR domains: FR1, FR2, FR3, and FR4. Accordingly, the sequences of CDRs and FRs usually appear in VH (or VL) in the following order: FR1-H1 (L1)-FR2-H2 (L2)-FR3-H3 (L3)-FR4.

In the present specification, the term "constant region" or "constant domain" refers to a region or a domain other than variable regions in an antibody. For example, an IgG antibody is a heterotetrameric glycoprotein of approximately 150,000 Da constituted by two identical light chains and two identical heavy chains connected through disulfide bonds. Each heavy chain has a variable region (VH) also called variable heavy chain domain or heavy chain variable domain, followed by a heavy chain constant region (CH) containing a CH1 domain, a hinge region, a CH2 domain, and a CH3 domain, from the N terminus toward the C terminus. Likewise, each light chain has a variable region (VL) also called variable light chain domain or light chain variable domain, followed by a constant light chain (CL) domain, from the N terminus toward the C terminus. The light chains of natural antibodies may be attributed to one of two types called kappa (κ) and lambda (λ) on the basis of the amino acid sequences of their constant domains.

In the present specification, the term "Fc region" is used for defining the C-terminal region of immunoglobulin heavy chains, including at least a portion of constant regions. This term includes a Fc region having a natural sequence and a mutant Fc region. In one embodiment, the heavy chain Fc region of human IgG1 spans from Cys226 or Pro230 to the carboxyl terminus of the heavy chain. However, the C-terminal lysine (Lys447) or glycine-lysine (Gly446-Lys447) of the Fc region may be present or absent. In the present specification, amino acid residues in a Fc region or a constant region are numbered according to the EU numbering system (also called EU index) described in Kabat et al., Sequences of Proteins of Immunological Interest, 5th Ed. Public Health Service, National Institutes of Health, Bethesda, MD 1991, unless otherwise specified.

The "class" of an antibody refers to the type of a constant domain or a constant region carried by the heavy chain of the antibody. Antibodies have 5 major classes: IgA, IgD, IgE, IgG, and IgM. Some of these classes may be further divided into subclasses (isotypes), for example, IgG1, IgG2, IgG3, IgG4, IgA1, and IgA2. Heavy chain constant domains corresponding to immunoglobulins of different classes are called alpha, delta, epsilon, gamma, and mu, respectively.

In the present specification, the "antigen binding domain" is limited only by binding to the antigen of interest. The antigen binding domain can be a domain having any structure as long as the domain used binds to the antigen of interest. Examples of such a domain include, but are not limited to, an antibody heavy chain variable region (VH), an antibody light chain variable region (VL), a single-domain antibody (sdAb), a module called A domain of approximately 35 amino acids contained in an *in vivo* cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854).

Preferred examples of the antigen binding domain of the present invention include an antigen binding domain that can exert an antigen binding function by a molecule constituted only by the antigen binding domain, and an antigen binding domain that can exert an antigen binding function by itself after being released from an additional peptide linked thereto. Examples of such an antigen binding domain include, but are not limited to, a single-domain antibody, scFv, Fv, Fab, Fab', and F(ab')₂.

One preferred example of the antigen binding domain of the present invention includes an antigen binding domain having a molecular weight of 60 kDa or smaller. Examples of such an antigen binding domain include, but are not limited to, single-domain antibodies, scFv, Fab, and Fab'. The antigen binding domain having a molecular weight of 60 kDa or smaller is usually likely to cause clearance by the kidney when existing as a monomer in blood (see J Biol Chem. 1988 Oct 15; 263 (29): 15064-70).

From another viewpoint, one preferred example of the antigen binding domain of the present invention includes an antigen binding domain having a half-life in blood of 12 hours or shorter. Examples of such an antigen binding domain include, but are not limited to, single-domain antibodies, scFv, Fab, and Fab'.

One preferred example of the antigen binding domain of the present invention includes a single-domain antibody (sdAb).

In the present specification, the term "single-domain antibody" is not limited by its structure as long as the domain can exert antigen binding activity by itself. It is known that a general antibody, for example, an IgG antibody, exhibits antigen binding activity in a state where a variable region is formed by the pairing of VH and VL, whereas the own domain structure of the single-domain antibody can exert antigen binding activity by itself without pairing with another domain. Usually, the single-domain antibody has a relatively low molecular weight and exists in the form of a monomer.

Examples of the single-domain antibody include, but are not limited to, antigen binding molecules congenitally lacking a light chain, such as VHH of an animal of the family *Camelidae* and shark V_{NAR}, and antibody fragments containing the whole or a portion of an antibody VH domain or the whole or a portion of an antibody VL domain. Examples of the single-domain antibody which is an antibody fragment containing the whole or a portion of an antibody VH or VL domain include, but are not limited to, artificially prepared single-domain antibodies originating from human antibody VH or human antibody VL as described in U.S. Patent No. 6,248,516 B1, etc. In some embodiments of the present invention, one single-domain antibody has three CDRs (CDR1, CDR2 and CDR3).

When the single domain antibody is VHH or a single domain VH antibody, exemplary CDRs of the single domain antibody include the following:
(a) hypervariable loops formed at amino acid residues 26-32 (CDR1), 53-55 (CDR2), and 96-101 (CDR3) (Chothia and Lesk, J. Mol. Biol. 196: 901-917 (1987));
(b) CDRs formed at amino acid residues 31-35b (CDR1), 50-65 (CDR2), and 95-102 (CDR3) (Kabat et al., Sequences of Proteins of Immunological Interest, 5th Ed. Public Health Service, National Institutes of Health, Bethesda, MD (1991));
(c) antigen contacts formed at amino acid residues 30-35b (CDR1), 47-58 (CDR2), and 93-101 (CDR3) (MacCallum et al., J. Mol. Biol. 262: 732-745 (1996)); and
(d) a combination of (a), (b), and/or (c) containing CDR amino acid residues 26-35 (CDR1), 26-35b (CDR1), 49-65 (CDR2), 93-102 (CDR3), or 94-102 (CDR3).

When the single domain antibody is a single domain VL antibody, exemplary CDRs of the single domain antibody include the following:
(a) hypervariable loops formed at amino acid residues 26-32 (CDR1), 50-52 (CDR2), and 91-96 (CDR3) (Chothia and Lesk, J. Mol. Biol. 196: 901-917 (1987));
(b) CDRs formed at amino acid residues 24-34 (CDR1), 50-56 (CDR2), and 89-97 (CDR3) (Kabat et al., Sequences of Proteins of Immunological Interest, 5th Ed. Public Health Service, National Institutes of Health, Bethesda, MD (1991));
(c) antigen contacts formed at amino acid residues 27c-36 (CDR1), 46-55 (CDR2), and 89-96 (CDR3) (MacCallum et al., J. Mol. Biol. 262: 732-745 (1996)); and
(d) a combination of (a), (b), and/or (c) containing CDR amino acid residues 46-56 (CDR2), 47-56 (CDR2), 48-56 (CDR2), or 49-56 (CDR2).

In the present specification, CDR residues and other residues (e.g., FR residues) in a variable domain are numbered according to Kabat et al. (supra), unless otherwise specified.

The single-domain antibody can be obtained from an animal capable of producing the single-domain antibody or by the immunization of the animal capable of producing the single-domain antibody. Examples of the animal capable of producing the single-domain antibody include, but are not limited to, animals of the family *Camelidae,* and transgenic animals harboring a gene capable of raising the single-domain antibody. The animals of the family *Camelidae* include camels, lamas, alpacas, one-hump camels and guanacos, etc. Examples of the transgenic animals harboring a gene capable of raising the single-domain antibody include, but are not limited to, transgenic animals described in International Publication No. WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody of the present invention.

Alternatively, the single-domain antibody can be obtained by ELISA, panning, or the like from a polypeptide library containing single-domain antibodies. Examples of the polypeptide library containing single-domain antibodies include, but are not limited to, naive antibody libraries obtained from various animals or humans (e.g., Methods in Molecular Biology 2012 911 (65-78); and Biochimica et Biophysica Acta - Proteins and Proteomics 2006 1764: 8 (1307-1319)), antibody libraries obtained by the immunization of various animals (e.g., Journal of Applied Microbiology 2014 117: 2 (528-536)), and synthetic antibody libraries prepared from antibody genes of various animals or humans (e.g., Journal of Biomolecular Screening 2016 21: 1 (35-43); Journal of Biological Chemistry 2016 291:24 (12641-12657); and AIDS 2016 30: 11 (1691-1701)).

In the present specification, the "antigen" is limited only by containing an epitope to which the antigen binding domain binds. Preferred examples of the antigen include, but are not limited to, animal- or human-derived peptides, polypeptides, and proteins. The target antigen according to the present invention is an antigen for use in the treatment of a disease caused by a target tissue. Preferred examples thereof include, but are not limited to, molecules expressed on the surface of target cells (e.g., cancer cells and inflammatory cells), molecules expressed on the surface of other cells in tissues containing target cells, molecules expressed on the surface of cells having an immunological role against target cells and tissues containing target cells, and large molecules present in the stromata of tissues containing target cells. Examples of the target antigen can include the following antigens.

Examples of the antigen can include the following molecules: 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic factor, av/b3 integrin, Axl, b2M, B7-1, B7-2, B7-H, B-lymphocyte stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer-associated antigens, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, botulinum toxin, *Clostridium perfringens* toxin, CKb8-1, CLC, CMV, CMVUL, CNTF, CNTN-1, COX, C-Ret, CRG-2, CT-1, CTACK, CTGF, CTLA-4, PD1, PDL1, LAG3, TIM3, galectin-9, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor-associated antigens, DAN, DCC, DcR3, DC-SIGN, decay accelerating factor, des(1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast-activating protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle-stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha 1, GFR-alpha 2, GFR-alpha 3, GITR, glucagon, Glut4, glycoprotein IIb/IIIa (GPIIb/IIIa), GM-CSF, gp130, gp72, GRO, growth hormone-releasing factor, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high-molecular-weight melanoma-associated antigen (HMW-MAA), HIV gp120, HIV IIIB gp 120 V3 loop, HLA, HLA-DR, HM1.24, HMFG PEM, HRG, Hrk, human heart myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-21, IL-23, IL-27, interferon (INF)-alpha, INF-beta, INF-gamma, inhibin, iNOS, insulin chain A, insulin chain B, insulin-like growth factor 1, integrin alpha 2, integrin alpha 3, integrin alpha 4, integrin alpha 4/beta 1, integrin alpha 4/beta 7, integrin alpha 5 (alpha V), integrin alpha 5/beta 1, integrin alpha 5/beta 3, integrin alpha 6, integrin beta 1, integrin beta 2, interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y-related antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, metalloproteinases, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Mucl), MUC18, mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), P1GF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T cell receptor (e.g., T cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta 1, TGF-beta 2, TGF-beta 3, TGF-beta 4, TGF-beta 5, thrombin, thymus Ck-1, thyroid stimulating hormone, Tie, TIMP, TIQ, tissue factor, TMEFF2, Tmpo, TMPRSS2, TNF, TNF-alpha, TNF-alpha/beta, TNF-beta 2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TLR (toll-like receptor) 1, TLR2, TLR3, TLR4, TLR5, TLR6, TLR7, TLR8, TLR9, TLR10, TSG, TSLP, tumor-associated antigen CA125, tumor-associated antigen-expressing Lewis-Y-related carbohydrate, TWEAK, TXB2, Ung, uPAR, uPAR-1, urokinase, VCAM, VCAM-1, VECAD, VE-cadherin, VE-cadherin-2, VEFGR-1 (flt-1), VEGF, VEGFR, VEGFR-3 (flt-4), VEGI, VIM, viral antigens, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, A□, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, chromogranin A, chromogranin B, tau, VAP1, high-molecular-weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, syndecan-1, syndecan-2, syndecan-3, syndecan-4, LPA, SIP, and receptors for hormones or growth factors.

Although the examples of the antigen listed above also include receptors, these receptors even existing in a soluble form in a body fluid can be used as the antigen to which the antigen binding domain of the present invention binds. One non-limiting example of the soluble form of such a receptor can include the protein which is soluble IL-6R as described by Mullberg et al. (J. Immunol. (1994) 152 (10), 4958-4968).

The examples of the antigen listed above include membrane molecules expressed on cell membranes, and soluble molecules secreted from cells to the outside of the cells. When the antigen binding domain of the present invention binds to a soluble molecule secreted from cells, the antigen binding domain preferably has neutralizing activity.

The solution containing the soluble molecule is not limited, and this soluble molecule may exist in a body fluid, i.e., every vascular liquid or every liquid filling between tissues or cells in living bodies. In a non-limiting aspect, the soluble molecule to which the antigen binding domain of the present invention binds can exist in an extracellular fluid. The extracellular fluid refers to a generic name for plasma, intercellular fluid, lymph, tight connective tissues, cerebrospinal fluid, spinal fluid, aspirates, synovial fluid, or such components in the bone and cartilage, alveolar fluid (bronchoalveolar lavage fluid), ascitic fluid, pleural effusion, cardiac effusion, cyst fluid, aqueous humor (hydatoid), or such transcellular fluids (various fluids in glandular cavities resulting from the active transport or secretory activity of cells, and fluids in the lumen of the gut and other body cavities) in vertebrates.

The term "tumor antigen" refers to an antigen expressed on a cancer cell, and means a biological molecule having antigenicity, the expression of which becomes recognized in association with the malignant alteration of cells. The tumor antigen of the present disclosure includes a tumor-specific antigen (antigen that is present only in tumor cells and is not found in other normal cells), and a tumor-associated antigen (antigen that is also present in other organs and tissues or heterogeneous and allogeneic normal cells, or antigen that is expressed during development and/or differentiation). Also, an aberrant sugar chain that appears on cell surface or a protein molecule during cell canceration is the tumor antigen and is also called cancer sugar chain antigen. In one aspect of the present invention, the target antigen is a tumor antigen.

Examples of the tumor antigen preferably include GPC3 which belongs as the receptor described above to the GPI-anchored receptor family and is expressed in some cancers including liver cancer (Int J Cancer. (2003) 103 (4), 455-65), EpCAM which is expressed in a plurality of cancers including lung cancer (Proc Natl Acad Sci U S A. (1989) 86 (1), 27-31) (its polynucleotide sequence and polypeptide sequence are described in RefSeq registration Nos. NM_002354.2 and NP_002345.2, respectively), EGFR, CA19-9, CA15-3, sialyl SSEA-1 (SLX), Her2, prostate stem cell antigen (PSCA), alpha-fetoprotein (AFP), cancer embryonic antigen (CEA), tumor antigen-125 (CA-125), calretinin, MUC-1, MUC-16, epithelial membrane protein (EMA), epithelial tumor antigen (ETA), tyrosinase, melanoma-associated antigen (MAGE), chromogranin, cytokeratin, desmin, glial fibrillary acidic protein (GFAP), gross cystic disease fluid protein (GCDFP-15), HMB-45 antigen, protein melan-A (melanoma antigen that is recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, a dimer form of pyruvate kinase isozyme type M2 (tumor M2-PK), GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of prostate member 1), TROP-2, Claudin 6, RNF43a, aberrant ras protein or aberrant p53 protein, integrin alpha v beta 3 (CD61), galectin, K-Ras (V-Ki-ras2 Kirsten rat sarcoma viral oncogene), and Ral-B.

Further examples thereof include: thyroid-stimulating hormone receptor (TSHR); CD171; CS-1 (CD2 subset 1, CRACC, SLAMF7, CD319 and 19A24); C-type lectin-like molecule-1 (CLL-1); ganglioside GD3 (aNeu5Ac(2-8)aNeu5Ac(2-3)bDGalp(1-4)bDGlcp(1-1)Cer); Tn antigen (Tn Ag); T antigen (T Ag); Fms-like tyrosine kinase 3 (FLT3); CD38; CD44v6; B7H3 (CD276); KIT (CD117); interleukin-13 receptor subunit alpha-2 (IL-13Ra2); interleukin 11 receptor alpha (IL-11Ra); interleukin 2 receptor alpha (IL-2Ra); prostate stem cell antigen (PSCA); protease serine 21 (PRSS21); vascular endothelial cell growth factor receptor 2 (VEGFR2); Lewis (Y) antigen; CD24; platelet-derived growth factor receptor beta (PDGFR- beta); stage-specific embryonic antigen-4 (SSEA-4); neural cell adhesion molecule (NCAM); carbonic anhydrase IX (CAIX); proteasome (prosome, macropain) subunit, beta, 9 (LMP2); ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer; TGS5; high-molecular-weight melanoma-associated antigen (HMWMAA); o-acetyl-GD2 ganglioside (OAcGD2); folate receptor beta; tumor endothelial marker 1 (TEM1/CD248); tumor endothelial marker 7 related (TEM7R); claudin 6 (CLDN6); G protein-coupled receptor glass C group 5, member D (GPRC5D); chromosome X open reading frame 61 (CXORF61); CD97; CD179a; anaplastic lymphoma kinase (ALK); polysialic acid; placenta specific 1 (PLAC1); a hexasaccharide moiety of globoH glycoceramide (GloboH); mammary gland differentiation antigen (NY-BR-1); uroplakin 2 (UPK2); hepatitis A virus cellular receptor 1 (HAVCR1); adrenaline receptor beta 3 (ADRB3); pannexin 3 (PANX3); G protein-coupled receptor 20 (GPR20); lymphocyte antigen 6 complex, locus K9 (LY6K); olfactory receptor 51E2 (OR51E2); TCR gamma alternate reading frame protein (TARP); Wilms' tumor protein (WT1); ETS translocation variant gene 6, located on chromosome 12p (ETV6-AML); sperm protein 17 (SPA17); X antigen family, member 1A (XAGE1); angiopoietin binding cell surface receptor 2 (Tie 2); melanoma cancer-testis antigen-1 (MAD-CT-1); melanoma cancer-testis antigen-2 (MAD-CT-2); Fos-related antigen 1; p53 mutants; human telomerase reverse transcriptase (hTERT); sarcoma translocation breakpoint; melanoma inhibitor of apoptosis (ML-IAP); ERG (transmembrane protease, serine 2 (TMPRSS2)-ETS fusion gene); N-acetylglucosaminyl-transferase V (NA17); paired box protein Pax-3 (PAX3); androgen receptor; cyclin B1; v-myc avian myelocytomatosis viral oncogene neuroblastoma-derived homolog (MYCN); Ras homolog family member C (RhoC); cytochrome P450 1B 1 (CYP1B1); CCCTC binding factor (zinc finger protein)-like (BORIS); squamous cell carcinoma antigen recognized by T cells 3 (SART3); paired box protein Pax-5 (PAX5); proacrosin binding protein p32 (OY-TES1); lymphocyte-specific protein tyrosine kinase (LCK); A kinase anchoring protein 4 (AKAP-4); synovial sarcoma, X breakpoint 2 (SSX2); CD79a; CD79b; CD72; leukocyte-associated immunoglobulin like receptor 1 (LAIR1); Fc fragment of IgA receptor (FCAR); leukocyte immunoglobulin-like receptor subfamily A member 2 (LILRA2); CD300 molecule-like family member f (CD300LF); C-type lectin domain family 12 member A (CLEC12A); bone marrow stromal antigen 2 (BST2); EGF-like module-containing mucin-like hormone receptor-like 2 (EMR2); lymphocyte antigen 75 (LY75); glypican-3 (GPC3); Fc receptor-like 5 (FCRL5); and immunoglobulin lambda-like polypeptide 1 (IGLL1).

The "MHC antigen" is a gene product of major histocompatibility complex (MHC). Among such antigens, glycoproteins expressed on cell membrane are mainly classified into MHC class I antigens and MHC class II antigens. The MHC class I antigens include HLA-A, -B, -C, -E, -F, -G, and -H, and the MHC class II antigens include HLA-DR, -DQ, and -DP. Tumor antigen-derived peptides presented on these MHC antigens are also included therein. A tumor antigen such as GP100, MART-1, or MAGE-1, or a complex with MHC presenting a mutated site, such as RAS or p53, is also regarded as one of the tumor antigens.

The "differentiation antigen" is a generic name for cell surface molecules that appear or disappear in association with the differentiation of bone marrow stem cells into macrophages, T cells, B cells or the like. The differentiation antigen may include CD1, CD2, CD4, CD5, CD6, CD7, CD8, CD10, CD11a, CD11b, CD11c, CD13, CD14, CD15s, CD16, CD18, CD19, CD20, CD21, CD22, CD23, CD25, CD27, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD70, CD71, CD73, CD95, CD99, CD102, CD106, CD117, CD122, CD126, and CDw130.

In general, the term "tumor" is a generic name for masses that develop on the surface of the body or in the inside of the body and can be touched or have a distinctively colored area. The tumor includes malignant tumor having three features, autonomous growth, infiltration and metastasis, and cachexia, and benign tumor characterized only by autonomous growth. The malignant tumor "cancer" refers to a disease characterized by the uncontrollable growth of aberrant cells. Cancer cells can spread locally or via bloodstream and the lymphatic system to other parts of the body. Examples of various cancers are described in the present disclosure and include, but are not limited to, breast cancer, prostate cancer, ovary cancer, cervical cancer, skin cancer, pancreatic cancer, colorectal cancer, kidney cancer, liver cancer, brain cancer, lymphoma, leukemia, lung cancer and the like. The terms "tumor" and "cancer" are interchangeably used in the present disclosure. Both the terms encompass, for example, solid and liquid, for example, diffuse or circulating, tumors. As used in the present disclosure, the term "cancer" or "tumor" encompasses premalignant as well as malignant cancers and tumors.

Examples of the cancer for the anticancer agent or the method for treating a cancer as mentioned later according to the present disclosure can include cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, germ cell tumor, lymphoma, and leukemia.

In one embodiment, the tumor antigen in association with a cancer type is a marker expressed by both normal cells and cancer cells, for example, a lineage marker such as CD19 on B cells. In a certain aspect, the tumor antigen of the present disclosure is derived from a cancer including, but is not limited to, primary or metastatic melanoma, thymoma, lymphoma, sarcoma, lung cancer, liver cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer, pancreatic cancer, and the like. In one embodiment, the tumor antigen is an antigen common in particular proliferative disorders. In one embodiment, the tumor antigen is a cell surface molecule that is overexpressed in cancer cells compared with normal cells, for example, by 1-fold overexpression, 2-fold overexpression, 3-fold overexpression or more as compared with normal cells. In some embodiments, the tumor antigen is a cell surface molecule that is inappropriately synthesized in cancer cells, for example, a molecule containing deletion, addition or mutation as compared with a molecule expressed in normal cells. In one embodiment, the tumor antigen is expressed exclusively on the cell surface of cancer cells, as a whole or as a fragment (e.g., MHC/peptide), and neither synthesized nor expressed on the surface of normal cells. In some embodiments, the chimeric receptor and the TRAB of the present disclosure include CAR and TRAB comprising an antigen binding domain (e.g., an antibody or an antibody fragment) binding to a peptide presented by MHC. Usually, peptides derived from endogenous proteins fill the pockets of major histocompatibility complex (MHC) class I molecules, and are recognized by T cell receptors (TCRs) on CD8⁺ T lymphocytes. The MHC class I complex is constitutively expressed by all nucleated cells. In cancers, virus-specific and/or tumor-specific peptide/MHC complexes are representative of a unique class of cell surface targets for immunotherapy. TCR-like antibodies targeting virus- or tumor antigen-derived peptides in the context of human leukocyte antigen (HLA)-A1 or HLA-A2 are described [see e.g., Sastry et al., J Virol. 2011 85 (5): 1935-1942; Sergeeva et al., Bood, 2011 117 (16): 4262-4272; Verma et al., J Immunol 2010 184 (4): 2156-2165; Willemsen et al., Gene Ther 2001 8 (21): 1601-1608; Dao et al., Sci Transl Med 2013 5 (176): 176ra33; and Tassev et al., Cancer Gene Ther 2012 19 (2): 84-100]. The TCR-like antibody can be identified, for example, by screening a library such as a human scFv phage display library.

The epitope, which means an antigenic determinant, present in the antigen means a site on the antigen to which the antigen binding domain disclosed in the present specification binds. Accordingly, for example, the epitope can be defined by its structure. Alternatively, the epitope may be defined by the antigen-binding activity of the antigen binding domain recognizing the epitope. When the antigen is a peptide or a polypeptide, the epitope may be identified by amino acid residues constituting the epitope. When the epitope is a sugar chain, the epitope may be identified by a particular sugar chain structure.

A linear epitope refers to an epitope comprising an epitope that is recognized by its primary sequence of amino acids. The linear epitope contains typically at least 3 and most commonly at least 5, for example, approximately 8 to approximately 10 or 6 to 20 amino acids, in its unique sequence.

In contrast to the linear epitope, a conformational epitope refers to an epitope that is contained in a primary sequence of amino acids containing a component other than the single defined component of the epitope to be recognized (e.g., an epitope whose primary sequence of amino acids may not be recognized by an antibody that determines the epitope). The conformational epitope may contain an increased number of amino acids, as compared with the linear epitope. As for the recognition of the conformational epitope, the antigen binding domain recognizes the three-dimensional structure of the peptide or the protein. For example, when a protein molecule is folded to form a three-dimensional structure, certain amino acids and/or polypeptide backbone constituting the conformational epitope are arranged in parallel to allow the antibody to recognize the epitope. Examples of the method for determining the conformation of the epitope include, but are not limited to, X-ray crystallography, two-dimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

The structure of the antigen binding domain binding to the epitope is called paratope. The paratope stably binds to the epitope through a hydrogen bond, electrostatic force, van der Waals' forces, a hydrophobic bond, or the like acting between the epitope and the paratope. This binding force between the epitope and the paratope is called affinity. The total binding force when a plurality of antigen binding domains bind to a plurality of antigens is called avidity. The affinity works synergistically when, for example, an antibody comprising a plurality of antigen binding domains (i.e., a polyvalent antibody) bind to a plurality of epitopes. Therefore, the avidity is higher than the affinity.

In a particular embodiment, the antigen binding domain provided in the present specification has a dissociation constant (Kd) of ≤1 µM, ≤100 nM, ≤10 nM, ≤1 nM, ≤0.1 nM, ≤0.01 nM or <0.001 nM (e.g., 10⁻⁸ M or less, for example, 10⁻⁸ M to 10⁻¹³ M, for example, 10⁻⁹ M to 10⁻¹³ M).

A method for confirming the binding of an antigen binding domain directed to the antigen, or an antigen binding molecule comprising the antigen binding domain to the epitope can be appropriately carried out according to the example given below.

For example, whether the antigen binding domain directed to a certain antigen recognizes a linear epitope present in the certain antigen molecule can be confirmed, for example, as follows: a linear peptide comprising an amino acid sequence constituting the extracellular domain of a certain antigen is synthesized for the purpose described above. The peptide can be chemically synthesized. Alternatively, the peptide is obtained by a genetic engineering approach using a region encoding an amino acid sequence corresponding to the extracellular domain in a certain antigen cDNA. Next, the antigen binding domain directed to a certain antigen is evaluated for its binding activity against the linear peptide comprising an amino acid sequence constituting the extracellular domain. For example, the binding activity of the antigen binding domain against the peptide can be evaluated by ELISA using an immobilized linear peptide as an antigen. Alternatively, the binding activity against the linear peptide may be determined on the basis of a level at which the linear peptide inhibits the binding of the antigen binding domain to a certain antigen-expressing cells. These tests can determine the binding activity of the antigen binding domain against the linear peptide.

Also, whether the antigen binding domain directed to a certain antigen recognizes the conformational epitope can be confirmed as follows: a certain antigen-expressing cells are prepared for the purpose described above. The recognition of the conformational epitope by the antigen binding domain directed to a certain antigen is confirmed, for example, when the antigen binding domain directed to a certain antigen strongly binds to the certain antigen-expressing cells upon contact with the cells, whereas the antigen binding domain does not substantially bind to an immobilized linear peptide comprising an amino acid sequence constituting the extracellular domain of IL-6R or a denatured (using a general denaturant such as guanidine) linear peptide comprising an amino acid sequence constituting the extracellular domain of a certain antigen. In this context, the term "not substantially bind" means that the binding activity is 80% or less, usually 50% or less, preferably 30% or less, particularly preferably 15% or less of binding activity against cells expressing a certain human antigen.

The method for confirming the antigen binding activity of the antigen binding domain also includes a method of measuring a Kd value by, for example, radiolabeled antigen binding assay (RIA). In one embodiment, RIA is carried out using the antigen binding domain of interest and its antigen. For example, the binding affinity in a solution of the antigen binding domain for the antigen is measured by equilibrating the antigen binding domain with the smallest concentration of a (125I)-labeled antigen in the presence of a titration series of an unlabeled antigen, and subsequently capturing the bound antigen by a plate coated with the antigen binding domain (see e.g., Chen et al., J. Mol. Biol. 293: 865-881(1999)).

According to an alternative embodiment, Kd is measured by a surface plasmon resonance method using BIACORE(R). For example, assay using BIACORE(R)-2000 or BIACORE(R)-3000 (BIAcore, Inc., Piscataway, NJ) is carried out at 25°C using a CM5 chip with about 10 response units (RU) of the antigen immobilized thereon. In one embodiment, a carboxymethylated dextran biosensor chip (CM5, BIAcore, Inc.) is activated using N-ethyl-N'-(3-dimethylaminopropyl)-carbodiimide hydrochloride (EDC) and N-hydroxysuccinimide (NHS) according to the supplier's instruction. The antigen is diluted to 5 µg/ml (about 0.2 µM) with 10 mM sodium acetate (pH 4.8) and then injected thereto at a flow rate of 5 µl/min so as to attain protein binding at about 10 response units (RU). After the antigen injection, 1 M ethanolamine is injected thereto in order to block unreacted groups. For kinetic measurement, 2-fold dilutions (0.78 nM to 500 nM) of the antigen binding domain in PBS containing 0.05% Polysorbate 20 (TWEEN-20(R)) as a surfactant (PBST) are injected thereto at a flow rate of about 25 µl/min at 25°C. An association rate (kon) and a dissociation rate (koff) are calculated by fitting sensorgrams of association and dissociation at the same time using a simple 1:1 Langmuir binding model (BIACORE(R) evaluation software version 3.2). An equilibrium dissociation constant (Kd) is calculated as a koff/kon ratio. Furthermore, an apparent dissociation constant (Kd) may be determined by use of equilibrium analysis. For these procedures, see the protocol attached to BIACORE(R). See, for example, Chen et al., J. Mol. Biol. 293: 865-881 (1999) and Methods Enzymol. 2000; 323: 325-40. In the surface plasmon resonance assay, the amount of the protein immobilized, the amount of the protein used in reaction, temperature, and solution composition can be variously changed by those skilled in the art. When the on-rate in the surface plasmon resonance assay described above exceeds 10⁶ M⁻¹s⁻¹, the on-rate can be determined by use of a fluorescence quenching technique of using a spectrometer (e.g. a stopped-flow spectrophotometer (Aviv Instruments, Inc.) or SLM-AMINCO(TM) spectrophotometer 8000 series (Thermo Spectronic/Thermo Fisher Scientific Inc.) using a stirring cuvette) to measure increase or decrease in fluorescence intensity (excitation = 295 nm; emission = 340 nm, band path: 16 nm) at 25°C for 20 nM antigen binding domain in PBS (pH 7.2) in the presence of gradually increased concentrations of the antigen.

Furthermore, the antigen binding activity of the antigen binding domain can also be measured by a known molecule-molecule interaction measurement method such as electrogenerated chemiluminescence.

Examples of the method for measuring the binding activity of the antigen binding domain directed to a certain antigen against the certain antigen-expressing cells include methods described in Antibodies: A Laboratory Manual (Ed Harlow, David Lane, Cold Spring Harbor Laboratory (1988) 359-420). Specifically, the binding activity can be evaluated on the basis of the principle of ELISA or FACS (fluorescence activated cell sorting) using the certain antigen-expressing cells as an antigen.

In the ELISA format, the binding activity of the antigen binding domain directed to a certain antigen against the certain antigen-expressing cells is quantitatively evaluated by comparing the levels of signals generated through enzymatic reaction. Specifically, a test antigen binding domain is added to an ELISA plate with the certain antigen-expressing cells immobilized thereon. Then, the test antigen binding domain bound with the cells is detected through the use of an enzyme-labeled antibody recognizing the test antigen binding domain. Alternatively, in the FACS, a dilution series of a test antigen binding domain is prepared, and the antibody binding titer for the certain antigen-expressing cells can be determined to compare the binding activity of the test antigen binding domain against the certain antigen-expressing cells.

The binding of the test antigen binding domain to the antigen expressed on the surface of cells suspended in a buffer solution or the like can be detected using a flow cytometer. For example, the following apparatuses are known as the flow cytometer:
FACSCanto(TM) II
FACSAria(TM)
FACSArray(TM)
FACSVantage(TM) SE
FACSCalibur(TM) (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter, Inc.)

One preferred example of the method for measuring the antigen binding activity of the antigen binding domain directed to a certain antigen includes the following method: first, a certain antigen-expressing cells reacted with a test antigen binding domain are stained with a FITC-labeled secondary antibody recognizing the test antigen binding domain. The test antigen binding domain is appropriately diluted with a suitable buffer solution to prepare the antigen binding domain at the desired concentration for use. The antigen binding domain can be used, for example, at any concentration from 10 µg/ml to 10 ng/ml. Next, fluorescence intensity and the number of cells are measured using FACSCalibur (Becton, Dickinson and Company). The amount of the antigen binding domain bound to the cells is reflected in the fluorescence intensity obtained by analysis using CELL QUEST Software (Becton, Dickinson and Company), i.e., a geometric mean value. In short, the binding activity of the test antigen binding domain indicated by the amount of the test antigen binding domain bound can be determined by obtaining the geometric mean value.

Whether the antigen binding domain directed to a certain antigen shares an epitope with a certain antigen binding domain can be confirmed by the competition between these antigen binding domains for the same epitope. The competition between the antigen binding domains is detected by cross-blocking assay or the like. The cross-blocking assay is preferably, for example, competitive ELISA assay.

Specifically, in the cross-blocking assay, a certain antigen protein-coated wells of a microtiter plate are preincubated in the presence or absence of a candidate competitor antigen binding domain. Then, a test antigen binding domain is added thereto. The amount of the test antigen binding domain bound with the certain antigen protein in the wells indirectly correlates with the binding capacity of the candidate competitor antigen binding domain that competes for the binding to the same epitope. In short, larger affinity of the competitor antigen binding domain for the same epitope means lower binding activity of the test antigen binding domain against the certain antigen protein-coated wells.

The amount of the test antigen binding domain bound with the wells via the certain antigen protein can be easily measured by labeling the antigen binding domain in advance. For example, a biotin-labeled antigen binding domain is assayed by using an avidin-peroxidase conjugate and an appropriate substrate. In particular, cross-blocking assay that utilizes enzyme labels such as peroxidase is called competitive ELISA assay. The antigen binding domain can be labeled with an alternative detectable or measurable labeling material. Specifically, radiolabels, fluorescent labels, and the like are known in the art.

Provided that the competitor antigen binding domain can block the binding of the antigen binding domain directed to a certain antigen by at least 20%, preferably at least 20 to 50%, more preferably at least 50% as compared with binding activity obtained in a control test carried out in the absence of the candidate competitor antigen binding domain associate, the test antigen binding domain is determined as an antigen binding domain substantially binding to the same epitope as that for the competitor antigen binding domain, or competing for the binding to the same epitope.

When the epitope to which the antigen binding domain directed to a certain antigen binds has an identified structure, whether a test antigen binding domain and a control antigen binding domain share an epitope can be evaluated by comparing the binding activity of these antigen binding domains against a peptide or a polypeptide prepared by introducing an amino acid mutation to a peptide constituting the epitope.

In such a method for measuring binding activity, for example, the binding activity of a test antigen binding domain and a control antigen binding domain against a linear peptide containing an introduced mutation can be compared in the ELISA format described above. In a method other than ELISA, the binding activity against the mutated peptide bound with a column may be measured by flowing the test antigen binding domain and the control antigen binding domain in the column, and then quantifying the antigen binding domain eluted in the eluate. A method for adsorbing a mutated peptide, for example, as a fusion peptide with GST, to a column is known in the art.

When the identified epitope is a conformational epitope, whether a test antigen binding domain and a control antigen binding domain share an epitope can be evaluated by the following method: first, a certain antigen-expressing cells and cells expressing the certain antigen with a mutation introduced to the epitope are prepared. The test antigen binding domain and the control antigen binding domain are added to cell suspensions containing these cells suspended in an appropriate buffer solution such as PBS. Subsequently, the cell suspensions are appropriately washed with a buffer solution, and a FITC-labeled antibody capable of recognizing the test antigen binding domain and the control antigen binding domain is then added thereto. The fluorescence intensity and the number of cells stained with the labeled antibody are measured using FACSCalibur (Becton, Dickinson and Company). The test antigen binding domain and the control antigen binding domain are appropriately diluted with a suitable buffer solution and used at concentrations thereby adjusted to the desired ones. These antigen binding domains are used, for example, at any concentration from 10 µg/ml to 10 ng/ml. The amount of the labeled antibody bound to the cells is reflected in the fluorescence intensity obtained by analysis using CELL QUEST Software (Becton, Dickinson and Company), i.e., a geometric mean value. In short, the binding activity of the test antigen binding domain and the control antigen binding domain indicated by the amount of the labeled antibody bound can be determined by obtaining the geometric mean value.

The competition of the antigen binding domain with another antigen binding domain for the same epitope can also be confirmed by use of radiolabeled antigen binding assay (RIA), BIACORE(R) surface plasmon resonance assay, electrogenerated chemiluminescence, or the like, in addition to ELISA or FACS described above.

The geometric mean comparison value (ΔGeo-Mean value for the mutated certain antigen molecule) thus obtained by analysis, which reflects the amount of the test antigen binding domain bound with the cells expressing the mutated certain antigen, is compared with the ΔGeo-Mean comparison value that reflects the amount of the test antigen binding domain bound to the certain antigen-expressing cells. In this case, the concentrations of the test antigen binding domain used for determining the ΔGeo-Mean comparison values for the cells expressing the mutated certain antigen and the certain antigen-expressing cells are particularly preferably adjusted to equal or substantially equal concentrations. An antigen binding domain already confirmed to recognize an epitope in certain antigen is used as the control antigen binding domain.

Provided that the ΔGeo-Mean comparison value of the test antigen binding domain for the cells expressing the mutated certain antigen is smaller than at least 80%, preferably 50%, more preferably 30%, particularly preferably 15% of the ΔGeo-Mean comparison value of the test antigen binding domain for the certain antigen-expressing cells, the test antigen binding domain "does not substantially bind to cells expressing the mutated certain antigen". The calculation expression for determining the Geo-Mean (geometric mean) value is described in the CELL QUEST Software User's Guide (BD biosciences). The epitope for the test antigen binding domain and the control antigen binding domain can be assessed as being the same when their comparison values can be regarded as being substantially equivalent as a result of comparison.

In the present specification, the term "carrying moiety" refers to a moiety other than an antigen binding domain in an antigen binding molecule. The carrying moiety of the present invention is usually a peptide or a polypeptide constituted by amino acids. In a specific embodiment, the carrying moiety in the antigen binding molecule is linked to the antigen binding domain via a cleavage site. The carrying moiety of the present invention may be a series of peptides or polypeptides connected through an amide bond, or may be a complex formed from a plurality of peptides or polypeptides through a covalent bond such as a disulfide bond or a noncovalent bond such as a hydrogen bond or hydrophobic interaction.

In some embodiments of the present invention, the antigen binding molecule after cleavage of the linker has higher antigen binding activity than that of the antigen binding molecule before cleavage of the linker. In other words, the antigen binding activity of the antigen binding domain of the antigen binding molecule is inhibited by the inhibiting domain in the presence of a moiety that is removed by the cleavage of the linker. Whether the antigen binding activity of the antigen binding domain is inhibited by the inhibiting domain is confirmed by a method such as FACS (fluorescence activated cell sorting), ELISA (enzyme-linked immunosorbent assay), ECL (electrogenerated chemiluminescence), a SPR (surface plasmon resonance) method (Biacore), BLI (biolayer interferometry) (Octet). In some embodiments of the present invention, the antigen binding activity of the antigen binding molecule after cleavage of the linker is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the binding activity of the antigen binding molecule before cleavage of the linker. In some more specific embodiments of the present invention, the binding of the antigen binding domain before the release to the antigen is not seen when the antigen binding activity of the antigen binding domain is measured by one method selected from among the methods described above.

In some aspects of the present invention, the antigen binding activity can be compared between before and after the cleavage of the linker. Specifically, the antigen binding activity measured using the antigen binding molecule after cleavage of the linker is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the antigen binding activity measured using the antigen binding molecule before cleavage of the linker. In some more specific embodiments, the binding of the antigen binding domain of the antigen binding molecule before cleavage of the linker to the antigen is not seen when the antigen binding activity is measured by one method selected from among the methods described above.

In some aspects of the present invention, the linker of the antigen binding molecule is cleavable by protease. In such aspects, therefore, the antigen binding activity can be compared between before and after the protease treatment of the antigen binding molecule. Specifically, the antigen binding activity measured using the antigen binding molecule after the protease treatment is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the antigen binding activity measured using the antigen binding molecule without the protease treatment. In some more specific embodiments, the binding of the antigen binding domain of the protease-untreated antigen binding molecule to the antigen is not seen when the antigen binding activity is measured by one method selected from among the methods described above.

In the present invention, the antigen binding molecule before cleavage of the linker has a longer half-life in blood than that of the antigen binding molecule after the cleavage. In some embodiments of the present invention, for the longer half-life of the antigen binding molecule, the antigen binding molecule before cleavage of the linker is designed so as to have a longer half-life in blood. In such embodiments, examples of the approach of extending the half-life in blood include, but are not limited to, a large molecular weight of the antigen binding molecule before the cleavage of the linker, FcRn binding activity possessed by the antigen binding molecule before the cleavage of the linker, albumin binding activity possessed by the antigen binding molecule before the cleavage of the linker, and the PEGylation of the antigen binding molecule before the cleavage of the linker.

In the present invention, the half-lives are preferably compared in terms of half-lives in blood in humans. If the half-lives in blood are difficult to measure in humans, the half-lives in blood in humans can be predicted on the basis of their half-lives in blood in mice (e.g., normal mice, transgenic mice expressing a human antigen, and transgenic mice expressing human FcRn) or monkeys (e.g., cynomolgus monkeys).

In one embodiment, the approach of extending the half-life in blood of the antigen binding molecule includes the imparting of FcRn binding activity to the antigen binding molecule before cleavage of the linker. The antigen binding molecule before cleavage of the linker can usually possess FcRn binding activity by a method of establishing a FcRn binding region in the antigen binding molecule before cleavage of the linker. The FcRn binding region refers to a region having binding activity against FcRn and may have any structure as long as the region used has binding activity against FcRn.

The antigen binding molecule containing a FcRn binding region is capable of being taken up into cells and then brought back into plasma through the salvage pathway of FcRn. For example, an IgG molecule has a relatively long circulation time in plasma (slow disappearance) because FcRn known as a salvage receptor of the IgG molecule functions. An IgG molecule taken up into the endosome through pinocytosis binds to FcRn expressed in the endosome under intraendosomal acidic conditions. An IgG molecule that has failed to bind to FcRn is moved to the lysosome and degraded therein, whereas the IgG molecule bound with FcRn is transferred to cell surface, then dissociated from the FcRn under neutral conditions in plasma, and thereby brought back into plasma.

The FcRn binding region is preferably a region binding directly to FcRn. Preferred examples of the FcRn binding region can include antibody Fc regions. However, a region capable of binding to a polypeptide, such as albumin or IgG, which has FcRn binding capacity is capable of binding indirectly to FcRn via albumin, IgG, or the like. Therefore, the FcRn binding region according to the present invention may be a region binding to such a polypeptide having FcRn binding capacity.

The binding activity of the FcRn binding region according to the present invention against FcRn, particularly, human FcRn may be measured by a method known to those skilled in the art, as mentioned in the above section about binding activity. The conditions therefor may be appropriately determined by those skilled in the art. The binding activity against human FcRn can be evaluated as KD (dissociation constant), apparent KD (apparent dissociation constant), kd (dissociation rate), or apparent kd (apparent dissociation rate), etc. These values can be measured by methods known to those skilled in the art. For example, Biacore (GE Healthcare Japan Corp.), Scatchard plot, a flow cytometer, and the like can be used.

The conditions for measuring the binding activity of the FcRn binding region against FcRn are not particularly limited and may be appropriately selected by those skilled in the art. The binding activity can be measured under conditions involving, for example, a MES buffer and 37°C, as described in WO2009/125825. Also, the binding activity of the FcRn binding region of the present invention against FcRn may be measured by a method known to those skilled in the art and can be measured using, for example, Biacore (GE Healthcare Japan Corp.).

As for pH for use in the measurement conditions, the binding affinity of the FcRn binding region for FcRn may be evaluated at any pH of 4.0 to 6.5. Preferably, a pH of 5.8 to 6.0, which is close to pH in the early endosome *in vivo,* is used for determining the binding affinity of the FcRn binding region for human FcRn. As for temperature for use in the measurement conditions, the binding affinity of the FcRn binding region for FcRn may be evaluated at any temperature of 10°C to 50°C. Preferably, a temperature of 15°C to 40°C is used for determining the binding affinity of the FcRn binding region for human FcRn. More preferably, any temperature from 20°C to 35°C, for example, any one of 20, 21, 22, 23, 24, 25, 26, 27, 28, 29, 30, 31, 32, 33, 34, and 35°C, is also used for determining the binding affinity of the FcRn binding region for FcRn. The temperature of 25°C is one non-limiting example of the temperature of the present invention.

One example of the FcRn binding region includes, but is not limited to, an IgG antibody Fc region. In the case of using an IgG antibody Fc region, its type is not limited, and for example, IgG1, IgG2, IgG3, or IgG4 Fc region may be used.

A natural IgG antibody Fc region as well as an altered Fc region variant having one or more amino acid substitutions may be used as long as the Fc region has FcRn binding activity. For example, an altered Fc region variant containing an amino acid sequence derived from an IgG antibody Fc region by the substitution of at least one amino acid selected from EU numbering positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434 and 436 by another amino acid may be used.

More specifically, an altered Fc region variant containing at least one amino acid substitution selected from
an amino acid substitution to substitute Gly at position 237 by Met,
an amino acid substitution to substitute Pro at position 238 by Ala,
an amino acid substitution to substitute Ser at position 239 by Lys,
an amino acid substitution to substitute Lys at position 248 by Ile,
an amino acid substitution to substitute Thr at position 250 by Ala, Phe, Ile, Met, Gln, Ser, Val, Trp, or Tyr,
an amino acid substitution to substitute Met at position 252 by Phe, Trp, or Tyr,
an amino acid substitution to substitute Ser at position 254 by Thr,
an amino acid substitution to substitute Arg at position 255 by Glu,
an amino acid substitution to substitute Thr at position 256 by Asp, Glu, or Gln,
an amino acid substitution to substitute Pro at position 257 by Ala, Gly, Ile, Leu, Met, Asn, Ser, Thr, or Val,
an amino acid substitution to substitute Glu at position 258 by His,
an amino acid substitution to substitute Asp at position 265 by Ala,
an amino acid substitution to substitute Asp at position 270 by Phe,
an amino acid substitution to substitute Asn at position 286 by Ala or Glu,
an amino acid substitution to substitute Thr at position 289 by His,
an amino acid substitution to substitute Asn at position 297 by Ala,
an amino acid substitution to substitute Ser at position 298 by Gly,
an amino acid substitution to substitute Val at position 303 by Ala,
an amino acid substitution to substitute Val at position 305 by Ala,
an amino acid substitution to substitute Thr at position 307 by Ala, Asp, Phe, Gly, His, Ile, Lys, Leu, Met, Asn, Pro, Gln, Arg, Ser, Val, Trp, or Tyr,
an amino acid substitution to substitute Val at position 308 by Ala, Phe, Ile, Leu, Met, Pro, Gln, or Thr,
an amino acid substitution to substitute Leu or Val at position 309 by Ala, Asp, Glu, Pro, or Arg,
an amino acid substitution to substitute Gln at position 311 by Ala, His, or Ile,
an amino acid substitution to substitute Asp at position 312 by Ala or His,
an amino acid substitution to substitute Leu at position 314 by Lys or Arg,
an amino acid substitution to substitute Asn at position 315 by Ala or His,
an amino acid substitution to substitute Lys at position 317 by Ala,
an amino acid substitution to substitute Asn at position 325 by Gly,
an amino acid substitution to substitute Ile at position 332 by Val,
an amino acid substitution to substitute Lys at position 334 by Leu,
an amino acid substitution to substitute Lys at position 360 by His,
an amino acid substitution to substitute Asp at position 376 by Ala,
an amino acid substitution to substitute Glu at position 380 by Ala,
an amino acid substitution to substitute Glu at position 382 by Ala,
an amino acid substitution to substitute Asn or Ser at position 384 by Ala,
an amino acid substitution to substitute Gly at position 385 by Asp or His,
an amino acid substitution to substitute Gln at position 386 by Pro,
an amino acid substitution to substitute Pro at position 387 by Glu,
an amino acid substitution to substitute Asn at position 389 by Ala or Ser,
an amino acid substitution to substitute Ser at position 424 by Ala,
an amino acid substitution to substitute Met at position 428 by Ala, Asp, Phe, Gly, His, Ile, Lys, Leu, Asn, Pro, Gln, Ser, Thr, Val, Trp, or Tyr,
an amino acid substitution to substitute His at position 433 by Lys,
an amino acid substitution to substitute Asn at position 434 by Ala, Phe, His, Ser, Trp, or Tyr, and
an amino acid substitution to substitute Tyr or Phe at position 436 by His (all according to the EU numbering)
in an IgG antibody Fc region may be used.

From another viewpoint, a Fc region containing at least one amino acid selected from
Met as the amino acid at position 237,
Ala as the amino acid at position 238,
Lys as the amino acid at position 239,
Ile as the amino acid at position 248,
Ala, Phe, Ile, Met, Gln, Ser, Val, Trp, or Tyr as the amino acid at position 250,
Phe, Trp, or Tyr as the amino acid at position 252,
Thr as the amino acid at position 254,
Glu as the amino acid at position 255,
Asp, Glu, or Gln as the amino acid at position 256,
Ala, Gly, Ile, Leu, Met, Asn, Ser, Thr, or Val as the amino acid at position 257,
His as the amino acid at position 258,
Ala as the amino acid at position 265,
Phe as the amino acid at position 270,
Ala or Glu as the amino acid at position 286,
His as the amino acid at position 289,
Ala as the amino acid at position 297,
Gly as the amino acid at position 298,
Ala as the amino acid at position 303,
Ala as the amino acid at position 305,
Ala, Asp, Phe, Gly, His, Ile, Lys, Leu, Met, Asn, Pro, Gln, Arg, Ser, Val, Trp, or Tyr as the amino acid at position 307,
Ala, Phe, Ile, Leu, Met, Pro, Gln, or Thr as the amino acid at position 308,
Ala, Asp, Glu, Pro, or Arg as the amino acid at position 309,
Ala, His, or Ile as the amino acid at position 311,
Ala or His as the amino acid at position 312,
Lys or Arg as the amino acid at position 314,
Ala or His as the amino acid at position 315,
Ala as the amino acid at position 317,
Gly as the amino acid at position 325,
Val as the amino acid at position 332,
Leu as the amino acid at position 334,
His as the amino acid at position 360,
Ala as the amino acid at position 376,
Ala as the amino acid at position 380,
Ala as the amino acid at position 382,
Ala as the amino acid at position 384,
Asp or His as the amino acid at position 385,
Pro as the amino acid at position 386,
Glu as the amino acid at position 387,
Ala or Ser as the amino acid at position 389,
Ala as the amino acid at position 424,
Ala, Asp, Phe, Gly, His, Ile, Lys, Leu, Asn, Pro, Gln, Ser, Thr, Val, Trp, or Tyr as the amino acid at position 428,
Lys as the amino acid at position 433,
Ala, Phe, His, Ser, Trp, or Tyr as the amino acid at position 434, and
His as the amino acid at position 436
(all according to the EU numbering)
in an IgG antibody Fc region may be used.

The antigen binding domain of the antigen binding molecule may have FcRn binding activity. In the embodiments in which the antigen binding molecule before cleavage of the linker has a longer half-life in blood than that of the antigen binding domain, the antigen binding domain may have no FcRn binding activity, as a matter of course, or the antigen binding domain may have FcRn binding activity as long as the FcRn binding activity is weaker than that of the antigen binding molecule before cleavage of the linker.

In one embodiment, the method for extending the half-life in blood of the carrying moiety involves binding the antigen binding molecule before cleavage of the linker to albumin. Since albumin does not undergo renal excretion and has FcRn binding activity, its half-life in blood is as long as 17 to 19 days (J Clin Invest. 1953 Aug; 32 (8): 746-768). Hence, it has been reported that a protein bound with albumin becomes bulky and capable of binding indirectly to FcRn and therefore has an increased half-life in blood (Antibodies 2015, 4(3), 141-156).

In one embodiment, the alternative method for extending the half-life in blood involves PEGylating the antigen binding molecule before cleavage of the linker. The PEGylation of a protein is considered to render the protein bulky and also suppress its degradation by protease in blood, thereby extending the half-life in blood of the protein (J Pharm Sci. 2008 Oct; 97 (10): 4167-83).

In some embodiments of the present invention, the antigen binding molecule before cleavage of the linker contains an antibody Fc region. In a specific embodiment, the antigen binding molecule before cleavage of the linker contains a CH2 domain and a CH3 domain of a human IgG antibody. In a specific embodiment, the antigen binding molecule before cleavage of the linker contains a moiety spanning from human IgG1 antibody heavy chain Cys226 or Pro230 to the carboxyl terminus of the heavy chain. However, the C-terminal lysine (Lys447) or glycine-lysine (Gly446-Lys447) of the Fc region may be present or absent.

In some embodiments of the present invention, the antigen binding molecule before cleavage of the linker contains an antibody constant region. In a more preferred embodiment, the antigen binding molecule before cleavage of the linker contains an IgG antibody constant region. In a further preferred embodiment, the antigen binding molecule before cleavage of the linker contains a human IgG antibody constant region.

In some embodiments of the present invention, the antigen binding molecule before cleavage of the linker contains: a region substantially similar in structure to an antibody heavy chain constant region; and a region substantially similar in structure to an antibody light chain, connected to the region via a covalent bond such as a disulfide bond or a noncovalent bond such as a hydrogen bond or hydrophobic interaction.

The linker of the antigen binding molecule is specifically cleaved at a rate of approximately 0.001 to 1500 × 10⁴ M⁻¹S⁻¹ or at least 0.001, 0.005, 0.01, 0.05, 0.1, 0.5, 1, 2.5, 5, 7.5, 10, 15, 20, 25, 50, 75, 100, 125, 150, 200, 250, 500, 750, 1000, 1250, or 1500 × 10⁴ M⁻¹S⁻¹.

In the present specification, the term "protease" refers to an enzyme such as endopeptidase or exopeptidase which hydrolyzes a peptide bond, typically, endopeptidase. The protease used in the present disclosure is limited only by being capable of cleaving the protease cleavage sequence and is not particularly limited by its type. In some embodiments, target tissue specific protease is used. The target tissue specific protease can refer to, for example, any of
(1) protease that is expressed at a higher level in the target tissue than in normal tissues,
(2) protease that has higher activity in the target tissue than in normal tissues,
(3) protease that is expressed at a higher level in the target cells than in normal cells, and
(4) protease that has higher activity in the target cells than in normal cells.

In a more specific embodiment, a cancer tissue specific protease or an inflammatory tissue specific protease is used.

In the present specification, the term "target tissue" means a tissue containing at least one target cell. In some embodiments of the present invention, the target tissue is a cancer tissue. In some embodiments of the present invention, the target tissue is an inflammatory tissue.

The term "cancer tissue" means a tissue containing at least one cancer cell. Thus, considering that, for example, the cancer tissue contains cancer cells and vascular vessels, every cell type that contributes to the formation of tumor mass containing cancer cells and endothelial cells is included in the scope of the present invention. In the present specification, the tumor mass refers to a foci of tumor tissue. The term "tumor" is generally used to mean benign neoplasm or malignant neoplasm.

In the present specification, examples of the "inflammatory tissue" include the following:
a joint tissue in rheumatoid arthritis or osteoarthritis,
a lung (alveolus) tissue in bronchial asthma or COPD,
a digestive organ tissue in inflammatory bowel disease, Crohn disease, or ulcerative colitis,
a fibrotic tissue in fibrosis in the liver, the kidney, or the lung,
a tissue under rejection of organ transplantation,
a vascular vessel or heart (cardiac muscle) tissue in arteriosclerosis or heart failure,
a visceral fat tissue in metabolic syndrome,
a skin tissue in atopic dermatitis and other dermatitides, and
a spinal nerve tissue in disk herniation or chronic lumbago.

Specifically expressed or specifically activated protease, or protease considered to be related to the disease condition of a target tissue (target tissue specific protease) is known for some types of target tissues. For example, International Publication Nos. WO2013/128194, WO2010/081173, and WO2009/025846 disclose protease specifically expressed in a cancer tissue. Also, J Inflamm (Lond). 2010; 7: 45, Nat Rev Immunol. 2006 Jul; 6 (7): 541-50, Nat Rev Drug Discov. 2014 Dec; 13 (12): 904-27, Respir Res. 2016 Mar 4; 17: 23, Dis Model Mech. 2014 Feb; 7 (2): 193-203, and Biochim Biophys Acta. 2012 Jan; 1824 (1): 133-45 disclose protease considered to be related to inflammation.

In addition to the protease specifically expressed in a target tissue, there also exists protease specifically activated in a target tissue. For example, protease may be expressed in an inactive form and then converted to an active form. Many tissues contain a substance inhibiting active protease and control the activity by the process of activation and the presence of the inhibitor (Nat Rev Cancer. 2003 Jul; 3 (7): 489-501). In a target tissue, the active protease may be specifically activated by escaping inhibition. The active protease can be measured by use of a method using an antibody recognizing the active protease (PNAS 2013 Jan 2; 110 (1): 93-98) or a method of fluorescently labeling a peptide recognizable by protease so that the fluorescence is quenched before cleavage, but emitted after cleavage (Nat Rev Drug Discov. 2010 Sep; 9 (9): 690-701. doi: 10.1038/nrd3053).

From one viewpoint, the term "target tissue specific protease" can refer to any of
(i) protease that is expressed at a higher level in the target tissue than in normal tissues,
(ii) protease that has higher activity in the target tissue than in normal tissues,
(iii) protease that is expressed at a higher level in the target cells than in normal cells, and
(iv) protease that has higher activity in the target cells than in normal cells.

Specific examples of the protease include, but are not limited to, cysteine protease (including cathepsin families B, L, S, etc.), aspartyl protease (cathepsins D, E, K, O, etc.), serine protease (including matriptase (including MT-SP1), cathepsins A and G, thrombin, plasmin, urokinase (uPA), tissue plasminogen activator (tPA), elastase, proteinase 3, thrombin, kallikrein, tryptase, and chymase), metalloproteinase (metalloproteinase (MMP1-28) including both membrane-bound forms (MMP14-17 and MMP24-25) and secreted forms (MMP1-13, MMP18-23 and MMP26-28), A disintegrin and metalloproteinase (ADAM), A disintegrin and metalloproteinase with thrombospondin motifs (ADAMTS), meprin (meprin alpha and meprin beta), CD10 (CALLA), prostate-specific antigen (PSA), legumain, TMPRSS3, TMPRSS4, human neutrophil elastase (HNE), beta secretase (BACE), fibroblast activation protein alpha (FAP), granzyme B, guanidinobenzoatase (GB), hepsin, neprilysin, NS3/4A, HCV-NS3/4, calpain, ADAMDEC1, renin, cathepsin C, cathepsin V/L2, cathepsin X/Z/P, cruzipain, otubain 2, kallikrein-related peptidases (KLKs (KLK3, KLK4, KLK5, KLK6, KLK7, KLK8, KLK10, KLK11, KLK13, and KLK14)), bone morphogenetic protein 1 (BMP-1), activated protein C, blood coagulation-related protease (Factor VIIa, Factor IXa, Factor Xa, Factor XIa, and Factor XIIa), HtrA1, lactoferrin, marapsin, PACE4, DESC1, dipeptidyl peptidase 4 (DPP-4), TMPRSS2, cathepsin F, cathepsin H, cathepsin L2, cathepsin O, cathepsin S, granzyme A, Gepsin calpain 2, glutamate carboxypeptidase 2, AMSH-like proteases, AMSH, gamma secretase, antiplasmin cleaving enzyme (APCE), decysin 1, N-acetylated alpha-linked acidic dipeptidase-like 1 (NAALADL1), and furin.

From another viewpoint, the target tissue specific protease can refer to a cancer tissue specific protease or an inflammatory tissue specific protease.

Examples of cancer tissue specific protease include protease specifically expressed in a cancer tissue disclosed in International Publication Nos. WO2013/128194, WO2010/081173, and WO2009/025846.

As for the type of cancer tissue specific protease, the protease having higher expression specificity in the cancer tissue to be treated is more effective for reducing adverse reactions. Preferable cancer tissue specific protease has a concentration in the cancer tissue at least 5 times, more preferably at least 10 times, further preferably at least 100 times, particularly preferably at least 500 times, most preferably at least 1000 times higher than its concentration in normal tissues. Also, preferable cancer tissue specific protease has activity in the cancer tissue at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The cancer tissue specific protease may be in a form bound with a cancer cell membrane or may be in a form secreted extracellularly without being bound with a cell membrane. When the cancer tissue specific protease is not bound with a cancer cell membrane, it is preferred for immunocyte-mediated cytotoxicity specific for cancer cells that the cancer tissue specific protease should exist within or in the vicinity of the cancer tissue. In the present specification, the "vicinity of the cancer tissue" means to fall within the scope of location where the protease cleavage sequence specific for the cancer tissue is cleaved so that the antigen binding domain exerts antigen binding activity. However, it is preferred that damage on normal cells should be minimized in this scope of location.

From an alternative viewpoint, cancer tissue specific protease is any of
(i) protease that is expressed at a higher level in the cancer tissue than in normal tissues,
(ii) protease that has higher activity in the cancer tissue than in normal tissues,
(iii) protease that is expressed at a higher level in the cancer cells than in normal cells, and
(iv) protease that has higher activity in the cancer cells than in normal cells.

One type of cancer tissue specific protease may be used alone, or two or more types of cancer tissue specific proteases may be combined. The number of types of cancer tissue specific protease can be appropriately set by those skilled in the art in consideration of the cancer type to be treated.

From these viewpoints, cancer tissue specific protease is preferably serine protease or metalloproteinase, more preferably matriptase (including MT-SP1), urokinase (uPA), or metalloproteinase, further preferably MT-SP1, uPA, MMP2, or MMP9, among the proteases listed above.

As for the type of inflammatory tissue specific protease, the protease having higher expression specificity in the inflammatory tissue to be treated is more effective for reducing adverse reactions. Preferable inflammatory tissue specific protease has a concentration in the inflammatory tissue at least 5 times, more preferably at least 10 times, further preferably at least 100 times, particularly preferably at least 500 times, most preferably at least 1000 times higher than its concentration in normal tissues. Also, preferable inflammatory tissue specific protease has activity in the inflammatory tissues at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The inflammatory tissue specific protease may be in a form bound with an inflammatory cell membrane or may be in a form secreted extracellularly without being bound with a cell membrane. When the inflammatory tissue specific protease is not bound with an inflammatory cell membrane, it is preferred for immunocyte-mediated cytotoxicity specific for inflammatory cells that the inflammatory tissue specific protease should exist within or in the vicinity of the inflammatory tissue. In the present specification, the "vicinity of the inflammatory tissue" means to fall within the scope of location where the protease cleavage sequence specific for the inflammatory tissue is cleaved so that the antigen binding domain exerts antigen binding activity. However, it is preferred that damage on normal cells should be minimized in this scope of location.

From an alternative viewpoint, inflammatory tissue specific protease is any of
(i) protease that is expressed at a higher level in the inflammatory tissue than in normal tissues,
(ii) protease that has higher activity in the inflammatory tissue than in normal tissues,
(iii) protease that is expressed at a higher level in the inflammatory cells than in normal cells, and
(iv) protease that has higher activity in the inflammatory cells than in normal cells.

One type of inflammatory tissue specific protease may be used alone, or two or more types of inflammatory tissue specific proteases may be combined. The number of types of inflammatory tissue specific protease can be appropriately set by those skilled in the art in consideration of the pathological condition to be treated.

From these viewpoints, t inflammatory tissue specific protease is preferably metalloproteinase among the proteases listed above. The metalloproteinase is more preferably ADAMTS5, MMP2, MMP7, MMP9, or MMP13.

The protease cleavage sequence is a particular amino acid sequence that is specifically recognized by target tissue specific protease when the antigen binding molecule is hydrolyzed by the target tissue specific protease in an aqueous solution.

The protease cleavage sequence is preferably an amino acid sequence that is hydrolyzed with high specificity by target tissue specific protease more specifically expressed in the target tissue or cells to be treated or more specifically activated in the target tissue/ cells to be treated, from the viewpoint of reduction in adverse reactions.

Specific examples of the protease cleavage sequence include target sequences that are specifically hydrolyzed by the above-listed protease specifically expressed in a cancer tissue disclosed in International Publication Nos. WO2013/128194, WO2010/081173, and WO2009/025846, the protease specific for an inflammatory tissue, and the like. A sequence artificially altered by, for example, introducing an appropriate amino acid mutation to a target sequence that is specifically hydrolyzed by known protease can also be used. Alternatively, a protease cleavage sequence identified by a method known to those skilled in the art as described in Nature Biotechnology 19, 661-667 (2001) may be used.

Furthermore, a naturally occurring protease cleavage sequence may be used. For example, TGFβ is converted to a latent form by protease cleavage. Likewise, a protease cleavage sequence in a protein that changes its molecular form by protease cleavage can also be used.

Examples of the protease cleavage sequence that can be used include, but are not limited to, sequences disclosed in International Publication No. WO2015/116933, International Publication No. WO2015/048329, International Publication No. WO2016/118629, International Publication No. WO2016/179257, International Publication No. WO2016/179285, International Publication No. WO2016/179335, International Publication No. WO2016/179003, International Publication No. WO2016/046778, International Publication No. WO2016/014974, Japanese Patent Application No. 2019-105464, U.S. Patent Publication No. US2016/0289324, U.S. Patent Publication No. US2016/0311903, PNAS (2000) 97: 7754-7759, Biochemical Journal (2010) 426: 219-228, and Beilstein J Nanotechnol. (2016) 7: 364-373.

The protease cleavage sequence is more preferably an amino acid sequence that is specifically hydrolyzed by suitable target tissue specific protease as mentioned above. The amino acid sequence that is specifically hydrolyzed by target tissue specific protease is preferably a sequence comprising any of the following amino acid sequences:
LSGRSDNH (cleavable by MT-SP1 or uPA),
PLALAG (cleavable by MMP2 or MMP9), and
VPLSLTMG (cleavable by MMP7).

Any of the following sequences can also be used as the protease cleavage sequence:
TSTSGRSANPRG (cleavable by MT-SP1 or uPA),
ISSGLLSGRSDNH (cleavable by MT-SP1 or uPA),
AVGLLAPPGGLSGRSDNH (cleavable by MT-SP1 or uPA),
GAGVPMSMRGGAG (cleavable by MMP1),
GAGIPVSLRSGAG (cleavable by MMP2),
GPLGIAGQ (cleavable by MMP2),
GGPLGMLSQS (cleavable by MMP2),
PLGLWA (cleavable by MMP2),
GAGRPFSMIMGAG (cleavable by MMP3),
GAGVPLSLTMGAG (cleavable by MMP7),
GAGVPLSLYSGAG (cleavable by MMP9),
AANLRN (cleavable by MMP11),
AQAYVK (cleavable by MMP11),
AANYMR (cleavable by MMP11),
AAALTR (cleavable by MMP11),
AQNLMR (cleavable by MMP11),
AANYTK (cleavable by MMP11),
GAGPQGLAGQRGIVAG (cleavable by MMP13),
PRFKIIGG (cleavable by pro-urokinase),
PRFRIIGG (cleavable by pro-urokinase),
GAGSGRSAG (cleavable by uPA),
SGRSA (cleavable by uPA),
GSGRSA (cleavable by uPA),
SGKSA (cleavable by uPA),
SGRSS (cleavable by uPA),
SGRRA (cleavable by uPA),
SGRNA (cleavable by uPA),
SGRKA (cleavable by uPA),
QRGRSA (cleavable by tPA),
GAGSLLKSRMVPNFNAG (cleavable by cathepsin B),
TQGAAA (cleavable by cathepsin B),
GAAAAA (cleavable by cathepsin B),
GAGAAG (cleavable by cathepsin B),
AAAAAG (cleavable by cathepsin B),
LCGAAI (cleavable by cathepsin B),
FAQALG (cleavable by cathepsin B),
LLQANP (cleavable by cathepsin B),
LAAANP (cleavable by cathepsin B),
LYGAQF (cleavable by cathepsin B),
LSQAQG (cleavable by cathepsin B),
ASAASG (cleavable by cathepsin B),
FLGASL (cleavable by cathepsin B),
AYGATG (cleavable by cathepsin B),
LAQATG (cleavable by cathepsin B),
GAGSGVVIATVIVITAG (cleavable by cathepsin L),
APMAEGGG (cleavable by meprin alpha or meprin beta),
EAQGDKII (cleavable by meprin alpha or meprin beta),
LAFSDAGP (cleavable by meprin alpha or meprin beta),
YVADAPK (cleavable by meprin alpha or meprin beta),
RRRRR (cleavable by furin),
RRRRRR (cleavable by furin),
GQSSRHRRAL (cleavable by furin),
SSRHRRALD (cleavable by TGF□),
RKSSIIIRMRDVVL (cleavable by plasminogen),
SSSFDKGKYKKGDDA (cleavable by staphylokinase),
SSSFDKGKYKRGDDA (cleavable by staphylokinase),
IEGR (cleavable by Factor Xa),
IDGR (cleavable by Factor Xa),
GGSIDGR (cleavable by Factor Xa),
GPQGIAGQ (cleavable by collagenase),
GPQGLLGA (cleavable by collagenase),
GIAGQ (cleavable by collagenase),
GPLGIAG (cleavable by collagenase),
GPEGLRVG (cleavable by collagenase),
YGAGLGVV (cleavable by collagenase),
AGLGVVER (cleavable by collagenase),
AGLGISST (cleavable by collagenase),
EPQALAMS (cleavable by collagenase),
QALAMSAI (cleavable by collagenase),
AAYHLVSQ (cleavable by collagenase),
MDAFLESS (cleavable by collagenase),
ESLPVVAV (cleavable by collagenase),
SAPAVESE (cleavable by collagenase),
DVAQFVLT (cleavable by collagenase),
VAQFVLTE (cleavable by collagenase),
AQFVLTEG (cleavable by collagenase),
PVQPIGPQ (cleavable by collagenase), and
LVPRGS (cleavable by thrombin).

In some embodiments, the protease cleavage sequence is cleavable by at least cysteine protease. In some embodiments, the protease cleavage sequence is cleavable by at least metalloprotease. In some embodiments, the protease cleavage sequence is cleavable by at least matriptase. In some embodiments, the protease cleavage sequence is cleavable by at least MT-SP1. In some embodiments, the protease cleavage sequence is cleavable by at least uPA. In some embodiments, the protease cleavage sequence is cleavable by at least matriptase and uPA. In some embodiments, the protease cleavage sequence is cleavable by at least MT-SP1 and uPA.

In one aspect, the protease cleavage sequence is selected from the group consisting of PLALAG, VPLSLTMG, GAGVPMSMRGGAG, GAGIPVSLRSGAG, GPLGIAGQ, GGPLGMLSQS, PLGLWA, GAGRPFSMIMGAG, GAGVPLSLTMGAG, GAGVPLSLYSGAG, AANLRN, AQAYVK, AANYMR, AAALTR, AQNLMR, AANYTK, and GAGPQGLAGQRGIVAG cleavable by MMP.

In one aspect, the protease cleavage sequence is selected from the group consisting of GPQGIAGQ, GPQGLLGA, GIAGQ, GPLGIAG, GPEGLRVG, YGAGLGVV, AGLGVVER, AGLGISST, EPQALAMS, QALAMSAI, AAYHLVSQ, MDAFLESS, ESLPVVAV, SAPAVESE, DVAQFVLT, VAQFVLTE, AQFVLTEG, and PVQPIGPQ cleavable by collagenase.

Any of the sequences represented by SEQ ID NOs: 1 to 725 can also be used as the protease cleavage sequence.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W; X7 is an amino acid selected from A, D, E, F, G, H, I, K, L, M, N, P, Q, R, S, T, V, W and Y; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; and X8 is an amino acid selected from A, D, E, F, G, H, I, K, L, M, N, P, Q, R, S, T, V, W and Y.

The following sequence can also be used as the protease cleavage sequence:
X1-X2-X3-X4-X5-X6-X7-X8
wherein X1 to X8 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; and X8 is an amino acid selected from A, D, E, F, G, I, K, N, T and W.

The following sequence can also be used as the protease cleavage sequence:
X1-X2-X3-X4-X5-X6-X7-X8
wherein X1 to X8 each represent one amino acid, wherein X1 is an amino acid selected from A, G, I, P, Q, S and Y; X2 is an amino acid selected from K and T; X3 is G; X4 is R; X5 is S; X6 is A; X7 is an amino acid selected from H, I and V; and X8 is an amino acid selected from H, V and Y.

The following sequence can also be used as the protease cleavage sequence:
X1-X2-X3-X4-X5-X6-X7-X8
wherein X1 to X8 each represent one amino acid, wherein X1 is Y; X2 is an amino acid selected from S and T; X3 is G; X4 is R; X5 is S; X6 is an amino acid selected from A and E; and X8 is an amino acid selected from H, P, V and Y.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, E, F, G, H, K, M, N, P, Q, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, F, L, M, P, Q, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, E, F, H, I, K, L, M, N, P, Q, R, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, G, H, I, K, L, M, N, Q, R, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from E, F, K, M, N, P, Q, R, S and W; X7 is an amino acid selected from A, D, E, F, G, H, I, K, L, M, N, P, Q, R, S, T, V, W and Y; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, F, G, L, M, P, Q, V and W; X8 is an amino acid selected from A, D, E, F, G, H, I, K, L, M, N, P, Q, R, S, T, V, W and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, D, E, F, G, H, I, K, M, N, P, Q, S, T, W and Y; X2 is an amino acid selected from A, D, E, F, H, K, L, M, P, Q, S, T, V, W and Y; X3 is an amino acid selected from A, D, E, F, G, H, I, K, L, M, N, P, Q, R, S, T, V, W and Y; X4 is R; X5 is an amino acid selected from A, D, E, F, G, H, I, K, L, M, N, P, Q, R, S, T, V, W and Y; X6 is an amino acid selected from A, D, E, F, H, I, K, L, M, N, P, Q, R, S, T, V, W and Y; X7 is an amino acid selected from A, D, E, F, G, H, I, K, L, M, N, P, Q, R, S, T, V, W and Y; X8 is an amino acid selected from A, D, E, F, G, I, K, N, T and W; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is an amino acid selected from A, G, I, P, Q, S and Y; X2 is an amino acid selected from K and T; X3 is G; X4 is R; X5 is S; X6 is A; X7 is an amino acid selected from H, I and V; X8 is an amino acid selected from H, V and Y; and X9 is an amino acid selected from R and G.

The following sequence can also be used as the protease cleavage sequence:
X1-X2-X3-X4-X5-X6-X7-X8-X9
wherein X1 to X9 each represent one amino acid, wherein X1 is Y; X2 is an amino acid selected from S and T; X3 is G; X4 is R; X5 is S; X6 is an amino acid selected from A and E; X8 is an amino acid selected from H, P, V and Y; and X9 is an amino acid selected from R and G.

In addition to using the protease cleavage sequences described above, a novel protease cleavage sequence may be obtained by screening. For example, from results of the crystal structure analysis of a known protease cleavage sequence, the novel protease cleavage sequence can be searched for by changing the interaction of active residues and/or recognition residues of the cleavage sequence and the enzyme. The novel protease cleavage sequence can also be searched for by altering amino acids in a known protease cleavage sequence and confirming interaction between the altered sequence and the protease. As another example, the protease cleavage sequence can be searched for by confirming the interaction of the protease with a library of peptides displayed using an *in vitro* display method such as phage display or ribosome display, or with an array of peptides immobilized onto a chip or beads. The interaction between the protease cleavage sequence and the protease can be confirmed by a method of confirming the cleavage of the sequence by the protease *in vitro* or *in vivo.*

The protease cleavage sequence of the present disclosure is specifically modified (cleaved) by the protease at a rate of approximately 0.001 to 1500 × 10⁴ M⁻¹S⁻¹ or at least 0.001, 0.005, 0.01, 0.05, 0.1, 0.5, 1, 2.5, 5, 7.5, 10, 15, 20, 25, 50, 75, 100, 125, 150, 200, 250, 500, 750, 1000, 1250, or 1500 × 10⁴ M⁻¹S⁻¹.

### Method for confirming protease cleavage

Examples of the method for evaluating the protease cleavage of the protease substrate or the protease cleavage sequence described in the present specification include methods described in Mol Cell Proteomics. 2014 Jun; 13 (6): 1585-97. doi: 10.1074/mcp.M113.033308. Epub 2014 Apr 4.

The type or concentration of the protease, a treatment temperature, and a treatment time for use in the evaluation can be appropriately selected. For example, the treatment can be performed at 37°C for 1 hour using PBS containing 1000 nM human uPA, PBS containing 1000 nM mouse uPA, PBS containing 500 nM human MT-SP1, or PBS containing 500 nM mouse MT-SP1.

Alternatively, a peptide array may be treated using serum (including human serum and mouse serum) instead of using the protease-containing solution, and a fluorescence value measured from a chip can be used.

The type or concentration of the serum, a treatment temperature, and a treatment time for use in the evaluation can be appropriately selected. For example, the treatment can be performed overnight at 37°C using human serum diluted into a concentration of 80% as a treatment solution.

A method for qualitatively confirming whether or not the protease cleavage sequence contained in a polypeptide has been cleaved by protease can involve subjecting a solution containing the protease cleavage sequence-containing polypeptide to SDS-PAGE (polyacrylamide gel electrophoresis) and measuring the molecular weights of the fragments. Also, this can be confirmed by comparing the molecular weights of a protease-untreated polypeptide and the polypeptide after protease treatment.

In the present specification, the term "cleaved" refers to a state where the polypeptide is separated into moieties after alteration of the protease cleavage sequence by protease and/or reduction of a cysteine-cysteine disulfide bond in the protease cleavage sequence. In the present specification, the term "uncleaved" refers to a state where moieties at both sides of the protease cleavage sequence in the polypeptide are linked in the absence of the protease cleavage of the protease cleavage sequence and/or in the absence of the reduction of a cysteine-cysteine disulfide bond in the protease cleavage sequence.

Cleaved fragments after protease treatment can be separated by electrophoresis such as SDS-PAGE and quantified to evaluate the protease cleavage sequence, and the cleavage rate of a molecule harboring the protease cleavage sequence. In a non-limiting aspect, the method for evaluating the cleavage rate of a molecule harboring the protease cleavage sequence is the following method: for example, in the case of evaluating the cleavage rate of an antibody variant harboring the protease cleavage sequence using recombinant human u-Plasminogen Activator/Urokinase (human uPA, huPA) (R&D Systems, Inc.; 1310-SE-010) or recombinant human Matriptase/ST14 Catalytic Domain (human MT-SP1, hMT-SP1) (R&D Systems, Inc.; 3946-SE-010), 100 µg/mL of the antibody variant is reacted with 40 nM huPA or 3 nM hMT-SP1 in PBS under a condition of 37°C for 1 hour, and then subjected to capillary electrophoresis immunoassay. The capillary electrophoresis immunoassay can employ, but not limited to, Wes (ProteinSimple, Inc.). An alternative to capillary electrophoresis immunoassay, such as SDS-PAGE, may be performed for separation, followed by detection by Western blotting, though the method is not limited thereto. Before and after cleavage, the light chain can be detected using anti-human lambda chain HRP-labeled antibody (Abcam PLC; ab9007) and may be detected using any antibody that can detect cleaved fragments. The area of each peak obtained after protease treatment is output using dedicated software for Wes (Compass for SW; ProteinSimple, Inc.), and the cleavage rate (%) of the antibody variant can be calculated according to the expression (Peak area of the cleaved light chain) × 100 / (Peak area of the cleaved light chain + Peak area of an uncleaved light chain). The calculation of the cleavage rate by use of the method mentioned above enables the cleavage rate to be compared among antibody variants harboring different cleavage sequences, for example, and also enables the cleavage rate of the same antibody variant to be compared among different animal models such as a normal mouse model and a tumor-transplanted mouse model.

For example, an antigen binding molecule having a linker comprising any of the protease cleavage sequences represented by SEQ ID NOs: 1 to 725 is useful as a protease substrate that is hydrolyzed by the action of protease. Specifically, in the present invention, linkers serving as the protease substrates listed in the present specification can be used. The linkers can be utilized as, for example, a library for selecting one having properties that meet the purpose for incorporation into an antigen binding molecule. Specifically, in order to selectively cleave the antigen binding molecule by a protease localized in a lesion, the linkers can be evaluated for their sensitivity to the protease. The linker-containing antigen binding molecule, after being administered to a living body, may come in contact with various proteases and then reach the lesion. Thus, such an antigen binding molecule desirably has sensitivity to the protease localized to the lesion while having as high resistance as possible to other proteases. In order to select a desired protease cleavage sequence according to the purpose, each protease substrate can be comprehensively analyzed in advance for sensitivity to various proteases to determine its protease resistance. A protease cleavage sequence that possesses necessary sensitivity and resistance can be found on the basis of the obtained protease resistance spectra.

Alternatively, the antigen binding molecule harboring the protease cleavage sequence undergoes not only an enzymatic effect ascribable to protease but also various environmental loads such as pH change, temperature, and oxidative-reductive stress and then reaches the lesion. As for such external factors, a protease cleavage sequence that possesses desired properties according to the purpose can also be selected on the basis of comparative information on resistance among the protease substrates.

In one embodiment of the present invention, a flexible linker is further attached to either one end or both ends of the protease cleavage sequence. The flexible linker at one end of the protease cleavage sequence can be referred to as a first flexible linker, and the flexible linker at the other end can be referred to as a second flexible linker. In a particular embodiment, the protease cleavage sequence and the flexible linker have any of the following formulas:
(protease cleavage sequence),
(first flexible linker)-(protease cleavage sequence),
(protease cleavage sequence)-(second flexible linker), and
(first flexible linker)-(protease cleavage sequence)-(second flexible linker).

The flexible linker according to the present embodiment is preferably a peptide linker. The first flexible linker and the second flexible linker each independently and arbitrarily exist and are identical or different flexible linkers each containing at least one flexible amino acid (Gly, etc.). The flexible linker contains, for example, a sufficient number of residues (amino acids arbitrarily selected from Arg, Ile, Gln, Glu, Cys, Tyr, Trp, Thr, Val, His, Phe, Pro, Met, Lys, Gly, Ser, Asp, Asn, Ala, etc., particularly Gly, Ser, Asp, Asn, and Ala, in particular, Gly and Ser, especially Gly, etc.) for the protease cleavage sequence to obtain the desired protease accessibility.

The flexible linker suitable for use at both ends of the protease cleavage sequence is usually a flexible linker that improves the access of protease to the protease cleavage sequence and elevates the cleavage efficiency of the protease. A suitable flexible linker may be readily selected and can be preferably selected from among different lengths such as 1 amino acid (Gly, etc.) to 20 amino acids, 2 amino acids to 15 amino acids, or 3 amino acids to 12 amino acids including 4 amino acids to 10 amino acids, 5 amino acids to 9 amino acids, 6 amino acids to 8 amino acids, or 7 amino acids to 8 amino acids. In some embodiments of the present invention, the flexible linker is a peptide linker of 1 to 7 amino acids.

Examples of the flexible linker include, but are not limited to, glycine polymers (G)n, glycine-serine polymers (including e.g., (GS)n, (GSGGS)n and (GGGS)n, wherein n is an integer of at least 1), glycine-alanine polymers, alanine-serine polymers, and other flexible linkers well known in conventional techniques.

Among them, glycine and glycine-serine polymers are receiving attention because these amino acids are relatively unstructured and easily function as neutral tethers between components.

Examples of the flexible linker consisting of the glycine-serine polymer include, but are not limited to,
Ser
Gly·Ser (GS)
Ser·Gly (SG)
Gly·Gly·Ser (GGS)
Gly·Ser·Gly (GSG)
Ser·Gly·Gly (SGG)
Gly·Ser·Ser (GSS)
Ser·Ser·Gly (SSG)
Ser·Gly·Ser (SGS)
Gly·Gly·Gly·Ser (GGGS)
Gly·Gly·Ser·Gly (GGSG)
Gly·Ser·Gly·Gly (GSGG)
Ser·Gly·Gly·Gly (SGGG)
Gly·Ser·Ser·Gly (GSSG)
Gly·Gly·Gly·Gly·Ser (GGGGS)
Gly·Gly·Gly·Ser·Gly (GGGSG)
Gly·Gly·Ser·Gly·Gly (GGSGG)
Gly·Ser·Gly·Gly·Gly (GSGGG)
Gly·Ser·Gly·Gly·Ser (GSGGS)
Ser·Gly·Gly·Gly·Gly (SGGGG)
Gly·Ser·Ser·Gly·Gly (GSSGG)
Gly·Ser·Gly·Ser·Gly (GSGSG)
Ser·Gly·Gly·Ser·Gly (SGGSG)
Gly·Ser·Ser·Ser·Gly (GSSSG)
Gly·Gly·Gly·Gly·Gly·Ser (GGGGGS)
Ser·Gly·Gly·Gly·Gly·Gly (SGGGGG)
Gly·Gly·Gly·Gly·Gly·Gly·Ser (GGGGGGS)
Ser·Gly·Gly·Gly·Gly·Gly·Gly (SGGGGGG)
(Gly·Gly·Gly·Gly·Ser (GGGGS))n
(Ser·Gly·Gly·Gly·Gly (SGGGG))n

In the present specification, the "association" can refer to, for example, a state where two or more polypeptide regions interact with each other. In general, a hydrophobic bond, a hydrogen bond, an ionic bond, or the like is formed between the intended polypeptide regions to form an associate. As one example of common association, an antibody typified by a natural antibody is known to retain a paired structure of a heavy chain variable region (VH) and a light chain variable region (VL) through a noncovalent bond or the like therebetween.

In the present specification, the "interface" usually refers to a face at which two regions associate or interact with each other. Amino acid residues forming the interface are usually one or more amino acid residues contained in each polypeptide region subjected to the association and more preferably refer to amino acid residues that approach each other upon association and participate in interaction. Specifically, the interaction includes a noncovalent bond such as a hydrogen bond, electrostatic interaction, or salt bridge formation between the amino acid residues approaching each other upon association.

In the present specification, the "amino acid residues forming the interface" specifically refers to amino acid residues contained in polypeptide regions constituting the interface. As one example, the polypeptide regions constituting the interface refer to polypeptide regions responsible for intramolecular or intermolecular selective binding in antibodies, ligands, receptors, substrates, etc. Examples of the amino acid residues forming the interface include, but are not limited to, amino acid residues approaching each other upon association. The amino acid residues approaching each other upon association can be found, for example, by analyzing the conformations of polypeptides and examining the amino acid sequences of polypeptide regions forming the interface upon association of the polypeptides.

In some embodiments of the present invention, VHH serving as the antigen binding domain associates with VL serving as the inhibiting domain. The amino acid residue involved in association with VL, in VHH can refer to, for example, an amino acid residue forming the interface between the VHH and the VL. Examples of the amino acid residue involved in association with VL, in VHH include, but are not limited to, amino acid residues at positions 37, 44, 45, and 47 (J. Mol. Biol. (2005) 350, 112-125). The activity of the VHH is inhibited by promoting the association between the VHH and the VL. Likewise, the amino acid residue involved in association with VHH, in VL can refer to, for example, an amino acid residue forming the interface between the VHH and the VL.

An amino acid residue involved in association with VL, in VHH can be altered in order to promote the association between the VHH and the VL. Examples of such an amino acid substitution include, but are not limited to, F37V, Y37V, E44G, Q44G, R45L, H45L, G47W, F47W, L47W, T47W, or/and S47W. Instead of altering each residue in VHH, VHH originally having an amino acid residue 37V, 44G, 45L, or/and 47W may be used.

Instead of the VHH amino acid, an amino acid residue involved in association with VHH, in VL may be altered, and amino acid alterations may also be introduced to both VHH and VL, as long as the purpose of promoting the association between the VHH and the VL can be achieved.

For the alteration of an amino acid in the amino acid sequence of a polypeptide, a method known in the art such as site-directed mutagenesis (Kunkel et al. (Proc. Natl. Acad. Sci. USA (1985) 82, 488-492)) or overlap extension PCR can be appropriately adopted. A plurality of methods known in the art can also be adopted as alteration methods for substituting an amino acid by an amino acid other than a natural amino acid (Annu. Rev. Biophys. Biomol. Struct. (2006) 35, 225-249; and Proc. Natl. Acad. Sci. U.S.A. (2003) 100 (11), 6353-6357). For example, a tRNA-containing cell-free translation system (Clover Direct (Protein Express)) having a non-natural amino acid bound with amber suppressor tRNA complementary to UAG codon (amber codon), which is a stop codon, is also preferably used.

In some alternative embodiments of the present invention, the antigen binding domain and the inhibiting domain can be associated with each other by using VHH as the antigen binding domain and using VH or VHH as the inhibiting domain. An amino acid residue involved in association with VH or VHH serving as the inhibiting domain, in VHH serving as the antigen binding domain can be identified and altered in order to promote the association of the antigen binding domain VHH with the inhibiting domain VH or VHH Also, an amino acid residue involved in association with VHH serving as the antigen binding domain, in VH or VHH serving as the inhibiting domain, can be identified and altered.

In the case of using a single-domain antibody other than VHH as the antigen binding domain, an amino acid residue involved in association, in the antigen binding domain or the inhibiting domain can also be identified and altered similarly to above.

In a particular embodiment, the protease cleavage sequence is located within the antibody constant region in the antigen binding molecule. In this case, the protease cleavage sequence can be located within the antibody constant region such that the antigen binding domain is released by protease cleavage. In a specific embodiment, the protease cleavage sequence is located within an antibody heavy chain constant region contained in the antigen binding molecule, and more specifically located on the antigen binding domain side with respect to amino acid position 140 (EU numbering) in the antibody heavy chain constant region, preferably on the antigen binding domain side with respect to amino acid position 122 (EU numbering) in the antibody heavy chain constant region. In an alternative specific embodiment, the protease cleavage sequence is located within an antibody light chain constant region contained in the antigen binding molecule, and more specifically located on the antigen binding domain side with respect to amino acid position 130 (Kabat numbering) in the antibody light chain constant region, preferably on the antigen binding domain side with respect to amino acid position 113 (Kabat numbering) in the antibody light chain constant region.

In a particular embodiment, the linker that is cleavable by protease is located near the boundary between the variable region and the constant region or near the boundary between CH1 and CH2 in the constant region. The phrase "near the boundary between the variable region and the constant region" refers to a site that resides upstream or downstream of the linking site between VH and CH1 or upstream or downstream of the linking site between VL and CL and does not largely influence the secondary structure of the antigen binding domain. This site comprises an elbow hinge region (from amino acid positions 109 (EU numbering) to 140 (EU numbering)). The phrase "near the boundary between CH1 and CH2" refers to a moiety that resides upstream or downstream of the linking site between CH1 and CH2 and does not largely influence the secondary structure of the antigen binding domain. This moiety comprises an upper hinge region (from amino acid positions 215 (EU numbering) to 220 (EU numbering)) and a lower hinge region (from amino acid positions 221 (EU numbering) to 230 (EU numbering)).

In a more specific embodiment, the linker that is cleavable by protease is located near the boundary between the antigen binding domain and an antibody constant region in the antigen binding molecule. The phrase "near the boundary between the antigen binding domain and the antibody constant region" can refer to near the boundary between the antigen binding domain and an antibody heavy chain constant region, or near the boundary between the antigen binding domain and an antibody light chain constant region. When the antigen binding domain is a single-domain antibody prepared from VH, or VHH and is connected to an antibody heavy chain constant region, the phrase "near the boundary between the antigen binding domain and the antibody constant region" can refer to between amino acid position 101 (Kabat numbering) of the single-domain antibody and amino acid position 140 (EU numbering) of the antibody heavy chain constant region and can preferably refer to between amino acid position 109 (Kabat numbering) of the single-domain antibody and amino acid position 122 (EU numbering) of the antibody heavy chain constant region. When the antigen binding domain is a single-domain antibody prepared from VH, or VHH and is connected to an antibody light chain constant region, the phrase "near the boundary between the antigen binding domain and the antibody light chain constant region" can refer to between amino acid position 101 (Kabat numbering) of the single-domain antibody and amino acid position 130 (Kabat numbering) of the antibody light chain constant region and can preferably refer to between amino acid position 109 (Kabat numbering) of the single-domain antibody and amino acid position 113 (Kabat numbering) of the antibody light chain constant region. When the antigen binding domain is a single-domain antibody prepared from VL, the phrase "near the boundary between the antigen binding domain and the antibody constant region" refers to a moiety that resides upstream or downstream of the linking site between VHH and CH2 and does not largely influence the secondary structure of the antigen binding domain. This moiety comprises a lower hinge region and starts from amino acid position 96 (Kabat numbering) of the single-domain antibody, preferably from amino acid position 104 (Kabat numbering) of the single-domain antibody.

In another embodiment of the present invention, the cleavage site and/or the protease cleavage sequence is located on the variable region side compared with amino acid position 140 (EU numbering) in an antibody heavy chain constant region, preferably on the variable region side compared with amino acid position 122 (EU numbering) in an antibody heavy chain constant region. In some specific embodiments, the cleavage site and/or the protease cleavage sequence is introduced at any position in a sequence from amino acid position 118 (EU numbering) to amino acid position 140 (EU numbering) in an antibody heavy chain constant region. In other more specific embodiments, the cleavage site and/or the protease cleavage sequence is located on the variable region side compared with amino acid position 130 (Kabat numbering) in an antibody light chain constant region, preferably on the variable region side compared with amino acid position 113 (Kabat numbering) in an antibody light chain constant region, or amino acid position 112 (Kabat numbering) in an antibody light chain constant region. In some specific embodiments, the cleavage site and/or the protease cleavage sequence is introduced at any position in a sequence from amino acid position 108 (Kabat numbering) to amino acid position 131 (Kabat numbering) in an antibody light chain constant region.

In one embodiment, the cleavage site and/or the protease cleavage sequence is located near the boundary between antibody VL and an antibody constant region. The phrase "near the boundary between antibody VL and an antibody light chain constant region" can refer to between amino acid position 96 (Kabat numbering) of the antibody VL and amino acid position 130 (EU numbering) (Kabat numbering position 130) of the antibody light chain constant region and can preferably refer to between amino acid position 104 (Kabat numbering) of the antibody VL and amino acid position 113 (EU numbering) (Kabat numbering position 113) of the antibody light chain constant region, or between amino acid position 105 (Kabat numbering) of the antibody VL and amino acid position 112 (EU numbering) (Kabat numbering position 112) of the antibody light chain constant region. When antibody VL is connected to an antibody heavy chain constant region, the "near the boundary between antibody VL and an antibody heavy chain constant region" can refer to between amino acid position 96 (Kabat numbering) of the antibody VL and amino acid position 140 (EU numbering) of the antibody heavy chain constant region and can preferably refer to between amino acid position 104 (Kabat numbering) of the antibody VL and amino acid position 122 (EU numbering) of the antibody heavy chain constant region, or between amino acid position 105 (Kabat numbering) of the antibody VL and amino acid position 122 (EU numbering) of the antibody heavy chain constant region.

In one embodiment, the cleavage site and/or the protease cleavage sequence is introduced near the CH2/CH3 interface of an antibody heavy chain constant region. In this context, the phrase "near the CH2/CH3 interface" refers to a region from amino acid positions 335 (EU numbering) to 345 (EU numbering).

A plurality of cleavage sites and/or protease cleavage sequences can be established in the antigen binding molecule and can be established at a plurality of locations selected from, for example, within an antibody constant region, within antibody VH, within antibody VL, near the boundary between antibody VH and an antibody constant region, and near the boundary antibody VL and an antibody constant region. Those skilled in the art can change the form of a molecule comprising antibody VH, antibody VL, and antibody constant regions, for example, by replacing the antibody VH with the antibody VL, with reference to the present invention. Such a molecular form is included in the scope of the present invention.

The term "IgG antibody-like molecule" used in the present specification is used to define a molecule having moieties substantially similar in structure to constant domains or constant regions as in an IgG antibody, and moieties substantially similar in structure to variable domains or variable regions as in the IgG antibody, and having conformation substantially similar to that of the IgG antibody. However, in the present specification, the "IgG antibody-like molecule" may or may not exert antigen binding activity while retaining the structures similar to those of the IgG antibody.

When the antigen binding molecule is an IgG antibody-like molecule, antigen binding domains may be respectively established at moieties corresponding to two variable regions of the IgG antibody. Such an embodiment should be understandable by those skilled in the art with reference to the present invention. The antigen binding domains incorporated in both arms may have the same antigen binding specificity or may differ in antigen binding specificity. Such an embodiment should be understandable by those skilled in the art with reference to the present invention. It is obvious that these embodiments are included in the scope of the present invention.

In the present specification, the term "specificity" refers to a property by which one of specifically binding molecules does not substantially bind to a molecule other than its one or more binding partner molecules. This term is also used when the antigen binding domain has specificity for an epitope contained in a particular antigen. The term is also used when the antigen binding domain has specificity for a particular epitope among a plurality of epitopes contained in a certain antigen. In this context, the term "not substantially bind" is determined according to the method described in the section about binding activity and means that the binding activity of a specific binding molecule for a molecule other than the binding partner(s) is 80% or less, usually 50% or less, preferably 30% or less, particularly preferably 15% or less, of its binding activity for the binding partner molecule(s).

The "treatment" (and its grammatically derived words, for example, "treat" and "treating") used in the present specification means clinical intervention that intends to alter the natural course of an individual to be treated and can be carried out both for prevention and during the course of a clinical pathological condition. The desirable effect of the treatment includes, but is not limited to, the prevention of the development or recurrence of a disease, the alleviation of symptoms, the attenuation of any direct or indirect pathological influence of the disease, the prevention of metastasis, reduction in the rate of progression of the disease, recovery from or alleviation of a disease condition, and ameliorated or improved prognosis. In some embodiments, the pharmaceutical composition of the present invention is used for delaying the onset of a disease or delaying the progression of the disease.

In the present invention, the pharmaceutical composition usually refers to a drug for the treatment or prevention of a disease or for examination or diagnosis. In the present invention, in the case of using a pharmaceutical composition in combination with the administration of an additional component, the pharmaceutical composition can be administered concurrently with, separately from, or continuously with the administration of the additional component. The pharmaceutical composition of the present invention may contain the additional component as a component.

The pharmaceutical composition of the present invention can be formulated by use of a method known to those skilled in the art. For example, the pharmaceutical composition can be parenterally used in an injection form of a sterile solution or suspension with water or any of other pharmaceutically acceptable liquids. The pharmaceutical composition can be formulated, for example, by appropriately combining the polypeptide with a pharmacologically acceptable carrier or medium, specifically, sterile water or physiological saline, a plant oil, an emulsifier, a suspending agent, a surfactant, a stabilizer, a flavoring agent, an excipient, a vehicle, an antiseptic, a binder, etc. and mixing them into a unit dosage form required for generally accepted pharmaceutical practice. The amount of the active ingredient in these formulations is set so as to give an appropriate volume in a prescribed range.

A sterile composition for injection can be formulated according to usual pharmaceutical practice using a vehicle such as injectable distilled water. Examples of the injectable aqueous solution include isotonic solutions containing physiological saline, glucose, or other adjuvants (e.g., D-sorbitol, D-mannose, D-mannitol, and sodium chloride). The aqueous solution can be used in combination with an appropriate solubilizer, for example, an alcohol (ethanol, etc.), a polyalcohol (propylene glycol, polyethylene glycol, etc.), or a nonionic surfactant (Polysorbate 80(TM), HCO-50, etc.).

Examples of the oil solution include sesame oil and soybean oil. The oil solution can also be used in combination with benzyl benzoate and/or benzyl alcohol as a solubilizer. The oil solution can be supplemented with a buffer (e.g., a phosphate buffer solution and a sodium acetate buffer solution), a soothing agent (e.g., procaine hydrochloride), a stabilizer (e.g., benzyl alcohol and phenol), and an antioxidant. The prepared injection solution is usually filled into an appropriate ampule.

The pharmaceutical composition of the present invention is preferably administered through a parenteral route. For example, a composition having an injection, transnasal, transpulmonary, or percutaneous dosage form is administered. The pharmaceutical composition can be administered systemically or locally by, for example, intravenous injection, intramuscular injection, intraperitoneal injection, or subcutaneous injection.

The administration method can be appropriately selected according to the age and symptoms of a patient. The dose of the pharmaceutical composition of the present invention can be set to the range of, for example, 0.0001 mg to 1000 mg per kg body weight per dose. Alternatively, the dose of the pharmaceutical composition containing the polypeptide can be set to a dose of, for example, 0.001 to 100000 mg per patient. However, the present invention is not necessarily limited by these numerical values. Although the dose and the administration method vary depending on the body weight, age, symptoms, etc. of a patient, those skilled in the art can set an appropriate dose and administration method in consideration of these conditions.

The polynucleotide according to the present invention is usually carried by (or inserted in) an appropriate vector and transfected into host cells. The vector is not particularly limited as long as the vector can stably retain an inserted nucleic acid. For example, when *E. coli* is used as the host, a pBluescript vector (manufactured by Stratagene Corp.) or the like is preferred as a vector for cloning. In the case of using the vector for the purpose of producing a polypeptide (e.g., a chimeric receptor, an IgG antibody, a bispecific antibody, and an antigen binding molecule) for use in carrying out the present invention, an expression vector is particularly useful. The expression vector is not particularly limited as long as the vector permits expression of the polypeptide *in vitro, in E. coli,* in cultured cells, or in organism individuals. The expression vector is preferably, for example, a pBEST vector (manufactured by Promega Corp.) for *in vitro* expression, a pET vector (manufactured by Invitrogen Corp.) for *E. coli,* a pME18S-FL3 vector (GenBank Accession No. AB009864) for cultured cells, and a pME18S vector (Mol Cell Biol. 8: 466-472 (1988)) for organism individuals. The insertion of the DNA of the present invention into the vector can be performed by a routine method, for example, ligase reaction using restriction sites (Current protocols in Molecular Biology edit. Ausubel et al. (1987) Publish. John Wiley & amp; Sons. Section 11.4-11.11).

The host cells are not particularly limited, and various host cells are used according to the purpose. Examples of the cells for expressing the polypeptide can include bacterial cells (e.g., *Streptococcus, Staphylococcus, E. coli, Streptomyces,* and *Bacillus subtilis*)*,* fungal cells (e.g., yeasts and *Aspergillus*)*,* insect cells (e.g., *Drosophila* S2 and *Spodoptera* SF9), animal cells (e.g., CHO, COS, HeLa, C127, 3T3, BHK, HEK293, and Bowes melanoma cells) and plant cells. The transfection of the vector to the host cells may be performed by a method known in the art, for example, a calcium phosphate precipitation method, an electroporation method (Current protocols in Molecular Biology edit. Ausubel et al., (1987) Publish. John Wiley & amp; Sons. Section 9.1-9.9), a Lipofectamine method (manufactured by GIBCO-BRL/Thermo Fisher Scientific Inc.), or a microinjection method.

An appropriate secretory signal can be incorporated into the polypeptide of interest in order to secrete the polypeptide expressed in the host cells to the lumen of the endoplasmic reticulum, periplasmic space, or an extracellular environment. The signal may be endogenous to the polypeptide of interest or may be a foreign signal.

When the polypeptide of the present invention is secreted into a medium, the recovery of the polypeptide in the production method is performed by the recovery of the medium. When the polypeptide of the present invention is produced into cells, the cells are first lysed, followed by the recovery of the polypeptide.

A method known in the art including ammonium sulfate or ethanol precipitation, acid extraction, anion- or cation-exchange chromatography, phosphocellulose chromatography, hydrophobic interaction chromatography, affinity chromatography, hydroxyapatite chromatography, and lectin chromatography can be used for recovering and purifying the polypeptide of the present invention from the recombinant cell cultures.

Examples of the antigen binding domain used in some embodiments of the present invention include a single-domain antibody. In these embodiments, the antigen binding activity of the single-domain antibody can be inhibited by associating with particular VL, associating with particular VH, or associating with particular VHH. The present invention also relates to a method for screening for such a single-domain antibody.

VL, VH or VHH having a known sequence, for example, VL, VH or VHH having a sequence registered in the IMGT or Kabat database, can be used as the VL, the VH or the VHH that inhibits the antigen binding activity of the single-domain antibody. Also, a VL, VH or VHH sequence newly identified from a human antibody library or the like can be used. The VL, the VH or the VHH that inhibits the binding activity of the single-domain antibody can be selected by preparing a protein by the combination of these sequences and measuring the binding activity by use of the method described above.

In one embodiment, the present invention provides a method for screening for a single-domain antibody whose antigen binding activity can be inhibited by associating with particular VL, comprising the following steps:
(a) obtaining a single-domain antibody having target antigen binding activity;
(b) associating the single-domain antibody obtained in the step (a) with a particular VL; and
(c) confirming that the binding activity of the single-domain antibody associated with the particular VL in the step (b) against the antigen is weakened or lost.

In the present invention, the phrase "binding activity is weakened" means that the binding activity against the target antigen is decreased as compared with that before the association, and the degree of this decrease is not limited.

In one embodiment, the present invention provides a method for screening for a single-domain antibody whose antigen binding activity can be inhibited by associating with particular VH, comprising the following steps:
(a) obtaining a single-domain antibody having target antigen binding activity;
(b) associating the single-domain antibody obtained in the step (a) with a particular VH; and
(c) confirming that the binding activity of the single-domain antibody associated with the particular VH in the step (b) against the antigen is weakened or lost.

In the present invention, the phrase "binding activity is weakened" means that the binding activity against the target antigen is decreased as compared with that before the association, and the degree of this decrease is not limited.

In one embodiment, the present invention provides a method for screening for a single-domain antibody whose antigen binding activity can be inhibited by associating with particular VHH, comprising the following steps:
(a) obtaining a single-domain antibody having target antigen binding activity;
(b) associating the single-domain antibody obtained in the step (a) with a particular VHH; and
(c) confirming that the binding activity of the single-domain antibody associated with the particular VHH in the step (b) against the antigen is weakened or lost.

In the present invention, the phrase "binding activity is weakened" means that the binding activity against the target antigen is decreased as compared with that before the association, and the degree of this decrease is not limited.

Examples of the method for associating the single-domain antibody with the particular VL, VH or VHH include a method of designing a molecule having the sequence of the single-domain antibody as a substitute for the sequence of one of VH and VL in an antibody or an antibody fragment comprising both VH and VL, such as a complete antibody, Fab, Fab', or (Fab)₂, and expressing a polypeptide having the sequence.

The present invention also relates to a method for producing a single-domain antibody whose antigen binding activity is inhibited by promoting the association of the single-domain antibody with particular VL, VH or VHH, promoting the association of the single-domain antibody with particular VL, promoting the association of the single-domain antibody with particular VH, or promoting the association of the single-domain antibody with particular VHH, in addition to screening for a single-domain antibody whose antigen binding activity is inhibited by associating with particular VL, associating with particular VH, or associating with particular VHH.

In one embodiment, the present invention provides a method for producing a single-domain antibody whose antigen binding activity is inhibited by associating with particular VL, comprising the following step:
(a) substituting an amino acid residue in a single-domain antibody that involves in association with antibody VL, to prepare an single-domain antibody variant retaining the binding activity of the single-domain antibody against the target antigen.

In a particular embodiment, the present invention provides the method for producing a single-domain antibody whose antigen binding activity is inhibited by associating with particular VL, further comprising the following steps:
(b) associating the single-domain antibody variant prepared in the step (a) with the particular VL; and
(c) confirming that the antigen binding activity of the single-domain antibody variant associated with the VL is weakened or lost.

In the present invention, the phrase "binding activity is weakened" means that the binding activity against the target antigen is decreased as compared with that before the association, and the degree of this decrease is not limited.

In one embodiment, the present invention provides a method for producing a single-domain antibody whose antigen binding activity is inhibited by associating with particular VH, comprising the following step:
(a) substituting an amino acid residue in a single-domain antibody that involves in association with antibody VH, to prepare an single-domain antibody variant retaining the binding activity of the single-domain antibody against the target antigen.

In a particular embodiment, the present invention provides the method for producing a single-domain antibody whose antigen binding activity is inhibited by associating with particular VH, further comprising the following steps:
(b) associating the single-domain antibody variant prepared in the step (a) with the particular VH; and
(c) confirming that the antigen binding activity of the single-domain antibody variant associated with the VH is weakened or lost.

In the present invention, the phrase "binding activity is weakened" means that the binding activity against the target antigen is decreased as compared with that before the association, and the degree of this decrease is not limited.

In one embodiment, the present invention provides a method for producing a single-domain antibody whose antigen binding activity is inhibited by associating with particular VHH, comprising the following step:
(a) substituting an amino acid residue in a single-domain antibody that involves in association with VHH, to prepare an single-domain antibody variant retaining the binding activity of the single-domain antibody against the target antigen.

In a particular embodiment, the present invention provides the method for producing a single-domain antibody whose antigen binding activity is inhibited by associating with particular VHH, further comprising the following steps:
(b) associating the single-domain antibody variant prepared in the step (a) with the particular VHH; and
(c) confirming that the antigen binding activity of the single-domain antibody variant associated with the VHH is weakened or lost.

In the present invention, the phrase "binding activity is weakened" means that the binding activity against the target antigen is decreased as compared with that before the association, and the degree of this decrease is not limited.

The step of associating the single-domain antibody with the particular VL, VH or VHH is performed by a method of designing a molecule having the sequence of the single-domain antibody as a substitute for the sequence of one of VH and VL in an antibody or an antibody fragment comprising both VH and VL, such as a complete antibody, Fab, Fab', or (Fab)₂, and expressing a polypeptide having the sequence.

According to a certain embodiment of the present invention, the single-domain antibody of the present invention whose antigen binding activity is inhibited or lost by associating with particular VL, VH or VHH can be obtained from a library comprising a plurality of fusion polypeptides of single-domain antibodies each linked to a first association sustaining domain.

In the present specification, an embodiment of the "library" can provide a library that permits efficient obtainment of a single-domain antibody whose antigen binding activity is inhibited or lost by associating with particular VL, VH or VHH

In the present specification, the "library" refers to a set of a plurality of fusion polypeptides having different sequences, or nucleic acids or polynucleotides encoding these fusion polypeptides. A plurality of fusion polypeptides contained in the library are fusion polypeptides differing in sequence from each other, not having a single sequence.

In the present specification, the term "differing in sequence from each other" in a plurality of fusion polypeptides differing in sequence from each other means that the individual fusion polypeptides in the library have distinct sequences. More preferably, the term means that the single-domain antibody moieties of the individual fusion polypeptides in the library have distinct sequences. Specifically, the number of the distinct sequences in the library reflects the number of independent clones differing in sequences in the library and is also referred to as a "library size". The library size of a usual phage display library is 10⁶ to 10¹² and may be expanded to 10¹⁴ by the application of a technique known in the art such as a ribosome display method. However, the actual number of phage particles for use in panning selection for the phage library is usually 10 to 10,000 times larger than the library size. This excessive multiple, also called the "number of equivalents of the library", represents that 10 to 10,000 individual clones may have the same amino acid sequence. Accordingly, the term "differing in sequence from each other" according to the present invention means that the individual polypeptides in the library excluding the number of equivalents of the library have distinct sequences and more specifically means that the library has 10⁶ to 10¹⁴ molecules, preferably 10⁷ to 10¹² molecules, of polypeptides differing in sequence from each other.

The term "plurality of" in the library consisting essentially of a plurality of fusion polypeptides according to the present invention usually refers to a set of two or more types of substances as to, for example, the polypeptide, polynucleotide molecule, vector, or virus of the present invention. Provided that, for example, two or more substances differ in particular trait from each other, this means that the substances are of two or more types. Examples thereof can include a mutant amino acid observed at a particular amino acid position in an amino acid sequence. For example, two or more polypeptides of the present invention having substantially the same, preferably identical sequences, except for particular mutant amino acids at surface-exposed, highly diverse amino acid positions are regarded as a plurality of polypeptides of the present invention. In another example, two or more polynucleotide molecules of the present invention having substantially the same, preferably identical sequences except for bases encoding particular mutant amino acids at surface-exposed, highly diverse amino acid positions are regarded as a plurality of polynucleotide molecules of the present invention.

A panning method that utilizes phage vectors is also preferably used as a method for screening the fusion polypeptides with binding activity as an index. A gene encoding each single-domain antibody and a gene encoding an IgG antibody CH1 domain or a light chain constant region can be linked in an appropriate form to form a fusion polypeptide. Genes encoding the fusion polypeptides thus formed can be inserted into phage vectors to obtain phages expressing the fusion polypeptides on the surface. After contact of the phages with the desired antigen, phages bound with the antigen can be recovered to recover DNAs encoding fusion polypeptides having the binding activity of interest. This operation can be repeated, if necessary, to enrich fusion polypeptides having the desired binding activity.

In addition to the phage display method, a technique using a cell-free translation system, a technique of presenting fusion polypeptides on cell or virus surface, a technique of using an emulsion, and the like are known as techniques of obtaining fusion polypeptides by panning using a library. For example, a ribosome display method of forming a complex of mRNA and a translated protein via ribosome by the removal of a stop codon, etc., a cDNA or mRNA display method of covalently binding a gene sequence to a translated protein using a compound such as puromycin, or a CIS display method of forming a complex of a gene and a translated protein using a nucleic acid binding protein can be used as the technique using a cell-free translation system. For example, the phage display method as well as an *E. coli* display method, a gram-positive bacterium display method, a yeast display method, a mammalian cell display method, or a virus display method can be used as the technique of presenting fusion polypeptides on cell or virus surface. For example, an *in vitro* virus display method using an emulsion containing a gene and a translation-related molecule can be used as the technique using an emulsion. These methods are already known in the art (Nat Biotechnol. 2000 Dec; 18(12): 1287-92, Nucleic Acids Res. 2006; 34(19): e127, Proc Natl Acad Sci U S A. 2004 Mar 2; 101(9): 2806-10, Proc Natl Acad Sci U S A. 2004 Jun 22; 101(25): 9193-8, Protein Eng Des Sel. 2008 Apr; 21(4): 247-55, Proc Natl Acad Sci U S A. 2000 Sep 26; 97(20): 10701-5, MAbs. 2010 Sep-Oct; 2(5): 508-18, Methods Mol Biol. 2012; 911: 183-98).

In an alternative embodiment, the present invention provides a library comprising a plurality of fusion polypeptides of single-domain antibodies linked to an IgG antibody light chain constant region, wherein the single-domain antibodies include a single-domain antibody whose antigen binding activity is inhibited or lost by associating with particular VL, VH or VHH, and a method for screening the library for a single-domain antibody whose antigen binding activity can be inhibited or could lost by associating with particular VL, VH or VHH

The "antigen binding activity of a predetermined value or lower" can refer to, for example, antigen binding activity that falls below a predetermined reference when the antigen binding activity is measured by the method listed in the present specification. Likewise, the "antigen binding activity of a predetermined value or higher" can refer to, for example, antigen binding activity that exceeds a predetermined reference when the antigen binding activity is measured by the method listed in the present specification. A fusion polypeptide having the antigen binding activity of a predetermined value or higher binds more strongly to the antigen than a fusion polypeptide having the antigen binding activity of a predetermined value or lower.

Hereinafter, some embodiments using an IgG antibody CH1 domain as the first association sustaining domain and using IgG antibody CL as the second association sustaining domain will be described.

A fusion polypeptide comprising the single-domain antibody of interest can be screened for from a library comprising a plurality of fusion polypeptides of single-domain antibodies each linked to an IgG antibody CH1 domain.

In some embodiments, the present invention provides a library comprising a plurality of fusion polypeptides of single-domain antibodies each linked to an IgG antibody CH1 domain, wherein the single-domain antibodies include a single-domain antibody whose antigen binding activity is inhibited or lost by associating with particular VL, VH or VHH, and a method for screening the library for a fusion polypeptide comprising a single-domain antibody whose antigen binding activity can be inhibited or could lost by associating with particular VL, VH or VHH

In a particular embodiment, the present invention provides a method for screening for a fusion polypeptide comprising a single-domain antibody whose antigen binding activity can be inhibited or could lost by associating with particular VL, from a library comprising a plurality of fusion polypeptides of single-domain antibodies each linked to an IgG antibody CH1 domain. Specifically, the present invention provides a method for screening for a single-domain antibody, comprising the following steps:
(a) *in vitro* displaying the fusion polypeptides of the library according to the present invention;
(b) providing an association partner of an IgG antibody light chain constant region fused with the particular VL;
(c) associating the fusion polypeptides displayed in the step (a) with the association partner provided in the step (b) and selecting a fusion polypeptide that does not bind to the antigen or has antigen binding activity of a predetermined value or lower in a state where the single-domain antibody associates with the VL; and
(d) selecting, from the fusion polypeptides thus selected in the step (c), a fusion polypeptide that binds to the antigen or has antigen binding activity of a predetermined value or higher in a state where the single-domain antibody contained therein does not associate with the VL.

The association partner provided in the step (b) further comprises a protease cleavage sequence. In this case, in the step (d), the association of the single-domain antibody with the VL is canceled by protease treatment, and the antigen binding activity of the single-domain antibody may be confirmed in a state where the single-domain antibody does not associate with the VL. The protease cleavage sequence in the association partner is not limited by its position as long as the association of the single-domain antibody with the VL is canceled by cleavage. As an example of the position, the protease cleavage sequence may be located, for example, near the boundary between the VL and the IgG antibody light chain constant region in the association partner, preferably at any position between amino acid position 96 (Kabat numbering) of the VL and amino acid position 130 (EU numbering) (Kabat numbering position 130) of the antibody light chain constant region, more preferably at any position between amino acid position 104 (Kabat numbering) of the VL and amino acid position 113 (EU numbering) (Kabat numbering position 113) of the antibody light chain constant region.

Instead of using the association partner comprising a protease cleavage sequence, the protease cleavage sequence may be introduced into the fusion polypeptides in the library, and the fusion polypeptides can be cleaved by protease so that the association of the single-domain antibody with the VL is canceled. The protease cleavage sequence in each fusion polypeptide is not limited by its position as long as the association of the single-domain antibody with the VL is canceled by cleavage and the single-domain antibody retains its antigen binding activity even after the cleavage. As an example of the position, the protease cleavage sequence may be located, for example, near the boundary between the single-domain antibody and the IgG antibody CH1 domain in the fusion polypeptide.

In the step (d), the full lengths of the fusion polypeptides selected in the step (c) or their moieties comprising the single-domain antibodies may be displayed again, and the antigen binding activity of the single-domain antibody can be confirmed in a state where the single-domain antibody does not associate with the VL.

In a particular embodiment, the present invention provides a method for screening for a fusion polypeptide comprising a single-domain antibody whose antigen binding activity can be inhibited or could lost by associating with particular VH, from a library comprising a plurality of fusion polypeptides of single-domain antibodies each linked to an IgG antibody light chain constant region. Specifically, the present invention provides a method for screening for a fusion polypeptide comprising a single-domain antibody, comprising the following steps:
(a) *in vitro* displaying the fusion polypeptides of the library according to the present invention;
(b) providing an association partner of an IgG antibody CH1 domain fused with the particular VH;
(c) associating the fusion polypeptides displayed in the step (a) with the association partner provided in the step (b) and selecting a fusion polypeptide that does not bind to the antigen or has antigen binding activity of a predetermined value or lower in a state where the single-domain antibody associates with the VH; and
(d) selecting, from the fusion polypeptides thus selected in the step (c), a fusion polypeptide that binds to the antigen or has antigen binding activity of a predetermined value or higher in a state where the single-domain antibody contained therein does not associate with the VH

The association partner provided in the step (b) further comprises a protease cleavage sequence. In this case, in the step (d), the association of the single-domain antibody with the VH is canceled by protease treatment, and the antigen binding activity of the single-domain antibody may be confirmed in a state where the single-domain antibody does not associate with the VH The protease cleavage sequence in the association partner is not limited by its position as long as the association of the single-domain antibody with the VH is canceled by cleavage. As an example of the position, the protease cleavage sequence may be located, for example, near the boundary between the VH and the IgG antibody CH1 domain in the association partner, preferably at any position between amino acid position 101 (Kabat numbering) of the VH and amino acid position 140 (EU numbering) of the antibody heavy chain constant region, more preferably at any position between amino acid position 109 (Kabat numbering) of the VH and amino acid position 122 (EU numbering) of the antibody heavy chain constant region.

Instead of using the association partner comprising a protease cleavage sequence, the protease cleavage sequence may be introduced into the fusion polypeptides in the library, and the fusion polypeptides can be cleaved by protease so that the association of the single-domain antibody with the VH is canceled. The protease cleavage sequence in each fusion polypeptide is not limited by its position as long as the association of the single-domain antibody with the VH is canceled by cleavage and the single-domain antibody retains its antigen binding activity even after the cleavage. As an example of the position, the protease cleavage sequence may be located, for example, near the boundary between the single-domain antibody and the IgG antibody light chain constant region in the fusion polypeptide.

In the step (d), the full lengths of the fusion polypeptides selected in the step (c) or their moieties comprising the single-domain antibodies may be displayed again, and the antigen binding activity of the single-domain antibody can be confirmed in a state where the single-domain antibody does not associate with the VH

An amino acid contained in each amino acid sequence described in the present invention may be posttranslationally modified (e.g., the modification of N-terminal glutamine to pyroglutamic acid by pyroglutamylation is a modification well known to those skilled in the art). Such an amino acid sequence containing the posttranslationally modified amino acid is also included in the amino acid sequence described in the present invention, as a matter of course.

A method for preparing an antibody having the desired binding activity is known to those skilled in the art. In the present invention, an antigen binding molecule against a molecule expressed on the surface of target cells (lesion cells) as an antigen (target antigen) can be used. When the target cells are tumor cells or cancer cells, the antigen is illustrated as a tumor antigen in the present specification. A method for preparing an antibody binding to the tumor antigen will be illustrated below.

The antibody binding to the tumor antigen can be obtained as a polyclonal or a monoclonal antibody by use of an approach known in the art. A mammal-derived monoclonal antibody can be preferably prepared as the antibody. The mammal-derived monoclonal antibody encompasses, for example, those produced by hybridomas and those produced by host cells transformed with expression vectors containing an antibody gene by a genetic engineering approach.

The monoclonal antibody-producing hybridomas can be prepared by use of a technique known in the art, for example, as follows: mammals are immunized with a tumor antigen protein used as a sensitizing antigen according to a usual immunization method. Immunocytes thus obtained are fused with parental cells known in the art by a usual cell fusion method. Next, cells producing a monoclonal antibody can be screened for by a usual screening method to select hybridomas producing the anti-tumor antigen antibody.

Specifically, the monoclonal antibody is prepared, for example, as follows: first, a tumor antigen gene can be expressed to obtain a tumor antigen protein for use as a sensitizing antigen for antibody obtainment. Specifically, a gene sequence encoding the tumor antigen is inserted into expression vectors known in the art, with which appropriate host cells are then transformed. The desired human tumor antigen protein is purified from the host cells or from a culture supernatant thereof by a method known in the art. In order to obtain a soluble tumor antigen from a culture supernatant, for example, a protein lacking a moiety constituting a hydrophobic region in a tumor antigen polypeptide sequence can be used. Also, purified natural GPC3 protein may be used as a sensitizing antigen.

This purified tumor antigen protein can be used as the sensitizing antigen for use in the immunization of mammals. A partial peptide of the tumor antigen can also be used as the sensitizing antigen. This partial peptide may be obtained by chemical synthesis from the amino acid sequence of the human tumor antigen. Alternatively, the partial peptide may be obtained by the incorporation of a portion of the tumor antigen gene into expression vectors followed by its expression. Furthermore, the partial peptide can also be obtained by the degradation of the tumor antigen protein with a proteolytic enzyme. The region and size of the tumor antigen peptide for use as such a partial peptide are not particularly limited by specific embodiments. The number of amino acids constituting the peptide used as the sensitizing antigen is preferably at least 5 or more, for example, 6 or more or 7 or more. More specifically, a peptide of 8 to 50, preferably 10 to 30 residues can be used as the sensitizing antigen.

Also, a fusion protein comprising a desired partial polypeptide or peptide of the tumor antigen protein fused with a different polypeptide can be used as the sensitizing antigen. For example, an antibody Fc fragment or a peptide tag can be preferably used for producing the fusion protein for use as the sensitizing antigen. Two or more types of genes respectively encoding the desired polypeptide fragments are fused in frame, and the fusion gene can be inserted into expression vectors as described above to prepare vectors for the expression of the fusion protein. The method for preparing the fusion protein is described in Molecular Cloning 2nd ed. (Sambrook, J. et al., Molecular Cloning 2nd ed., 9.47-9.58 (1989), Cold Spring Harbor Lab. Press). As one example, a method for obtaining GPC3 for use as a sensitizing antigen and an immunization method using this sensitizing antigen are also specifically described in WO2003/000883, WO2004/022754, and WO2006/006693.

The mammals to be immunized with the sensitizing antigen are not limited to specific animals. The mammals to be immunized are preferably selected in consideration of compatibility with the parental cells for use in cell fusion. In general, rodents, for example, mice, rats, or hamsters, rabbits, monkeys, or the like are preferably used.

These animals are immunized with the sensitizing antigen according to a method known in the art. For example, a general immunization method involves administering the sensitizing antigen to the mammals by intraperitoneal or subcutaneous injection to thereby perform immunization. Specifically, the sensitizing antigen diluted with PBS (phosphate-buffered saline), saline, or the like at an appropriate dilution ratio is mixed with a usual adjuvant, for example, a Freund's complete adjuvant, if desired, and emulsified. Then, the resulting sensitizing antigen is administered to the mammals several times at 4- to 21-day intervals. Also, an appropriate carrier may be used in the immunization with the sensitizing antigen. Particularly, in the case of using a partial peptide having a small molecular weight as the sensitizing antigen, immunization with the sensitizing antigen peptide bound with a carrier protein such as albumin or keyhole limpet hemocyanin may be desirable in some cases.

Alternatively, the hybridomas producing the desired antibody can also be prepared as described below by use of DNA immunization. The DNA immunization is an immunization method which involves immunostimulating immunized animals by expressing *in vivo* the sensitizing antigen in the immunized animals given vector DNAs that have been constructed in a form capable of expressing the antigenic protein-encoding gene in the immunized animals. The DNA immunization can be expected to be superior to the general immunization method using the administration of the protein antigen to animals to be immunized as follows:
- the DNA immunization can provide immunostimulation with the membrane protein structure maintained; and
- the DNA immunization eliminates the need of purifying the immunizing antigen.

In order to obtain the monoclonal antibody of the present invention by the DNA immunization, first, a DNA expressing the tumor antigen protein is administered to the animals to be immunized. The DNA encoding the tumor antigen can be synthesized by a method known in the art such as PCR. The obtained DNA is inserted into appropriate expression vectors, which are then administered to the animals to be immunized. For example, commercially available expression vectors such as pcDNA3.1 can be preferably used as the expression vectors. A method generally used can be used as a method for administering the vectors to the organisms. For example, gold particles with the expression vectors adsorbed thereon can be transferred into the cells of animal individuals to be immunized using a gene gun to thereby perform the DNA immunization. Further, an antibody recognizing the tumor antigen may be prepared by use of a method described in International Publication No. WO2003/104453.

A rise in the titer of the antibody binding to the tumor antigen is confirmed in the serum of the mammals thus immunized. Then, immunocytes are collected from the mammals and subjected to cell fusion. Particularly, spleen cells can be used as preferred immunocytes.

Mammalian myeloma cells are used in the cell fusion with the immunocytes. The myeloma cells preferably have an appropriate selection marker for screening. The selection marker refers to a character that can survive (or cannot survive) under particular culture conditions. For example, hypoxanthine-guanine phosphoribosyltransferase deficiency (hereinafter, abbreviated to HGPRT deficiency) or thymidine kinase deficiency (hereinafter, abbreviated to TK deficiency) is known in the art as the selection marker. Cells having the HGPRT or TK deficiency is sensitive to hypoxanthine-aminopterin-thymidine (hereinafter, abbreviated to HAT-sensitive). The HAT-sensitive cells are killed in a HAT selective medium because the cells fail to synthesize DNA. By contrast, these cells, when fused with normal cells, become able to grow even in the HAT selective medium because the fused cells can continue DNA synthesis through the use of the salvage pathway of the normal cells.

The cells having the HGPRT or TK deficiency can be selected in a medium containing 6-thioguanine or 8-azaguanine (hereinafter, abbreviated to 8AG) for the HGPRT deficiency or 5'-bromodeoxyuridine for the TK deficiency. The normal cells are killed by incorporating these pyrimidine analogs into their DNAs. By contrast, the cells deficient in these enzymes can survive in the selective medium because the cells cannot incorporate the pyrimidine analogs therein. In addition, a selection marker called G418 resistance confers resistance to a 2-deoxystreptamine antibiotic (gentamicin analog) through a neomycin resistance gene. Various myeloma cells suitable for cell fusion are known in the art.

For example, P3 (P3x63Ag8.653) (J. Immunol. (1979) 123 (4), 1548-1550), P3x63Ag8U.1 (Current Topics in Microbiology and Immunology (1978) 81, 1-7), NS-1 (C. Eur. J. Immunol. (1976) 6 (7), 511-519), MPC-11 (Cell (1976) 8 (3), 405-415), SP2/0 (Nature (1978) 276 (5685), 269-270), FO (J. Immunol. Methods (1980) 35 (1-2), 1-21), S194/5.XX0.BU.1 (J. Exp. Med. (1978) 148 (1), 313-323), R210 (Nature (1979) 277 (5692), 131-133) can be preferably used as such myeloma cells.

Basically, the cell fusion of the immunocytes with the myeloma cells is carried out according to a method known in the art, for example, the method of Kohler and Milstein et al. (Methods Enzymol. (1981) 73, 3-46).

More specifically, the cell fusion can be carried out, for example, in a usual nutrient medium in the presence of a cell fusion promoter. For example, polyethylene glycol (PEG) or hemagglutinating virus of Japan (HVJ) is used as the fusion promoter. In addition, an auxiliary such as dimethyl sulfoxide is added thereto for use, if desired, for enhancing fusion efficiency.

The ratio between the immunocytes and the myeloma cells used can be arbitrarily set. For example, the amount of the immunocytes is preferably set to 1 to 10 times the amount of the myeloma cells. For example, an RPMI1640 medium or a MEM medium suitable for the growth of the myeloma cell line as well as a usual medium for use in this kind of cell culture is used as the medium for use in the cell fusion. Preferably, a solution supplemented with serum (e.g., fetal calf serum (FCS)) can be further added to the medium.

For the cell fusion, the immunocytes and the myeloma cells are well mixed in the predetermined amounts in the medium. A PEG solution (e.g., average molecular weight: on the order of 1000 to 6000) preheated to approximately 37□C is usually added thereto at a concentration of 30 to 60% (w/v). The mixed solution is gently mixed so that desired fusion cells (hybridomas) are formed. Subsequently, the appropriate medium listed above is sequentially added to the cell cultures, and its supernatant is removed by centrifugation. This operation can be repeated to remove the cell fusion agents or the like unfavorable for hybridoma growth.

The hybridomas thus obtained can be cultured in a usual selective medium, for example, a HAT medium (medium containing hypoxanthine, aminopterin, and thymidine), for selection. The culture using the HAT medium can be continued for a time long enough to kill cells (non-fused cells) other than the desired hybridomas (usually, the time long enough is several days to several weeks). Subsequently, hybridomas producing the desired antibody are screened for and single-cell cloned by a usual limiting dilution method.

The hybridomas thus obtained can be selected by use of a selective medium appropriate for the selection marker of the myeloma used in the cell fusion. For example, the cells having the HGPRT or TK deficiency can be selected by culture in a HAT medium (medium containing hypoxanthine, aminopterin, and thymidine). Specifically, when HAT-sensitive myeloma cells are used in the cell fusion, only cells successfully fused with normal cells can be grown selectively in the HAT medium. The culture using the HAT medium is continued for a time long enough to kill cells (non-fused cells) other than the desired hybridomas. Specifically, the culture can generally be carried out for several days to several weeks to select the desired hybridomas. Subsequently, hybridomas producing the desired antibody can be screened for and single-cell cloned by a usual limiting dilution method.

The screening of the desired antibody and the single-cell cloning can be preferably carried out by a screening method based on antigen-antibody reaction known in the art. For example, a monoclonal antibody binding to GPC3 can bind to GPC3 expressed on cell surface. Such a monoclonal antibody can be screened for, for example, by FACS (fluorescence activated cell sorting). FACS is a system that can analyze the binding of an antibody to cell surface, by bringing cells into contact with a fluorescent antibody, analyzing the cells with laser light, and measuring fluorescence emitted from individual cells.

In order to screen for a hybridoma producing the monoclonal antibody of the present invention by FACS, first, cells expressing GPC3 are prepared. The cells for screening are preferably mammalian cells forced to express the tumor antigen used. Untransformed mammalian cells used as host cells can be used as a control to selectively detect the binding activity of an antibody against the tumor antigen on cell surface. Specifically, a hybridoma producing an antibody binding to a cell forced to express GPC3 without binding to the host cells can be selected to obtain a hybridoma producing a monoclonal antibody against the tumor antigen.

Alternatively, the antibody can be evaluated for its binding activity against immobilized tumor antigen-expressing cells on the basis of the principle of ELISA. GPC3-expressing cells are immobilized onto each well of, for example, an ELISA plate. The hybridoma culture supernatant is contacted with the immobilized cell in the well to detect an antibody binding to the immobilized cell. In the case of a mouse-derived monoclonal antibody, the antibody bound with the cell can be detected using an anti-mouse immunoglobulin antibody. Hybridomas producing the desired antibody having the ability to bind to the antigen, thus selected by screening, can be cloned by a limiting dilution method or the like.

The monoclonal antibody-producing hybridomas thus prepared can be subcultured in a usual medium. The hybridomas can also be stored over a long period in liquid nitrogen.

The hybridomas are cultured according to a usual method, and the desired monoclonal antibody can be obtained from the culture supernatant thereof. Alternatively, the hybridomas may be administered to mammals compatible therewith and grown, and the monoclonal antibody can be obtained from the ascitic fluids thereof. The former method is suitable for obtaining highly pure antibodies.

An antibody encoded by an antibody gene cloned from the antibody-producing cells such as hybridomas may also be preferably used. The cloned antibody gene is incorporated in appropriate vectors, which are then transferred to hosts so that the antibody encoded by the gene is expressed. Methods for the antibody gene isolation, the incorporation into vectors, and the transformation of host cells have already been established by, for example, Vandamme et al. (Eur. J. Biochem. (1990) 192 (3), 767-775). A method for producing a recombinant antibody as mentioned below is also known in the art.

For example, cDNAs encoding the variable regions (V regions) of the antibody binding to the tumor antigen are obtained from the hybridoma cells producing the antibody. For this purpose, usually, total RNA is first extracted from the hybridomas. For example, the following methods can be used as a method for mRNA extraction from the cells: - guanidine ultracentrifugation method (Biochemistry (1979) 18 (24), 5294-5299), and - AGPC method (Anal. Biochem. (1987) 162 (1), 156-159).

The extracted mRNAs can be purified using mRNA Purification Kit (manufactured by GE Healthcare Bio-Sciences Corp.) or the like. Alternatively, a kit for directly extracting total mRNA from cells is also commercially available, such as QuickPrep mRNA Purification Kit (manufactured by GE Healthcare Bio-Sciences Corp.). The mRNAs may be obtained from the hybridomas using such a kit. From the obtained mRNAs, the cDNAs encoding antibody V regions can be synthesized using reverse transcriptase. The cDNAs can be synthesized using, for example, AMV Reverse Transcriptase First-strand cDNA Synthesis Kit (manufactured by Seikagaku Corp.). Alternatively, a 5'-RACE method (Proc. Natl. Acad. Sci. USA (1988) 85 (23), 8998-9002; and Nucleic Acids Res. (1989) 17 (8), 2919-2932) using SMART RACE cDNA amplification kit (manufactured by Clontech Laboratories, Inc.) and PCR may be appropriately used for the cDNA synthesis and amplification. In the course of such cDNA synthesis, appropriate restriction sites mentioned later can be further introduced into both ends of the cDNAs.

The cDNA fragments of interest are purified from the obtained PCR products and subsequently ligated with vector DNAs. The recombinant vectors thus prepared are transferred to *E. coli* or the like. After colony selection, desired recombinant vectors can be prepared from the *E. coli* that has formed the colony. Then, whether or not the recombinant vectors have the nucleotide sequences of the cDNAs of interest is confirmed by a method known in the art, for example, a dideoxynucleotide chain termination method.

The 5'-RACE method using primers for variable region gene amplification is conveniently used for obtaining the genes encoding variable regions. First, cDNAs are synthesized with RNAs extracted from the hybridoma cells as templates to obtain a 5'-RACE cDNA library. A commercially available kit such as SMART RACE cDNA amplification kit is appropriately used in the synthesis of the 5'-RACE cDNA library.

The antibody gene is amplified by PCR with the obtained 5'-RACE cDNA library as a template. Primers for mouse antibody gene amplification can be designed on the basis of an antibody gene sequence known in the art. These primers have nucleotide sequences differing depending on immunoglobulin subclasses. Thus, the subclass is desirably determined in advance using a commercially available kit such as Iso Strip mouse monoclonal antibody isotyping kit (Roche Diagnostics K.K.).

Specifically, primers capable of amplifying genes encoding γ1, y2a, y2b, and y3 heavy chains and κ and λ light chains can be used, for example, for the purpose of obtaining a gene encoding mouse IgG. Primers that anneal to portions corresponding to constant regions close to variable regions are generally used as 3' primers for amplifying IgG variable region genes. On the other hand, primers included in the 5' RACE cDNA library preparation kit are used as 5' primers.

The PCR products thus obtained by amplification can be used to reshape an immunoglobulin composed of heavy chains and light chains in combination. The desired antibody can be screened for with the binding activity of the reshaped immunoglobulin against the antigen as an index. For example, the binding of the antibody to GPC3 is further preferably specific for the purpose of obtaining the antibody against GPC3. The antibody used in the present invention can be screened for, for example, by the following steps:
(1) contacting each antibody comprising V regions encoded by the cDNAs obtained from the hybridomas, with antigen-expressing cells;
(2) detecting the binding between the antigen-expressing cell and the antibody; and
(3) selecting the antibody binding to the antigen-expressing cell.

A method for detecting the binding between the antibody and the tumor antigen-expressing cell is known in the art. Specifically, the binding between the antibody and the tumor antigen-expressing cell can be detected by an approach such as FACS mentioned above. A fixed preparation of tumor antigen-expressing cells can be appropriately used for evaluating the binding activity of the antibody.

A panning method using phage vectors is also preferably used as a method for screening for the antibody with its binding activity as an index. When antibody genes are obtained as libraries of heavy chain and light chain subclasses from a polyclonal antibody-expressing cell population, a screening method using phage vectors is advantageous. Genes encoding heavy chain and light chain variable regions can be linked via an appropriate linker sequence to form a gene encoding single-chain Fv (scFv). The gene encoding scFv can be inserted to phage vectors to obtain phages expressing scFv on their surface. The phages thus obtained are contacted with the desired antigen. Then, antigen-bound phages can be recovered to recover a DNA encoding scFv having the binding activity of interest. This operation can be repeated, if necessary, to enrich scFvs having the desired binding activity.

After the obtainment of the cDNA encoding each V region of the antibody binding to the tumor antigen of interest, this cDNA is digested with restriction enzymes that recognize the restriction sites inserted in both ends of the cDNA. The restriction enzymes preferably recognize and digest a nucleotide sequence that appears low frequently in the nucleotide sequence constituting the antibody gene. The insertion of restriction sites that provide cohesive ends is preferred for inserting one copy of the digested fragment in the correct direction in a vector. The thus-digested cDNAs encoding the V regions of the anti-GPC3 antibody can be inserted to appropriate expression vectors to obtain antibody expression vectors. In this case, genes encoding antibody constant regions (C regions) and the genes encoding the V regions are fused in frame to obtain a chimeric antibody. In this context, the chimeric antibody refers to an antibody comprising constant and variable regions of different origins. Thus, heterogeneous (e.g., mouse-human) chimeric antibodies as well as human-human homogeneous chimeric antibodies are also encompassed by the chimeric antibody according to the present invention. The V region genes can be inserted to expression vectors preliminarily having constant region genes to construct chimeric antibody expression vectors. Specifically, for example, recognition sequences for restriction enzymes that digest the V region genes can be appropriately located on the 5' side of an expression vector carrying the DNAs encoding the desired antibody constant regions (C regions). This expression vector having the C region genes and the V region genes are digested with the same combination of restriction enzymes and fused in frame to construct a chimeric antibody expression vector.

In order to produce the monoclonal antibody, the antibody gene is incorporated into expression vectors such that the antibody gene is expressed under the control of expression control regions. The expression control regions for antibody expression include, for example, an enhancer and a promoter. Also, an appropriate signal sequence can be added to the amino terminus such that the expressed antibody is extracellularly secreted. For example, a peptide having an amino acid sequence MGWSCIILFLVATATGVHS is used as a signal sequence in Examples described later. Any other suitable signal sequence can be added. The expressed polypeptide is cleavable at the carboxyl terminal moiety of this sequence. The cleaved polypeptide can be extracellularly secreted as a mature polypeptide. Furthermore, appropriate host cells can be transformed with these expression vectors to obtain recombinant cells expressing the DNA encoding the antibody binding to the targeted tumor antigen.

For the antibody gene expression, DNAs encoding the heavy chain (H chain) and the light chain (L chain) of the antibody are separately incorporated into different expression vectors. The same host cell can be co-transfected with these vectors carrying the H chain gene and the L chain gene and thereby allowed to express an antibody molecule comprising the H chain and the L chain. Alternatively, the DNAs encoding the H chain and L chain may be incorporated into a single expression vector, with which host cells can be transformed (see International Publication No. WO94/011523).

Many combinations of host cells and expression vectors are known in the art for preparing the antibody by transferring the isolated antibody gene into appropriate hosts. All of these expression systems can be applied to the isolation of the domain comprising antibody variable regions according to the present invention. In the case of using eukaryotic cells as the host cells, animal cells, plant cells, or fungus cells can be appropriately used. Specifically, examples of the animal cells can include the following cells:
(1) mammalian cells such as CHO, COS, myeloma, BHK (baby hamster kidney), Hela, and Vero,;
(2) amphibian cells such as *Xenopus* oocytes; and
(3) insect cells such as sf9, sf21, and Tn5.

Alternatively, antibody gene expression systems using cells derived from the genus *Nicotiana* (e.g., *Nicotiana tabacum*) as the plant cells are known in the art. Cultured callus cells can be appropriately used for the plant cell transformation.

The following cells can be used as the fungus cells:
cells derived from yeasts of the genus *Saccharomyces* (e.g., *Saccharomyces cerevisiae*) and the genus *Pichia* (e.g., *Pichiapastoris*), and
cells derived from filamentous fungi of the genus *Aspergillus* (e.g., *Aspergillus niger*).

Antibody gene expression systems using prokaryotic cells are also known in the art. In the case of using, for example, bacterial cells, cells of bacteria such as *E. coli* and *Bacillus subtilis* can be appropriately used. The expression vectors containing the antibody gene of interest are transferred into these cells by transformation. The transformed cells are cultured *in vitro,* and the desired antibody can be obtained from the cultures of the transformed cells.

In addition to the host cells, transgenic animals may be used for the production of the recombinant antibody. Specifically, the desired antibody can be obtained from animals transfected with the gene encoding this antibody. For example, the antibody gene can be inserted in frame into a gene encoding a protein specifically produced in milk to thereby construct a fusion gene. For example, goat β casein can be used as the protein secreted into milk. A DNA fragment containing the fusion gene having the antibody gene insert is injected into goat embryos, which are in turn introduced into female goats. From milk produced by transgenic goats (or progeny thereof) brought forth by the goats that have received the embryos, the desired antibody can be obtained as a fusion protein with the milk protein. In order to increase the amount of milk containing the desired antibody produced from the transgenic goats, hormone can be administered to the transgenic goats (Bio/Technology (1994) 12 (7), 699-702).

In the case of administering the antigen binding molecule described in the present specification to humans, a domain derived from a genetically recombinant antibody that has been altered artificially can be appropriately adopted as the domain comprising antibody variable regions in the antigen binding molecule, for example, for the purpose of reducing heteroantigenicity in humans. The genetically recombinant antibody encompasses, for example, humanized antibodies. Such an altered antibody is appropriately produced using a method known in the art.

Each antibody variable region that is used for preparing the domain comprising antibody variable regions in the antigen binding molecule described in the present specification is usually constituted by three complementarity-determining regions (CDRs) flanked by four framework regions (FRs). The CDRs are regions that substantially determine the binding specificity of the antibody. The CDRs have highly diverse amino acid sequences. On the other hand, the amino acid sequences constituting the FRs often exhibit high identity even among antibodies differing in binding specificity. Therefore, in general, the binding specificity of an antibody can reportedly be transplanted to another antibody by CDR grafting.

The humanized antibody is also called reshaped human antibody. Specifically, for example, a humanized antibody comprising non-human animal (e.g., mouse) antibody CDRs grafted in a human antibody is known in the art. General gene recombination approaches are also known for obtaining the humanized antibody. Specifically, for example, overlap extension PCR is known in the art as a method for grafting mouse antibody CDRs to human FRs. In the overlap extension PCR, nucleotide sequences encoding mouse antibody CDRs to be grafted are added to primers for human antibody FR synthesis. The primers are prepared for each of the four FRs. In the mouse CDR grafting to the human FRs, in general, the selection of human FRs highly homologous to the mouse FRs is reportedly advantageous in maintaining the CDR functions. Specifically, in general, it is preferred to use human FRs comprising amino acid sequences highly identical to those of the mouse FRs adjacent to the mouse CDRs to be grafted.

The nucleotide sequences to be linked are designed such that the sequences are connected in frame. DNAs encoding human FRs are individually synthesized with their respective primers. The resulting PCR products contain the mouse CDR-encoding DNA added to each human FR-encoding DNA. The mouse CDR-encoding nucleotide sequences are designed such that the nucleotide sequence in each product overlaps with another. Subsequently, the overlapping CDR portions in the products synthesized with the human antibody gene as a template are annealed to each other for complementary strand synthesis reaction. Through this reaction, the human FR sequences are linked via the mouse CDR sequences.

Finally, the full-length gene of the V region comprising three CDRs and four FRs thus linked is amplified using primers that respectively anneal to the 5' and 3' ends thereof and have the added recognition sequences for appropriate restriction enzymes. The DNA thus obtained and the DNA encoding the human antibody C region can be inserted into expression vectors such that these DNAs are fused in frame to prepare vectors for human-type antibody expression. These vectors carrying the DNAs are transferred to hosts to establish recombinant cells. Then, the recombinant cells are cultured for the expression of the DNA encoding the humanized antibody to produce the humanized antibody into the cultures of the cultured cells (see European Patent Publication No. EP239400 and International Publication No. WO1996/002576).

The humanized antibody thus prepared can be evaluated for its binding activity against the antigen by qualitative or quantitative assay to thereby select suitable human antibody FRs that allow the CDRs to form a favorable antigen binding site when linked via the CDRs. If necessary, FR amino acid residue(s) may be substituted such that the CDRs of the resulting reshaped human antibody form an appropriate antigen binding site. For example, a mutation can be introduced in the amino acid sequence of human FR by the application of the PCR method used in the mouse CDR grafting to the human FRs. Specifically, a mutation of a partial nucleotide sequence can be introduced to the primers annealing to a FR nucleotide sequence. The FR nucleotide sequence synthesized using such primers contains the mutation thus introduced. The mutant antibody having the substituted amino acid(s) can be evaluated for its binding activity against the antigen by assay in the same way as above to thereby select mutated FR sequences having the desired properties (Sato, K.et al., Cancer Res., (1993) 53, 851-856).

Furthermore, transgenic animals having all repertoires of human antibody genes (see International Publication Nos. WO1993/012227, WO1992/003918, WO1994/002602, WO1994/025585, WO1996/034096, and WO1996/033735) can be used as animals to be immunized by DNA immunization to obtain the desired human antibody.

In addition, a technique of obtaining a human antibody by panning using a human antibody library is also known. For example, human antibody V regions are expressed as a single-chain antibody (scFv) on the surface of phages by a phage display method. A phage expressing scFv binding to the antigen can be selected. The gene of the selected phage can be analyzed to determine DNA sequences encoding the V regions of the human antibody binding to the antigen. After the determination of the DNA sequence of the scFv binding to the antigen, the V region sequences are fused in frame with the sequences of the desired human antibody C regions. Then, this fusion product can be inserted to appropriate expression vectors to prepare expression vectors. The expression vectors are transferred to the suitable expression cells as listed above. The cells are allowed to express the gene encoding the human antibody to obtain the human antibody. These methods have already been known in the art (see International Publication Nos. WO1992/001047, WO1992/020791, WO1993/006213, WO1993/011236, WO1993/019172, WO1995/001438, and WO1995/015388).

### Domain comprising antibody variable regions having T cell receptor complex binding activity

In the present specification, the "domain comprising antibody variable regions having T cell receptor complex binding activity" refers to a moiety of an anti-T cell receptor complex antibody comprising a region that specifically binds to and is complementary to a portion or the whole of a T cell receptor complex. The T cell receptor complex may be a T cell receptor itself or may be an adaptor molecule constituting the T cell receptor complex together with the T cell receptor. The adaptor is preferably CD3.

### Domain comprising antibody variable regions having T cell receptor binding activity

In the present specification, the "domain comprising antibody variable regions having T cell receptor binding activity" refers to a moiety of an anti-T cell receptor antibody comprising a region that specifically binds to and is complementary to a portion or the whole of a T cell receptor. The moiety of the T cell receptor to which the domain of the present invention binds may be a variable region or may be a constant region, and is preferably an epitope present in a constant region. Examples of the sequence of the constant region can include the sequences of a T cell receptor alpha chain of RefSeq registration No. CAA26636.1, a T cell receptor beta chain of RefSeq registration No. C25777, a T cell receptor gamma 1 chain of RefSeq registration No. A26659, a T cell receptor gamma 2 chain of RefSeq registration No. AAB63312.1, and a T cell receptor delta chain of RefSeq registration No. AAA61033.1.

### Domain comprising antibody variable regions having CD3 binding activity

In the present specification, the "domain comprising antibody variable regions having CD3 binding activity" refers to a moiety of an anti-CD3 antibody comprising a region that specifically binds to and is complementary to a portion or the whole of CD3. Preferably, the domain comprises a light chain variable region (VL) and a heavy chain variable region (VH) of the anti-CD3 antibody.

The domain comprising antibody variable regions having CD3 binding activity according to the present invention is capable of binding to any epitope as long as the epitope is present in a gamma chain, delta chain or epsilon chain sequence constituting human CD3. In the present disclosure, a domain comprising a light chain variable region (VL) and a heavy chain variable region (VH) of an anti-CD3 antibody that binds to an epitope present in an extracellular region of an epsilon chain of a human CD3 complex is preferably used. A CD3 binding domain comprising a light chain variable region (VL) and a heavy chain variable region (VH) of an anti-CD3 antibody described in Examples as well as a light chain variable region (VL) and a heavy chain variable region (VH) of OKT3 antibody (Proc. Natl. Acad. Sci. USA (1980) 77, 4914-4917) or any of various anti-CD3 antibodies known in the art is preferably used as such a domain. Also, a domain comprising antibody variable regions originating from an anti-CD3 antibody having the desired properties, which is obtained by immunizing the desired animal by the method described above using a gamma chain, a delta chain or an epsilon chain constituting human CD3, may be appropriately used. An appropriately humanized antibody as described above or a human antibody is appropriately used as the anti-CD3 antibody that gives rise to the domain comprising antibody variable regions having CD3 binding activity. As for the structure of the gamma chain, the delta chain or the epsilon chain constituting CD3, their polynucleotide sequences are described in RefSeq registration Nos. NM_000073.2, NM_000732.4 and NM_000733.3, and their polypeptide sequences are described in RefSeq registration Nos. NP_000064.1, NP_000723.1 and NP_000724.1.

### Specific

The term "specific" refers to a state in which one of specifically binding molecules does not exhibit any significant binding to a molecule other than its one or more binding partner molecules. This term is also used when the domain comprising antibody variable regions is specific for a particular epitope among a plurality of epitopes contained in a certain antigen. When an epitope to which the domain comprising antibody variable regions binds is contained in a plurality of different antigens, an antigen binding molecule having this domain comprising antibody variable regions can bind to various antigens comprising the epitope.

### Epitope

The epitope, which means an antigenic determinant, present in the antigen means a site on the antigen to which the domain comprising antibody variable regions in the antigen binding molecule disclosed in the present specification binds. Accordingly, for example, the epitope can be defined by its structure. Alternatively, the epitope may be defined by the antigen binding activity of the antigen binding molecule recognizing the epitope. When the antigen is a peptide or a polypeptide, the epitope may be identified by amino acid residues constituting the epitope. When the epitope is a sugar chain, the epitope may be identified by a particular sugar chain structure.

A linear epitope refers to an epitope comprising an epitope that is recognized by its primary sequence of amino acids. The linear epitope contains typically at least 3 and most commonly at least 5, for example, approximately 8 to approximately 10 or 6 to 20 amino acids, in its unique sequence.

In contrast to the linear epitope, the conformational epitope refers to an epitope that is contained in a primary sequence of amino acids containing a component other than the single defined component of the epitope to be recognized (e.g., an epitope whose primary sequence of amino acids may not be recognized by an antibody that determines the epitope). The conformational epitope may contain an increased number of amino acids, as compared with the linear epitope. As for the recognition of the conformational epitope, an antibody recognizes the three-dimensional structure of the peptide or the protein. For example, when a protein molecule is folded to form a three-dimensional structure, certain amino acids and/or polypeptide backbone constituting the conformational epitope are arranged in parallel to allow the antibody to recognize the epitope. Examples of the method for determining the conformation of the epitope include, but are not limited to, X-ray crystallography, two-dimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

A method for confirming the binding of an antigen binding molecule binding to a tumor antigen to an epitope will be illustrated below.

For example, whether the antigen binding molecule binding to the tumor antigen recognizes a linear epitope present in the tumor antigen molecule can be confirmed, for example, as follows: a linear peptide comprising an amino acid sequence constituting the extracellular domain of the tumor antigen is synthesized for the purpose described above. The peptide can be chemically synthesized. Alternatively, the peptide is obtained by a genetic engineering approach using a region encoding an amino acid sequence corresponding to the extracellular domain in tumor antigen cDNA. Next, the test antigen binding molecule having a domain comprising antibody variable regions having binding activity against the tumor antigen is evaluated for its binding activity against the linear peptide comprising an amino acid sequence constituting the extracellular domain. For example, the binding activity of the antigen binding molecule against the peptide can be evaluated by ELISA using an immobilized linear peptide as an antigen. Alternatively, the binding activity against the linear peptide may be determined on the basis of a level at which the linear peptide inhibits the binding of the antigen binding molecule to tumor antigen-expressing cells. These tests can determine the binding activity of the antigen binding molecule against the linear peptide.

Also, whether the test antigen binding molecule having a domain comprising antibody variable regions having binding activity against the tumor antigen recognizes the conformational epitope can be confirmed as follows: tumor antigen-expressing cells are prepared for the purpose described above. The recognition of the conformational epitope by test antigen binding molecule having a domain comprising antibody variable regions having binding activity against the tumor antigen is confirmed, for example, when the antigen binding molecule strongly binds to the tumor antigen-expressing cells upon contact with the cells, whereas the antigen binding molecule does not substantially bind to an immobilized linear peptide comprising an amino acid sequence constituting the extracellular domain of the tumor antigen. In this context, the term "not substantially bind" means that the binding activity is 80% or less, usually 50% or less, preferably 30% or less, particularly preferably 15% or less of binding activity against cells expressing the human tumor antigen.

Examples of the method for measuring the binding activity of the test antigen binding molecule comprising the antigen binding domain directed to the tumor antigen against the tumor antigen-expressing cells include methods described in Antibodies: A Laboratory Manual (Ed Harlow, David Lane, Cold Spring Harbor Laboratory (1988) 359-420). Specifically, the binding activity can be evaluated on the basis of the principle of ELISA or FACS (fluorescence activated cell sorting) using the GPC3-expressing cells as an antigen.

In the ELISA format, the binding activity of the test antigen binding molecule comprising the antigen binding domain directed to the target tumor antigen against the tumor antigen-expressing cells is quantitatively evaluated by comparing the levels of signals generated through enzymatic reaction. Specifically, the test antigen binding molecule is added to an ELISA plate with the tumor antigen-expressing cells immobilized thereon. Then, the test antigen binding molecule bound with the cells is detected through the use of an enzyme-labeled antibody recognizing the test antigen binding molecule. Alternatively, in the FACS, a dilution series of the test antigen binding molecule is prepared, and the antibody binding titer for the tumor antigen-expressing cells can be determined to compare the binding activity of the test antigen binding molecule against the tumor antigen-expressing cells.

The binding of the test antigen binding molecule to the antigen expressed on the surface of cells suspended in a buffer solution or the like can be detected using a flow cytometer. For example, the following apparatuses are known as the flow cytometer:
FACSCanto(TM) II
FACSAria(TM)
FACSArray(TM)
FACSVantage(TM) SE
FACSCalibur(TM) (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter, Inc.)

One preferred example of the method for measuring the antigen binding activity of the test antigen binding molecule includes the following method: first, target tumor antigen-expressing cells reacted with the test antigen binding molecule are stained with a FITC-labeled secondary antibody recognizing the test antigen binding domain. The test antigen binding molecule is appropriately diluted with a suitable buffer solution to prepare the antigen binding molecule at the desired concentration for use. The antigen binding molecule can be used, for example, at any concentration from 10 µg/ml to 10 ng/ml. Next, fluorescence intensity and the number of cells are measured using FACSCalibur (Becton, Dickinson and Company). The amount of the antibody bound to the cells is reflected in the fluorescence intensity obtained by analysis using CELL QUEST Software (Becton, Dickinson and Company), i.e., a geometric mean value. In short, the binding activity of the test antigen binding molecule indicated by the amount of the test antigen binding molecule bound can be determined by obtaining the geometric mean value.

Whether the test antigen binding molecule shares an epitope with a certain antigen binding molecule can be confirmed by the competition between these antigen binding domains for the same epitope. The competition between the antigen binding molecules is detected by cross-blocking assay or the like. The cross-blocking assay is preferably, for example, competitive ELISA assay.

Specifically, in the cross-blocking assay, tumor antigen protein-coated wells of a microtiter plate are preincubated in the presence or absence of a candidate competitor antigen binding molecule. Then, the test antigen binding molecule is added thereto. The amount of the test antigen binding molecule bound with the tumor antigen protein in the wells indirectly correlates with the binding capacity of the candidate competitor antigen binding molecule that competes for the binding to the same epitope. In short, larger affinity of the competitor antigen binding molecule for the same epitope means lower binding activity of the test antigen binding molecule against the tumor antigen protein-coated wells.

The amount of the test antigen binding molecule bound with the wells via the tumor antigen protein can be easily measured by labeling the antigen binding molecule in advance. For example, a biotin-labeled antigen binding molecule is assayed by using an avidin-peroxidase conjugate and an appropriate substrate. In particular, cross-blocking assay that utilizes enzyme labels such as peroxidase is called competitive ELISA assay. The antigen binding molecule can be labeled with an alternative detectable or measurable labeling material. Specifically, radiolabels, fluorescent labels, and the like are known in the art.

Provided that the competitor antigen binding molecule can block the binding of the test antigen binding molecule comprising the antigen binding domain directed to the tumor antigen by at least 20%, preferably at least 20 to 50%, more preferably at least 50% as compared with binding activity obtained in a control test carried out in the absence of the candidate competitor antigen binding molecule, the test antigen binding molecule is determined as an antigen binding molecule substantially binding to the same epitope as that for the competitor antigen binding molecule, or competing for the binding to the same epitope.

When the epitope to which the test antigen binding molecule comprising the antigen binding domain directed to the tumor antigen binds has an identified structure, whether the test antigen binding molecule and a control antigen binding molecule share an epitope can be evaluated by comparing the binding activity of these antigen binding molecules against a peptide prepared by introducing an amino acid mutation to a peptide constituting the epitope.

In such a method for measuring binding activity, for example, the binding activity of a test antigen binding molecule and a control antigen binding molecule against a linear peptide containing an introduced mutation can be compared in the ELISA format described above to be measured. In a method other than ELISA, the binding activity against the mutated peptide bound with a column may be measured by flowing the test antigen binding molecule and the control antigen binding molecule in the column, and then quantifying the antigen binding molecule eluted in the eluate. A method for adsorbing a mutated peptide, for example, as a fusion peptide with GST, to a column is known in the art.

When the identified epitope is a conformational epitope, whether a test antigen binding molecule and a control antigen binding molecule share an epitope can be evaluated by the following method: first, tumor antigen-expressing cells and cells expressing the tumor antigen with a mutation introduced to the epitope are prepared. The test antigen binding molecule and the control antigen binding molecule are added to cell suspensions containing these cells suspended in an appropriate buffer solution such as PBS. Subsequently, the cell suspensions are appropriately washed with a buffer solution, and a FITC-labeled antibody capable of recognizing the test antigen binding molecule and the control antigen binding molecule is then added thereto. The fluorescence intensity and the number of cells stained with the labeled antibody are measured using FACSCalibur (Becton, Dickinson and Company). The test antigen binding molecule and the control antigen binding molecule are appropriately diluted with a suitable buffer solution and used at concentrations thereby adjusted to the desired ones. These antigen binding molecules are used, for example, at any concentration from 10 µg/ml to 10 ng/ml. The amount of the labeled antibody bound to the cells is reflected in the fluorescence intensity obtained by analysis using CELL QUEST Software (Becton, Dickinson and Company), i.e., a geometric mean value. In short, the binding activity of the test antigen binding molecule and the control antigen binding molecule indicated by the amount of the labeled antibody bound can be determined by obtaining the geometric mean value.

In the present method, whether to not substantially bind to cells expressing the tumor antigen having a mutation can be determined, for example, by the following method: first, a test antigen binding molecule and a control antigen binding molecule bound with the cells expressing the mutated tumor antigen are stained with a labeled antibody. Subsequently, the fluorescence intensity of the cells is detected. In the case of using FACSCalibur in the fluorescence detection by flow cytometry, the obtained fluorescence intensity can be analyzed using the CELL QUEST Software. From geometric mean values obtained in the presence and absence of the antigen binding molecule, their comparison value (ΔGeo-Mean) can be calculated according to expression 1 given below to determine the rate of increase in fluorescence intensity caused by the binding of the antigen binding molecule.$\begin{matrix}Δ Geo - Mean = Geo - Mean \left(in the presence of the antigen binding molecule\right) / Geo - \\ Mean \left(in the absence of the antigen binding molecule\right)\end{matrix}$

### Fv (variable fragment)

In the present specification, the term "Fv (variable fragment)" means the minimum unit of an antibody-derived antigen binding domain consisting of a pair of a light chain variable region (VL) and a heavy chain variable region (VH) of an antibody. In 1988, Skerra and Pluckthun found that an antibody can be prepared from an *E. coli* periplasm fraction in a homogeneous state with activity retained, by inserting an antibody gene downstream of a bacterial signal sequence and inducing the expression of the gene in *E. coli* (Science (1988) 240 (4855), 1038-1041). In Fv prepared from the periplasm fraction, VH and VL were associated in a form binding to an antigen.

In the present specification, Fv preferably includes, for example, a pair of Fvs which is any of the following antigen binding molecules: antigen binding molecules comprising (1) a bivalent antigen binding domain which is bivalent scFv in which one monovalent scFv of the bivalent scFv is linked to one polypeptide constituting an Fc region via a heavy chain Fv fragment constituting a CD3 binding domain, and the other monovalent scFv is linked to the other polypeptide constituting the Fc region via a light chain Fv fragment forming the CD3 binding domain; (2) a domain comprising an Fc region having no binding activity against an Fc gamma receptor and having amino acids constituting an Fc region of IgG1, IgG2a, IgG3 or IgG4; and (3) at least a monovalent CD3 binding domain, wherein the light chain Fv fragment and the heavy chain Fv fragment are associated to constitute a CD3 binding domain in a form binding to the antigen CD3.

### scFv, single-chain antibody, or sc(Fv)₂

In the present specification, the term "scFv", "single-chain antibody", or "sc(Fv)₂" means an antibody fragment of a single polypeptide chain that contains variable regions derived from both heavy and light chains, but not constant regions. In general, a single-chain antibody further contains a polypeptide linker between the VH domain and the VL domain, which enables formation of the desired structure that presumably permits antigen binding. The single-chain antibody is discussed in detail by Pluckthun in The Pharmacology of Monoclonal Antibodies, Vol. 113, Rosenburg and Moore ed., Springer-Verlag, New York, 269-315 (1994). See also International Publication No. WO1988/001649 and U.S. Patent Nos. 4,946,778 and 5,260,203. In a particular aspect, the single-chain antibody can be bispecific and/or humanized.

scFv is an antigen binding domain in which VH and VL constituting Fv are linked via a peptide linker (Proc. Natl. Acad. Sci. U.S.A. (1988) 85 (16), 5879-5883). VH and VL can be retained in close proximity by the peptide linker.

sc(Fv)₂ is a single-chain antibody in which four variable regions of two VLs and two VHs are linked via linkers such as peptide linkers to form a single chain (J Immunol. Methods (1999) 231 (1-2), 177-189). These two VHs and two VLs may be derived from different monoclonal antibodies. Such sc(Fv)₂ also preferably includes a bispecific sc(Fv)₂ that recognizes two epitopes present in the same antigen, for example, as disclosed in Journal of Immunology (1994) 152 (11), 5368-5374. sc(Fv)₂ can be produced by methods known to those skilled in the art. sc(Fv)₂ can be produced, for example, by linking scFvs by a linker such as a peptide linker.

Examples of the configuration of the antigen binding domains constituting the sc(Fv)₂ described in the present specification include an antibody in which two VHs and two VLs are aligned as VH, VL, VH, and VL (i.e., [VH]-linker-[VL]-linker-[VH]-linker-[VL]) in this order starting at the N-terminus of the single-chain polypeptide. The order of two VHs and two VLs is not particularly limited to the configuration described above and may be any order of arrangement. Examples thereof can also include the following arrangements:
[VL]-linker- [VH]-linker- [VH] -linker-[VL],
[VH]-linker-[VL]-linker-[VL]-linker-[VH],
[VH]-linker-[VH]-linker-[VL]-linker-[VL],
[VL] -linker- [VL]-linker-[VH]-linker-[VH], and
[VL]-linker-[VH]-linker-[VL]-linker-[VH].

The molecular form of the sc(Fv)₂ is also described in detail in WO2006/132352. On the basis of the description therein, those skilled in the art can appropriately prepare the desired sc(Fv)₂ in order to prepare the antigen binding molecule disclosed in the present specification.

The antigen binding molecule of the present invention may be conjugated with a carrier polymer such as PEG or an organic compound such as an anticancer agent. Also, a sugar chain can be preferably added to the antigen binding molecule of the present invention by the insertion of a glycosylation sequence for the purpose of producing the desired effects.

For example, an arbitrary peptide linker that can be introduced by genetic engineering, or a synthetic compound linker (e.g., a linker disclosed in Protein Engineering, 9 (3), 299-305, 1996) can be used as the linker to link the antibody variable regions. In the present invention, a peptide linker is preferred. The length of the peptide linker is not particularly limited and can be appropriately selected by those skilled in the art according to the purpose. The length is preferably 5 or more amino acids (the upper limit is not particularly limited and is usually 30 or less amino acids, preferably 20 or less amino acids), particularly preferably 15 amino acids. When the sc(Fv)₂ contains three peptide linkers, all of these peptide linkers used may have the same lengths or may have different lengths.

Examples of the peptide linker can include
Ser,
Gly-Ser,
Gly-Gly-Ser,
Ser-Gly-Gly,
Gly-Gly-Gly-Ser,
Ser-Gly-Gly-Gly,
Gly-Gly-Gly-Gly-Ser,
Ser-Gly-Gly-Gly-Gly,
Gly-Gly-Gly-Gly-Gly-Ser,
Ser-Gly-Gly-Gly-Gly-Gly,
Gly-Gly-Gly-Gly-Gly-Gly-Ser,
Ser-Gly-Gly-Gly-Gly-Gly-Gly,
(Gly-Gly-Gly-Gly-Ser)n, and
(Ser-Gly-Gly-Gly-Gly)n,
wherein n is an integer of 1 or larger.

However, the length or sequence of the peptide linker can be appropriately selected by those skilled in the art according to the purpose.

The synthetic compound linker (chemical cross-linking agent) is a cross-linking agent usually used in the cross-linking of peptides, for example, N-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl]sulfone (BSOCOES), or bis[2-(sulfosuccinimidoxycarbonyloxy)ethyl]sulfone (sulfo-BSOCOES).

These cross-linking agents are commercially available.

Three linkers are usually necessary for linking four antibody variable regions. All of these linkers used may be the same linkers or may be different linkers.

"Fab" is constituted by a single light chain, and a CH1 region and a variable region of a single heavy chain. The heavy chain of the Fab molecule cannot form a disulfide bond with another heavy chain molecule.

"F(ab')₂" and "Fab'" are produced by treating an immunoglobulin (monoclonal antibody) with protease such as pepsin and papain, and mean an antibody fragment produced by digestion near a disulfide bond present between two H chains in a hinge region. For example, papain treatment cleaves IgG upstream of the disulfide bond present between two H chains in a hinge region to produce two homologous antibody fragments in which an L chain consisting of VL (L chain variable region) and CL (L chain constant region) is linked to an H chain fragment consisting of VH (H chain variable region) and CH gamma 1 (gamma 1 region in an H chain constant region) via a disulfide bond at their C-terminal regions. Each of these two homologous antibody fragments is called Fab'.

"F(ab')₂" comprises two light chains and two heavy chains containing a constant region (CH1 domains and a portion of CH2 domains) so as to form the interchain disulfide bond between these two heavy chains. The F(ab')₂ constituting the antigen binding molecule disclosed in the present specification can be preferably obtained by the partial digestion of, for example, a full-length monoclonal antibody having the desired antigen binding domains with a proteolytic enzyme such as pepsin followed by the removal of an Fc fragment adsorbed on a protein A column. Such a proteolytic enzyme is not particularly limited as long as the enzyme is capable of digesting a full-length antibody to restrictively form F(ab')₂ under appropriately set reaction conditions (e.g., pH) of the enzyme. Examples thereof can include pepsin and ficin.

An Fc region constituting the antigen binding molecule disclosed in the present specification can be preferably obtained by partially digesting an antibody such as a monoclonal antibody with protease such as pepsin, and then adsorbing the resulting fragment onto a protein A or protein G column, followed by elution with an appropriate elution buffer or the like. The protease is not particularly limited as long the protease is capable of digesting an antibody such as a monoclonal antibody under appropriately set reaction conditions (e.g., pH) of the enzyme. Examples thereof can include pepsin and ficin.

The antigen binding molecule described in the present specification comprises an Fc region having reduced binding activity against an Fc gamma receptor and having amino acids constituting an IgG1, IgG2, IgG3 or IgG4 Fc region.

Antibody isotypes are determined according to the structures of constant regions. Constant regions of the isotypes IgG1, IgG2, IgG3, and IgG4 are called C gamma 1, C gamma 2, C gamma 3, and C gamma 4, respectively.

The Fc region refers to a region, except for F(ab')₂, comprising two light chains and two heavy chains comprising a portion of a constant region including a region between the CH1 and CH2 domains such that interchain disulfide bonds are formed between the two heavy chains. The Fc region constituting the antigen binding molecule disclosed in the present specification can be preferably obtained by partially digesting an IgG1, IgG2, IgG3, or IgG4 monoclonal antibody or the like with protease such as pepsin, and then re-eluting a fraction adsorbed on a protein A. The protease is not particularly limited as long the protease is capable of digesting a full-length antibody to restrictively form F(ab')₂ under appropriately set reaction conditions (e.g., pH) of the enzyme. Examples thereof can include pepsin and ficin.

The Fcγ receptor refers to a receptor capable of binding to the Fc region of an IgG1, IgG2, IgG3, or IgG4 monoclonal antibody and means any member of the protein family substantially encoded by Fcγ receptor genes. In humans, this family includes, but is not limited to: FcyRI (CD64) including isoforms FcyRIa, FcyRIb, and FcyRIc; FcyRII (CD32) including isoforms FcyRIIa (including allotypes H131 and R131), FcyRIIb (including FcyRIIb-1 and FcyRIIb-2), and FcyRIIc; and FcγRIII (CD16) including isoforms FcγRIIIa (including allotypes V158 and F158) and FcyRIIIb (including allotypes FcγRIIIb-NA1 and FcyRIIIb-NA2); and any yet-to-be-discovered human FcyR or FcyR isoform or allotype. The FcyR includes those derived from humans, mice, rats, rabbits, and monkeys. The FcyR is not limited to these molecules and may be derived from any organism. The mouse FcyRs include, but are not limited to, FcyRI (CD64), FcyRII (CD32), FcyRIII (CD16), and FcyRIII-2 (CD16-2), and any yet-to-be-discovered mouse FcyR or FcyR isoform or allotype. Preferred examples of such Fcγ receptors include human FcyRI (CD64), FcyRIIa (CD32), FcyRIIB (CD32), FcyRIIIA (CD16), and/or FcyRIIIB (CD16). The polynucleotide sequence and amino acid sequence of FcyRI are described in RefSeq registration Nos. NM_000566.3 and NP_000557.1, respectively. The polynucleotide sequence and amino acid sequence of FcyRIIA are described in RefSeq registration Nos. BC020823.1 and 30AAH20823.1, respectively. The polynucleotide sequence and amino acid sequence of FcyRIIB are described in RefSeq registration Nos. BC146678.1 and AAI46679.1, respectively. The polynucleotide sequence and amino acid sequence of FcyRIIIA are described in RefSeq registration Nos. BC033678.1 and AAH33678.1, respectively. The polynucleotide sequence and amino acid sequence of FcyRIIIB are described in RefSeq registration Nos. BC128562.1 and AAI28563.1, respectively. Whether or not the Fcγ receptor has binding activity against the Fc region of an IgG1, IgG2, IgG3, or IgG4 monoclonal antibody can be confirmed by FACS or the ELISA format described above as well as by ALPHAScreen (amplified luminescent proximity homogeneous assay screen), the BIACORE method based on a surface plasmon resonance (SPR) phenomenon, or the like (Proc. Natl. Acad. Sci. USA (2006) 103 (11), 4005-4010).

The "Fc ligand" or the "effector ligand" refers to a molecule derived from any organism, preferably a polypeptide, which binds to an antibody Fc region to form an Fc/Fc ligand complex. The binding of the Fc ligand to Fc preferably induces one or more effector functions. The Fc ligand includes, but is not limited to, Fc receptors, FcγR, FcαR, FcεR, FcRn, C1q, and C3, mannan binding lectin, mannose receptor, *Staphylococcus* protein A, *Staphylococcus* protein G, and viral FcyR. The Fc ligand also includes Fc receptor homologs (FcRH) (Davis et al., (2002) Immunological Reviews 190, 123-136) which are a family of Fc receptors homologous to FcyR. The Fc ligand may also include unfound molecules binding to Fc.

Whether or not the Fc region has reduced binding activity against any Fcγ receptor of FcγI, FcγIIA, FcγIIB, FcγIIIA and FcγIIIB can be confirmed by FACS or the ELISA format described above as well as by ALPHAScreen (amplified luminescent proximity homogeneous assay screen), the BIACORE method based on a surface plasmon resonance (SPR) phenomenon, or the like (Proc. Natl. Acad. Sci. USA (2006) 103 (11), 4005-4010).

The ALPHAScreen method is carried out by the ALPHA technology using two types of beads (donor and acceptor) on the basis of the following principle: luminescence signals are detected only when these two beads are located in proximity through the biological interaction between a molecule bound with the donor bead and a molecule bound with the acceptor bead. A laser-excited photosensitizer in the donor bead converts ambient oxygen to singlet oxygen having an excited state. The singlet oxygen diffuses around the donor bead and reaches the acceptor bead located in proximity thereto to thereby cause chemiluminescent reaction in the bead, which finally emits light. In the absence of the interaction between the molecule bound with the donor bead and the molecule bound with the acceptor bead, singlet oxygen produced by the donor bead does not reach the acceptor bead. Thus, no chemiluminescent reaction occurs.

For example, a biotin-labeled antigen binding molecule is allowed to bind to the donor bead, while a glutathione S transferase (GST)-tagged Fcγ receptor is allowed to bind to the acceptor bead. In the absence of a competing antigen binding molecule having a mutated Fc region, an antigen binding molecule having a wild-type Fc region interacts with the Fcγ receptor to generate signals of 520 to 620 nm. The untagged antigen binding molecule having a mutated Fc region competes with the antigen binding molecule having a wild-type Fc region for the interaction with the Fcγ receptor. Decrease in fluorescence caused as a result of the competition can be quantified to thereby determine relative binding affinity. The antigen binding molecule (e.g., antibody) biotinylation using sulfo-NHS-biotin or the like is known in the art. The Fcγ receptor can be tagged with GST by an appropriately adopted method which involves, for example: fusing a polynucleotide encoding the Fcγ receptor in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is injected on the surface of the sensor chip. Upon binding of the analyte to the ligand, the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). Kinetics, i.e., an association rate constant (ka) and a dissociation rate constant (kd), can be determined from the curve of the sensorgram, while affinity (KD) can be determined from the ratio between these constants. Inhibition assay is also preferably used in the BIACORE method. Examples of the inhibition assay are described in Proc. Natl. Acad. Sci. USA (2006) 103 (11), 4005-4010.

A plurality of therapeutic antibodies that exhibit an antitumor effect exert an antitumor effect on cancer cells through the inhibition of signals necessary for cancer cell growth, the induction of cell death signals, or ADCC (antibody dependent cell-mediated cytotoxicity or antibody-dependent cellular cytotoxicity) or CDC (complement dependent cytotoxicity). An antibody Fc region binds to an Fc receptor present on effector cells such as NK cells or macrophages so that these effector cells exert cytotoxicity to a target cancer cell bound with the antibody. This cytotoxicity is ADCC. A complement complex binds to a complement binding site present in an antibody structure. A complement component present in the complex forms a hole on the cell membrane of a cell bound with the antibody so that the influx of water or ions into the cell is promoted to disrupt the cell, causing cytotoxicity. This cytotoxicity is CDC. Among the Fc receptors, the Fcγ receptor refers to a receptor capable of binding to the Fc region of an IgG1, IgG2, IgG3, or IgG4 monoclonal antibody. When binding activity against the Fcγ receptor is low, T cells and the receptor expressed in NK cells, macrophages, or the like are not bridged in a manner independent of a cancer antigen. Hence, cancer antigen-independent cytokine induction does not occur. An antibody comprising an Fc region having reduced binding activity against any Fcγ receptor of FcyI, FcγIIA, FcγIIB, FcγIIIA and/or FcγIIIB is desirable as an antibody or an IgG antibody-like molecule serving as a primary molecule and is desirable as a bispecific antibody serving as a secondary molecule.

In the present specification, the reduced binding activity against an Fcγ receptor means that the test antigen binding molecule exhibits competitive activity of, for example, 50% or lower, preferably 45% or lower, 40% or lower, 35% or lower, 30% or lower, 20% or lower, or 15% or lower, particularly preferably 10% or lower, 9% or lower, 8% or lower, 7% or lower, 6% or lower, 5% or lower, 4% or lower, 3% or lower, 2% or lower, or 1% or lower, compared with the competitive activity of a control antigen binding molecule on the basis of the analysis method described above.

An antigen binding molecule having an IgG1, IgG2, IgG3, or IgG4 monoclonal antibody Fc region can be appropriately used as the control antigen binding molecule. Examples of the structure of the Fc region include a sequence of RefSeq registration No. AAC82527.1 with A added to the N terminus, a sequence of RefSeq registration No. AAB59393.1 with A added to the N terminus, a sequence of RefSeq registration No. CAA27268.1 with A added to the N terminus, and a sequence of RefSeq registration No. AAB59394.1 with A added to the N terminus. In the case of using an antigen binding molecule having a mutant of the Fc region of an antibody of a certain isotype as a test substance, an antigen binding molecule having the Fc region of the antibody of this certain isotype is used as a control to test the effect of the mutation in the mutant on the binding activity against an Fcγ receptor. The antigen binding molecule having the Fc region mutant thus confirmed to have reduced binding activity against an Fcγ receptor is appropriately prepared.

For example, a 231A-238S deletion (WO 2009/011941), C226S, C229S, P238S, (C220S)(J. Rheumatol (2007) 34, 11), C226S, C229S (Hum. Antibod. Hybridomas (1990) 1 (1), 47-54), C226S, C229S, E233P, L234V, or L235A (Blood (2007) 109, 1185-1192) (these amino acids are defined according to the EU numbering) mutant is known in the art as such a mutant.

Preferred examples thereof include antigen binding molecules having an Fc region derived from the Fc region of an antibody of a certain isotype by the substitution of any of the following constituent amino acids: amino acids at positions 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, and 332 defined according to the EU numbering. The isotype of the antibody from which the Fc region is originated is not particularly limited, and an Fc region originated from an IgG1, IgG2, IgG3, or IgG4 monoclonal antibody can be appropriately used. An Fc region originated from an IgG1 antibody is preferably used.

For example, an antigen binding molecule having an Fc region derived from an IgG1 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):
(a) L234F, L235E, and P331S,
(b) C226S, C229S, and P238S,
(c) C226S and C229S,
(d) C226S, C229S, E233P, L234V, and L235A,
(e) L234A, L235A or L235R, and N297A, and
(f) L235A or L235R, S239K, and N297A
or by the deletion of an amino acid sequence from positions 231 to 238 defined according to the EU numbering can also be appropriately used.

An antigen binding molecule having an Fc region derived from an IgG2 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):
(g) H268Q, V309L, A330S, and P331S,
(h) V234A,
(i) G237A,
(j) V234A and G237A,
(k) A235E and G237A,
(1) V234A, A235E, and G237A
defined according to the EU numbering can also be appropriately used.

An antigen binding molecule having an Fc region derived from an IgG3 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):
(m) F241A,
(n) D265A, and
(o) V264A
defined according to the EU numbering can also be appropriately used.

An antigen binding molecule having an Fc region derived from an IgG4 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):
(p) L235A, G237A, and E318A,
(q) L235E, and
(r) F234A and L235A
defined according to the EU numbering can also be appropriately used.

Other preferred examples thereof include antigen binding molecules having an Fc region derived from the Fc region of an IgG1 antibody by the substitution of any of the following constituent amino acids: amino acids at positions 233, 234, 235, 236, 237, 327, 330, and 331 defined according to the EU numbering, by an amino acid at the corresponding EU numbering position in the Fc region of the counterpart IgG2 or IgG4.

Other preferred examples thereof include antigen binding molecules having an Fc region derived from the Fc region of an IgG1 antibody by the substitution of any one or more of the following constituent amino acids: amino acids at positions 234, 235, and 297 defined according to the EU numbering, by a different amino acid. The type of the amino acid present after the substitution is not particularly limited. An antigen binding molecule having an Fc region with any one or more of amino acids at positions 234, 235, and 297 substituted by alanine is particularly preferred.

Other preferred examples thereof include antigen binding molecules having an Fc region derived from the Fc region of an IgG1 antibody by the substitution of the constituent amino acid at position 265 defined according to the EU numbering, by a different amino acid. The type of the amino acid present after the substitution is not particularly limited. An antigen binding molecule having an Fc region with an amino acid at position 265 substituted by alanine is particularly preferred.

Many studies have been made so far on antibody-dependent cellular cytotoxicity (ADCC) and complement-dependent cytotoxicity (CDC) which are the effector functions of antibodies of IgG class. Among the antibodies of human IgG class, antibodies of IgG1 subclass have the highest ADCC activity and CDC activity. Also, antibody-dependent cellular phagocytosis (ADCP) which is the phagocytosis of target cells via antibodies of IgG class is suggested as one of the effector functions of antibodies. Antibodies of IgG1 subclass are capable of exerting these effector functions against tumor. Therefore, the antibodies of IgG1 subclass are used as most of antibody drugs for cancer antigens.

Meanwhile, IgG antibodies for mediating an antibody effector function ADCC or ADCP, or antibody-dependent cellular phagocytosis (ADCP) activity for the phagocytosis of target cells require the binding of the Fc region of the IgG antibody to an Fcγ receptor (FcyR) present on the surface of effector cells such as killer cells, natural killer cells, or activated macrophages.

Enhancement in cytotoxic effector functions such as ADCC and ADCP is a promising approach for enhancing the antitumor effects of anticancer antibodies. It is suggested that an antibody having an Fc region that exhibits the optimized binding to an Fcγ receptor mediates a stronger effector function and thereby exerts an effective antitumor effect. Accordingly, various reports (e.g., WO2013047752) have been made so far as antibody engineering approaches of enhancing or improving the antitumor activity of antibody drugs against cancer antigens.

For the binding between an Fc region and an Fcγ receptor, some amino acid residues in an antibody hinge region and CH2 domain and sugar chains added to Asn 297 (EU numbering) of the CH2 domain have been found to be important (Clark, M., Chemical Immunology (1997) 65, 88-110; Greenwood J, Clark M, Waldmann H., Eur. J. Immunol. (1993) 23, 1098-1104; and Morgan A, Jones ND, Nesbitt AM, Chaplin L, Bodmer MW, Emtage JS., Immunology (1995) 86, 319-324). Fc region mutants having various Fcγ receptor binding characteristics have been studied so far by focusing on this binding site, to obtain Fc region mutants having higher affinity for activating Fcγ receptors (WO2000/042072 and WO2006/019447).

Since binding activity against an Fcγ receptor plays an important role in the cytotoxic activity of antibodies targeting membrane antigens as mentioned above, human IgG1 isotype having high binding activity against FcyR is used when cytotoxic activity is necessary. Further, enhancement in cytotoxic activity by enhancing binding activity against an Fcγ receptor is a technique widely used. An attempt has also been made to enhance the Fcγ receptor binding activity of antibodies targeting soluble antigens (WO2013047752).

The secondary molecule antibody having ADCC activity is preferably an IgG antibody or an IgG antibody-like molecule having an Fc region containing at least one or more amino acids selected from the group consisting of:
Lys or Tyr at amino acid position 221,
Phe, Trp, Glu or Tyr at amino acid position 222,
Phe, Trp, Glu or Lys at amino acid position 223,
Phe, Trp, Glu or Tyr at amino acid position 224,
Glu, Lys or Trp at amino acid position 225,
Glu, Gly, Lys or Tyr at amino acid position 227,
Glu, Gly, Lys or Tyr at amino acid position 228,
Ala, Glu, Gly or Tyr at amino acid position 230,
Glu, Gly, Lys, Pro or Tyr at amino acid position 231,
Glu, Gly, Lys or Tyr at amino acid position 232,
Ala, Asp, Phe, Gly, His, Ile, Lys, Leu, Met, Asn, Gln, Arg, Ser, Thr, Val, Trp or Tyr at amino acid position 233,
Ala, Asp, Glu, Phe, Gly, His, Ile, Lys, Met, Asn, Pro, Gln, Arg, Ser, Thr, Val, Trp or Tyr at amino acid position 234,
Ala, Asp, Glu, Phe, Gly, His, Ile, Lys, Met, Asn, Pro, Gln, Arg, Ser, Thr, Val, Trp or Tyr at amino acid position 235,
Ala, Asp, Glu, Phe, His, Ile, Lys, Leu, Met, Asn, Pro, Gln, Arg, Ser, Thr, Val, Trp or Tyr at amino acid position 236,
Asp, Glu, Phe, His, Ile, Lys, Leu, Met, Asn, Pro, Gln, Arg, Ser, Thr, Val, Trp or Tyr at amino acid position 237,
Asp, Glu, Phe, Gly, His, Ile, Lys, Leu, Met, Asn, Gln, Arg, Ser, Thr, Val, Trp or Tyr at amino acid position 238,
Asp, Glu, Phe, Gly, His, Ile, Lys, Leu, Met, Asn, Pro, Gln, Arg, Thr, Val, Trp or Tyr at amino acid position 239,
Ala, Ile, Met or Thr at amino acid position 240,
Asp, Glu, Leu, Arg, Trp or Tyr at amino acid position 241,
Leu, Glu, Leu, Gln, Arg, Trp or Tyr at amino acid position 243,
His at amino acid position 244,
Ala at amino acid position 245,
Asp, Glu, His or Tyr at amino acid position 246,
Ala, Phe, Gly, His, Ile, Leu, Met, Thr, Val or Tyr at amino acid position 247,
Glu, His, Gln or Tyr at amino acid position 249,
Glu or Gln at amino acid position 250,
Phe at amino acid position 251,
Phe, Met or Tyr at amino acid position 254,
Glu, Leu or Tyr at amino acid position 255,
Ala, Met or Pro at amino acid position 256,
Asp, Glu, His, Ser or Tyr at amino acid position 258,
Asp, Glu, His or Tyr at amino acid position 260,
Ala, Glu, Phe, Ile or Thr at amino acid position 262,
Ala, Ile, Met or Thr at amino acid position 263,
Asp, Glu, Phe, Gly, His, Ile, Lys, Leu, Met, Asn, Pro, Gln, Arg, Ser, Thr, Trp or Tyr at amino acid position 264,
Ala, Leu, Phe, Gly, His, Ile, Lys, Leu, Met, Asn, Pro, Gln, Arg, Ser, Thr, Val, Trp or Tyr at amino acid position 265,
Ala, Ile, Met or Thr at amino acid position 266,
Asp, Glu, Phe, His, Ile, Lys, Leu, Met, Asn, Pro, Gln, Arg, Thr, Val, Trp or Tyr at amino acid position 267,
Asp, Glu, Phe, Gly, Ile, Lys, Leu, Met, Pro, Gln, Arg, Thr, Val or Trp at amino acid position 268,
Phe, Gly, His, Ile, Lys, Leu, Met, Asn, Pro, Arg, Ser, Thr, Val, Trp or Tyr at amino acid position 269,
Glu, Phe, Gly, His, Ile, Leu, Met, Pro, Gln, Arg, Ser, Thr, Trp or Tyr at amino acid position 270,
Ala, Asp, Glu, Phe, Gly, His, Ile, Lys, Leu, Met, Asn, Gln, Arg, Ser, Thr, Val, Trp or Tyr at amino acid position 271,
Asp, Phe, Gly, His, Ile, Lys, Leu, Met, Pro, Arg, Ser, Thr, Val, Trp or Tyr at amino acid position 272,
Phe or Ile at amino acid position 273,
Asp, Glu, Phe, Gly, His, Ile, Leu, Met, Asn, Pro, Arg, Ser, Thr, Val, Trp or Tyr at amino acid position 274,
Leu or Trp at amino acid position 275,
Asp, Glu, Phe, Gly, His, Ile, Leu, Met, Pro, Arg, Ser, Thr, Val, Trp or Tyr at amino acid position 276,
Asp, Glu, Gly, His, Ile, Lys, Leu, Met, Asn, Pro, Gln, Arg, Ser, Thr, Val or Trp at amino acid position 278,
Ala at amino acid position 279,
Ala, Gly, His, Lys, Leu, Pro, Gln, Trp or Tyr at amino acid position 280,
Asp, Lys, Pro or Tyr at amino acid position 281,
Glu, Gly, Lys, Pro or Tyr at amino acid position 282,
Ala, Gly, His, Ile, Lys, Leu, Met, Pro, Arg or Tyr at amino acid position 283,
Asp, Glu, Leu, Asn, Thr or Tyr at amino acid position 284,
Asp, Glu, Lys, Gln, Trp or Tyr at amino acid position 285,
Glu, Gly, Pro or Tyr at amino acid position 286,
Asn, Asp, Glu or Tyr at amino acid position 288,
Asp, Gly, His, Leu, Asn, Ser, Thr, Trp or Tyr at amino acid position 290,
Asp, Glu, Gly, His, Ile, Gln or Thr at amino acid position 291,
Ala, Asp, Glu, Pro, Thr or Tyr at amino acid position 292,
Phe, Gly, His, Ile, Leu, Met, Asn, Pro, Arg, Ser, Thr, Val, Trp or Tyr at amino acid position 293,
Phe, Gly, His, Ile, Lys, Leu, Met, Asn, Pro, Arg, Ser, Thr, Val, Trp or Tyr at amino acid position 294,
Asp, Glu, Phe, Gly, His, Ile, Lys, Met, Asn, Pro, Arg, Ser, Thr, Val, Trp or Tyr at amino acid position 295,
Ala, Asp, Glu, Gly, His, Ile, Lys, Leu, Met, Asn, Gln, Arg, Ser, Thr or Val at amino acid position 296,
Asp, Glu, Phe, Gly, His, Ile, Lys, Leu, Met, Pro, Gln, Arg, Ser, Thr, Val, Trp or Tyr at amino acid position 297,
Ala, Asp, Glu, Phe, His, Ile, Lys, Met, Asn, Gln, Arg, Thr, Val, Trp or Tyr at amino acid position 298,
Ala, Asp, Glu, Phe, Gly, His, Ile, Lys, Leu, Met, Asn, Pro, Gln, Arg, Ser, Val, Trp or Tyr at amino acid position 299,
Ala, Asp, Glu, Gly, His, Ile, Lys, Leu, Met, Asn, Pro, Gln, Arg, Ser, Thr, Val or Trp at amino acid position 300,
Asp, Glu, His or Tyr at amino acid position 301,
Ile at amino acid position 302,
Asp, Gly or Tyr at amino acid position 303,
Asp, His, Leu, Asn or Thr at amino acid position 304,
Glu, Ile, Thr or Tyr at amino acid position 305,
Ala, Asp, Asn, Thr, Val or Tyr at amino acid position 311,
Phe at amino acid position 313,
Leu at amino acid position 315,
Glu or Gln at amino acid position 317,
His, Leu, Asn, Pro, Gln, Arg, Thr, Val or Tyr at amino acid position 318,
Asp, Phe, Gly, His, Ile, Leu, Asn, Pro, Ser, Thr, Val, Trp or Tyr at amino acid position 320, Ala, Asp, Phe, Gly, His, Ile, Pro, Ser, Thr, Val, Trp or Tyr at amino acid position 322,
Ile at amino acid position 323,
Asp, Phe, Gly, His, Ile, Leu, Met, Pro, Arg, Thr, Val, Trp or Tyr at amino acid position 324, Ala, Asp, Glu, Phe, Gly, His, Ile, Lys, Leu, Met, Pro, Gln, Arg, Ser, Thr, Val, Trp or Tyr at amino acid position 325,
Ala, Asp, Glu, Gly, Ile, Leu, Met, Asn, Pro, Gln, Ser, Thr, Val, Trp or Tyr at amino acid position 326,
Ala, Asp, Glu, Phe, Gly, His, Ile, Lys, Leu, Met, Asn, Pro, Arg, Thr, Val, Trp or Tyr at amino acid position 327,
Ala, Asp, Glu, Phe, Gly, His, Ile, Lys, Met, Asn, Pro, Gln, Arg, Ser, Thr, Val, Trp or Tyr at amino acid position 328,
Asp, Glu, Phe, Gly, His, Ile, Lys, Leu, Met, Asn, Gln, Arg, Ser, Thr, Val, Trp or Tyr at amino acid position 329,
Cys, Glu, Phe, Gly, His, Ile, Lys, Leu, Met, Asn, Pro, Arg, Ser, Thr, Val, Trp or Tyr at amino acid position 330,
Asp, Phe, His, Ile, Leu, Met, Gln, Arg, Thr, Val, Trp or Tyr at amino acid position 331,
Ala, Asp, Glu, Phe, Gly, His, Lys, Leu, Met, Asn, Pro, Gln, Arg, Ser, Thr, Val, Trp or Tyr at amino acid position 332,
Ala, Asp, Glu, Phe, Gly, His, Ile, Leu, Met, Pro, Ser, Thr, Val or Tyr at amino acid position 333,
Ala, Glu, Phe, Ile, Leu, Pro or Thr at amino acid position 334,
Asp, Phe, Gly, His, Ile, Leu, Met, Asn, Pro, Arg, Ser, Val, Trp or Tyr at amino acid position 335,
Glu, Lys or Tyr at amino acid position 336,
Glu, His or Asn at amino acid position 337,
Asp, Phe, Gly, Ile, Lys, Met, Asn, Gln, Arg, Ser or Thr at amino acid position 339,
Ala or Val at amino acid position 376,
Gly or Lys at amino acid position 377,
Asp at amino acid position 378,
Asn at amino acid position 379,
Ala, Asn or Ser at amino acid position 380,
Ala or Ile at amino acid position 382,
Glu at amino acid position 385,
Thr at amino acid position 392,
Leu at amino acid position 396,
Lys at amino acid position 421,
Asn at amino acid position 427,
Phe or Leu at amino acid position 428,
Met at amino acid position 429,
Trp at amino acid position 434,
Ile at amino acid position 436, and
Gly, His, Ile, Leu or Tyr at amino acid position 440,
among sites represented by EU numbering in the Fc region.

### Preparation of primary molecule

The primary molecule is an antigen binding molecule binding to an antigen expressed in lesion cells and comprising a linker that is cleavable by protease. As one example, the primary molecule is prepared by inserting the linker that is cleavable by protease to (1) an antibody binding to an antigen expressed in lesion cells, which is produced by the procedures described above.

More specifically, a partial sequence of the antibody for linker insertion is added to a DNA sequence encoding a portion or the whole of the linker to prepare 3' and 5' PCR primers. PCR reaction is performed on the 5' and 3' sides of DNA by using the nucleotide sequence of the heavy chain or the light chain of the antibody for linker insertion as a template. Next, the obtained PCR products are inserted to expression vectors known in the art using In-Fusion(R) HD Cloning Kit (Clontech Laboratories, Inc.) or the like. Cultured cells are cotransfected with the obtained expression vector and the light chain or heavy chain plasmid to be paired therewith by a method known in the art. The antibody thus expressed is purified by a method known in the art using a protein A column or the like to obtain an antibody harboring the linker.

The "binding" of the present disclosure usually refers to binding through interaction principally involving a noncovalent bond such as electrostatic force, van der Waals' forces, or a hydrogen bond. Preferred examples of the binding pattern of the present disclosure include, but are not limited to, the antigen-antibody reaction of an antigen binding region, an antigen binding molecule, an antibody, or an antibody fragment binding to an antigen.

The primary molecule of the present disclosure is limited only by binding to an antigen expressed in lesion cells, and a molecule having any structure can be used as long as the molecule in an uncleaved or cleaved state can bind to an antigen expressed in the lesion cell of interest. Examples of the primary molecule include, but are not limited to, antibodies, single-domain antibodies (sdAbs), a module called A domain of approximately 35 amino acids contained in an *in vivo* cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854).

In one embodiment of the present invention, a flexible linker is further attached to either one end or both ends of the protease cleavage sequence. The flexible linker at one end of the protease cleavage sequence can be referred to as a first flexible linker, and the flexible linker at the other end can be referred to as a second flexible linker. In a particular embodiment, the protease cleavage sequence and the flexible linker have any of the following formulas:
(protease cleavage sequence),
(first flexible linker)-(protease cleavage sequence),
(protease cleavage sequence)-(second flexible linker), and
(first flexible linker)-(protease cleavage sequence)-(second flexible linker).

The flexible linker according to the present embodiment is preferably a peptide linker. The first flexible linker and the second flexible linker each independently and arbitrarily exist and are identical or different flexible linkers each containing at least one flexible amino acid (Gly, etc.). The flexible linker contains, for example, a sufficient number of residues (amino acids arbitrarily selected from Arg, Ile, Gln, Glu, Cys, Tyr, Trp, Thr, Val, His, Phe, Pro, Met, Lys, Gly, Ser, Asp, Asn, Ala, etc., particularly Gly, Ser, Asp, Asn, and Ala, in particular, Gly and Ser, especially Gly, etc.) for the protease cleavage sequence to obtain the desired protease accessibility.

The flexible linker suable for use at both ends of the protease cleavage sequence is usually a flexible linker that improves the access of protease to the protease cleavage sequence and elevates the cleavage efficiency of the protease. A suitable flexible linker may be readily selected and can be preferably selected from among different lengths such as 1 amino acid (Gly, etc.) to 20 amino acids, 2 amino acids to 15 amino acids, or 3 amino acids to 12 amino acids including 4 amino acids to 10 amino acids, 5 amino acids to 9 amino acids, 6 amino acids to 8 amino acids, or 7 amino acids to 8 amino acids. In some embodiments of the present invention, the flexible linker is a peptide linker of 1 to 7 amino acids.

Examples of the flexible linker include, but are not limited to, glycine polymers (G)n, glycine-serine polymers (including e.g., (GS)n, (GSGGS)n and (GGGS)n, wherein n is an integer of at least 1), glycine-alanine polymers, alanine-serine polymers, and other flexible linkers well known in conventional techniques.

Among them, glycine and glycine-serine polymers are receiving attention because these amino acids are relatively unstructured and easily function as neutral tethers between components.

Examples of the flexible linker consisting of the glycine-serine polymer can include, but are not limited to,
Ser;
Gly·Ser (GS);
Ser·Gly (SG);
Gly·Gly·Ser (GGS);
Gly·Ser·Gly (GSG);
Ser·Gly·Gly (SGG);
Gly·Ser·Ser (GSS);
Ser·Ser·Gly (SSG);
Ser·Gly·Ser (SGS);
Gly·Gly·Gly·Ser (GGGS);
Gly·Gly·Ser·Gly (GGSG);
Gly·Ser·Gly·Gly (GSGG);
Ser·Gly·Gly·Gly (SGGG);
Gly·Ser·Ser·Gly (GSSG);
Gly·Gly·Gly·Gly·Ser (GGGGS);
Gly·Gly·Gly·Ser·Gly (GGGSG);
Gly·Gly·Ser·Gly·Gly (GGSGG);
Gly·Ser·Gly·Gly·Gly (GSGGG);
Gly·Ser·Gly·Gly·Ser (GSGGS);
Ser·Gly·Gly·Gly·Gly (SGGGG);
Gly·Ser·Ser·Gly·Gly (GSSGG);
Gly·Ser·Gly·Ser·Gly (GSGSG);
Ser·Gly·Gly·Ser·Gly (SGGSG);
Gly·Ser·Ser·Ser·Gly (GSSSG);
Gly·Gly·Gly·Gly·Gly·Ser (GGGGGS);
Ser·Gly·Gly·Gly·Gly·Gly (SGGGGG);
Gly·Gly·Gly·Gly·Gly·Gly·Ser (GGGGGGS);
Ser·Gly·Gly·Gly·Gly·Gly·Gly (SGGGGGG);
(Gly·Gly·Gly·Gly·Ser (GGGGS))n; and
(Ser·Gly·Gly·Gly·Gly (SGGGG))n
wherein n is an integer of 1 or larger.

Those skilled in the art can appropriately select the length or sequence of the peptide linker according to a purpose.

The antigen binding molecule of the present disclosure is a polypeptide comprising a cleavage sequence. The cleavage sequence is cleavable by protease. The cleavage sequence may be placed at any position in the polypeptide as long as an antigen binding fragment resulting from the cleavage can bind to an antigen expressed in target cells (lesion cells). The polypeptide may comprise one or more cleavage sequences.

In one embodiment of the present invention, the antigen binding molecule of the present invention yields an antigen binding fragment by the cleavage of the cleavage sequence, and this antigen binding fragment has antigen binding activity as the antigen binding molecule after cleavage of the linker.

A method for detecting the emergence of the antigen binding fragment after cleavage of the linker of the antigen binding molecule includes a detection method using an antibody for detection recognizing the fragment. The detection using the antibody for antigen binding fragment detection can be confirmed by a well-known method such as such as FACS, ELISA format, ALPHAScreen (amplified luminescent proximity homogeneous assay screen), or the BIACORE method based on a surface plasmon resonance (SPR) phenomenon, BLI (bio-layer interferometry) (Octet) (Proc. Natl. Acad. Sci. USA (2006) 103 (11), 4005-4010). For example, in the case of detecting the antigen binding fragment using Octet, the antibody for detection recognizing the antigen binding fragment is biotinylated and contacted with a biosensor. Then, the emergence of the antigen binding fragment can be detected by measuring binding to the antigen binding fragment in a sample. Specifically, the amounts of the antigen binding fragment in samples containing the antigen binding molecule before protease treatment or the antigen binding fragment after protease treatment are measured using the detection antibody. The emergence of the antigen binding fragment can be detected by comparing the amounts of the antigen binding fragment detected in the samples before and after protease treatment. Also, the amounts of the antigen binding fragment in samples containing protease and the antigen binding molecule before cleavage of the linker or the antigen binding fragment after cleavage of the linker, and samples containing no protease and containing the antigen binding molecule before cleavage of the linker or the antigen binding fragment after cleavage of the linker are measured using the detection antibody. The emergence of the antigen binding fragment can be detected by comparing the amounts of the antigen binding fragment detected in the samples with and without protease. More specifically, the emergence of the antigen binding fragment can be detected by a method described in Examples of the present application. When the antigen binding molecule before cleavage of the linker forms a fusion protein, the amounts of the antigen binding fragment in samples containing the fusion protein before or after protease treatment are measured using the antibody for antigen binding fragment detection. The emergence of the antigen binding fragment can be detected by comparing the amounts of the antigen binding fragment detected in the samples before and after protease treatment. Also, the amounts of the antigen binding fragment in samples containing protease and the fusion protein, and samples containing no protease and containing the fusion protein are measured using the antibody for antigen binding fragment detection. The emergence of the antigen binding fragment can be detected by comparing the amounts of the antigen binding fragment detected in the samples with and without protease. More specifically, the emergence of the antigen binding fragment can be detected by a method described in Examples of the present application.

In one embodiment of the present invention, the cleavage sequence comprises a protease cleavage sequence and is cleaved by protease.

In the present specification, the term "protease" refers to an enzyme such as endopeptidase or exopeptidase which hydrolyzes a peptide bond, typically, endopeptidase. The protease used in the present invention is limited only by being capable of cleaving the protease cleavage sequence and is not particularly limited by its type. In some embodiments, target tissue specific protease is used. The target tissue specific protease can refer to, for example, any of
(1) protease that is expressed at a higher level in the target tissue than in normal tissues,
(2) protease that has higher activity in the target tissue than in normal tissues,
(3) protease that is expressed at a higher level in the target cells than in normal cells, and
(4) protease that has higher activity in the target cells than in normal cells.

In a more specific embodiment, a cancer tissue specific protease or an inflammatory tissue specific protease is used.

In the present specification, the term "target tissue" means a tissue containing at least one target cell. In some embodiments of the present invention, the target tissue is a cancer tissue. In some embodiments of the present invention, the target tissue is an inflammatory tissue.

The term "cancer tissue" means a tissue containing at least one cancer cell. Thus, considering that, for example, the cancer tissue contains cancer cells and vascular vessels, every cell type that contributes to the formation of tumor mass containing cancer cells and endothelial cells is included in the scope of the present invention. In the present specification, the tumor mass refers to a foci of tumor tissue. The term "tumor" is generally used to mean benign neoplasm or malignant neoplasm.

In the present specification, examples of the "inflammatory tissue" include the following:
a joint tissue in rheumatoid arthritis or osteoarthritis,
a lung (alveolus) tissue in bronchial asthma or COPD,
a digestive organ tissue in inflammatory bowel disease, Crohn disease, or ulcerative colitis,
a fibrotic tissue in fibrosis in the liver, the kidney, or the lung,
a tissue under rejection of organ transplantation,
a vascular vessel or heart (cardiac muscle) tissue in arteriosclerosis or heart failure,
a visceral fat tissue in metabolic syndrome,
a skin tissue in atopic dermatitis and other dermatitides, and
a spinal nerve tissue in disk herniation or chronic lumbago.

Specifically expressed or specifically activated protease, or protease considered to be related to the disease condition of a target tissue (target tissue specific protease) is known for some types of target tissues. For example, International Publication Nos. WO2013/128194, WO2010/081173, and WO2009/025846 disclose protease specifically expressed in a cancer tissue. Also, J Inflamm (Lond). 2010; 7: 45, Nat Rev Immunol. 2006 Jul; 6 (7): 541-50, Nat Rev Drug Discov. 2014 Dec; 13 (12): 904-27, Respir Res. 2016 Mar 4; 17: 23, Dis Model Mech. 2014 Feb; 7 (2): 193-203, and Biochim Biophys Acta. 2012 Jan; 1824 (1): 133-45 disclose protease considered to be related to inflammation.

In addition to the protease specifically expressed in a target tissue, there also exists protease specifically activated in a target tissue. For example, protease may be expressed in an inactive form and then converted to an active form. Many tissues contain a substance inhibiting active protease and control the activity by the process of activation and the presence of the inhibitor (Nat Rev Cancer. 2003 Jul; 3 (7): 489-501). In a target tissue, the active protease may be specifically activated by escaping inhibition. The active protease can be measured by use of a method using an antibody recognizing the active protease (PNAS 2013 Jan 2; 110 (1): 93-98) or a method of fluorescently labeling a peptide recognizable by protease so that the fluorescence is quenched before cleavage, but emitted after cleavage (Nat Rev Drug Discov. 2010 Sep; 9 (9): 690-701. doi: 10.1038/nrd3053).

From one viewpoint, the term "target tissue specific protease" can refer to any of
(i) protease that is expressed at a higher level in the target tissue than in normal tissues,
(ii) protease that has higher activity in the target tissue than in normal tissues,
(iii) protease that is expressed at a higher level in the target cells than in normal cells, and
(iv) protease that has higher activity in the target cells than in normal cells.

Specific examples of the protease include, but are not limited to, cysteine protease (including cathepsin families B, L, S, etc.), aspartyl protease (cathepsins D, E, K, O, etc.), serine protease (including matriptase (including MT-SP1), cathepsins A and G, thrombin, plasmin, urokinase (uPA), tissue plasminogen activator (tPA), elastase, proteinase 3, thrombin, kallikrein, tryptase, and chymase), metalloproteinase (metalloproteinase (MMP1-28) including both membrane-bound forms (MMP14-17 and MMP24-25) and secreted forms (MMP1-13, MMP18-23 and MMP26-28), A disintegrin and metalloproteinase (ADAM), A disintegrin and metalloproteinase with thrombospondin motifs (ADAMTS), meprin (meprin alpha and meprin beta), CD10 (CALLA), prostate-specific antigen (PSA), legumain, TMPRSS3, TMPRSS4, human neutrophil elastase (HNE), beta secretase (BACE), fibroblast activation protein alpha (FAP), granzyme B, guanidinobenzoatase (GB), hepsin, neprilysin, NS3/4A, HCV-NS3/4, calpain, ADAMDEC1, renin, cathepsin C, cathepsin V/L2, cathepsin X/Z/P, cruzipain, otubain 2, kallikrein-related peptidases (KLKs (KLK3, KLK4, KLK5, KLK6, KLK7, KLK8, KLK10, KLK11, KLK13, and KLK14)), bone morphogenetic protein 1 (BMP-1), activated protein C, blood coagulation-related protease (Factor VIIa, Factor IXa, Factor Xa, Factor XIa, and Factor XIIa), HtrAl, lactoferrin, marapsin, PACE4, DESC1, dipeptidyl peptidase 4 (DPP-4), TMPRSS2, cathepsin F, cathepsin H, cathepsin L2, cathepsin O, cathepsin S, granzyme A, Gepsin calpain 2, glutamate carboxypeptidase 2, AMSH-like proteases, AMSH, gamma secretase, antiplasmin cleaving enzyme (APCE), decysin 1, N-acetylated alpha-linked acidic dipeptidase-like 1 (NAALADL1), and furin.

From another viewpoint, the target tissue specific protease can refer to a cancer tissue specific protease or an inflammatory tissue specific protease.

Examples of cancer tissue specific protease include protease specifically expressed in a cancer tissue disclosed in International Publication Nos. WO2013/128194, WO2010/081173, and WO2009/025846.

As for the type of cancer tissue specific protease, the protease having higher expression specificity in the cancer tissue to be treated is more effective for reducing adverse reactions. Preferable cancer tissue specific protease has a concentration in the cancer tissue at least 5 times, more preferably at least 10 times, further preferably at least 100 times, particularly preferably at least 500 times, most preferably at least 1000 times higher than its concentration in normal tissues. Also, preferable cancer tissue specific protease has activity in the cancer tissue at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The cancer tissue specific protease may be in a form bound with a cancer cell membrane or may be in a form secreted extracellularly without being bound with a cell membrane. When the cancer tissue specific protease is not bound with a cancer cell membrane, it is preferred for immunocyte-mediated cytotoxicity specific for cancer cells that the cancer tissue specific protease should exist within or in the vicinity of the cancer tissue. It is preferred that damage on normal cells should be minimized in this scope of location.

From an alternative viewpoint, the protease specifically expressed in a cancer tissue ("cancer tissue specific protease") is any of
(i) protease that is expressed at a higher level in the cancer tissue than in normal tissues,
(ii) protease that has higher activity in the cancer tissue than in normal tissues,
(iii) protease that is expressed at a higher level in the cancer cells than in normal cells, and
(iv) protease that has higher activity in the cancer cells than in normal cells.

One type of cancer tissue specific protease may be used singly, or two or more types of cancer tissue specific proteases may be combined. The number of types of cancer tissue specific protease can be appropriately set by those skilled in the art in consideration of the cancer type to be treated.

From these viewpoints, cancer tissue specific protease is preferably serine protease or metalloproteinase, more preferably matriptase (including MT-SP1), urokinase (uPA), or metalloproteinase, further preferably MT-SP1, uPA, MMP-2, or MMP-9, among the proteases listed above.

As for the type of inflammatory tissue specific protease, the protease having higher expression specificity in the inflammatory tissue to be treated is more effective for reducing adverse reactions. Preferable inflammatory tissue specific protease has a concentration in the inflammatory tissue at least 5 times, more preferably at least 10 times, further preferably at least 100 times, particularly preferably at least 500 times, most preferably at least 1000 times higher than its concentration in normal tissues. Also, preferable inflammatory tissue specific protease has activity in the inflammatory tissues at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The inflammatory tissue specific protease may be in a form bound with an inflammatory cell membrane or may be in a form secreted extracellularly without being bound with a cell membrane. When the inflammatory tissue specific protease is not bound with an inflammatory cell membrane, it is preferred for immunocyte-mediated cytotoxicity specific for inflammatory cells that the inflammatory tissue specific protease should exist within or in the vicinity of the inflammatory tissue. It is preferred that damage on normal cells should be minimized in this scope of location.

From an alternative viewpoint, inflammatory tissue specific protease is any of
(i) protease that is expressed at a higher level in the inflammatory tissue than in normal tissues,
(ii) protease that has higher activity in the inflammatory tissue than in normal tissues,
(iii) protease that is expressed at a higher level in the inflammatory cells than in normal cells, and
(iv) protease that has higher activity in the inflammatory cells than in normal cells.

One type of inflammatory tissue specific protease may be used singly, or two or more types of inflammatory tissue specific proteases may be combined. The number of types of inflammatory tissue specific protease can be appropriately set by those skilled in the art in consideration of the pathological condition to be treated.

From these viewpoints, the inflammatory tissue specific protease is preferably metalloproteinase among the proteases listed above. The metalloproteinase is more preferably ADAMTS5, MMP-2, MMP-7, MMP-9, or MMP-13.

In some more specific embodiments of the present invention, the antigen binding molecule (primary molecule) is an antibody. In the case of using an antibody as the antigen binding molecule, the antigen binding molecule after cleavage of the linker may comprise an antigen binding fragment comprising the whole or a portion of variable regions and a portion of the cleaved linker. In some further specific embodiments, the antigen binding molecule is an IgG antibody. In the case of using an IgG antibody as the antigen binding molecule, its type is not limited and IgG1, IgG2, IgG3, IgG4, or the like can be used. In the case of using an IgG antibody as the antigen binding molecule, its binding to an antigen expressed in target cells or lesion cells is also achieved by an antigen binding fragment comprising the whole or a portion of variable regions.

In some embodiments of the present invention, the cleavage of the cleavage sequence and/or protease cleavage sequence in the antigen binding molecule separates the domain having binding activity against an antigen expressed in lesion cells in the antigen binding molecule. However, binding to the antigen expressed in lesion cells is still attained. For example, in the case of using an IgG antibody as the antigen binding molecule, the cleavage sequence and/or the protease cleavage sequence is disposed in an antibody variable region, which, in a cleaved state, can no longer form the entire antibody variable regions but is capable of binding to the antigen expressed in lesion cells.

In some embodiments of the present invention, VH and VL contained in the antigen binding molecule associate with each other. The association between antibody VH and antibody VL may be canceled, for example, by cleaving the protease cleavage sequence of the linker contained in the antigen binding molecule. The cancelation of the association can be used interchangeably with, for example, the whole or partial cancelation of the state where two or more polypeptide regions interact with each other. The interaction between VH and VL may be wholly canceled, or the interaction between VH and VL may be partially canceled.

The antigen binding molecule of the present invention includes an antigen binding molecule in which the association between antibody VL or a portion thereof and antibody VH or a portion thereof is canceled by the cleavage of the linker.

In some embodiments of the present invention, the antigen binding molecule comprises antibody VH and antibody VL, wherein the antibody VH and the antibody VL in the antigen binding molecule are associated with each other in an uncleaved state of the protease cleavage sequence of the linker in the antigen binding molecule, and the association between the antibody VH and the antibody VL in the antigen binding molecule is canceled by the cleavage of the protease cleavage sequence of the linker. The protease cleavage sequence of the linker in the antigen binding molecule may be placed at any position in the antigen binding molecule as long as the antigen binding molecule even after cleavage can bind to an antigen expressed in lesion cells.

In further some embodiments of the present invention, the antigen binding molecule comprises antibody VH, antibody VL, and antibody constant regions.

As mentioned by Rothlisberger et al. (J Mol Biol. 2005 Apr 8; 347 (4): 773-89), antibody VH and VL domains or CH and CL domains are known to interact with each other via many amino acid side chains. VH-CH1 and VL-CL are known to be able to form a stable structure as a Fab domain. As previously reported, amino acid side chains between VH and VL generally interact with a dissociation constant in the range of 10⁻⁵ M to 10⁻⁸ M. When only VH and VL domains exist, only a small proportion may form an associated state.

In some embodiments of the present invention, the antigen binding molecule is designed such that linker comprising the protease cleavage sequence is established in the antigen binding molecule antibody VH and antibody VL, and the entire heavy chain-light chain interaction is present between two peptides in the Fab structure before cleavage, whereas the interaction between the peptide containing the VH (or a portion of the VH) and the peptide containing the VL (or a portion of the VL) is attenuated by the cleavage of the protease cleavage sequence so that the association between the VH and the VL is canceled.

In one embodiment of the present invention, the linker comprising the protease cleavage sequence is located within an antibody constant region. In a more specific embodiment, the linker comprising the protease cleavage sequence is located on the variable region side compared with amino acid position 140 (EU numbering) in an antibody heavy chain constant region, preferably on the variable region side compared with amino acid position 122 (EU numbering) in an antibody heavy chain constant region. In some specific embodiments, the protease cleavage sequence is introduced at any position in a sequence from amino acid position 118 (EU numbering) to amino acid position 140 (EU numbering) in an antibody heavy chain constant region. In other more specific embodiments, the protease cleavage sequence is located on the variable region side compared with amino acid position 130 (Kabat numbering) in an antibody light chain constant region, preferably on the variable region side compared with amino acid position 113 (Kabat numbering) in an antibody light chain constant region, or amino acid position 112 (Kabat numbering) in an antibody light chain constant region. In some specific embodiments, the protease cleavage sequence is introduced at any position in a sequence from amino acid position 108 (Kabat numbering) to amino acid position 131 (Kabat numbering) in an antibody light chain constant region.

In one embodiment of the present invention, the linker comprising the protease cleavage sequence is located within antibody VH or antibody VL serving as an antigen binding molecule. In a more specific embodiment, the protease cleavage sequence is located on the antibody constant region side with respect to amino acid position 7 (Kabat numbering) of antibody VH, preferably on the antibody constant region side with respect to amino acid position 40 (Kabat numbering) of antibody VH, more preferably on the antibody constant region side with respect to amino acid position 101 (Kabat numbering) of antibody VH, further preferably on the antibody constant region side with respect to amino acid position 109 (Kabat numbering) of antibody VH or on the antibody constant region side with respect to amino acid position 111 (Kabat numbering) of antibody VH In a more specific embodiment, the protease cleavage sequence is located on the antibody constant region side with respect to amino acid position 7 (Kabat numbering) of antibody VL, preferably on the antibody constant region side with respect to amino acid position 39 (Kabat numbering) of antibody VL, more preferably on the antibody constant region side with respect to amino acid position 96 (Kabat numbering) of antibody VL, further preferably on the antibody constant region side with respect to amino acid position 104 (Kabat numbering) of antibody VL or on the antibody constant region side with respect to amino acid position 105 (Kabat numbering) of antibody VL.

In some more specific embodiments, the protease cleavage sequence is introduced at positions of residues constituting a loop structure in antibody VH or antibody VL and residues close to the loop structure. The loop structure in antibody VH or antibody VL refers to a moiety that does not form a secondary structure such as α-helix or β-sheet in the antibody VH or the antibody VL. The positions of residues constituting a loop structure and residues close to the loop structure can specifically refer to ranges from amino acid positions 7 (Kabat numbering) to 16 (Kabat numbering), from amino acid positions 40 (Kabat numbering) to 47 (Kabat numbering), from amino acid positions 55 (Kabat numbering) to 69 (Kabat numbering), from amino acid positions 73 (Kabat numbering) to 79 (Kabat numbering), from amino acid positions 83 (Kabat numbering) to 89 (Kabat numbering), from amino acid positions 95 (Kabat numbering) to 99 (Kabat numbering), and from amino acid positions 101 (Kabat numbering) to 113 (Kabat numbering) in the antibody VH, and from amino acid positions 7 (Kabat numbering) to 19 (Kabat numbering), from amino acid positions 39 (Kabat numbering) to 46 (Kabat numbering), from amino acid positions 49 (Kabat numbering) to 62 (Kabat numbering), and from amino acid positions 96 (Kabat numbering) to 107 (Kabat numbering) in the antibody VL.

In another aspect, when the antigen binding molecule is an antibody, the linker is introduced near a CH2/CH3 interface of an antibody heavy chain constant region. In this context, the phrase "near a CH2/CH3 interface" is a region from EU numbering positions 335 to 345.

In one embodiment, the protease cleavage sequence is located near the boundary between antibody VH and an antibody constant region. The phrase "near the boundary between antibody VH and an antibody heavy chain constant region" can refer to between amino acid position 101 (Kabat numbering) of the antibody VH and amino acid position 140 (EU numbering) of the antibody heavy chain constant region and can preferably refer to between amino acid position 109 (Kabat numbering) of the antibody VH and amino acid position 122 (EU numbering) of the antibody heavy chain constant region, or between amino acid position 111 (Kabat numbering) of the antibody VH and amino acid position 122 (EU numbering) of the antibody heavy chain constant region. When antibody VH is connected to an antibody light chain constant region, the "near the boundary between antibody VH and an antibody light chain constant region" can refer to between amino acid position 101 (Kabat numbering) of the antibody VH and amino acid position 130 (Kabat numbering) of the antibody light chain constant region and can preferably refer to between amino acid position 109 (Kabat numbering) of the antibody VH and amino acid position 113 (Kabat numbering) of the antibody light chain constant region, or between amino acid position 111 (Kabat numbering) of the antibody VH and amino acid position 112 (Kabat numbering) of the antibody light chain constant region.

In one embodiment, the protease cleavage sequence is located near the boundary between antibody VL and an antibody constant region. The phrase "near the boundary between antibody VL and an antibody light chain constant region" can refer to between amino acid position 96 (Kabat numbering) of the antibody VL and amino acid position 130 (Kabat numbering) of the antibody light chain constant region and can preferably refer to between amino acid position 104 (Kabat numbering) of the antibody VL and amino acid position 113 (Kabat numbering) of the antibody light chain constant region, or between amino acid position 105 (Kabat numbering) of the antibody VL and amino acid position 112 (Kabat numbering) of the antibody light chain constant region. When antibody VL is connected to an antibody heavy chain constant region, the "near the boundary between antibody VL and an antibody heavy chain constant region" can refer to between amino acid position 96 (Kabat numbering) of the antibody VL and amino acid position 140 (EU numbering) of the antibody heavy chain constant region and can preferably refer to between amino acid position 104 (Kabat numbering) of the antibody VL and amino acid position 122 (EU numbering) of the antibody heavy chain constant region, or between amino acid position 105 (Kabat numbering) of the antibody VL and amino acid position 122 (EU numbering) of the antibody heavy chain constant region.

A plurality of protease cleavage sequences can be established in the primary molecule and can be established at a plurality of locations selected from, for example, within an antibody constant region, within antibody VH, within antibody VL, near the boundary between antibody VH and an antibody constant region, and near the boundary between antibody VL and an antibody constant region. Those skilled in the art can change the form of a molecule comprising antibody VH, antibody VL, and antibody constant regions, for example, by replacing the antibody VH with the antibody VL, with reference to the present invention. Such a molecular form is included in the scope of the present invention.

### Preparation of secondary molecule (T cell-redirecting antibody)

The secondary molecule is a T cell-redirecting antibody having antibody variable regions binding to a T cell receptor complex, and antibody variable regions binding to the antigen binding molecule after cleavage of the linker; or a chimeric receptor having an extracellular domain binding to the antigen binding molecule after cleavage of the linker. As one example, the T cell-redirecting antibody is prepared using (2) an anti-CD3 antibody and (3) an antibody binding to the antigen binding molecule after cleavage of the linker, which are obtained by the procedures described above.

### Bispecific antibody

In a preferred aspect, the T cell-redirecting antibody of the present invention can be a bispecific antibody. In this context, the bispecific antibody is an antibody having two different specificities. An IgG bispecific antibody can be secreted by a hybrid hybridoma (quadroma) resulting from the fusion of two IgG antibody-producing hybridomas (Milstein C et al., Nature (1983) 305, 537-540). In the case of using an Fc region having reduced binding activity against an Fcγ receptor as an Fc region of the bispecific antibody, an Fc region originating from a bispecific antibody is also appropriately used.

The IgG bispecific antibody is secreted by introducing a total of four genes, genes of L chains and H chains constituting the two IgG antibodies of interest, to cells, and coexpressing these genes. However, the number of combinations of IgG H and L chains produced by such a method is theoretically as many as 10. It is difficult to purify IgG consisting of H and L chains in the combination of interest from the ten IgG types. In addition, the amount of a secreted bispecific antibody having the combination of interest is considerably reduced theoretically. This requires a large culture scale and further increases production cost.

Hence, a technique for promoting the association between H chains and between L and H chains in the combination of interest can be applied to the bispecific antibody of the present invention.

For example, a technique of suppressing the unintended association between H chains by introducing charge repulsion to the interface between the second constant regions (CH2) or the third constant regions (CH3) of the antibody H chains (WO2006/106905) can be applied to association for the multispecific antibody.

In the technique of suppressing the unintended association between H chains by introducing charge repulsion to the CH2 or CH3 interface, examples of amino acid residues contacting with each other at the interface between the H chain constant regions can include a residue at EU numbering position 356, a residue at EU numbering position 439, a residue at EU numbering position 357, a residue at EU numbering position 370, a residue at EU numbering position 399, and a residue at EU numbering position 409 in one CH3 region, and their partner residues in another CH3 region. More specifically, for example, an antibody comprising two H chain CH3 regions can be prepared as an antibody in which one to three pairs of amino acid residues selected from the following amino acid residue pairs (1) to (3) in the first H chain CH3 region have the same charge: (1) amino acid residues at EU numbering positions 356 and 439 contained in the H chain CH3 region; (2) amino acid residues at EU numbering positions 357 and 370 contained in the H chain CH3 region; and (3) amino acid residues at EU numbering positions 399 and 409 contained in the H chain CH3 region.

The antibody can be further prepared as an antibody in which one to three pairs of amino acid residues are selected from the amino acid residue pairs (1) to (3) in the second H chain CH3 region different from the first H chain CH3 region so as to correspond to the amino acid residue pairs (1) to (3) having the same charge in the first H chain CH3 region and to have opposite charge from their corresponding amino acid residues in the first H chain CH3 region.

Each amino acid residue described in the pairs (1) to (3) is located close to its partner in the associated H chains. Those skilled in the art can find positions corresponding to the amino acid residues described in each of the pairs (1) to (3) as to the desired H chain CH3 regions or H chain constant regions by homology modeling or the like using commercially available software and can appropriately alter amino acid residues at the positions.

In the antibody described above, each of the "amino acid residues having charge" is preferably selected from, for example, amino acid residues included in any of the following groups (a) and (b):
(a) glutamic acid (E) and aspartic acid (D); and
(b) lysine (K), arginine (R), and histidine (H).

In the antibody described above, the phrase "having the same charge" means that, for example, all of two or more amino acid residues are amino acid residues included in any one of the groups (a) and (b). The phrase "carrying opposite charge" means that, for example, at least one amino acid residue among two or more amino acid residues may be an amino acid residue included in any one of the groups (a) and (b), while the remaining amino acid residue(s) is amino acid residue(s) included in the other group.

In a preferred embodiment, the antibody may have the first H chain CH3 region and the second H chain CH3 region cross-linked through a disulfide bond.

The amino acid residue to be altered according to the present invention is not limited to the amino acid residues in the antibody variable region or the antibody constant region mentioned above. Those skilled in the art can find amino acid residues constituting the interface as to a polypeptide mutant or a heteromultimer by homology modeling or the like using commercially available software and can alter amino acid residues at the positions so as to regulate the association.

The association for the bispecific antibody of the present invention can also be carried out by an alternative technique known in the art. An amino acid side chain present in the Fc region of one antibody H chain is substituted by a larger side chain (knob), and its partner amino acid side chain present in the Fc region of the other H chain is substituted by a smaller side chain (hole). The knob can be placed into the hole to efficiently associate the polypeptides of the Fc domains differing in amino acid sequence (WO1996/027011; Ridgway JB et al., Protein Engineering (1996) 9, 617-621; Merchant AM et al. Nature Biotechnology (1998) 16, 677-681; and US20130336973).

In addition to this technique, a further alternative technique known in the art may be used for forming the bispecific antibody of the present invention. A portion of CH3 of one antibody H chain is converted to its counterpart IgA-derived sequence, and its complementary portion in CH3 of the other H chain is converted to its counterpart IgA-derived sequence. Use of the resulting strand-exchange engineered domain CH3 can cause efficient association between the polypeptides differing in sequence through complementary CH3 association (Protein Engineering Design & Selection, 23; 195-202, 2010). By use of this technique known in the art, the bispecific antibody of interest can also be efficiently formed.

Alternatively, the bispecific antibody may be formed by, for example, an antibody preparation technique using antibody CH1-CL association and VH-VL association as described in WO2011/028952, WO2014/018572, or Nat Biotechnol. 2014 Feb; 32 (2): 191-8, a technique of preparing a bispecific antibody using separately prepared monoclonal antibodies (Fab arm exchange) as described in WO2008/119353 and WO2011/131746, a technique of controlling the association between antibody heavy chain CH3 domains as described in WO2012/058768 and WO2013/063702, a technique of preparing a bispecific antibody constituted by two types of light chains and one type of heavy chain as described in WO2012/023053, or a technique of preparing a bispecific antibody using two bacterial cell lines each expressing an antibody half-molecule consisting of one H chain and one L chain as described in Christoph et al. (Nature Biotechnology Vol. 31, p. 753-758 (2013)).

Even if the bispecific antibody of interest cannot be formed efficiently, the bispecific antibody of the present invention may be obtained by the separation and purification of the bispecific antibody of interest from among produced antibodies. For example, the previously reported method involves introducing amino acid substitution to the variable domains of two types of H chains to impart thereto difference in isoelectric point (pI) so that two types of homodimers and the heterodimerized antibody of interest can be separately purified by ion-exchanged chromatography (WO2007114325). A method using protein A to purify a heterodimerized antibody consisting of a mouse IgG2a H chain capable of binding to protein A and a rat IgG2b H chain incapable of binding to protein A has previously been reported as a method for purifying the heterodimer (WO98050431 and WO95033844). Alternatively, amino acid residues at EU numbering positions 435 and 436 that constitute the protein A binding site of IgG may be substituted by amino acids, such as Tyr and His, which offer the different strength of protein A binding, and the resulting H chain, or H chains differing in strength of protein A binding, obtained according to a method described in Reference Example 5, are used to change the interaction of each H chain with protein A. As a result, only the heterodimerized antibody can be efficiently purified by use of a protein A column.

A common L chain capable of imparting binding ability to a plurality of different H chains may be obtained and used as a common L chain for the bispecific antibody. Bispecific IgG can be efficiently expressed by introducing such a common L chain and a plurality of different H chains to cells for IgG expression (Nature Biotechnology (1998) 16, 677-681). A method for selecting a common L chain that exhibits high binding ability in response to any different H chains can also be utilized for the selection of a common H chain (WO2004/065611).

An Fc region having improved C-terminal heterogeneity can be appropriately used as the Fc region of the present invention. More specifically, the present invention provides an Fc region lacking glycine 446 and lysine 447 defined according to the EU numbering in the amino acid sequences of two polypeptides constituting an Fc region originating from IgG1, IgG2, IgG3 or IgG4.

A plurality, for example, two or more, of these techniques may be used in combination. These techniques may be appropriately separately applied to two H chains to be associated. These techniques can further be used in combination with the Fc region having reduced binding activity against an FcD receptor mentioned above. The antigen binding molecule of the present invention may be prepared as a separately produced antigen binding molecule having the same amino acid sequence based on such a variant.

In the present invention, antibody variable regions that are "functionally equivalent" are not particularly limited as long as the antibody variable regions are antibody H chain and/or L chain variable regions that satisfy the conditions mentioned above. Such antibody variable regions may have the substitution, deletion, addition, and/or insertion of one or more amino acids (e.g., 1, 2, 3, 4, 5, or 10 amino acids) in the amino acid sequences of the variable regions described in Tables 1 to 3 mentioned above. A method well known to those skilled in the art for introducing the substitution, deletion, addition, and/or insertion of one or more amino acids into an amino acid sequence is a method of introducing mutations into proteins. Those skilled in the art can prepare variable regions that are functionally equivalent to the antibody variable regions having the functions mentioned above by appropriately introducing mutations into amino acid sequences using, for example, site-directed mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci U S A. 82, 488-492).

In the case of altering an amino acid residue, the amino acid is desirably mutated into a different amino acid having the conserved properties of the amino acid side chain. Examples of the amino-acid side chain properties can include hydrophobic amino acids (A, I, L, M, F, P, W, Y, and V), hydrophilic amino acids (R, D, N, C, E, Q, G, H, K, S, and T), amino acids having aliphatic side chains (G, A, V, L, I, and P), amino acids containing hydroxy group-containing side chains (S, T, and Y), amino acids having sulfur atomcontaining side chains (C and M), amino acids having carboxylic acid- and amide-containing side chains (D, N, E, and Q), amino acids having base-containing side chains (R, K, and H), and amino acids having aromatic side chains (H, F, Y, and W) (amino acids are represented by one-letter codes in parentheses). Amino acid substitution within each of these groups is called conservative substitution. It has already been known that a polypeptide having an amino acid sequence modified from a certain amino acid sequence by the deletion and/or addition of one or more amino acid residues and/or the substitution thereof by other amino acids maintains its biological activity (Mark, D. F. et al., Proc. Natl. Acad. Sci. USA (1984) 81: 5662-6; Zoller, M. J. and Smith, M., Nucleic Acids Res. (1982) 10: 6487-500; Wang, A. et al., Science (1984) 224: 1431-3; Dalbadie-McFarland, G. et al., Proc. Natl. Acad. Sci. USA (1982) 79: 6409-13). The variable region of the present invention having such amino acid alteration has an amino acid sequence identity of at least 70%, more preferably at least 75%, still more preferably at least 80%, further preferably at least 85%, still further preferably at least 90%, most preferably at least 95%, to the amino acid sequence of the CDR sequences, the FR sequences, or the whole variable region of the variable region before the alteration. In the present specification, the sequence identity is defined as the percentage of residues identical to the residues of the original amino acid sequence of the H chain or L chain variable region determined by aligning the sequences and appropriately introducing gaps so as to maximize the sequence identity, as necessary. The identity of amino acid sequences can be determined by a method mentioned later.

The "functionally equivalent antibody variable region" may be obtained, for example, from a nucleic acid that hybridizes under stringent conditions to a nucleic acid consisting of a nucleotide sequence encoding any of the amino acid sequences of the variable regions described in Tables 1 to 3 mentioned above. Examples of the stringent hybridization conditions for isolating a nucleic acid that hybridizes under stringent conditions to a nucleic acid consisting of a nucleotide sequence encoding the amino acid sequence of a variable region can include conditions of 6 M urea, 0.4% SDS, 0.5 × SSC, and 37°C, and hybridization conditions with stringency equivalent thereto. The isolation of a more highly homologous nucleic acid can be expected using more stringent conditions, for example, conditions of 6 M urea, 0.4% SDS, 0.1 × SSC, and 42°C. The washing conditions after the hybridization are, for example, washing with 0.5 × SSC (1 × SSC is 0.15 M NaCl and 0.015 M sodium citrate, pH 7.0) and 0.1% SDS at 60°C, more preferably washing with 0.2 × SSC and 0.1% SDS at 60°C, still more preferably washing with 0.2 × SSC and 0.1% SDS at 62°C, even more preferably washing using 0.2 × SSC and 0.1% SDS at 65°C, yet more preferably washing using 0.1 × SSC and 0.1% SDS at 65°C. The isolated nucleic acid can be sequenced by a method known in the art mentioned later. The homology of the isolated nucleic acid has at least 50% or higher, preferably 70% or higher, more preferably 90% or higher (e.g., 95%, 96%, 97%, 98%, 99%, or higher) sequence identity, in terms of the whole nucleotide sequence.

The nucleic acid that hybridizes under stringent conditions to a nucleic acid consisting of a nucleotide sequence encoding the amino acid sequence of a variable region may be isolated by using, instead of the method based on the hybridization technique described above, a gene amplification method, for example, polymerase chain reaction (PCR), using primers synthesized on the basis of information on the nucleotide sequence encoding the amino acid sequence of the variable region.

The identity of nucleotide sequences or amino acid sequences can be determined using algorithm BLAST by Karlin and Altschul (Proc. Natl. Acad. Sci. USA (1993) 90: 5873-7). Programs called BLASTN and BLASTX have been developed on the basis of this algorithm (Altschul et al., J. Mol. Biol. (1990) 215: 403-10). In the case of analyzing nucleotide sequences according to BLASTN based on BLAST, the parameters are set to, for example, score = 100 and wordlength = 12. In the case of analyzing amino acid sequences according to BLASTX based on BLAST, the parameters are set to, for example, score = 50 and wordlength = 3. In the case of using BLAST and Gapped BLAST programs, default parameters for each program are used. Specific approaches for these analysis methods are known in the art (see the website of NCBI (National Center for Biotechnology Information), BLAST (Basic Local Alignment Search Tool); http://www.ncbi.nlm.nih.gov).

The Fc region contained in the bispecific antibody of the present invention is not particularly limited as long as the Fc region has reduced binding activity against an Fcγ receptor. Preferred examples of the Fc region of the present invention can include a combination of an Fc region moiety of E22Hh and an Fc region moiety of E22Hk, a combination of an Fc region moiety of E2702GsKsc and an Fc region moiety of E2704sEpsc, and combination of an Fc region moiety of E2702sKsc and an Fc region moiety of E2704sEpsc described in WO2016/047722A1.

In the present invention, the pharmaceutical composition of the present invention may be encapsulated, if necessary, in microcapsules (e.g., hydroxymethylcellulose, gelatin, and poly(methyl methacrylate) microcapsules), and used in a colloidal drug delivery system (liposomes, albumin microspheres, microemulsion, nanoparticles, and nanocapsules, etc.) (see e.g., Remington's Pharmaceutical Science 16th edition, Oslo Ed. (1980)). Methods for preparing agents into sustained-release agents are also known in the art, and such methods may be applied to the bispecific antigen binding molecule of the present invention (J. Biomed. Mater. Res. (1981) 15, 267-277, Chemtech. (1982) 12, 98-105; U.S. Patent No. 3773719; EP Patent Publication Nos. EP58481 and EP133988; and Biopolymers (1983) 22, 547-556).

The pharmaceutical composition, the cytotoxicity-inducing agent or the cell growth-inhibiting agent of the present invention can be administered to patients by oral or parenteral administration. Parenteral administration is preferred. Specific examples of such an administration method include administration by injection, transnasal administration, transpulmonary administration, and transdermal administration. Examples of the administration by injection include intravenous injection, intramuscular injection, intraperitoneal injection, and subcutaneous injection. The pharmaceutical composition, the cytotoxicity-inducing agent or the cell growth-inhibiting agent of the present invention can be administered systemically or locally, for example, through administration by injection. The administration method can be appropriately selected according to the age and symptoms of a patient. The dose can be selected in the range of 0.0001 mg to 1000 mg per kg body weight for a single administration. Alternatively, for example, the dose may be selected in the range of 0.001 mg/body to 100000 mg/body per patient. However, the pharmaceutical composition, the cytotoxicity-inducing agent or the cell growth-inhibiting agent of the present invention is not limited by these doses.

The pharmaceutical composition, the cytotoxicity-inducing agent or the cell growth-inhibiting agent of the present invention can be formulated according to a routine method (e.g., Remington's Pharmaceutical Science, latest edition, Mark Publishing Company, Easton, U.S.A), and may also contain a pharmaceutically acceptable carrier or additive. Examples thereof include surfactants, excipients, coloring agents, flavoring agents, preservatives, stabilizers, buffers, suspending agents, tonicity agents, binders, disintegrants, lubricants, fluidity promoting agents, and corrigents. The pharmaceutically acceptable carrier or additive is not limited thereto, and other carriers commonly used can be appropriately used. Specific examples of the carrier can include light anhydrous silicic acid, lactose, crystalline cellulose, mannitol, starch, carmellose calcium, carmellose sodium, hydroxypropylcellulose, hydroxypropylmethylcellulose, polyvinylacetal diethylaminoacetate, polyvinylpyrrolidone, gelatin, medium chain fatty acid triglyceride, polyoxyethylene hardened castor oil 60, saccharose, carboxymethylcellulose, corn starch, and inorganic salts.

### Chimeric receptor

A chimeric receptor having an extracellular domain binding to the antigen binding molecule after cleavage of the linker may be used as the secondary molecule. As one example, the chimeric receptor is prepared using an antibody binding to the antigen binding molecule after cleavage of the linker, which is obtained by the procedures described above.

The term "chimeric receptor" refers to a recombinant polypeptide that comprises at least an extracellular binding domain, a transmembrane domain and an intracellular signaling domain and induces specificity and intracellular signal production for target cells, for example, cancer cells, when expressed in immune effector cells. The term "chimeric antigen receptor" or "CAR" means a chimeric receptor whose extracellular binding domain binds to an antigen.

The term "extracellular binding domain" means any proteinous molecule or a portion thereof capable of specifically binding to a predetermined molecule such as an antigen, and includes, for example, a single chain antibody (scFv) in which a light chain variable region (VL) and a heavy chain variable region (VH) of a monoclonal antibody specific for a tumor antigen or the like are linked in series. The extracellular binding domain may be used interchangeably with an extracellular recognition domain.

The term "transmembrane domain" is positioned between the extracellular binding domain and the intracellular signaling domain and comprises a polypeptide having a function of penetrating a cell membrane.

The term "intracellular signal transduction domain" means any oligopeptide domain or polypeptide domain known to work to transduce signals that cause activation or inhibition of an intracellular biological process, for example, activation of immunocytes such as T cells or NK cells, and comprises at least one "stimulatory molecule signal transduction domain" derived from a stimulatory molecule of T cells mentioned later, and may further comprise at least one "costimulatory molecule signal transduction domain" derived from a costimulatory molecule of T cells mentioned later.

As used in the present disclosure, the "domain" means, for example, one region in a polypeptide that is folded into a particular structure independently of other regions and/or has a particular function. The domain can be, for example, a cytoplasmic moiety of a molecule or a portion thereof. As used in the present disclosure, the "cytoplasmic domain" of a molecule means a full-length cytoplasmic domain or a portion thereof that transduces intracellular signals when activated.

In one embodiment, the chimeric receptor is a molecule comprising domains defined below.

In one embodiment, the chimeric receptor comprises a chimeric fusion protein comprising an extracellular binding domain, an extracellular hinge domain, a transmembrane domain, and an intracellular signal transduction domain comprising a stimulatory molecule signal transduction domain derived from a stimulatory molecule.

In one embodiment, the chimeric receptor comprises a chimeric fusion protein comprising an extracellular binding domain, an extracellular hinge domain, a transmembrane domain, and an intracellular signal transduction domain comprising a costimulatory molecule signal transduction domain derived from a costimulatory molecule and a functional signal transduction domain derived from a stimulatory molecule.

In one embodiment, the chimeric receptor comprises a chimeric fusion protein comprising an extracellular binding domain, a transmembrane domain, and an intracellular signal transduction domain comprising at least two functional signal transduction domains derived from one or more costimulatory molecules and a functional signal transduction domain derived from a stimulatory molecule.

In one embodiment, the chimeric receptor comprises a chimeric fusion protein comprising an extracellular binding domain, a transmembrane domain, and an intracellular signal transduction domain comprising at least two costimulatory molecule signal transduction domains derived from one or more costimulatory molecules, a stimulatory molecule signal transduction domain derived from a stimulatory molecule, and an additional functional domain and/or motif.

In one embodiment, the chimeric receptor described in the present specification can be used as a chimeric antigen receptor (CAR).

One embodiment discloses a chimeric receptor comprising i) an extracellular domain capable of binding to a predetermined antigen, ii) a transmembrane domain, and iii) an intracellular segment comprising one or more intracellular signal transduction domains selected from a cytoplasmic costimulatory domain and a cytoplasmic domain of an interleukin receptor chain and a CD3 zeta intracellular signal transduction domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

In one embodiment, an extracellular hinge domain and a transmembrane domain may be contained between the extracellular binding domain and the intracellular signaling domain. The term "extracellular hinge domain" means a domain that links the extracellular binding domain to the transmembrane domain. The extracellular hinge domain is not particularly limited as long as the extracellular hinge domain can link the extracellular binding domain to the transmembrane domain. The extracellular hinge domain may be derived from a natural protein or may be artificially designed. The extracellular hinge domain can be constituted by, for example, approximately 10 to 300 amino acids, preferably approximately 20 to 100 amino acids. The extracellular hinge domain preferably neither hinders the ability of the extracellular binding domain to bind to an Fc region of the Fc region altered antibody of the present disclosure nor hinders signal transduction mediated by the intracellular signaling domain. The term "transmembrane domain" is not particularly limited as long as the transmembrane domain is positioned between the extracellular binding domain and the intracellular signaling domain and is a polypeptide having a function of penetrating a cell membrane. The transmembrane domain may be derived from a natural protein or may be artificially designed. The transmembrane domain derived from a natural protein can be obtained from any membrane-associated protein or transmembrane protein.

In one embodiment, the chimeric receptor comprises an additional leader sequence at the amino terminus (N terminus) of the chimeric receptor fusion protein.

In one embodiment, the chimeric receptor further comprises a leader sequence at the N terminus of the extracellular binding domain. In this context, the leader sequence may be cleaved from the antigen recognition domain (e.g., scFv) during cell processing and the localization of the chimeric receptor to a cell membrane.

The term "scFv" means a fusion protein comprising at least one antibody fragment comprising a light chain variable region and at least one antibody fragment comprising a heavy chain variable region. In one embodiment, the scFv means a fusion protein that can be expressed as a single polypeptide chain in which the light chain variable region and the heavy chain variable region are continuously linked via, for example, a synthetic linker, for example, a short flexible polypeptide linker, and the scFv further retains the specificity of its original antibody. In the present disclosure, the scFv may have the light chain variable region (VL) and the heavy chain variable region (VH) in any order, unless otherwise specified. For example, as for the ends of the N terminus and C terminus of the polypeptide, the scFv may comprise VL-linker-VH or may comprise VH-linker-VL. Various methods for preparing scFv are known and include methods described in U.S. Patent No. 4694778, Science, vol. 242, pp. 423-442 (1988), Nature, vol. 334, p. 54454 (1989), and Science, vol. 242, pp. 1038-1041 (1988).

The term "flexible polypeptide linker" or "linker" used regarding scFv refers to a peptide linker consisting of amino acids, such as glycine and/or serine residues, used singly or in combination for linking a heavy chain variable region and a light chain variable region together. In one embodiment, the flexible polypeptide linker is a Gly/Ser linker and comprises an amino acid sequence (Gly-Gly-Gly-Ser)ₙ wherein n is an integer of 1 or larger. For example, n = 1, n = 2, n = 3, n = 4, n = 5, n = 6, n = 7, n = 8, n = 9 and n = 10. In one embodiment, examples of the flexible polypeptide linker include, but are not limited to (Gly₄Ser)₅, (Gly₄Ser)₄ and (Gly₄Ser)₃. In another embodiment, the linker contains a plurality of repeats of (Gly₂Ser), (GlySer) or (Gly₃Ser). Linkers described in WO2012/138475, which are incorporated herein by reference, are also included in the scope of the present disclosure.

In one embodiment, examples of the extracellular hinge domain include extracellular hinge domains of CD8 alpha, CD8 beta, CD28, CD4, NKp30, NKp44, and NKp46. Alternatively, a hinge region of an immunoglobulin (e.g., IgG4) may be used.

In one embodiment, examples of the protein from which the transmembrane domain is derived can include T cell receptor alpha and beta chains, CD3 zeta, CD28, CD3 epsilon, CD45, CD4, CD5, CD8 alpha, CD8 beta, CD9, CD16, CD22, CD33, CD37, CD64, CD80, CD86, CD134, CD137, ICOS, CD154, GITR, NKp30, NKp44, and NKp46. In one embodiment, the protein from which the transmembrane domain is derived is CD28 or CD3 epsilon.

The term "signal transduction domain" refers to a functional moiety of a protein that acts by conveying intracellular information in order to regulate cell activity via a defined signal transduction pathway through the production of a second messenger or through the functionalization of an effector in response to such a messenger.

As used in the present disclosure, the "intracellular signal transduction domain" refers to an intracellular moiety of a molecule. The intracellular signaling domain can generate signals that promote an immune effector function of cells containing a chimeric receptor such as CAR, for example, CART cells. Examples of the immune effector function of CART cells include cytolytic activity and helper activity, for example, cytokine secretion. In an embodiment, the intracellular signaling domain is a moiety of a protein that transduces effector function signals and allows cells to carry out a specified function. Although the whole intracellular signaling domain may be adopted, it is not necessarily required to use the whole chain in many cases. A truncated moiety that transduces effector function signals can be used instead of an intact chain as long as a truncated moiety of the intracellular signaling domain is used. Thus, the term "intracellular signaling domain" is meant to include every truncated moiety of the intracellular signaling domain sufficient for transducing effector function signals.

In an embodiment, the intracellular signal transduction domain may comprise a primary intracellular signal transduction domain. Typical examples of the primary intracellular signaling domain include those derived from molecules involved in primary stimulation or antigen dependent stimulation. In an embodiment, the intracellular signaling domain may comprise a costimulatory intracellular domain. Typical examples of the costimulatory intracellular signaling domain include those derived from molecules involved in costimulatory signals or antigen independent stimulation. In the case of, for example, CART, the primary intracellular signaling domain may comprise an intracytoplasmic sequence of a T cell receptor, or the costimulatory intracellular signaling domain may comprise an intracytoplasmic sequence of a co-receptor or a costimulatory molecule.

As used in the present disclosure, the term "CD3 zeta" means cluster of differentiation 3 (CD3) T cell coreceptor of every mammalian species, preferably a human. In mammals, CD3 comprises a CD3 zeta chain, a CD3 delta chain and two CD3 epsilon chains. The CD3 zeta chain (e.g., NCBI RefSeq: NP_932170.1) comprises an intracellular signaling domain that can be used for engineering the chimeric receptor. In the particular chimeric receptor of the present disclosure, the primary signaling sequence of CD3 zeta is the full-length cytoplasmic region sequence of GenBank NM000734.3 (nucleotides 299 to 634) or a portion thereof, or equivalent residues from a non-human species, for example, a mouse, a rodent, a monkey, an anthropoid and the like.

In one embodiment, the intracellular signal transduction domain may comprise a cytoplasmic domain of an interleukin receptor chain. In one embodiment, the intracellular signal transduction domain used may be CD28, 4-1BB, ICOS, or CD3 zeta-CD28-4-1BB or CD3 zeta-CD28-OX40 in which a plurality of signal transduction domains are connected. In one embodiment, the protein from which the transmembrane domain is derived is CD28 or CD3 epsilon, and the intracellular signal transduction domain may be CD28, 4-1BB, ICOS, or CD3 zeta-CD28-4-1BB or CD3 zeta-CD28-OX40 in which a plurality of signal transduction domains are connected.

One embodiment discloses a chimeric receptor comprising i) an extracellular domain capable of binding to a predetermined antigen, ii) a transmembrane domain, and iii) an intracellular segment comprising one or more intracellular signal transduction domains selected from a cytoplasmic costimulatory domain and/or a cytoplasmic domain of an interleukin receptor chain and a CD3 zeta intracellular signal transduction domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

As used in the present disclosure, the term "CD3 zeta" means cluster of differentiation 3 (CD3) T cell coreceptor of every mammalian species, preferably a human. In mammals, CD3 comprises a CD3 zeta chain, a CD3 delta chain and two CD3 epsilon chains. The CD3 zeta chain (e.g., NCBI RefSeq: NP_932170.1) comprises an intracellular signal transduction domain that can be used for engineering the chimeric receptor of the present disclosure.

The term "stimulation" refers to primary response that is induced by the binding of a stimulatory molecule (e.g., a TCR/CD3 complex or CAR) to its ligand of the same origin (or a tumor antigen for CAR), and thereby mediates a signal transduction event, for example, but not limited to, signal transduction mediated by a TCR/CD3 complex, or signal transduction mediated by an appropriate NK receptor or signaling domain of CAR The stimulation may mediate changed expression of a certain molecule.

The term "stimulatory molecule" refers to a molecule that is expressed by immunocytes, for example, T cells, NK cells or B cells, which provide an intracytoplasmic signaling sequence regulating the activation of the immunocytes in the form of stimulation, in at least some aspects of an immunocyte signaling pathway. In one aspect, the signal is a primary signal that is triggered, for example, by the binding between a TCR/CD3 complex and an MHC molecule presenting a peptide, and thereby mediates T cell response including, but not limited to, growth, activation, differentiation, and the like. The primary intracytoplasmic signaling sequence (also referred to as a "primary signaling domain") which acts in the form of stimulation may contain a signaling motif, which is known as an immunoreceptor tyrosine-based activation motif or ITAM In the present disclosure, examples of ITAM containing an intracytoplasmic signaling sequence having particular application include, but are not limited to, those derived from CD3 zeta, common FcR gamma (FCER1G), Fc gamma RIIa, FcR beta (Fc epsilon R1b), CD3 gamma, CD3 delta, CD3 epsilon, CD22, CD79a, CD79b, CD278 ("ICOS"), Fc epsilon RI, CD66d, CD32, DAP10, DAP12, CLEC2, CLEC7A (Dectin 1), CLEC9A, EZRIN, RADIXIN and MOESIN. In any one or two particular chimeric receptors of the present disclosure, the intracellular signaling domain comprises an intracellular signaling sequence, for example, a primary signaling sequence of CD3 zeta.

The term "costimulatory molecule" refers to a cognate binding partner on a T cell that specifically binds to a costimulatory ligand and thereby mediates the costimulatory response, for example, but not limited to, growth, of the T cell (secondary signal). The costimulatory molecule is a cell surface molecule other than an antigen receptor or its ligand that is responsible for an efficient immune response. The term "costimulatory intracellular signaling domain" refers to an intracellular moiety of the costimulatory molecule. The intracellular signaling domain may comprise the whole intracellular moiety, or the whole natural intracellular signaling domain of the molecule from which the intracellular moiety is obtained, or a functional fragment or derivative thereof. Examples of the costimulatory molecule include, but are not limited to, ligands that specifically bind to MHC class I molecule, TNF receptor protein, immunoglobulin-like protein, cytokine receptor, integrin, signaling lymphocytic activation molecule (SLAM protein), activating NK cell receptor, BTLA, Toll ligand receptor, OX40 (CD134), CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 `(KLRF1), NKp44, NKp30, NKp46, CD19, CD4, CD5, CD8 alpha, CD8 beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD154 and CD83.

In one embodiment, the costimulatory molecule is selected from, for example, 4-1BB (i.e., CD137), CD27, CD28 and/or OX40, and enhances T cell receptor stimulation.

The term "4-1BB" refers to a member of the TNFR superfamily having an amino acid sequence provided as GenBank accession No. AAA62478.2, or equivalent residues from a non-human species, for example, a mouse, a rodent, a monkey, an anthropoid and the like. The term "4-1BB costimulatory domain" is defined as amino acid residues 214 to 255 of GenBank accession No. AAA62478.2, or equivalent residues from a non-human species, for example, a mouse, a rodent, a monkey, an anthropoid and the like. In one aspect, the "4-1BB costimulatory domain" is the full-length sequence from nucleotides 886 to 1026 of GenBank NM001561.5 or a portion thereof, or equivalent residues from a non-human species, for example, a mouse, a rodent, a monkey, an anthropoid and the like.

The term "antigen" or "Ag" refers to a molecule that initiates immune reaction. This immune reaction may include any of antibody production or activation of specific immunocompetent cells, or both. Every high molecule including substantially all proteins or peptides may be useful as the antigen. The antigen may be derived from recombinant or genomic DNA. Every DNA comprising a nucleotide sequence or a partial nucleotide sequence encoding a protein that initiates immune reaction eventually encodes an "antigen" similar to the term antigen used in the present disclosure. The antigen is not always encoded only by the full-length nucleotide sequence of a gene. It is readily understood that: use of partial nucleotide sequences of more than one gene is included in the present disclosure; and these nucleotide sequences are arranged in various combinations so as to encode polypeptides that initiate more desirable immune reaction, though the present disclosure is not limited thereby. The antigen may not always be encoded by a "gene". The antigen may be produced or synthesized, or may be derived from a biological sample, or may be a high molecule other than a polypeptide. Examples of such a biological sample can include, but are not limited to, tissue samples, tumor samples, cells and fluids containing other biological components. When the chimeric receptor of the present disclosure has an amino acid sequence derived from an antibody as a portion of the extracellular binding domain, a molecule to which the extracellular binding domain binds may be referred to as an antigen.

The IgG antibody having enhanced antibody-dependent cellular cytotoxicity (ADCC) used in the present invention can be prepared with reference to, for example, WO 2013/002362 A1, WO 2014/104165 A1, WO 2005/014651 A1, and WO 2006/046751 A1.

In one aspect, the IgG antibody is an antibody comprising an Fc region, wherein the Fc region is constituted by a heterodimer comprising a first polypeptide and a second polypeptide, and the antibody compared with an antibody wherein the Fc region is constituted by a homodimer comprising only the first polypeptide and an antibody wherein the Fc region is constituted by a homodimer comprising only the second polypeptide has enhanced binding activity against an Fcγ receptor. In the IgG antibody, at least one or more amino acid mutations may be introduced in the Fc region.

In another aspect, in the IgG antibody, at least one or more amino acid mutations may be introduced at amino acid sites selected from the group consisting of Ala at EU numbering position 231, Pro at EU numbering position 232, Glu at EU numbering position 233, Leu at EU numbering position 234, Leu at EU numbering position 235, Gly at EU numbering position 236, Gly at EU numbering position 237, Pro at EU numbering position 238, Ser at EU numbering position 239, Val at EU numbering position 240, Asp at EU numbering position 265, Val at EU numbering position 266, Ser at EU numbering position 267, His at EU numbering position 268, Glu at EU numbering position 269, Asp at EU numbering position 270, Pro at EU numbering position 271, Gln at EU numbering position 295, Tyr at EU numbering position 296, Ser at EU numbering position 298, Tyr at EU numbering position 300, Ser at EU numbering position 324, Asn at EU numbering position 325, Lys at EU numbering position 326, Ala at EU numbering position 327, Leu at EU numbering position 328, Pro at EU numbering position 329, Ala at EU numbering position 330, Pro at EU numbering position 331, Ile at EU numbering position 332, Glu at EU numbering position 333, Lys at EU numbering position 334, Thr at EU numbering position 335, Ile at EU numbering position 336, and Ser at EU numbering position 337 in the CH2 region of the Fc region.

In this context, the Fcγ receptor is not particularly limited and is, for example, a receptor selected from the group consisting of FcyRIa, FcyRIIa R, FcyRIIa H, FcyRIIb, and FcyRIIIa. Preferably, the Fcγ receptor is FcyRIIIa.

In an alternative aspect, in the IgG antibody, at least one or more amino acid mutations selected from the group consisting of the substitution of amino acid L at EU numbering position 234 by Y, the substitution of amino acid L at EU numbering position 235 by Y or Q, the substitution of amino acid G at EU numbering position 236 by W, the substitution of amino acid S at EU numbering position 239 by M, the substitution of amino acid H at EU numbering position 268 by D, the substitution of amino acid D at EU numbering position 270 by E, the substitution of amino acid S at EU numbering position 298 by A, the substitution of amino acid K at EU numbering position 326 by D, the substitution of amino acid A at EU numbering position 327 by D, the substitution of amino acid L at EU numbering position 328 by W, the substitution of amino acid A at EU numbering position 330 by M or K and the substitution of amino acid K at EU numbering position 334 by E or L are introduced in the amino acid sequence of the first polypeptide and/or the second polypeptide constituting the Fc region.

In an alternative aspect, in the IgG antibody, a mutation is introduced in at least one amino acid selected from Leu at EU numbering position 234, Leu at EU numbering position 235, Gly at EU numbering position 236, Ser at EU numbering position 239, His at EU numbering position 268, Asp at EU numbering position 270, Ser at EU numbering position 298, Lys at EU numbering position 326, Ala at EU numbering position 327, Leu at EU numbering position 328, Ala at EU numbering position 330 and Lys at EU numbering position 334 in the amino acid sequence of the first polypeptide and/or the second polypeptide constituting the Fc region.

In an alternative aspect, in the IgG antibody, a mutation is introduced in at least one amino acid selected from Leu at EU numbering position 234, Leu at EU numbering position 235, Gly at EU numbering position 236, Ser at EU numbering position 239, His at EU numbering position 268, Asp at EU numbering position 270, Ser at EU numbering position 298, Ala at EU numbering position 327, Leu at EU numbering position 328 and Lys at EU numbering position 334 in the amino acid sequence of any one of the first polypeptide and the second polypeptide constituting the Fc region, and a mutation is introduced in at least one amino acid selected from Asp at EU numbering position 270, Lys at EU numbering position 326, Ala at EU numbering position 330 and Lys at EU numbering position 334 in the amino acid sequence of the other polypeptide.

In an alternative aspect, in the IgG antibody, at least one amino acid mutation selected from the group consisting of the substitution of amino acid L at EU numbering position 234 by Y, the substitution of amino acid L at EU numbering position 235 by Y or Q, the substitution of amino acid G at EU numbering position 236 by W, the substitution of amino acid S at EU numbering position 239 by M, the substitution of amino acid H at EU numbering position 268 by D, the substitution of amino acid D at EU numbering position 270 by E, the substitution of amino acid S at EU numbering position 298 by A, the substitution of amino acid A at EU numbering position 327 by D, the substitution of amino acid L at EU numbering position 328 by W and the substitution of amino acid K at EU numbering position 334 by L is introduced in the amino acid sequence of any one of the first polypeptide and the second polypeptide constituting the Fc region, and at least one amino acid mutation selected from the group consisting of the substitution of amino acid D at EU numbering position 270 by E, the substitution of amino acid K at EU numbering position 326 by D, the substitution of amino acid A at EU numbering position 330 by M or K and the substitution of amino acid K at EU numbering position 334 by E is introduced in the amino acid sequence of the other polypeptide.

In an alternative aspect, in the IgG antibody, any of the following mutation groups (i) to (vi) is introduced in the amino acid sequence of any one of the first polypeptide and the second polypeptide constituting the Fc region, and any of the following mutation groups (vii) to (ix) is introduced in the amino acid sequence of the other polypeptide:
(i) the substitution of amino acid L at EU numbering position 234 by Y, the substitution of amino acid L at EU numbering position 235 by Y, the substitution of amino acid G at EU numbering position 236 by W, the substitution of amino acid H at EU numbering position 268 by D and the substitution of amino acid S at EU numbering position 298 by A;
(ii) the substitution of amino acid L at EU numbering position 234 by Y, the substitution of amino acid L at EU numbering position 235 by Y, the substitution of amino acid G at EU numbering position 236 by W, the substitution of amino acid H at EU numbering position 268 by D, the substitution of amino acid D at EU numbering position 270 by E and the substitution of amino acid S at EU numbering position 298 by A;
(iii) the substitution of amino acid L at EU numbering position 234 by Y, the substitution of amino acid L at EU numbering position 235 by Q, the substitution of amino acid G at EU numbering position 236 by W, the substitution of amino acid S at EU numbering position 239 by M, the substitution of amino acid H at EU numbering position 268 by D, the substitution of amino acid D at EU numbering position 270 by E and the substitution of amino acid S at EU numbering position 298 by A;
(iv) the substitution of amino acid L at EU numbering position 234 by Y, the substitution of amino acid L at EU numbering position 235 by Y, the substitution of amino acid G at EU numbering position 236 by W, the substitution of amino acid H at EU numbering position 268 by D, the substitution of amino acid S at EU numbering position 298 by A and the substitution of amino acid A at EU numbering position 327 by D;
(v) the substitution of amino acid L at EU numbering position 234 by Y, the substitution of amino acid L at EU numbering position 235 by Y, the substitution of amino acid G at EU numbering position 236 by W, the substitution of amino acid S at EU numbering position 239 by M, the substitution of amino acid H at EU numbering position 268 by D, the substitution of amino acid S at EU numbering position 298 by A and the substitution of amino acid A at EU numbering position 327 by D;
(vi) the substitution of amino acid L at EU numbering position 234 by Y, the substitution of amino acid L at EU numbering position 235 by Y, the substitution of amino acid G at EU numbering position 236 by W, the substitution of amino acid S at EU numbering position 239 by M, the substitution of amino acid H at EU numbering position 268 by D, the substitution of amino acid S at EU numbering position 298 by A, the substitution of amino acid A at EU numbering position 327 by D, the substitution of amino acid L at EU numbering position 328 by W and the substitution of amino acid K at EU numbering position 334 by L;
(vii) the substitution of amino acid K at EU numbering position 326 by D, the substitution of amino acid A at EU numbering position 330 by M and the substitution of amino acid K at EU numbering position 334 by E;
(viii) the substitution of amino acid D at EU numbering position 270 by E, the substitution of amino acid K at EU numbering position 326 by D, the substitution of amino acid A at EU numbering position 330 by M and the substitution of amino acid K at EU numbering position 334 by E; and
(ix) the substitution of amino acid D at EU numbering position 270 by E, the substitution of amino acid K at EU numbering position 326 by D, the substitution of amino acid A at EU numbering position 330 by K and the substitution of amino acid K at EU numbering position 334 by E.

In one aspect of the present invention, the IgG antibody having enhanced ADCC activity is a polypeptide constituted by a heterodimer comprising a first polypeptide and a second polypeptide, wherein any one of the first polypeptide and the second polypeptide comprises an Fc region having the following mutation group (i) or (ii) introduced therein and has an altered Fc region function as compared with a polypeptide comprising an unmutated Fc region:
(i) the mutation of an amino acid at EU numbering position 234 into L, S, F, E, V, D, Q, I, M, T, A, G or H, the mutation of an amino acid at EU numbering position 235 into Y or Q, the mutation of an amino acid at EU numbering position 236 into W, the mutation of an amino acid at EU numbering position 239 into M or I, the mutation of an amino acid at EU numbering position 268 into D, and the mutation of an amino acid at EU numbering position 298 into A; and
(ii) the mutation of an amino acid at EU numbering position 270 into E, the mutation of an amino acid at EU numbering position 326 into D, the mutation of an amino acid at EU numbering position 330 into A, K, M, F, I, Y or H, and the mutation of an amino acid at EU numbering position 334 into E.

In another aspect of the present invention, the IgG antibody having enhanced ADCC activity is a polypeptide wherein any one of the first polypeptide and the second polypeptide comprises an Fc region having the following mutation group (i) or (ii) introduced therein and, and the other polypeptide has the following mutation group (iii) introduced therein:
(i) the mutation of an amino acid at EU numbering position 234 into L, S, F, E, V, D, Q, I, M, T, A, G or H, the mutation of an amino acid at EU numbering position 235 into Y or Q, the mutation of an amino acid at EU numbering position 236 into W, the mutation of an amino acid at EU numbering position 239 into M or I, the mutation of an amino acid at EU numbering position 268 into D, and the mutation of an amino acid at EU numbering position 298 into A, and the mutation of an amino acid at EU numbering position 327 into D;
(ii) the mutation of an amino acid at EU numbering position 234 into L, S, F, E, V, D, Q, I, M, T, A, G or H, the mutation of an amino acid at EU numbering position 235 into Y or Q, the mutation of an amino acid at EU numbering position 236 into W, the mutation of an amino acid at EU numbering position 239 into M or I, the mutation of an amino acid at EU numbering position 268 into D, the mutation of an amino acid at EU numbering position 270 into E, and the mutation of an amino acid at EU numbering position 298 into A; and
(iii) the mutation of an amino acid at EU numbering position 270 into E, the mutation of an amino acid at EU numbering position 326 into D, the mutation of an amino acid at EU numbering position 330 into A, K, M, F, I, Y or H, and the mutation of an amino acid at EU numbering position 334 into E.

In an alternative aspect of the present invention, the IgG antibody having enhanced ADCC activity is a polypeptide having (i) E, D, T or L as an amino acid at EU numbering position 234.

In an alternative aspect of the present invention, the IgG antibody having enhanced ADCC activity is [4] the polypeptide according to [2] having (ii) L, F, E or D as an amino acid at EU numbering position 234.

In an alternative aspect of the present invention, the IgG antibody having enhanced ADCC activity is [5] the polypeptide according to [2] having (i) V, I, T, M or L as an amino acid at EU numbering position 234.

In an alternative aspect of the present invention, the IgG antibody having enhanced ADCC activity is [6] the polypeptide according to [2] having (i) V, E, D, T, I, L or F as an amino acid at EU numbering position 234, and M or I as an amino acid at EU numbering position 239, and (iii) A or K as an amino acid at EU numbering position 330.

In an alternative aspect of the present invention, the IgG antibody having enhanced ADCC activity is [7] the polypeptide according to [2] having (ii) F, E, D, S or L as an amino acid at EU numbering position 234, and M or I as an amino acid at EU numbering position 239, and (iii) A, F or K as an amino acid at EU numbering position 330.

In an alternative aspect of the present invention, the IgG antibody having enhanced ADCC activity is a polypeptide having enhanced binding activity against an Fcγ receptor by the functional alteration of the Fc region. In this context, the Fcγ receptor is selected from the group consisting of FcyRIa, FcyRIIaR, FcyRIIaH, FcyRIIb, FcyRIIIaF and FcyRIIIaV.

In one embodiment, examples of the target antigen according to the present invention preferably include receptors, tumor antigens, MHC antigens, and differentiation antigens.

Examples of the receptor can include receptors belonging to receptor families such as hematopoietic factor receptor family, cytokine receptor family, tyrosine kinase receptor family, serine/threonine kinase receptor family, TNF receptor family, G protein-coupled receptor family, GPI-anchored receptor family, tyrosine phosphatase receptor family, adhesion factor family, and hormone receptor family. The receptors belonging to these receptor families, and their features are described in many literatures, for example, reviews of Cooke BA., King RJB., van der Molen HJ. ed. New Comprehensive Biochemistry Vol. 18B "Hormones and their Actions Part II" pp. 1-46 (1988) Elsevier Science Publishers BV., or Masayuki Miyasaka ed., Cell Technology suppl. Handbook series "Adhesion Factor Handbook" (1994) (Gakken Medical Shujunsha Co., Ltd., Tokyo, Japan) as well as Patthy (Cell (1990) 61 (1), 13-14), Ullrich et al. (Cell (1990) 61 (2), 203-212), Massague (e with acute accent code) (Cell (1992) 69 (6), 1067-1070), Miyajima et al. (Annu. Rev. Immunol. (1992) 10, 295-331), Taga et al. (FASEB J. (1992) 6, 3387-3396), Fantl et al. (Annu. Rev. Biochem. (1993), 62, 453-481), Smith et al. (Cell (1994) 76 (6) 959-962), and Flower DR. (Biochim. Biophys. Acta (1999) 1422 (3) 207-234).

Specific examples of the receptors belonging to the receptor families preferably include human or mouse erythropoietin (EPO) receptor (Blood (1990) 76 (1), 31-35; and Cell (1989) 57 (2), 277-285), human or mouse granulocyte colony-stimulating factor (G-CSF) receptor (Proc. Natl. Acad. Sci. USA. (1990) 87 (22), 8702-8706, mG-CSFR, Cell (1990) 61 (2), 341-350), human or mouse thrombopoietin (TPO) receptor (Proc Natl Acad Sci U S A. (1992) 89 (12), 5640-5644; and EMBO J. (1993) 12 (7), 2645-53), human or mouse insulin receptor (Nature (1985) 313 (6005), 756-761), human or mouse Flt-3 ligand receptor (Proc. Natl. Acad. Sci. USA. (1994) 91 (2), 459-463), human or mouse platelet-derived growth factor (PDGF) receptor (Proc. Natl. Acad. Sci. USA. (1988) 85 (10) 3435-3439), human or mouse interferon (IFN)-alpha, beta receptor (Cell (1990) 60 (2), 225-234; and Cell (1994) 77 (3), 391-400), human or mouse leptin receptor, human or mouse growth hormone (GH) receptor, human or mouse interleukin (IL)-10 receptor, human or mouse insulin-like growth factor (IGF)-I receptor, human or mouse leukemia inhibitory factor (LIF) receptor, and human or mouse ciliary neurotrophic factor (CNTF) receptor.

The term "tumor antigen" refers to an antigen expressed on a cancer cell, and means a biological molecule having antigenicity, the expression of which becomes recognized in association with the malignant alteration of cells. In one aspect of the present disclosure, the target antigen is a tumor antigen. The tumor antigen includes a tumor-specific antigen (antigen that is present only in tumor cells and is not found in other normal cells), and a tumor-associated antigen (antigen that is also present in other organs and tissues or heterogeneous and allogeneic normal cells, or antigen that is expressed during development and/or differentiation). Also, an aberrant sugar chain that appears on cell surface or a protein molecule during cell canceration is the tumor antigen and is also called cancer sugar chain antigen.

Examples of the tumor antigen preferably include GPC3 which belongs as the receptor described above to the GPI-anchored receptor family and is expressed in some cancers including liver cancer (Int J Cancer. (2003) 103 (4), 455-65), EpCAM which is expressed in a plurality of cancers including lung cancer (Proc Natl Acad Sci U S A. (1989) 86 (1), 27-31) (its polynucleotide sequence and polypeptide sequence are described in RefSeq registration Nos. NM_002354.2 and NP_002345.2, respectively), EGFR, CA19-9, CA15-3, sialyl SSEA-1 (SLX), Her2, prostate stem cell antigen (PSCA), alpha-fetoprotein (AFP), cancer embryonic antigen (CEA), tumor antigen-125 (CA-125), calretinin, MUC-1, MUC-16, epithelial membrane protein (EMA), epithelial tumor antigen (ETA), tyrosinase, melanoma-associated antigen (MAGE), chromogranin, cytokeratin, desmin, glial fibrillary acidic protein (GFAP), gross cystic disease fluid protein (GCDFP-15), HMB-45 antigen, protein melan-A (melanoma antigen that is recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, a dimer form of pyruvate kinase isozyme type M2 (tumor M2-PK), GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of prostate member 1), TROP-2, Claudin 6, RNF43a, aberrant ras protein or aberrant p53 protein, integrin alpha v beta 3 (CD61), galectin, K-Ras (V-Ki-ras2 Kirsten rat sarcoma viral oncogene), and Ral-B.

Further examples thereof include: thyroid-stimulating hormone receptor (TSHR); CD171; CS-1 (CD2 subset 1, CRACC, SLAMF7, CD319 and 19A24); C-type lectin-like molecule-1 (CLL-1); ganglioside GD3 (aNeu5Ac(2-8)aNeu5Ac(2-3)bDGalp(1-4)bDGlcp(1-1)Cer); Tn antigen (Tn Ag); T antigen (T Ag); Fms-like tyrosine kinase 3 (FLT3); CD38; CD44v6; B7H3 (CD276); KIT (CD117); interleukin-13 receptor subunit alpha-2 (IL-13Ra2); interleukin 11 receptor alpha (IL-11Ra); interleukin 2 receptor alpha (IL-2Ra); prostate stem cell antigen (PSCA); protease serine 21 (PRSS21); vascular endothelial cell growth factor receptor 2 (VEGFR2); Lewis (Y) antigen; CD24; platelet-derived growth factor receptor beta (PDGFR- beta); stage-specific embryonic antigen-4 (SSEA-4); neural cell adhesion molecule (NCAM); carbonic anhydrase IX (CAIX); proteasome (prosome, macropain) subunit, beta, 9 (LMP2); ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer; TGS5; high-molecular-weight melanoma-associated antigen (HMWMAA); o-acetyl-GD2 ganglioside (OAcGD2); folate receptor beta; tumor endothelial marker 1 (TEM1/CD248); tumor endothelial marker 7 related (TEM7R); claudin 6 (CLDN6); G protein-coupled receptor glass C group 5, member D (GPRC5D); chromosome X open reading frame 61 (CXORF61); CD97; CD179a; anaplastic lymphoma kinase (ALK); polysialic acid; placenta specific 1 (PLAC1); a hexasaccharide moiety of globoH glycoceramide (GloboH); mammary gland differentiation antigen (NY-BR-1); uroplakin 2 (UPK2); hepatitis A virus cellular receptor 1 (HAVCR1); adrenaline receptor beta 3 (ADRB3); pannexin 3 (PANX3); G protein-coupled receptor 20 (GPR20); lymphocyte antigen 6 complex, locus K9 (LY6K); olfactory receptor 51E2 (OR51E2); TCR gamma alternate reading frame protein (TARP); Wilms' tumor protein (WT1); ETS translocation variant gene 6, located on chromosome 12p (ETV6-AML); sperm protein 17 (SPA17); X antigen family, member 1A (XAGE1); angiopoietin binding cell surface receptor 2 (Tie 2); melanoma cancer-testis antigen-1 (MAD-CT-1); melanoma cancer-testis antigen-2 (MAD-CT-2); Fos-related antigen 1; p53 mutants; human telomerase reverse transcriptase (hTERT); sarcoma translocation breakpoint; melanoma inhibitor of apoptosis (ML-IAP); ERG (transmembrane protease, serine 2 (TMPRSS2)-ETS fusion gene); N-acetylglucosaminyl-transferase V (NA17); paired box protein Pax-3 (PAX3); androgen receptor; cyclin B1; v-myc avian myelocytomatosis viral oncogene neuroblastoma-derived homolog (MYCN); Ras homolog family member C (RhoC); cytochrome P450 1B1 (CYP1B1); CCCTC binding factor (zinc finger protein)-like (BORIS); squamous cell carcinoma antigen recognized by T cells 3 (SART3); paired box protein Pax-5 (PAX5); proacrosin binding protein p32 (OY-TES1); lymphocyte-specific protein tyrosine kinase (LCK); A kinase anchoring protein 4 (AKAP-4); synovial sarcoma, X breakpoint 2 (SSX2); CD79a; CD79b; CD72; leukocyte-associated immunoglobulin like receptor 1 (LAIR1); Fc fragment of IgA receptor (FCAR); leukocyte immunoglobulin-like receptor subfamily A member 2 (LILRA2); CD300 molecule-like family member f (CD300LF); C-type lectin domain family 12 member A (CLEC12A); bone marrow stromal antigen 2 (BST2); EGF-like module-containing mucin-like hormone receptor-like 2 (EMR2); lymphocyte antigen 75 (LY75); glypican-3 (GPC3); Fc receptor-like 5 (FCRL5); and immunoglobulin lambda-like polypeptide 1 (IGLL1).

The "MHC antigen" is a gene product of major histocompatibility complex (MHC). Among such antigens, glycoproteins expressed on cell membrane are mainly classified into MHC class I antigens and MHC class II antigens. The MHC class I antigens include HLA-A, -B, -C, -E, -F, -G, and -H, and the MHC class II antigens include HLA-DR, -DQ, and -DP. Tumor antigen-derived peptides presented on these MHC antigens are also included therein. A tumor antigen such as GP100, MART-1, or MAGE-1, or a complex with MHC presenting a mutated site, such as RAS or p53, is also regarded as one of the tumor antigens.

The "differentiation antigen" is a generic name for cell surface molecules that appear or disappear in association with the differentiation of bone marrow stem cells into macrophages, T cells, B cells or the like. The differentiation antigen may include CD1, CD2, CD4, CD5, CD6, CD7, CD8, CD10, CD11a, CD11b, CD11c, CD13, CD14, CD15s, CD16, CD18, CD19, CD20, CD21, CD22, CD23, CD25, CD27, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD70, CD71, CD73, CD95, CD99, CD102, CD106, CD117, CD122, CD126, and CDw130.

### Domain recognizing antigen binding molecule after cleavage of linker

The bispecific antibody and the chimeric receptor of the present disclosure have a domain recognizing the antigen binding molecule after cleavage of the linker. In one aspect, the bispecific antibody and the chimeric receptor have higher binding activity against the antigen binding molecule after cleavage of the linker than that against the antigen binding molecule before cleavage of the linker. The domain comprising antibody variable regions is provided from variable domains of one or more antibodies. Preferably, the domain comprising antibody variable regions comprises an antibody light chain variable region (VL) and an antibody heavy chain variable region (VH). Examples of such a domain comprising antibody variable regions preferably include "scFv (single chain Fv)", "single chain antibody", "Fv", "scF_{V2} (single chain F_{V2})", "Fab" and "F(ab')₂".

The term "specific" refers to a state in which a specifically binding molecule does not exhibit the same binding activity or exhibits drastically reduced binding activity against a molecule other than its one or more binding partner molecules. This term is also used when the domain comprising antibody variable regions is specific for a particular epitope among a plurality of epitopes contained in a certain antigen. When an epitope to which the domain comprising antibody variable regions binds is contained in a plurality of different antigens, an antigen binding molecule having this domain comprising antibody variable regions can bind to various antigens containing the epitope.

The terms "compete" and "cross-compete" are interchangeably used in the present disclosure in order to refer to the ability of an antibody molecule. Interference to binding may be direct or may be indirect (e.g., through an antibody molecule or the allosteric alteration of a target). The extent to which an antibody molecule can interfere with the binding of another antibody molecule to a target and thus, whether it can be regarded as competing can be determined by use of, for example, competitive binding assay as described in the present disclosure. In some embodiments, the competitive binding assay is quantitative competitive assay. In other embodiments, when the binding of a first antibody molecule to a target is reduced by 10% or more, for example, 20% or more, 30% or more, 40% or more, 50% or more, 55% or more, 60% or more, 65% or more, 70% or more, 75% or more, 80% or more, 85% or more, 90% or more, 95% or more, 98% or more, or 99% or more, in the competitive binding assay (e.g., the competitive assay described in the present disclosure), the first antibody molecule is regarded as competing with a second antibody molecule for the binding to the target.

As used in the present disclosure, the term "epitope" refers to a component of an antigen that specifically interacts with an antibody molecule. Such a component typically comprises a factor such as an amino acid side chain or a sugar side chain, or is a portion of such a factor. The epitope can be defined by a method known in the art or disclosed in the present disclosure, for example, by crystallography or hydrogen-deuterium exchange. At least one or some components on an antibody molecule, which specifically interact with the epitope, are typically positioned within CDRs. Typically, the epitope has a feature of a specific three-dimensional structure. Typically, the epitope has a feature of a specific charge. Some epitopes are linear epitopes while other epitopes are conformational epitopes.

The epitope may be defined by the binding activity of an antibody molecule that recognizes the epitope against an antigen. When the antigen is a peptide or a polypeptide, the epitope may be defined by an amino acid residue constituting the epitope. When the epitope is a sugar chain, the epitope may be defined by a particular sugar chain structure.

When the chimeric receptor of the present disclosure has an amino acid sequence derived from an antibody as a portion of the extracellular binding domain, a binding site of a molecule to which the extracellular binding domain binds may be referred to as an epitope.

The linear epitope refers to an epitope comprising an epitope that is recognized by its primary sequence of amino acids. The linear epitope contains typically at least 3 and most commonly at least 5, for example, approximately 8 to approximately 10 or 6 to 20 amino acids, in its unique sequence.

In contrast to the linear epitope, the conformational epitope refers to an epitope that is contained in a primary sequence of amino acids containing a component other than the single defined component of the epitope to be recognized (e.g., an epitope whose primary sequence of amino acids may not be recognized by an antibody that determines the epitope). The conformational epitope may contain an increased number of amino acids, as compared with the linear epitope. As for the recognition of the conformational epitope, an antibody recognizes the three-dimensional structure of the peptide or the protein. For example, when a protein molecule is folded to form a three-dimensional structure, certain amino acids and/or polypeptide backbone constituting the conformational epitope are arranged in parallel to allow the antibody to recognize the epitope. Examples of the method for determining the conformation of the epitope include, but are not limited to, X-ray crystallography, two-dimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

The term "nucleic acid" or "polynucleotide" refers to deoxyribonucleic acid (DNA) or ribonucleic acid (RNA), and a polymer thereof in either single- or double-stranded form. The term "nucleic acid" includes a gene, cDNA, or mRNA. In one embodiment, the nucleic acid molecule is a synthetic (e.g., chemically synthesized) nucleic acid molecule or a recombinant nucleic acid molecule. This term encompasses a nucleic acid containing analogs or derivatives of natural nucleotides that have binding characteristics similar to those of a reference nucleic acid and are metabolized in a manner similar to that of naturally occurring nucleotides, unless particularly limited. A particular nucleic acid sequence also virtually includes conservatively altered mutants thereof (e.g., by degenerate codon substitution), alleles, orthologs, SNPs, and complementary sequences as well as explicitly presented sequences, unless otherwise specified. Specifically, the degenerate codon substitution may be achieved by producing a sequence in which the third position of one or more selected (or all) codons is substituted with mixed base and/or deoxyinosine residues [Batzer et al., Nucleic Acid Res. 19: 5081 (1991); Ohtsuka et al., J. Biol. Chem. 260: 2605-2608 (1985); and Rossolini et al., Mol. Cell. Probes 8: 91-98 (1994)].

As used in the present disclosure, the term "nucleic acid sequence" refers to a sequence of nucleoside or nucleotide monomers consisting of natural bases, sugars and intersugar (backbone) bonds. This term also includes a modified or substituted sequence comprising a non-natural monomer or a portion thereof. The nucleic acid sequence of the present application can be a deoxyribonucleic acid sequence (DNA) or a ribonucleic acid sequence (RNA) and contains natural bases including adenine, guanine, cytosine, thymidine and uracil. These sequences may also contain modified bases. Examples of such a modified base include aza and deaza adenine, guanine, cytosine, thymidine and uracil; and xanthine and hypoxanthine.

As used in the present disclosure, the term "isolated nucleic acid" refers to a nucleic acid substantially free of cellular materials or culture media when produced by a recombinant DNA technique, or free of chemical precursors or other chemicals when chemically synthesized. The isolated nucleic acid is also substantially free of sequences naturally flanking the nucleic acid (i.e., sequences positioned at the 5' and 3' ends of the nucleic acid) from which the nucleic acid is derived. The term "nucleic acid" includes DNA and RNA, can be either double-stranded or single-stranded, and corresponds to a sense or antisense strand. The term "nucleic acid" further includes a complementary nucleic acid sequence, for example, cDNA.

The term "coding (encoding)" refers to the inherent characteristics of a specific sequence of nucleotides in a polynucleotide, such as a gene, a cDNA, or an mRNA, useful as a template for the synthesis of other polymers and high molecules in biological processes having either a defined sequence of nucleotides (e.g., rRNA, tRNA and mRNA) or a defined sequence of amino acids, and biological characteristics resulting therefrom. Thus, a gene, cDNA, or RNA encodes a protein when the transcription and translation of mRNA corresponding to the gene produce the protein in cells or other biological systems. Both a coding strand, the nucleotide sequence of which is identical to a mRNA sequence and is usually shown in a sequence listing, and a non-coding strand which is used as a template for the transcription of a gene or cDNA, can be regarded as encoding a protein or other products of the gene or the cDNA.

The "nucleotide sequence encoding an amino acid sequence" encompasses all of nucleotide sequences that are degenerate forms of each other and nucleotide sequences encoding the same amino acid sequence, unless otherwise specified. The phrase "nucleotide sequence encoding a protein or RNA" may encompasses an intron, in some cases, to the extent that the nucleotide sequence encoding the protein is capable of containing the intron. The nucleic acid molecule is operably linkable to at least one regulatory element for the expression of the chimeric receptor.

The terms "peptide", "polypeptide", and "protein" are interchangeably used and refer to a compound constituted by amino acid residues covalently linked through peptide bonds. The protein or the peptide must contain at least two amino acids, with no limitation on the maximum number of amino acids capable of constituting the sequence of the protein or the peptide. The polypeptide includes every peptide or protein comprising two or more amino acids linked to each other through peptide bonds. As used in the present disclosure, this term refers to both a short chain also generally called peptide, oligopeptide and oligomer in the art, and a longer chain generally called protein in the art, of which there are many types. Examples of the "polypeptide" particularly include biologically active fragments, substantially homologous polypeptides, oligopeptides, homodimers, heterodimers, mutants of polypeptides, altered polypeptides, derivatives, analogs, and fusion protein. Examples of the peptide include natural peptides, recombinant peptides, and combinations thereof.

The polypeptide according to the present disclosure usually refers to a peptide having a length on the order of 10 amino acids or longer, and a protein. When a linkage of amino acids connected through a peptide bond from the N terminus to the C terminus is regarded as a single peptide chain, the polypeptide of the present disclosure may be a complex protein formed from a plurality of single peptide chains through interaction such as a S-S bond, hydrophobic interaction, or an ion bond.

The term "isolated polypeptide", also called "isolated protein", refers to a polypeptide substantially free of cellular materials or culture media when produced by a recombinant DNA technique, or free of chemical precursors or other chemicals when chemically synthesized.

The term "amino acid" includes all of natural amino acids and modified amino acids.

As used in the present disclosure, the term "conservative amino acid variation" is the substitution of an amino acid residue by another amino acid residue without impairing the desired characteristics of the protein.

The term "conservative sequence alteration" refers to amino acid mutation that does not significantly influence or change the binding characteristics of an antibody or an antibody fragment containing the amino acid sequence. Examples of such conservative alteration include amino acid substitution, addition and deletion. The alteration can be introduced into the antibody or the antibody fragment of the present disclosure by standard techniques known in the art, such as site-directed mutagenesis and PCR-mediated mutagenesis. The conservative amino acid substitution is substitution that replaces an amino acid residue with an amino acid residue having a similar side chain. Families of amino acid residues having similar side chains are defined in the art. These families include amino acids having a basic side chain (e.g., lysine, arginine, and histidine), amino acids having an acidic side chain (e.g., aspartic acid and glutamic acid), amino acids having an uncharged polar side chain (e.g., glycine, asparagine, glutamine, serine, threonine, tyrosine, cysteine, and tryptophan), amino acids having a nonpolar side chain (e.g., alanine, valine, leucine, isoleucine, proline, phenylalanine, and methionine), amino acids having a beta-branched side chain (e.g., threonine, valine, and isoleucine) and amino acids having an aromatic side chain (e.g., tyrosine, phenylalanine, tryptophan, and histidine). Thus, one or more amino acid residues within the chimeric receptor of the present disclosure may be replaced with other amino acid residues of the same side chain family, and the chimeric receptor thus changed can be tested by use of functional assay described in the present disclosure.

The term "expression" refers to the transcription and/or translation of a particular nucleotide sequence driven by a promoter.

The "host cell", the "host cell line", and the "host cell cultures" in the present disclosure are interchangeably used and refer to cells harboring a foreign nucleic acid (including the progeny of such cells). The host cell includes a "transformant" and a "transformed cell", which include a primary transformed cell and progeny derived from the cell, regardless of the number of passages. The progeny may not be completely identical in terms of nucleic acid contents to a parent cell, and may have a mutation. Mutant progeny having the same function or biological activity as that used for screening or selecting the original transformed cells for are also included in the present disclosure.

The "vector" in the present disclosure refers to a nucleic acid molecule that can propagate another nucleic acid to which the vector is linked. This term includes a vector as a self-replicating nucleic acid structure, and a vector integrated into the genomes of host cells harboring the vector. A certain vector can bring about the expression of a nucleic acid operatively link to the vector itself. Such a vector is also referred to as an "expression vector" in the present disclosure.

The transfection means the uptake of an expression vector by a host cell for which whether or not to actually express any coding sequence is not clear. Many transfection methods are known to the ordinarily skilled artisan, for example, CaPO₄ precipitation and electroporation. In general, successful transfection is recognized when a sign of the operation of the vector appears within the host cell.

The term "promoter" refers to a DNA sequence that is recognized by the synthetic mechanism of cells, or an introduced synthetic mechanism, necessary for starting the specific transcription of a polynucleotide sequence.

The term "lentivirus" refers to a genus of the family *Retroviridae.* The lentivirus is unique, among the retroviruses, in being able to infect non-dividing cells. The lentivirus can deliver a significant amount of genetic information into DNA of host cells and is therefore one of the most efficient methods of a gene delivery vector. HIV, SIV, and FIV are all examples of the lentivirus.

The term "lentivirus vector" refers to a vector derived from at least a portion of the lentivirus genome. Examples thereof particularly include self-inactivating lentivirus vectors as disclosed in Milone et al., Mol. Ther. 17 (8): 1453-1464 (2009). Other examples of the lentivirus vector that may be used in clinics include, but are not limited to, LENTIVECTOR(R) gene delivery technology manufactured by Oxford BioMedica plc, LENTIMAX(TM) vector system manufactured by Lentigen Technology, Inc., and the like. Nonclinical types of lentivirus vectors are also available, and these vectors can be appropriately selected and prepared by those skilled in the art.

The term "antigen presenting cell" or "APC" refers to an immune system cell, such as an accessory cell (e.g., a B-cell, a dendritic cell, and the like), which presents a foreign antigen complexed with major histocompatibility complex (MHC) on its surface. T cells may recognize the complex using their T cell receptors (TCRs). APC processes the antigen and presents the antigen to T cells.

As used in the present disclosure in the present specification, the term "immune effector cell" refers to a cell involved in the promotion of immune response, for example, immune effector response. Examples of the immune effector cell include T cells, for example, alpha/beta T cells and gamma/delta T cells, B cells, natural killer (NK) cells, natural killer T (NK-T) cells, mast cells, and myeloid series-derived phagocytes.

Examples of the T cells can include T cells derived from humans, and T cells derived from non-human mammals such as dogs, cats, pigs and mice. The T cells can also be obtained by isolation and purification from immunocytes infiltrating into a body fluid such as blood or bone marrow fluid, a tissue of the spleen, the thymus gland, lymph node, or the like, or a cancer tissue such as primary tumor, metastatic tumor, or cancerous ascites. Examples of such T cells can include α (alpha)/β (beta) T cells, γ (gamma)/δ (delta) T cells, CD8⁺ T cells, CD4⁺ T cells, tumor infiltrating T cells, memory T cells, naive T cells, and NKT cells.

As used in the present disclosure in the present specification, the term "immune effector function or immune effector response" refers to, for example, a function or response of immune effector cells that enhances or promotes the immune attack of target cells. The immune effector function or response refers to, for example, the characteristics of T or NK cells that promote the killing or inhibition of growth or proliferation of target cells. In the case of T cells, primary stimulation and co-stimulation are examples of the immune effector function or response.

The term "effector function" refers to a specified function of cells. The effector function of T cells can be, for example, cytolytic activity or helper activity such as cytokine secretion.

The term "autologous" refers to every material derived from the same individual as an individual to which the material is later to be re-introduced.

The term "allogeneic" refers to every material derived from a different animal of the same species as that of an individual to which the material is introduced. Two or more individuals are said to be allogeneic to each other when genes at one or more loci are not identical. In some aspects, allogeneic material from individuals of the same species may differ genetically to an extent sufficient for interacting antigenically.

The term "heterologous" refers to a graft derived from an animal of a different species.

As used in the present disclosure, the term "subject" means all members of the animal kingdom including humans. In a preferred aspect, the subject is a human.

In the context of the present disclosure, the terms "treatment", "treat", and the like mean the relief or alleviation of at least one symptom associated with a disease condition, or the delay or reversal of progression of such a condition, as long as the terms relate to any of disease conditions recited herein. Within the scope of this meaning of the present disclosure, the term "treatment" also means arrest, the delay of onset (i.e., the time to the clinical manifestation of a disease) and/or reduction in the risk of developing or worsening a disease. For example, in connection with a cancer, the term "treatment" may mean the elimination or reduction of a patient's tumor burden, or the prevention, delay or blocking of metastasis, etc.

As used in the present disclosure and as well understood in the art, the "therapy" is an approach for obtaining beneficial or desired results, including clinical results. The beneficial or desired clinical results can include, but are not limited to, the alleviation or amelioration of one or more symptoms or pathological conditions, the diminishment of the extent of a disease, the stabilization (i.e., not worsening) of a disease state, the prevention of spread of a disease, the delay or slowing of progression of a disease, the amelioration or alleviation of a disease condition, and remission (partial or complete), regardless of whether to be detectable or undetectable. The "therapy" may also mean the prolongation of a survival period compared with an expected survival period without the treatment. The term "alleviate" a disease or a disorder means that the extent and/or undesirable clinical manifestation of a disorder or a disease condition is lessened and/or the time course of progression is slowed or lengthened, as compared with the case of not treating the disorder.

The term "cancer" refers to a disease characterized by the uncontrollable growth of aberrant cells. Cancer cells can spread locally or via bloodstream and the lymphatic system to other parts of the body. Examples of various cancers are described in the present disclosure and include, but are not limited to, breast cancer, prostate cancer, ovary cancer, cervical cancer, skin cancer, pancreatic cancer, colorectal cancer, kidney cancer, liver cancer, brain cancer, lymphoma, leukemia, lung cancer and the like. The terms "tumor" and "cancer" are interchangeably used in the present disclosure in the present specification. Both the terms encompass, for example, solid and liquid, for example, diffuse or circulating, tumors. As used in the present disclosure, the term "cancer" or "tumor" encompasses premalignant as well as malignant cancers and tumors.

Examples of the cancer for the anticancer agent or the method for treating a cancer as mentioned later according to the present disclosure can include cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, germ cell tumor, lymphoma, and leukemia.

In some embodiments, the tumor antigen in association with a cancer type is a marker expressed by both normal cells and cancer cells, for example, a lineage marker such as CD 19 on B cells. In a certain aspect, the tumor antigen of the present disclosure is derived from a cancer including, but is not limited to, primary or metastatic melanoma, thymoma, lymphoma, sarcoma, lung cancer, liver cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer, pancreatic cancer, and the like. In some embodiment, the tumor antigen is an antigen common in particular proliferative disorders. In some embodiment, the tumor antigen is a cell surface molecule that is overexpressed in cancer cells compared with normal cells, for example, by 1-fold overexpression, 2-fold overexpression, 3-fold overexpression or more as compared with normal cells. In some embodiments, the tumor antigen is a cell surface molecule that is inappropriately synthesized in cancer cells, for example, a molecule containing deletion, addition or mutation as compared with a molecule expressed in normal cells. In some embodiment, the tumor antigen is expressed exclusively on the cell surface of cancer cells, as a whole or as a fragment (e.g., MHC/peptide), and neither synthesized nor expressed on the surface of normal cells. In some embodiments, the chimeric receptor of the present disclosure include CAR comprising an antigen binding domain (e.g., an antibody or an antibody fragment) that binds to a peptide presented by MHC. Usually, peptides derived from endogenous proteins fill the pockets of major histocompatibility complex (MHC) class I molecules, and are recognized by T cell receptors (TCRs) on CD8⁺ T lymphocytes. The MHC class I complex is constitutively expressed by all nucleated cells. In cancers, virus-specific and/or tumor-specific peptide/MHC complexes are representative of a unique class of cell surface targets for immunotherapy. TCR-like antibodies targeting virus- or tumor antigen-derived peptides in the context of human leukocyte antigen (HLA)-A1 or HLA-A2 are described [see e.g., Sastry et al., J Virol.2011 85(5): 1935-1942; Sergeeva et al., Bood, 2011 117(16): 4262-4272; Verma et al., J Immunol 2010 184(4): 2156-2165; Willemsen et al., Gene Ther 2001 8(21): 1601-1608; Dao et al., Sci Transl Med 2013 5(176): 176ra33;Tassev et al., Cancer Gene Ther 2012 19(2): 84-100]. The TCR-like antibody can be identified, for example, by screening a library such as a human scFv phage display library.

As used in the present disclosure, the phrase "treating or preventing a cancer" means the inhibition of cancer cell replication, the provision of antitumor immunity, the inhibition of cancer spread (metastasis), the inhibition of tumor growth, reduction in the number of cancer cells or tumor growth, or the amelioration of cancer-related symptoms. As used in the present disclosure, the terms "prevent," "preventing" and "prevention" refer to an action that is performed before a subject begins to suffer from the condition or before relapse of the condition, unless otherwise specified. The prevention does not have to bring about the complete prevention of the condition. This term encompasses the partial prevention or alleviation of the condition or a symptom of the condition, or reduction in the risk of developing the condition.

The term "anticancer effect" refers to a biological effect that can be demonstrated by various approaches including but are not limited to, for example, decrease in tumor volume, decrease in the number of cancer cells, decrease in the number of metastases, increase in life expectancy, decrease in cancer cell growth, decrease in cancer cell survival, and amelioration of various physiological symptoms associated with a cancerous condition. The "anticancer effect" can also be demonstrated by the ability of the peptide, the polynucleotide, the cell and the antibody described in the present disclosure in the prevention of the occurrence of a cancer at a primary location. The term "antitumor effect" refers to a biological effect that can be demonstrated by various approaches including but are not limited to, for example, decrease in tumor volume, decrease in the number of tumor cells, decrease in tumor cell growth, and decrease in tumor cell survival.

As used herein, the term "therapeutically effective" applied to a dosage or an amount refers to an amount of a compound or a pharmaceutical composition (e.g., a composition comprising T lymphocytes (and/or NK cells) comprising the chimeric receptor of the present disclosure, and if desired, further comprising a tumor-specific cytotoxic monoclonal antibody or another antitumor molecule comprising an Fc moiety (e.g., a mixed molecule consisting of a ligand (e.g., a cytokine or an immune cell receptor) bound to a tumor surface receptor combined with an immunoglobulin Fc moiety or Fc-containing DNA or RNA), or a composition comprising the primary antibody of the present disclosure) sufficient for causing the desired activity when the compound or the pharmaceutical composition is administered to a subject in need of treatment. In the context of the present disclosure, the term "therapeutically effective" refers to an amount of a compound or a pharmaceutical composition effective for delaying the manifestation of, arresting the progression of, or relieving or alleviating at least one symptom of a disorder treated by a procedure according to the method of the present disclosure. It should be noted that when a combination of active ingredients is administered, the effective amount of the combination may or may not include the respective amounts of the ingredients that would be effective if administered individually.

In the present disclosure, the term "pharmaceutically acceptable" refers to a molecular entity and other ingredients of such a composition that are physiologically tolerable and do not typically produce undesirable reactions when the composition is administered to a mammal (e.g., a human). Preferably, in the present disclosure, the "pharmaceutically acceptable" means approved by a regulatory agency of the Federal or a state government or listed in the U.S. Pharmacopeia or other generally recognized pharmacopeia for use in mammals, more specifically, humans.

### Chimeric receptor of present disclosure

In one aspect of the present disclosure, the chimeric receptor of the present disclosure comprises two signaling domains described herein, i.e., CD3 zeta and 4-1BB/CD137, or CD3 zeta as well as one or more signaling domains. In a particular aspect, several signaling domains are fused to each other for additive or synergistic effects. Non-limiting examples of the useful additional signaling domain include a portion or the whole of one or more of TCR zeta chain, CD27, CD28, OX40/CD134, 4-1BB/CD137, Fc epsilon RIγ, ICOS/CD278, ILRB/CD122, IL-2RG/CD132, DAP-10 and CD40.

The present disclosure discloses a chimeric receptor comprising i) an extracellular domain capable of binding to a predetermined antigen via the antigen binding molecule after cleavage of the linker, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

The present disclosure also includes a chimeric receptor comprising i) an extracellular domain capable of binding via the antigen binding molecule after cleavage of the linker, ii) a transmembrane domain, and iii) an intracellular segment comprising one or more intracellular signal transduction domains including a cytoplasmic domain of an IL receptor chain and optionally at least one supplementary cytoplasmic domain. In an embodiment, these domains are optionally fused directly or indirectly in the foregoing order starting from the N terminus. In one embodiment, these domains within the intracellular segment are fused in the inverse order.

In some embodiments, the IL receptor chain is proximal to the transmembrane domain and/or is near or forms the N terminus of the intracellular segment of the chimeric receptor. In other embodiments, the IL receptor chain is near or forms the C terminus of the intracellular segment of the chimeric receptor. In some embodiments, the IL receptor chain is upstream or N-terminal from a CD3 zeta intracellular signaling domain comprising an exogenous STAT3 association motif YXXQ.

In an embodiment where the intracellular segment comprises only a signaling domain of the IL receptor chain, cells expressing the chimeric receptor can be activated by a predetermined antigen presented in an MHC complex via endogenous TCR and/or by a CD80/86 molecule via endogenous CD28, for example by B cells.

The present disclosure also provides a cell expressing a chimeric receptor. Such a cell can have, for example, a high growth rate and/or a high survival rate, produces high amounts of cytokines, and/or can have high cytotoxic activity against a cell having, on its surface, a predetermined/preselected antigen to which the chimeric receptor binds, as compared with a parent cell that does not express the chimeric receptor. For example, as shown in Examples, T cells transduced with the chimeric receptor of the present disclosure have high ability to divide and grow in primary antibody-dependent and cancer antigen- or tumor antigen-dependent manners, and provide an antitumor effect and improve an overall survival, in mice receiving the cells as treatment. Accordingly, an antitumor effect in humans is also expected.

### Extracellular domain

The extracellular domain used for the chimeric receptor of the present disclosure is a domain comprising a proteinous molecule or a portion thereof capable of binding to the targeted antigen binding molecule after cleavage of the linker, and includes, for example, an antigen binding domain of an antibody and a ligand binding domain of a receptor. This domain binds to and interacts with an antigen present on cell surface, thereby conferring specificity for cells expressing the chimeric receptor. For example, the extracellular domain used for the chimeric receptor of the present disclosure comprises variable regions of an antibody (e.g., an H chain and an L chain), a single chain or a binding fragment thereof, or TCR (TCR alpha, TCR beta, TCR gamma, or TCR delta). For example, an antibody Fab fragment, antibody variable regions [H chain V region (VH) and L chain V region (VL)] or an extracellular ligand binding domain of a receptor can be used. Particularly, in an embodiment, a single chain variable fragment (scFv) can be used.

The extracellular domain of the chimeric receptor of the present disclosure may bind to only one targeted antigen binding molecule after cleavage of the linker, or may bind to two or more targeted antigen binding molecules after cleavage of the linker. In addition, the present disclosure includes both a chimeric receptor comprising one extracellular domain and a chimeric receptor comprising two or more extracellular domains.

Examples of the antigen to which the antigen binding molecule after cleavage of the linker (to which the extracellular domain of the chimeric receptor of the present disclosure binds) binds include viral antigens, bacterial (particularly, infectious bacteria) antigens, parasite antigens, cell surface markers on target cells related to particular pathological conditions (e.g., tumor antigens), and surface molecules of immunocytes causing autoimmunity.

One aspect of the present disclosure provides a chimeric receptor capable of binding to an antigen derived from the family *Retroviridae* (e.g., human immunodeficiency virus, for example, HIV-1 and HIV-LP), the family *Picornaviridae* (e.g., poliovirus, hepatitis A virus, enterovirus, human coxsackievirus, rhinovirus, and echovirus), rubella virus, coronavirus, vesicular stomatitis virus, rabies virus, Ebola virus, parainfluenza virus, mumps virus, measles virus, respiratory syncytial virus, influenza virus, hepatitis B virus, parvovirus, the family *Adenoviridae,* the family *Herpesviridae* [e.g., type 1 and type 2 herpes simplex virus (HSV), varicella-zoster virus, cytomegalovirus (CMV), and herpes virus], the family *Poxviridae* (e.g. smallpox virus, vaccinia virus, and pox virus), or hepatitis C virus, via the antigen binding molecule after cleavage of the linker.

Another aspect of the present disclosure provides a chimeric receptor capable of binding to an antigen derived from a bacterial strain of the genus *Staphylococci,* the genus *Streptococcus, Escherichia coli, Pseudomonas,* or the genus *Salmonella,* via the antigen binding molecule after cleavage of the linker. Particularly, the present disclosure provides a chimeric receptor capable of binding to an antigen derived from an infectious bacterium, for example, *Helicobacter pyloris, Legionella pneumophilia,* a bacterial strain of *Mycobacteria* sps. (e.g., *M. tuberculosis, M. avium, M. intracellulare, M. kansasii,* or *M. gordonae*)*, Staphylococcus aureus, Neisseria gonorrhoeae, Neisseria meningitides, Listeria monocytogenes, Streptococcus pyogenes,* Group A *Streptococcus,* Group B *Streptococcus (Streptococcus agalactiae), Streptococcus pneumoniae,* or *Clostridium tetani,* via the antigen binding molecule after cleavage of the linker.

In another aspect of the present disclosure, the antibody that binds to the extracellular binding domain of the chimeric receptor is capable of binding to a tumor antigen such as 5T4, alpha 5 beta 1-integrin, 707-AP, AFP, ART-4, B7H4, BAGE, beta-catenin/m, Bcr-abl, MN/C IX antibody, CA125, CAMEL, CAP-1, CASP-8, CD4, CD19, CD20, CD22, CD25, CDC27/m, CD30, CD33, CD52, CD56, CD80, CDK4/m, CEA, CT, Cyp-B, DAM, EGFR, ErbB3, ELF2M, EMMPRIN, EpCam, ETV6-AML1, G250, GAGE, GnT-V, Gp100, HAGE, HER-2/new, HLA-A^{∗}0201-R170I, HPV-E7, HSP70-2M, HST-2, hTERT (or hTRT), iCE, IGF-1R, IL-2R, IL-5, KIAA0205, LAGE, LDLR/FUT, MAGE, MART-1/melan-A, MART-2/Ski, MC1R, myosin/m, MUC1, MUC-16, MUM-1, MUM-2, MUM-3, NA88-A, PAP, proteinase-3, p190 minor bcr-abl, Pml/RAR alpha, PRAME, PSA, PSM, PSMA, RAGE, RU1 or RU2, SAGE, SART-1 or SART-3, survivin, TEL/AML1, TGF beta, TPI/m, TRP-1, TRP-2, TRP-2/INT2, VEGF, WT1, NY-Eso-1 or NY-Eso-B. In the present disclosure, the antibody is also capable of binding to a cell surface adhesion molecule, a surface molecule of inflammatory cells found in autoimmune diseases, or a TCR causing autoimmunity.

### Intracellular segment

The intracellular segment of the chimeric receptor according to the present disclosure is a proteinous molecule that can comprise one or more intracellular signaling domains and is capable of transducing signals into cells when the extracellular domain present in the same molecule binds to (interacts with) its cognate antigen/ligand.

In an aspect, the intracellular segment of the chimeric receptor comprises a CD3 zeta intracellular signaling domain comprising an exogenous STAT3 association motif. In addition, the intracellular segment of the chimeric receptor comprises one or more intracellular signaling domains selected from a cytoplasmic domain of an IL receptor chain and/or a cytoplasmic costimulatory domain. The intracellular segment comprises an endogenous or exogenous JAK binding motif and a STAT5 association motif.

A primary cytoplasmic signaling sequence regulates the primary activation of a TCR complex. For example, the CD3 zeta intracellular signaling domain provides a primary cytoplasmic signal. The primary cytoplasmic signaling sequence may comprise a signaling motif known as an immunoreceptor tyrosine-based activation motif (ITAM) [Nature, vol. 338, pp. 383-384 (1989)]. On the other hand, a primary cytoplasmic signaling sequence that acts in an inhibitory manner may comprise a signaling motif known as an immunoreceptor tyrosine-based inhibition motif (ITIM) [J Immunol., vol. 162, No. 2, pp. 897-902 (1999)]. In the present disclosure, an intracellular signaling domain having ITAM and/or ITIM can be used.

In the present disclosure, the CD3 zeta intracellular domain comprises an immunoreceptor tyrosine-based activation motif (ITAM). Examples of the intracellular signal transduction domain having ITAM that can be used instead of or to replace CD3 zeta include intracellular signal transduction domains having ITAM derived from FcR gamma, FcR beta, CD3 gamma, CD3 delta, CD3 epsilon, CD5, CD22, CD79a, CD79b, and CD66d. Specifically, examples of the intracellular domain comprising one or more ITAM motifs include peptides having sequences from amino acids 52 to 164 of CD3 zeta (NCBI RefSeq: NP932170.1), from amino acids 45 to 86 of Fc epsilon RI gamma (NCBI RefSeq: NP004097.1), from amino acids 201 to 244 of Fc epsilon RIbeta (NCBI RefSeq: NP_000130.1), from amino acids 139 to 182 of CD3 gamma (NCBI RefSeq: NP_000064.1), from amino acids 128 to 171 of CD3 delta (NCBI RefSeq: NP_000723.1), from amino acids 153 to 207 of CD3 epsilon (NCBI RefSeq: NP_000724.1), from amino acids 402 to 495 of CD5 (NCBI RefSeq: NP_055022.2), from amino acids 707 to 847 of CD22 (NCBI RefSeq: NP_001762.2), from amino acids 166 to 226 of CD79a (NCBI RefSeq: NP_001774.1), from amino acids 182 to 229 of CD79b (NCBI RefSeq: NP_000617.1), and from amino acids 177 to 252 of CD66d (NCBI RefSeq: NP_001806.2), and mutants having the same functions as those of these peptides. The amino acids based on amino acid sequence information of NCBI RefSeq ID or GenBank described in the present disclosure are numbered on the basis of the full length of a precursor (comprising a signal peptide sequence, etc.) of each protein.

In an embodiment, the amino acid residue represented by "X" in the STAT3 association motif YXXQ can be any natural amino acid including any modified natural amino acid that retains STAT3 binding. In one embodiment, the amino acid X is independently selected from leucine, arginine, histidine, phenylalanine, lysine, proline, methionine, valine, glutamine, threonine, and aspartic acid. The amino acid X is, for example, arginine. The amino acid X is, for example, histidine.

In an embodiment, the two amino acid residues flanking the tyrosine residue in the STAT3 association motif YXXQ are arginine-histidine. In yet another embodiment, the exogenous STAT3 association motif is YRHQ.

The exogenous STAT3 association motif YXXQ may be introduced in any moiety of the intracellular domain of CD3 zeta. In an embodiment, the YXXQ association motif is inserted near a C-terminal region. Without wishing to be bound by a particular theory, many endogenous YXXQ motifs are probably located near or within 100 aa from the C terminus. Also, the YXXQ motif located near the C-terminal region has been shown to be more functional at a more proximal site in GP130 and LIFR studies (Schmitz J et al., J Immunol. 2000; 164: 848-54; and Tomida M et al., Blood. 1999; 93: 1934-41).

In an embodiment, the exogenous STAT3 association motif YXXQ is introduced in any moiety of the intracellular domain of CD3 zeta located within 200 amino acid residues from the C terminus of the chimeric receptor. For example, the STAT3 association motif is introduced within 200 amino acid residues, within 150 amino acid residues, within 100 amino acid residues, within 90 amino acid residues, within 80 amino acid residues, within 70 amino acid residues, within 60 amino acid residues, within 50 amino acid residues, within 40 amino acid residues, within 30 amino acid residues, within 20 amino acid residues or within 10 amino acid residues from the C terminus of the chimeric receptor. In one embodiment, the exogenous STAT3 association motif is introduced at a location other than ITAM

In an embodiment, the CD3 zeta intracellular domain comprising an exogenous STAT3 association motif comprises at least one ITAM motif. In one embodiment, the CD3 zeta intracellular domain comprising an exogenous STAT3 association motif comprises two ITAM motifs. In a further embodiment, the CD3 zeta intracellular domain comprising an exogenous STAT3 association motif comprises three ITAM motifs.

Those skilled in the art will appreciate that several methods may be used to introduce a STAT3 association motif into the intracellular signaling domain of CD3 zeta. For example, the exogenous STAT3 association motif can be introduced by substituting amino acid residues Leu-His-Met at amino acid residues 104 to 106 of the intracellular signaling domain of CD3 zeta by tyrosine at residue 104 and any other two amino acid residues at positions 105 and 106 flanking the tyrosine residue. The amino acid residues 104-105-106 of the intracellular signaling domain of CD3 zeta correspond to amino acid residues 156-157-158 of the full-length CD3 zeta (e.g., NCBI RefSeq: NP_932170.1).

As described, the chimeric receptor according to an embodiment comprises an intracellular segment comprising one or more intracellular signaling domains selected from a cytoplasmic domain of an IL receptor chain and a cytoplasmic costimulatory domain.

In the present disclosure, the cytoplasmic domain of an IL receptor chain can be selected from any chain of the IL receptor. For example, a cytoplasmic domain comprising amino acids 266 to 551 of an IL-2 receptor beta chain (NCBI REFSEQ: NP_000869.1) (amino acids 256 to 538 of an IL-21 receptor alpha chain (NCBI REFSEQ: NP_068570.1)), amino acids 284 to 369 of a common IL-2 receptor gamma chain (NCBI REFSEQ: NP_000197.1), amino acids 265 to 459 of IL-7R alpha (NCBI REFSEQ: NP_002176.2), amino acids 292 to 521 of IL-9R alpha (NCBI REFSEQ: NP_002177.2) or amino acids 257 to 825 of IL-4R alpha (NCBI REFSEQ: NP_000409.1) may be used. The whole region of the cytoplasmic domain of the IL receptor chain may be used.

Alternatively, a truncated fragment of the cytoplasmic domain of the IL receptor chain may also be used. The truncated fragment comprises, for example, up to 250 amino acids of the ILR cytoplasmic domain, or is 50 to 200 amino acids or 80 to 150 amino acids of the ILR cytoplasmic domain.

In an embodiment, the cytoplasmic domain of the IL receptor chain, optionally the truncated fragment of the cytoplasmic domain of the IL receptor chain, comprises at least a STAT association motif, optionally a STAT5 association motif, and a JAK binding motif (also known as a box-1 motif). In an embodiment, the cytoplasmic domain of the IL receptor chain or the truncated fragment thereof comprises a STAT5 association motif and a JAK binding motif.

In an embodiment, the cytoplasmic domain and/or the truncated fragment of the IL receptor chain includes mutants having the same function, for example, mutants that induce STAT signal transduction, optionally STAT5 signal transduction and/or JAK signal transduction.

In one aspect of the present disclosure, a cytoplasmic domain of an IL-2 receptor (IL-2R) beta chain may be used. Examples of the cytoplasmic domain of an IL-2R beta chain that may be used in the present disclosure include amino acids 266 to 551 of an IL-2R beta chain (NCBI RefSeq: NP_000869.1). One embodiment includes a peptide having any of sequences from amino acid positions 266 to 337 and from amino acid positions 530 to 551. In one aspect of the present disclosure, a truncated fragment of the cytoplasmic domain of the IL-2R beta chain may be used. This truncated fragment may comprise i) a JAK binding motif (e.g., amino acids 278 to 286 of NCBI RefSeq: NP_000869.1), also called BOX-1 motif, which permits association with tyrosine kinase JAK1, and ii) a STAT association motif, optionally a STAT5 or STAT3 association motif. Other moieties of the IL receptor chain can be changed, for example, by conservative amino acid variation.

In an embodiment, the intracellular segment may comprise an exogenous JAK binding motif, or a signaling molecule comprising a JAK binding motif. The JAK binding motif is derived from, for example, IL2R gamma (IL2RG), erythropoietin receptor (EpoR), thrombopoietin receptor (TpoR), granulocyte macrophage colony stimulating factor receptor (GM-CSFR), and growth hormone receptor (GHR).

The IL-2R beta chain comprises three functional STAT5 binding motifs, YFFF, YCTF and YLSL, for use in STAT5 association. Mutations of these tyrosine residues can abolish the IL-2 reactivity of the IL-2R beta chain (Friedmann et al., 1996). The erythropoietin receptor (EpoR) comprises two tyrosine residues that mediate STAT5 activation, i.e., Y343 and Y401 both of which have a YXXL motif as described above (Klingmuller et al., 1996). Thus, YXXL can be a preferred motif for STAT5 recruitment. Other amino acid residues are also functional, for example, as shown with the IL-2R beta chain STAT5 binding motif. In one embodiment, the STAT5 association motif is an IL-2R beta chain STAT5 association motif and comprises tyrosine residue-510 (tyrosine residue 510 is amino acid 536 of NCBI RefSeq: NP_000869.1).

In an embodiment, the STAT5 association motif can be derived from IL2R gamma, EpoR, TpoR, GM-CSFR and GHR

In an embodiment, the STAT5 association motif of the IL-2R beta chain comprises amino acid residues YXXL. In an embodiment, the amino acid residue represented by "X" in the STAT5 association motif can be any natural amino acid including any modified natural amino acid that retains STAT5 binding.

Likewise, the intracellular segment comprises one or more JAK binding motifs that may be located or introduced in any of the intracellular signaling domains.

In one aspect of the present disclosure, a cytoplasmic domain of an IL-21 receptor (EL-21R) alpha chain may be used. Examples of the cytoplasmic domain of the IL-21R alpha chain used in the present disclosure include intracellular signal transduction domains comprising amino acid positions 256 to 538 (NCBI RefSeq: NP_068570.1) of the IL-21R alpha chain. In one aspect of the present disclosure, a truncated fragment of the cytoplasmic domain of the IL-21R alpha chain may be used. The truncated fragment comprises a box-1 motif (amino acids 266 to 274 of NCBI RefSeq: NP_068570.1) necessary for association with tyrosine kinase JAK1, and comprises a STAT association motif. In an embodiment, the STAT association motif comprises tyrosine residue-500 (amino acid 519 of NCBI RefSeq: NP_000869.1) and three residues flanking at the C-terminal side of tyrosine residue 500, i.e., YLRQ, necessary for STAT1/3 association.

Other examples of the intracellular signaling domain include cytoplasmic regions derived from a TCR complex and/or a costimulatory molecule, and any mutant having the same functions as those of these sequences. Other examples thereof include cytoplasmic signaling domains listed in Table 2 of Sadelain et al., 2009, which is incorporated herein by reference.

The activation of natural T cells is transduced by two different types of intracellular signaling domains, i.e., a domain for inducing antigen-dependent primary activation via a TCR complex (e.g., a primary cytoplasmic signal provided by, for example, CD3 zeta) and a domain that acts in an antigen-independent manner to provide a secondary or costimulatory signal (secondary cytoplasmic signal).

Examples of the intracellular domain comprising the secondary or costimulatory cytoplasmic signaling domain that may be used in the present disclosure include sequences derived from CD2, CD4, CD5, CD8 alpha, CD8 beta, CD28, CD134, CD137 (4-1BB), ICOS, and CD154, for example, truncated fragments thereof comprising a signaling motif. Specific examples thereof include peptides having any of sequences from amino acids 236 to 351 of CD2 (NCBI RefSeq: NP_001758.2), from amino acids 421 to 458 of CD4 (NCBI RefSeq: NP_000607.1), from amino acids 402 to 495 of CD5 (NCBI RefSeq: NP_055022.2), from amino acids 207 to 235 of CD8 alpha (NCBI RefSeq: NP_001759.3), from amino acids 196 to 210 of CD8 beta (GenBank: AAA35664.1), from amino acids 180 to 220 of CD28 (NCBI RefSeq: NP_006130.1), from amino acids 214 to 255 of CD137 (4-1BB, NCBI RefSeq: NP_001552.2), from amino acids 241 to 277 of CD134 (OX40, NCBI RefSeq: NP_003318.1), and from amino acids 166 to 199 of ICOS (NCBI RefSeq: NP_036224.1), and mutants having the same functions as those of these peptides.

In one aspect, the disclosure preferably includes a chimeric receptor comprising an intracellular segment having one or more, for example, 2 or 3 intracellular signaling domains in addition to the intracellular signaling domain of CD3 zeta comprising an exogenous STAT3 association motif.

The present disclosure also includes a chimeric receptor comprising an intracellular segment having two or more same intracellular signaling domains linked in series. In one aspect, the present disclosure provides a chimeric receptor having a cytoplasmic domain of an IL receptor located on the N-terminal side of an intracellular signaling domain of CD3 zeta, i.e., a chimeric receptor comprising a cytoplasmic domain of an IL receptor and an intracellular signaling domain of CD3 zeta linked in this order from the N-terminal side. The present disclosure also includes a chimeric receptor obtained by further adding an intracellular domain of CD28 (e.g., a cytoplasmic costimulatory domain of CD28) to the chimeric receptor mentioned above, i.e., a chimeric receptor comprising an intracellular signaling domain of CD28, a cytoplasmic domain of an IL receptor, and an intracellular signaling domain of CD3 zeta comprising an exogenous STAT3 motif, linked in this order from the N-terminal side.

In an embodiment, the chimeric receptor comprises an intracellular segment comprising a CD3 zeta intracellular signaling domain comprising an exogenous STAT3 association motif and an intracellular signaling domain selected from a cytoplasmic domain of an interleukin receptor chain and a cytoplasmic costimulatory domain, wherein at least one intracellular signaling domain comprises an endogenous or exogenous JAK binding motif and a STAT5 association motif.

In one embodiment, the chimeric receptor comprises a CD3 zeta intracellular signaling domain having an exogenous STAT3 association motif, a cytoplasmic domain of an IL receptor chain fragment comprising an endogenous or exogenous JAK binding motif and a STAT5 association motif, and a cytoplasmic costimulatory domain of CD28.

In the chimeric receptor of the present disclosure, an oligopeptide linker or a polypeptide linker can be inserted between the domains of the intracellular segment so as to link the domains therein and/or to link these domains to other domains. For example, a linker having a length of 2 to 10 amino acids can be used. Particularly, a linker having a glycine-serine continuous sequence can be used. For example, a linker IDGGGGSGGGGSGGGGS can be inserted between the CD28 cytoplasmic domain and the partial cytoplasmic IL-2 receptor beta domain. For example, a linker KLGGSGP can be inserted between the partial cytoplasmic IL-2 receptor beta domain and the intracellular domain of the CD3 zeta chain.

Another aspect provides a chimeric receptor comprising i) an extracellular domain capable of binding to a predetermined antigen, ii) a transmembrane domain, and iii) an intracellular segment comprising one or more intracellular signaling domains including a cytoplasmic domain of an interleukin receptor chain and optionally a supplementary cytoplasmic domain.

The cytoplasmic domain of an IL receptor chain may be selected from any chain of the IL receptor described in the present specification. The whole region of the cytoplasmic domain of the IL receptor chain may be used. Alternatively, a truncated fragment of the cytoplasmic domain of the IL receptor chain may also be used. Examples of the full length and the truncated fragment thereof are given in the present specification.

In an embodiment, the truncated fragment may comprise at least one tyrosine kinase association motif (also known as a box-1 motif) and a STAT (signal transducer and activator of transcription) association motif described in the present specification. The truncated fragment comprises, for example, up to 250 amino acids of the ILR cytoplasmic domain, or is 50 to 200 amino acids or 80 to 150 amino acids of the ILR cytoplasmic domain.

The STAT association motif of the IL-2R beta chain comprises tyrosine residue-510 (tyrosine residue 510 is amino acid 536 of NCBI RefSeq: NP_000869.1). In an embodiment, the STAT association motif comprises tyrosine residue 510 and 4 residues flanking at the C-terminal side of tyrosine residue 510, i.e., YLSLQ.

Other STAT association motifs are also known and include, for example, YXXQ, optionally YXPQ, of IL-6, YXXQ of IL-10, YLPSNID of IL-12, YLSLQ, YCTFP and YFFFH of IL-2, YVTMS of IL-7, YLPQE of IL-9, and YKAFS and YKPFQ of IL-4. Any of the STAT signaling domains may be used and/or can be introduced into the ILR chain.

In an embodiment, the intracellular segment of the chimeric receptor comprises at least one supplementary signaling domain other than those present in the IL receptor, in addition to the cytoplasmic domain of the IL receptor. Examples of the intracellular signaling domain include a cytoplasmic region derived from a TCR complex and/or a costimulatory molecule, and any mutant having the same functions as those of these sequences. Other examples thereof include cytoplasmic signal transduction domains listed in Table 2 of Sadelain et al., 2009, which is incorporated herein by reference.

The present disclosure includes a chimeric receptor comprising an intracellular segment comprising one or more, for example, 2 or 3 intracellular signaling domains in addition to the cytoplasmic domain of the IL receptor. The chimeric receptor comprises, for example, a cytoplasmic domain of an IL receptor and an intracellular signaling domain of CD3 zeta. The chimeric receptor comprises, for example, a cytoplasmic domain of an IL receptor, an intracellular signaling domain of CD3 zeta and a cytoplasmic costimulatory domain of CD28.

In an embodiment, the chimeric receptor comprises an intracellular segment comprising a CD3 zeta intracellular signaling domain, and one or more cytoplasmic costimulatory domains, wherein the intracellular segment comprises a JAK binding motif, a STAT5 and/or STAT3 association motif.

### Transmembrane domain and spacer domain

The chimeric receptor of the present disclosure comprises a transmembrane domain. The transmembrane domain may be derived from a natural polypeptide or may be artificially designed. The transmembrane domain derived from a natural polypeptide can be obtained from a membrane-associated or transmembrane protein. For example, a transmembrane domain of a T cell receptor alpha or beta chain, a CD3 zeta chain, CD28, CD3 epsilon, CD45, CD4, CD5, CD8, CD9, CD16, CD22, CD33, CD37, CD64, CD80, CD86, CD134, CD137, ICOS, CD154, or GITR can be used. The artificially designed transmembrane domain is a polypeptide mainly comprising hydrophobic residues such as leucine and valine. For example, a triplet of phenylalanine, tryptophan and valine can be found at each end of the synthetic transmembrane domain. Optionally, a short-chain oligopeptide linker or a polypeptide linker, for example, a linker having a length of 2 to 10 amino acids can be arranged between the transmembrane domain and the intracellular segment as described in the present specification. Particularly, a linker sequence having a glycine-serine continuous sequence can be used.

The transmembrane domain used can be, for example, a transmembrane domain having any of sequences from amino acids 153 to 179 of CD28 (NCBI RefSeq: NP_006130.1).

In the chimeric receptor of the present disclosure, a spacer domain can be arranged between the extracellular domain and the transmembrane domain, or between the intracellular segment and the transmembrane domain. The spacer domain means any oligopeptide or polypeptide that works to link the transmembrane domain to the extracellular domain and/or the transmembrane domain to the intracellular segment. The spacer domain comprises up to 300 amino acids, for example, approximately 10 to 100 amino acids, or approximately 25 to 50 amino acids.

The spacer domain preferably has a sequence that promotes the binding of the chimeric receptor to an antigen via the antigen binding molecule after cleavage of the linker and enhances signal transduction into cells. Examples of the amino acid that is expected to promote the binding include cysteine, charged amino acids, and serine and threonine within a potential glycosylation site. These amino acids can be used as amino acids constituting the spacer domain.

In an embodiment, the spacer domain is a polypeptide comprising or consisting of amino acids 118 to 178 of CD8 alpha (NCBI RefSeq: NP_001759.3), i.e., a hinge region of CD8 alpha, amino acids 135 to 195 of CD8 beta (GenBank: AAA35664.1), amino acids 315 to 396 of CD4 (NCBI RefSeq: NP_000607.1), amino acids 114 to 152 of CD28 (NCBI RefSeq: NP_006130.1), or a portion thereof. Further, the spacer domain may be an artificially synthesized sequence.

The chimeric receptor of the present disclosure can be designed so as to form a polymer, particularly, a dimer. For example, in order to polymerize (dimerize) the chimeric receptor, for example, via a disulfide bond, cysteine is inserted into the spacer domain and/or the transmembrane domain.

Further, in the chimeric receptor of the present disclosure, a signal peptide sequence can be linked to the N terminus. The signal peptide sequence resides at the N termini of many secretory proteins and membrane proteins, and has a length of 15 to 30 amino acids. Most protein molecules having an intracellular domain described in the present specification are membrane proteins, and have a signal peptide sequence. The signal peptide derived from such a secretory protein or a membrane protein can be used as the signal peptide for the chimeric receptor of the present disclosure. Any signal peptide can be used. The signal peptide can be, for example, an oncostatin M. signal peptide. The signal peptide can be derived from a human and may be derived from a non-human source, for example, insect cells or a virus. In an embodiment, the signal peptide is a human signal peptide.

### (2) Nucleic acid encoding chimeric receptor

The present disclosure provides a nucleic acid encoding the chimeric receptor described in the present specification. The nucleic acid encoding the chimeric receptor can be easily prepared from the amino acid sequence of the defined chimeric receptor by a routine method. A nucleotide sequence encoding an amino acid sequence can be obtained from NCBI RefSeq ID or GenBank accession number mentioned above for the amino acid sequence of each domain, and the nucleic acid of the present disclosure can be prepared by use of standard molecular biological and/or chemical procedures. For example, a nucleic acid can be synthesized on the basis of the nucleotide sequence, and the nucleic acid of the present disclosure can be prepared by combining DNA fragments obtained from a cDNA library through polymerase chain reaction (PCR).

The nucleic acid of the present disclosure can be linked to another nucleic acid so as to be expressed under the control of a preferred promoter. Examples of the promoter include promoters that constitutively promote the expression of a gene or an operably linked construct, and promoters that induce the expression of a gene or an operably linked construct by the action of a drug or the like (e.g., tetracycline or doxorubicin). The nucleic acid of the present disclosure can be also linked to a nucleic acid comprising other regulatory elements, for example, an enhancer sequence or a terminator sequence, which cooperate with a promoter or a transcription initiation site, in order to obtain the efficient transcription of the nucleic acid. In addition to the nucleic acid of the present disclosure, a gene capable of serving as a marker for confirming expression of the nucleic acid (e.g., a drug resistance gene, a gene encoding a reporter enzyme, or a gene encoding a fluorescent protein) may be incorporated.

In an embodiment, the nucleic acid is a codon-optimized nucleic acid for expression in a particular host.

The present disclosure provides a composition comprising the nucleic acid of the present disclosure as an active ingredient, together with a pharmaceutically acceptable excipient. Preferred pharmaceutically acceptable excipients are well known to those skilled in the art. Examples of the pharmaceutically acceptable excipient include phosphate buffered saline (e.g., 0.01 M phosphate, 0.138 M NaCl, 0.0027 M KC1, pH 7.4), aqueous solutions containing inorganic acid salts such as hydrochloride, hydrobromide, phosphate, or sulfate, saline, solutions of glycol or ethanol, and salts of organic acids such as acetate, propionate, malonate and benzoate. Adjuvants such as a wetting agent or an emulsifier, and a pH buffering agent may be used. Excipients described in Remington's Pharmaceutical Sciences (Mack Pub. Co., N.J. 1991) (which is incorporated herein by reference) can be properly used as the pharmaceutically acceptable excipient. The composition of the present disclosure can be formulated into a known form preferred for parenteral administration, for example, injection or infusion. Further, the composition of the present disclosure may contain formulation additives such as a suspending agent, a preservative, a stabilizer and/or a dispersant, and a preservative for prolonging efficacy during preservation. The composition may be in a dry form for reconstitution with an appropriate sterile liquid before use. For administration mediated by fine particles, particles such as gold particles of a microscopic size can be coated with DNA.

In the case of introducing the nucleic acid of the present disclosure into cells *ex vivo,* the nucleic acid of the present disclosure may be combined with a substance that promotes the migration of the nucleic acid into cells, for example, a reagent for introducing a nucleic acid, such as a liposome or a cationic lipid, in addition to the excipient mentioned above. Alternatively, a vector carrying the nucleic acid of the present disclosure is also useful, as mentioned later. Particularly, a composition in a form preferred for administration to a living body, containing the nucleic acid of the present disclosure carried by a preferred vector is suitable for *in vivo* gene therapy.

The composition comprising the nucleic acid of the present disclosure as an active ingredient can be administered for the treatment of, for example, a disease such as a cancer [blood cancer (leukemia), solid tumor etc.], an inflammatory disease/autoimmune disease (e.g., asthma and eczema), hepatitis, and an infectious disease, the cause of which is a virus such as influenza and HIV, a bacterium, or a fungus, for example, tuberculosis, MRSA, VRE, or deep mycosis, depending on an antigen to which the chimeric receptor encoded by the nucleic acid binds via the antigen binding molecule after cleavage of the linker. The composition comprising the nucleic acid of the present disclosure as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, or can be appropriately formulated for such administration, though the administration route is not particularly limited.

### Method for producing cell expressing chimeric receptor

A method for producing a cell expressing the chimeric receptor of the present disclosure comprises the step of introducing a nucleic acid encoding the chimeric receptor described in the present specification into a cell. This step is performed *ex vivo.* For example, a cell can be transformed *ex vivo* with a virus vector or a non-virus vector carrying the nucleic acid of the present disclosure so as to produce a cell expressing the chimeric receptor of the present disclosure.

In the method of the present disclosure, a cell derived from a mammal, for example, a human cell, or a cell derived from a non-human mammal such as a monkey, a mouse, a rat, a pig, a horse, or a dog can be used.

In one embodiment, the mammal is a human.

The present disclosure provides a chimeric receptor, a nucleic acid encoding the chimeric receptor, a cell expressing the chimeric receptor, and a composition comprising any of the foregoing. In an embodiment, one or more of the foregoing may be used in the field of adoptive gene immunotherapy targeting antigens such as tumor antigens, and/or in screening or other *in vitro* assays. The chimeric antigen receptor of the present disclosure can be introduced into cells to obtain, for example, enhancement or elevation in the expression level of the chimeric antigen receptor in the cells. Such cells are capable of exerting cytotoxic activity against cells expressing a target antigen.

One aspect provides a method for preparing a cell expressing the chimeric receptor of the present disclosure, comprising:
a) isolating an immunocyte from a mammal (optionally, the immunocyte is a T cell);
b) transfecting or transducing the isolated immunocyte (optionally, T cell) with a nucleic acid encoding the chimeric receptor described in the present specification; and
c) optionally isolating and/or culturing and expanding a chimeric receptor-expressing cell (optionally, a chimeric receptor-expressing T cell) after the transfection or the transduction.

In one embodiment, autologous T lymphocytes, autologous NK cells, or autologous macrophages are activated and/or grown *ex vivo* before re-introduction to a subject. In one embodiment, the T lymphocytes or the NK cells are allogeneic T lymphocytes or allogeneic NK cells. In one embodiment, the allogeneic T lymphocytes are T lymphocytes whose expression of an endogenous T cell receptor is blocked or eliminated. In one embodiment, allogeneic T lymphocytes or allogeneic NK cells are activated and/or grown *ex vivo* before introduction to a subject. In one embodiment, the chimeric receptor is introduced to T lymphocytes, NK cells or macrophages by a method selected from the group consisting of retroviral transduction, lentiviral transduction, DNA electroporation and RNA electroporation, DNA or RNA transfection, and genetic alteration by gene editing.

The NK cells used in the method of the present disclosure are devoid of or rarely express a major histocompatibility complex I and/or II molecule, and are capable of growing preferentially by exposure to cells genetically modified so as to express membrane-associated IL-15 and 4-1BB ligand (CDI37L). Such a cell line includes, but is not limited to, K562 [ATCC, CCL 243; Lozzio et al., Blood 45 (3): 321-334 (1975); and Klein et al., Int. J. Cancer 18: 421-431 (1976)] and Wilms' tumor cell line HFWT [Fehniger T A, Caligiuri M A. Int Rev Immunol 20 (3-4): 503-534 (2001); and Harada H, et al., Exp Hematol 32 (7): 614-621 (2004)], endometrial tumor cell line HHUA, melanoma cell line HMV-II, hepatoblastoma cell line HuH-6, lung small-cell cancer cell lines Lu-130 and Lu-134-A, neuroblastoma cell lines NB 19 and N1369, testicular NEC 14-derived embryonic cancer cell lines, neck cancer cell line TCO-2 and bone marrow-metastatic neuroblastoma cell line TNB 1[Harada H., et al., Jpn. J. Cancer Res 93: 313-319 (2002)]. Preferably, the cell line used is devoid of or rarely expresses both the MHC I and II molecules, as in the K562 and HFWT cell lines. A solid support may be used instead of the cell line. Such a support preferably is attached on its surface to at least one molecule capable of binding to NK cells and inducing an initial activation event and/or proliferative response or capable of binding a molecule having such an affect, thereby acting as a scaffold. The support may be attached on its surface to CD137 ligand protein, an anti-CD 137 antibody, IL-15 protein or an anti-IL-15 receptor antibody. Preferably, the support has an anti-IL-15 receptor antibody and an anti-CD137 antibody attached on its surface.

In one embodiment, in any method of the present disclosure involving T lymphocyte activation, T lymphocytes can be activated in the presence of one or more agents selected from the group consisting of anti-CD3/CD28, IL-2 and phytohemagglutinin. In any method of the present disclosure involving NK cell activation, NK cells can be activated in the presence of one or more agents selected from the group consisting of CD137 ligand protein, anti-CD137 antibody, IL-15 protein, an anti-IL-15 receptor antibody, IL-2 protein, IL-12 protein, IL-21 protein and a K562 cell line.

The cell used in the method of the present disclosure is not particularly limited, and any cell can be used. For example, a cell collected, isolated, or purified from a body fluid, a tissue or an organ, for example, blood (peripheral blood, umbilical cord blood etc.) or bone marrow, or a cell obtained by differentiating or reprogramming (in order to prepare induce pluripotent stem cells (iPSCs)) the cell mentioned above can be used (see e.g., Themeli et al., 2013). A peripheral blood mononuclear cell (PBMC), an immune cell [including, for example, a T cell, a dendritic cell, a B cell, a hematopoietic stem cell, a macrophage, a monocyte, a NK cell or a hematopoietic cell (a neutrophil or a basophil)], an umbilical cord blood mononuclear cell, a fibroblast, a precursor adipocyte, a hepatocyte, a skin keratinocyte, a mesenchymal stem cell, an adipose stem cell, various cancer cell lines, or a neural stem cell can be used. For example, a NK cell or a T cell, a precursor cell of a T cell (a hematopoietic stem cell, a lymphocyte precursor cell etc.) or a cell population containing these cells can be used. Examples of the T cell include CD8-positive T cells, CD4-positive T cells, regulatory T cells, cytotoxic T cells, and tumor infiltrating lymphocytes. The cell population containing a T cell and a precursor cell of a T cell includes PBMCs. These cells may be collected from a living body, may be obtained by culturing and expanding cells collected from a living body, or may be established as a cell line. If the transplantation of the produced chimeric receptor-expressing cell or a cell differentiated from the produced chimeric receptor-expressing cell into a living body is desired, the nucleic acid can be introduced into a cell collected from the living body itself or a conspecific living body thereof.

In conjunction with the polynucleotide, the present disclosure also provides a vector comprising such a polynucleotide (including a vector in which such a polynucleotide is operably linked to at least one regulatory element for expression of the chimeric receptor). Non-limiting examples of the useful vector of the present disclosure include virus vectors such as retrovirus vectors and lentivirus vectors.

In a particular aspect, such a vector also comprises a suicide gene. The term "suicide gene" used herein refers to a gene that causes a cell expressing the suicide gene to die. The suicide gene can be a gene that imparts sensitivity to an agent, e.g., a drug, to a cell in which the gene is expressed, and causes the cell to die when the cell is contacted with or exposed to the agent. The suicide gene is known in the art (see e.g., Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and includes, for example, herpes simplex virus (HSV) thymidine kinase (TK) gene, and genes of cytosine deaminase, purine nucleoside phosphorylase, nitroreductase and caspases such as caspase 8.

The nucleic acid encoding the chimeric receptor of the present disclosure can be introduced to a vector, and the vector can be introduced into cells. For example, a virus vector such as a retrovirus vector (including an oncoretrovirus vector, a lentivirus vector, and a pseudotyped vector), an adenovirus vector, an adeno-associated virus (AAV) vector, a simian virus vector, a vaccinia virus vector or a Sendai virus vector, an Epstein-Barr virus (EBV) vector, and a HSV vector can be used. For example, a virus vector devoid of replicating ability so as not to self-replicate in infected cells can be used.

In addition, a non-virus vector can also be used in the present disclosure in combination with a liposome or a condensing agent such as a cationic lipid, as described in WO96/10038, WO97/18185, WO97/25329, WO97/30170 and WO97/31934 (which are incorporated herein by reference). The nucleic acid of the present disclosure may be introduced into cells by calcium phosphate transduction, DEAE-dextran, electroporation, or particle bombardment.

In the case of using, for example, a retrovirus vector, the method of the present disclosure can be performed by selecting a suitable packaging cell based on a LTR sequence and a packaging signal sequence that is possessed by the vector, and preparing a retrovirus particle using the packaging cell. Examples of the packaging cell include PG13 (ATCC CRL-10686), PA317 (ATCC CRL-9078), GP+E-86 and GP+envAm-12 (U.S. Patent No. 5,278,056), and Psi-Crip [Proceedings of the National Academy of Sciences of the United States of America, vol. 85, pp. 6460-6464 (1988)]. The retrovirus particle may be prepared using a 293 cell or a 293T cell having high transfection efficiency. Many types of retrovirus vectors produced on the basis of retrovirus and packaging cells that can be used for packaging of retrovirus vectors are widely commercially available from many companies.

The present disclosure also provides a host cell comprising the chimeric receptor. Non-limiting examples of the useful host cell include T lymphocytes and NK cells, which may be autologous or allogeneic (whose endogenous T cell receptor is removed or maintained). In a certain aspect, the host cell is an autologous T lymphocyte isolated from a patient having a cancer. In a particular aspect, such an autologous T lymphocyte is activated and grown *ex vivo.*

The chimeric receptor of the present disclosure can be introduced into the host cell by any method known in the art. Non-limiting examples of the particularly useful method include retroviral transduction, lentiviral transduction and DNA and mRNA electroporation. As shown in Examples below, mRNA electroporation causes effective expression of the chimeric receptor of the present disclosure in T lymphocytes. Examples of references describing the retroviral transduction include Anderson et al., U.S. Patent No. 5,399,346; Mann et al., Cell 33: 153 (1983); Temin et al., U.S. Pat. No. 4,650,764; Temin et al., U.S. Patent No. 5,124,263; Dougherty et al., International Publication No. WO 95/07358 (published on March 16, 1995); and Kuo et al., Blood 82: 845 (1993). International Publication No. WO 95/07358 describes high efficiency transduction of primary B lymphocytes. For example, for specific techniques of retroviral transduction and mRNA electroporation that can be used, see also the Examples section below.

Host cell activation and growth can usually be used to attain integration of a virus vector into the genome and expression of the gene encoding the chimeric receptor of the present disclosure. However, provided that mRNA electroporation is used, neither activation nor growth is required (though electroporation is more effective when carried out on activated cells). As a result of viral transduction, the host cell (T lymphocyte or NKT cell) expresses the chimeric receptor of the present disclosure for a long period while potentially producing a stronger effect than that upon mRNA electroporation when the receptor is transiently expressed (typically for 3 to 5 days). However, the viral transduction is complicated, expensive and difficult to carry out, whereas the mRNA electroporation is much simpler and much easier to carry out. Further, the transient expression is useful if there is potential toxicity and should be helpful in the initial phases of clinical trials for possible adverse reactions.

One aspect is a method of preparing the cell disclosed in the present specification, comprising transfecting or transducing a cell with the nucleic acid or the vector described in the present specification.

In one embodiment, isolated immune cells are isolated T cells.

In an embodiment, isolated cells are CD3⁺ and are optionally stimulated with an anti-CD3 antibody, optionally in a soluble or membrane-bound form, for example, OKT3 or mOKT3, and/or with APC before transduction or transfection. In one embodiment, the APC are artificial APC (aAPC). In another embodiment, the APC expresses a membrane form of an anti-CD3 monoclonal antibody.

In one embodiment, the transfection or transduction step is repeated. For example, the transfection or transduction step can be performed twice, or three times, or four times, or until, for example, an adequate level of expression is achieved. The transfection or transduction step can be performed, for example, five times.

In one embodiment, cells are transfected or transduced for two or more consecutive days. Cells are transfected or transduced, for example, for two consecutive days, three consecutive days, or four consecutive days.

In one embodiment, the chimeric receptor-transduced cells are stimulated with irradiated cells expressing a predetermined antigen. The chimeric receptor-transduced cells are stimulated with irradiated cells, for example, at an effector:target ratio of 100:1, 75:1, 50:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:50 or 1:100. 1:1 is preferred.

### Cell expressing chimeric receptor and use thereof

The cell expressing the chimeric receptor of the present disclosure is a cell allowed to harbor or express a nucleic acid encoding the chimeric receptor described in the present specification by the production method described in the present specification.

The cell of the present disclosure binds to a particular antigen via the chimeric receptor. Signals are thereby transduced into the cell, and as a result, the cell is activated. The activation of the cell expressing the chimeric receptor differs depending on the type of host cells and the intracellular domains of the chimeric receptor, and can be confirmed on the basis of, for example, cytokine release, improvement in cell growth rate, change in cell surface molecule, as an index. For example, the release of a cytotoxic cytokine (a tumor necrosis factor, lymphotoxin, etc.) from the activated cell causes destruction of target cells expressing an antigen. In addition, the cytokine release or the change in cell surface molecule stimulates other immunocytes, for example, B cells, dendritic cells, NK cells, and macrophages.

The T lymphocytes used in the method of the present disclosure are most preferably patient's own cells (i.e., autologous cells) that are isolated in advance from a blood sample and preferably activated and grown *ex vivo* (e.g., for 3 to 5 days) by use of a standard method, for example, anti-CD3/CD28 beads, IL-2 or phytohemagglutinin. Alternatively, allogeneic T lymphocytes can be used (preferably allogeneic T lymphocytes whose expression of an endogenous T cell receptor is blocked or eliminated). See Torikai et al., Blood, 2012 119: 5697-5705. T lymphocytes and NK cells thus isolated (and, if desired, activated and/or grown) from a patient are transduced (or electroporated) with a polynucleotide encoding the chimeric receptor of the present disclosure (or a vector comprising such a polynucleotide) so that the chimeric receptor is expressed on the cell surface of the T cells or the NK cells. The modified cells can then be administered to the patient (e.g., 1 day after drip infusion of a therapeutic antibody).

One aspect provides use of the chimeric receptor-expressing cell, the nucleic acid, the vector, the cell or the composition described in the present specification for treating a disease.

Another aspect is a method for treating or preventing a disease in a mammal, comprising administering an effective amount of the cell or the composition disclosed in the present specification to the mammal in need thereof.

A therapeutic drug comprising the chimeric receptor-expressing cell as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, though the administration route is not limited.

A further aspect is a method comprising integrating a nucleic acid encoding the chimeric receptor of the present disclosure into a living body using a virus vector or the like, and directly expressing the chimeric receptor. Examples of the virus vector include, but are not limited to, adenovirus vectors. Alternatively, the nucleic acid encoding the chimeric receptor may be integrated directly into a living body by electroporation, an approach of directly administering the nucleic acid, or the like without the use of the virus vector, and the chimeric receptor may be intermittently secreted in a living body by administering cells genetically altered so as to secrete and express the chimeric receptor to the living body.

According to the present disclosure, patients can be treated by a procedure which involves infusing a therapeutically effective dose of T lymphocytes or NK cells comprising the chimeric receptor of the present disclosure in the range of approximately 10⁵ to 10¹⁰ or more cells per kilogram body weight (cells/kg). The infusion can be repeated as frequently and as many times as possible as long as the patients can tolerate until the desired response is achieved. The appropriate infusion dosage and schedule may differ among patients, but can be determined by a physician who treats a particular patient. Typically, an initial dose of approximately 10⁶ cells/kg is infused and increased to 10⁸ or more cells/kg. IL-2 can also be used in combination therewith for the post-infusion growth of the infused cells. The amount of IL-2 can be approximately 1 to 5 × 10⁶ international units per square meter of body surface.

The therapeutic drug comprises the cell expressing the chimeric receptor as an active ingredient and may further comprise a preferred excipient. Examples of the excipient include the pharmaceutically acceptable excipients mentioned above for the composition comprising the nucleic acid of the present disclosure as an active ingredient, various cell culture media, and isotonic sodium chloride.

Pharmaceutical composition of present disclosure

A further aspect of the present disclosure provides a pharmaceutical composition. In one aspect, the present disclosure provides a pharmaceutical composition comprising (i) a polynucleotide encoding the chimeric receptor of the present disclosure or a vector comprising such a polynucleotide and (ii) a pharmaceutically acceptable carrier or additive.

Appropriate additives for use in the pharmaceutical composition of the present disclosure are well known to those skilled in the art and can include, for example, a tissue culture medium (e.g., for the *ex vivo* survival of cells) or an aqueous salt solution (e.g., upon injection of cells to patients). Detailed description on the pharmaceutically acceptable additives is available from Remington's Pharmaceutical Sciences (Mack Pub. Co., N.J. 1991).

The disease against to which the pharmaceutical composition of the present invention is administered is not particularly limited as long as the disease exhibits sensitivity to the therapy used. In one embodiment, the disease is a cancer. In one embodiment, the subject is suspected of having a cancer, or has a cancer.

The cancer is, for example, blood cancer or solid tumor. The blood cancer is, for example, leukemia, lymphoma or myeloma. The solid tumor is, for example, cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, and germ cell tumor.

In an embodiment, the disease is an inflammatory disease/autoimmune disease (e.g., asthma and eczema), hepatitis, and an infectious disease, the cause of which is a virus such as influenza and HIV, a bacterium, or a fungus, for example, tuberculosis, MRSA, VRE, or deep mycosis. In one embodiment, the subject is suspected of having such an inflammatory disease, or has such an inflammatory disease.

The pharmaceutical composition of the present disclosure binding to an antigen possessed by cells desired to be reduced or eliminated for the treatment of the diseases mentioned above, i.e., a tumor antigen, a viral antigen, a bacterial antigen, or the like, is administered for the treatment of these diseases.

Accordingly, one aspect includes a method for decreasing the number of cells expressing a predetermined antigen in a subject, comprising administering an effective amount of the pharmaceutical composition described in the present specification to the subject in need thereof, wherein the chimeric receptor-expressing cell specifically binds to the predetermined antigen via the antigen binding molecule after cleavage of the linker, binding to the extracellular binding domain.

The cell of the present disclosure can also be utilized for the prevention of an infectious disease after bone marrow transplantation or exposure to radiation, donor lymphocyte transfusion for the purpose of remission of recurrent leukemia, and the like.

The chimeric receptor of the present disclosure imparts antibody-dependent cellular cytotoxicity to T lymphocytes and enhances antibody-dependent cellular cytotoxicity (ADCC) in NK cells. The binding of the receptor through an antibody (or another antitumor molecule comprising an Fc moiety) that binds to tumor cells triggers T cell activation, sustained proliferation and specific cytotoxicity against cancer cells targeted by the antibody (or such another antitumor molecule comprising an Fc moiety).

The chimeric receptor of the present disclosure promotes T cell therapy that allows a single receptor to be used for diverse cancer cell types. This strategy can be eventually advantageous when an immune escape mechanism exploited by tumor is taken into consideration. At the same time, diverse antigens can be targeted (Grupp et al., N. Engl. J. Med. 2013; 368 (16):1509-1518). Antibody-mediated cytotoxicity can be stopped anytime by the mere discontinuation of antibody administration. Since T cells expressing the chimeric receptor of the present disclosure are activated only by an antibody bound to target cells, unbound immunoglobulins do not exert any stimulation into the infused T cells. Clinical safety can be further enhanced by using mRNA electroporation for the transient expression of the chimeric receptor in order to limit any potential autoimmune reactivity.

In one aspect of the method, the T lymphocytes or the NK cells are autologous T lymphocytes or NK cells isolated from the subject. In a particular aspect, the autologous T lymphocytes or NK cells are activated and/or grown *ex vivo* before re-introduction to the subject. In another aspect, the T lymphocytes or the NK cells are allogeneic T lymphocytes or NK cells. In a certain aspect, the T lymphocytes are allogeneic T lymphocytes whose expression of an endogenous T cell receptor is blocked or eliminated. In a particular aspect, the allogeneic T lymphocytes are activated and/or grown *ex vivo* before introduction to the subject. The T lymphocytes can be activated by any method known in the art in the presence of, for example, anti-CD3/CD28, IL-2 and/or phytohemagglutinin. The NK cells can be activated by any method known in the art in the presence of one or more agents selected from the group consisting of, for example, CD137 ligand protein, anti-CD137 antibody, IL-15 protein, an anti-IL-15 receptor antibody, IL-2 protein, IL-12 protein, IL-21 protein and a K562 cell line. For example, for description on useful methods for growing NK cells, see U.S. Patent Nos. 7,435,596 and 8,026,097.

In one aspect of the method, following introduction (or re-introduction) of the T lymphocytes or the NK cells to the subject, a therapeutically effective amount of a PD-1/PD-L1 signal inhibitor and/or a VEGF signal inhibitor is administered to the subject.

### Combination treatment of present disclosure

The composition and the method described in the present specification may be used in combination with another type of treatment for a cancer, such as chemotherapy, surgery, radiation, or gene therapy. Such treatment can be applied concurrently or sequentially (in any order) with the immunotherapy according to the present disclosure.

In combined use with an additional therapeutic agent, an appropriate therapeutically effective dose of each agent can be decreased owing to additive or synergistic effects.

The treatment of the present disclosure can be combined with, for example, another immunomodulatory treatment such as a therapeutic vaccine (including, but not limited to, GVAX, DC vaccines, etc.), a checkpoint inhibitor (including, but not limited to, agents blocking CTLA4, PD1, LAG3, TIM3, etc.) or an activator (including, but not limited to, agents enhancing 41BB, OX40, etc.).

The pharmaceutical composition of the present disclosure may also comprise one or more additional active compounds, preferably compounds having complementary activity without adversely affecting each other, if necessary for a particular indication to be treated. Non-limiting examples of the possible additional active compounds include PD-1/PD-L1 signal inhibitors and VEGF signal inhibitors.

In another aspect, the pharmaceutical composition of the present disclosure further comprises a monoclonal antibody (e.g., rituximab, trastuzumab, or hul4.18K322A) that can exert cytotoxicity against cancer cells, or another antitumor molecule comprising an Fc moiety (e.g., a composite molecule comprising a ligand (e.g., a cytokine or an immune cell receptor) binding to a tumor surface receptor combined with an immunoglobulin Fc moiety or Fc-containing DNA or RNA).

An appropriate dose of the antibody used depends on the type of cancer to be treated, the severity and course of the disease, previous treatment, patient's clinical history and response to the antibody, and the discretion of the treating physician. The antibody can be administered to the patient at once or over a series of treatments. The progress of the treatment according to the present disclosure can be easily monitored by a conventional technique and assay.

The administration of the antibody can be carried out by any appropriate route including systemic administration and direct administration to a site of the disease (e.g., to primary tumor).

Non-limiting examples of the additional therapeutic agent useful for combination with the immunotherapy of the present disclosure include the following:
(i) anti-angiogenic agents (e.g., TNP-470, platelet factor 4, thrombospondin-1, tissue inhibitors of metalloproteases (TIMP1 and TIMP2), prolactin (16 kD fragment), angiostatin (38 kD fragment of plasminogen), endostatin, bFGF soluble receptor, transforming growth factor beta, interferon alpha, soluble KDR and FLT-1 receptor, placental proliferin-related protein, and those described in Carmeliet and Jain (2000));
(ii) VEGF antagonists or VEGF receptor antagonists such as anti-VEGF antibodies, VEGF variants, soluble VEGF receptor fragments, aptamers capable of blocking VEGF or VEGFR, neutralizing anti-VEGFR antibodies, inhibitors of VEGFR tyrosine kinase and any combination thereof;
(iii) chemotherapeutic compounds such as pyrimidine analogs (e.g., 5-fluorouracil, floxuridine, capecitabine, gemcitabine and cytarabine), purine analogs, folate antagonists and related inhibitors (e.g., mercaptopurine, thioguanine, pentostatin and 2-chlorodeoxyadenosine (cladribine)); antiproliferative/antimitotic agents including natural products such as vinca alkaloids (e.g., vinblastine, vincristine, and vinorelbine), microtubule disruptors such as taxane (e.g., paclitaxel and docetaxel), vincristine, vinblastine, nocodazole, epothilones and navelbine, epipodophyllotoxin (e.g., etoposide and teniposide), and DNA damaging agents (e.g., actinomycin, amsacrine, anthracyclines, bleomycin, busulfan, camptothecin, carboplatin, chlorambucil, cisplatin, cyclophosphamide, Cytoxan, dactinomycin, daunorubicin, doxorubicin, epirubicin, hexamethylmelamine, oxaliplatin, ifosfamide, melphalan, mechlorethamine, mitomycin, mitoxantrone, nitrosourea, plicamycin, procarbazine, taxol, taxotere, teniposide, triethylene thiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (e.g., L-asparaginase which systemically metabolizes L-asparagine and removes cells lacking the ability to synthesize their own asparagine); antiplatelet agents; antiproliferative/antimitotic alkylating agents such as nitrogen mustards (e.g., mechlorethamine, cyclophosphamide and analogs, melphalan, and chlorambucil), ethylenimines and methylmelamines (e.g., hexamethylmelamine and thiotepa), alkyl sulfonates-busulfan, nitrosoureas (e.g., carmustine (BCNU) and analogs, and streptozocin), and trazenes-dacarbazinine (DTIC); antiproliferative/antimitotic antimetabolites such as folic acid analogs (e.g., methotrexate); platinum coordination complexes (e.g., cisplatin and carboplatin), procarbazine, hydroxyurea, mitotane, and aminoglutethimide; hormones, hormone analogs (e.g., estrogen, tamoxifen, goserelin, bicalutamide, and nilutamide) and aromatase inhibitors (e.g., letrozole and anastrozole); anticoagulants (e.g., heparin, synthetic heparin salts and other thrombin inhibitors); fibrinolytic agents (e.g., tissue plasminogen activator, streptokinase and urokinase), aspirin, dipyridamole, ticlopidine, clopidogrel, and abciximab; antimigratory agents; antisecretory agents (e.g., brefeldin); immunosuppressive agents (e.g., cyclosporine, tacrolimus (FK-506), sirolimus (rapamycin), azathioprine, and mycophenolate mofetil); anti-angiogenic compounds (e.g., TNP-470, genistein, and bevacizumab) and growth factor inhibitors (e.g., fibroblast growth factor (FGF) inhibitors); angiotensin receptor blockers; nitric oxide donors; anti-sense oligonucleotides; antibodies (e.g., trastuzumab); cell cycle inhibitors and differentiation inducers (e.g., tretinoin); mTOR inhibitors, topoisomerase inhibitors (e.g., doxorubicin (adriamycin), amsacrine, camptothecin, daunorubicin, dactinomycin, teniposide, epirubicin, etoposide, idarubicin and mitoxantrone, topotecan, and irinotecan), corticosteroids (e.g., cortisone, dexamethasone, hydrocortisone, methylprednisolone, prednisone, and prednisolone); growth factor signaling kinase inhibitors; mitochondrial dysfunction inducers and caspase activators; and chromatin disruptors.

For further examples of useful agents, see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

The above disclosure generally describes the present application. More complete understanding can be obtained by reference to specific examples given below. These examples are described merely for the purpose of illustration and are not intended to limit the scope of the present application. Change in form and substitution of equivalents are also contemplated as circumstances might suggest or render expedient. Although particular terms are used in the present disclosure, such terms are intended in a descriptive sense and not for purposes of limitation.

All references cited herein are incorporated herein by reference.

### EXAMPLES

Hereinafter, the present invention will be described in more detail with reference to Examples. However, these Examples do not limit the scope of the present invention.

### [Example 1] Concept of antibody technology using antibody that is specifically cleaved at lesion site and antibody recognizing the cleavage

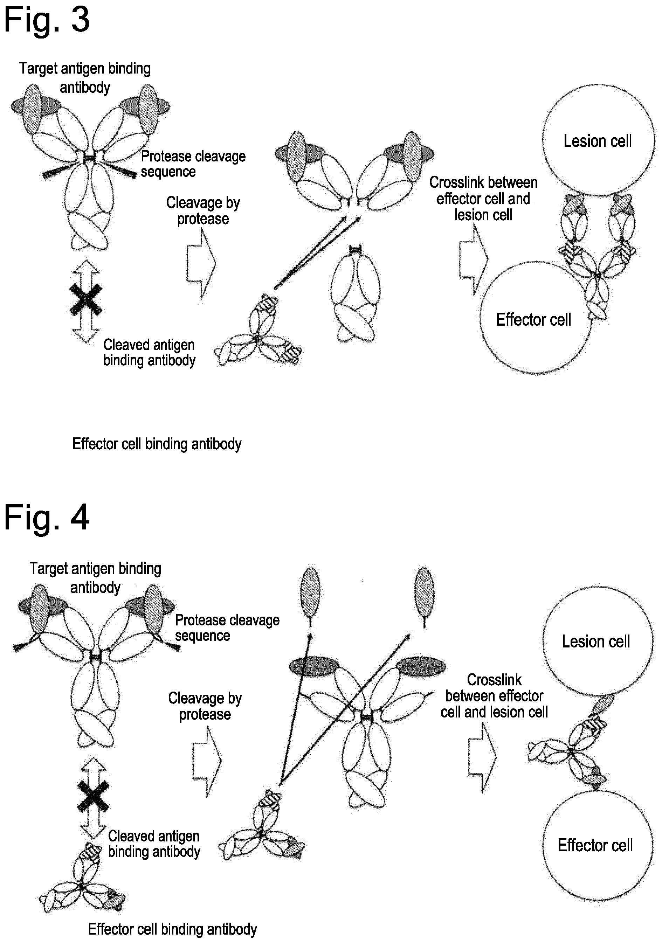

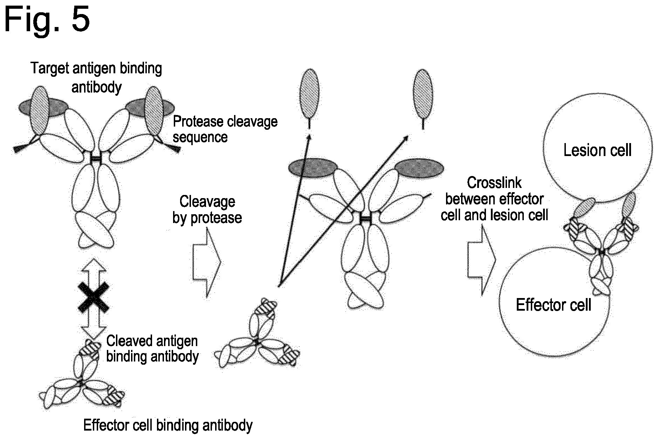

Figure 1 shows existing antibody technology (Probody(R)) of expanding tissue specificity and therapeutic window by imparting sensitivity to protease having an expression level elevated at a lesion site such as a cancer tissue or an inflammatory tissue to an antibody. The Probody(R) is a molecule in which an antibody is connected to a masking peptide that masks the antigen binding site of the antibody and a peptide sequence that is cleavable by protease expressed at a lesion site (Sci Transl Med. 2013 Oct 16; 5 (207): 207ra144). The antigen binding site of the antibody is masked by the masking peptide and cannot thereby bind to the antigen, in an uncleaved state of the peptide sequence. The cleavage peptide sequence of the Probody(R) is cleavable by protease expressed at a target pathological site so that the masking peptide is dissociated to produce an antibody molecule having antigen binding activity, which is in turn capable of binding to the antigen specific for the target pathological tissue. On the other hand, the problems of the Probody(R) are disclosed in International Publication Nos. WO2018/097307 and WO2018/097308. In order to solve these problems, the present inventors devised a group of versatile molecules which were molecules comprising an activated antibody that was produced by protease and having a short half-life in blood, and exerted no pharmacological effect without cleavage by the protease (Figures 2 to 5).

In a method for preparing such a group of molecules, antibodies binding to a target antigen (primary molecules; antigen binding molecules) as well as two groups of molecules, i.e., secondary molecules activating effector cells, such as T cell-redirecting antibodies and antibodies mediating ADCC activity or chimeric receptors, are first obtained. An antigen binding to a target antigen and harboring a protease-cleavable linker having a sequence that is cleavable by protease specific for a lesion site is used as a primary molecule. Meanwhile, the ability to bind to the antigen binding molecule obtained through the cleavage of the linker by the protease is imparted to the antibody or the chimeric receptor (secondary molecule) activating effector cells. These two molecules do not bind to each other in an uncleaved state of the linker. Hence, in normal tissues, neither are the effector cells bridged to the target antigen nor activation occurs. On the other hand, when the linker is cleaved by the protease, the antigen binding molecule obtained through the cleavage of the linker binds to the secondary molecule to form a complex. The complex bridges the target antigen to the effector cells in the presence of the antigen so that the effector cells are activated. The primary molecule that is cleavable by protease may be a variant (e.g., an IgG antibody-like molecule and a heavy chain antibody) fused with a single domain antibody such as VHH or may be an IgG antibody harboring a cleavable linker. The binding of the secondary molecule to the antigen binding molecule obtained through the cleavage of the linker may be recognized at the end of a peptide resulting from the cleavage or may be recognized at the exposed molecular interface of the antigen binding molecule obtained through the cleavage of the linker. Possible application examples of this scheme are therapy using antibodies that exert ADCC or TDCC activity depending on both protease and a target antigen highly expressed at a lesion site, CAR-T cells, ADC (antibody drug conjugate), immunocytokines, oncolytic viruses, virus vectors for gene treatment, or the like.

In the present invention, when the antigen binding molecule is an antibody or an IgG antibody-like molecule, the antigen binding molecule before protease cleavage has a long circulation time in blood. On the other hand, the antigen binding molecule obtained through the cleavage of the linker is rapidly metabolized when the salvage mechanism mediated by Fc-FcRn interaction is inhibited by the removal of Fc through the cleavage of the linker. In fact, an antibody fragment (Fab) has half the half-life in blood of full-length IgG (Toxicol Pathol 1999 Sep-Oct (5): 536-44.), and the half-life in blood of VHH is 80 minutes (Br J Pharmacol. 2010 Jun; 160 (4): 1016-28). Hence, there is low possibility that the antigen binding molecule obtained through the cleavage of the linker circulates and acts on an antigen expressed in normal tissues to activate effector cells. Furthermore, the secondary molecule activating effector cells does not bind to the uncleaved primary molecule and thus has no risk of causing adverse reactions even at the time of high-concentration administration.

### [Example 2] Preparation of antibody having protease-cleavable linker and antibody binding to antigen binding molecule obtained through cleavage of linker of the antibody

### (2-1) Expression and purification of antibody harboring linker that is cleavable by protease

A nucleotide sequence encoding an antibody harboring a linker that is cleavable by protease was prepared by a method known in the art using primers encoding the linker. More specifically, a partial sequence of the antibody for cleavable linker insertion was added to a DNA sequence encoding a portion or the whole of the cleavable linker to prepare 3' and 5' PCR primers. By using the nucleotide sequence of the antibody that is subjected to the protease-cleavable linker insertion as a templateon, PCR reaction was performed on the DNA at 5' and 3' sides of the nucleotide sequence. Next, the obtained PCR products were inserted to expression vectors known in the art using In-Fusion(R) HD Cloning Kit (Clontech Laboratories, Inc.) or the like. A human embryonic kidney cell-derived FreeStyle 293-F line or Expi293 line (Invitrogen Corp.) was cultured by the method designated by the manufacturer, and a plasmid encoding the nucleotide sequence was introduced to the cells by lipofection. The cells were cultured for 4 days in a CO₂ incubator (37°C, 8% CO₂, 90 rpm), and an antibody comprising an Fc region was purified from the culture supernatant by a method known to those skilled in the art using rProtein A Sepharose(TM) Fast Flow (Amersham Biosciences Corp.). The absorbance of the purified antibody solution was measured at 280 nm using a spectrophotometer. The concentration of the purified antibody was calculated using an absorption coefficient calculated by PACE from the obtained measurement value (Protein Science 1995; 4: 2411-2423).

### (2-2) Expression and purification of bispecific antibody specifically binding to an antigen binding molecule whose linkage is cleavable, and human CD3

Antibody arm 20A10H-F760nN17/20A10L-k0MT binding to the end of collagen II peptide cleavable by MMP13 or the like (WO2011/034128) was prepared by coexpressing heavy chain 20A10H-F760nN17 having heavy chain variable region 20A10H (SEQ ID NO: 726) and heavy chain constant region F760nN17 (SEQ ID NO: 727) in combination with light chain 20A10L-k0MT having light chain variable region 20A10L (SEQ ID NO: 728) and light chain constant region k0MT (SEQ ID NO: 729) in combination.

Antibody arm 20952H-F760nN17/20952L-k0MTC binding to the end of human IgG1 peptide cleavable by IdeS (WO2012/067624) was prepared by coexpressing heavy chain 20952H-F760nN17 having heavy chain variable region 20952H (SEQ ID NO: 730) and heavy chain constant region F760nN17 (SEQ ID NO: 727) in combination with light chain 20952L-k0MTC having light chain variable region 20952L (SEQ ID NO: 731) and light chain constant region k0MTC (SEQ ID NO: 746) in combination.

Anti-human CD3 antibody arm TRO1H113-F760nP17/L0011-k0a was prepared by coexpressing heavy chain TR01H113-F760nP17 having heavy chain variable region TR01H113 (SEQ ID NO: 732) and heavy chain constant region F760nP17 (SEQ ID NO: 733) in combination with light chain L0011-k0a having light chain variable region L0011 (SEQ ID NO: 734) and light chain constant region k0a (SEQ ID NO: 729) in combination. A constant region that exhibited reduced binding to an Fcγ receptor and was altered so as to attain hetero-association of two heavy chains was used as the constant regions F760nN17 and F760nN17 of these antibody arms.

20A10H-F760nN17/20A10L-k0MT, 20952H-F760nN17/20952L-k0MTC, and TR01H113-F760nP17/L0011-k0a were each expressed and purified by the method shown in Example 3-2. These antibodies were mixed by a method known to those skilled in the art that utilized difference in charge among their constant regions (Proc. Natl. Acad. Sci., 110, 5145-5150, 2013), to prepare bispecific antibodies (20A10H-F760nN17/20A10L-k0MT//TR01Hl 13-F760nP17/L0011-k0a, 20952H-F760nN17/20952L-k0MTC//TR01H113-F760nP17/L0011-k0a, and H0000-F760nN17/GL4-k0a//TR01H1 13-F760nP17/L0011-k0a, respectively) against the antigen binding molecule obtained through the protease cleavage of the linker and human CD3.

Antibodies in the present specification were designated according to the following rule: (heavy chain variable region)-(heavy chain constant region)/(light chain variable region)-(light chain constant region). For example, antibody name 20A10H-F760nN17/20A10L-k0MT means that this antibody has heavy chain variable region 20A10H, heavy chain constant region F760nN17, light chain variable region 20A10L, and light chain constant region k0MT. Hetero-associates constituted by two different antibodies are indicated by the individual antibodies with // inserted between their names. For example, 20A10H-F760nN17/20A10L-k0MT//TR01H113-F760nP17/L0011-k0a means that this antibody has 20A10H-F760nN17/20A10L-k0MT as one of the antibody arms and TR01H113-F760nP17/L0011-k0a as the other antibody arm.

### (2-3) Expression and purification of antibody with enhanced ADCC activity specifically binding to antigen binding molecule obtained through protease cleavage of linker

Antibody 20A1 0H_Kn125/20A1 0L-k0MT//20A10H_H1076/20A10L-k0MT recognizing the end of collagen II peptide cleavable by MMP13 or the like was prepared by coexpressing heavy chain 20A10H-Kn125 having heavy chain variable region 20A10H (SEQ ID NO: 726) and heavy chain constant region Kn125 (SEQ ID NO: 735) in combination and heavy chain 20A10H-H1076 having heavy chain variable region 20A10H (SEQ ID NO: 726) and heavy chain constant region H1076 (SEQ ID NO: 736) in combination with light chain 20A10L-k0MT having light chain variable region 20A10L (SEQ ID NO: 728) and light chain constant region k0MT (SEQ ID NO: 729) in combination.

Antibody 20952H-H1076.G236E/20952L-k0MTC//20952H-Kn125/20952L-k0MTC recognizing the end of human IgGI peptide cleavable by IdeS was prepared by coexpressing heavy chain20952H-H1076.G236E having heavy chain variable region 20952H (SEQ ID NO: 730) and heavy chain constant region H1076.G236E (SEQ ID NO: 737) in combination and heavy chain 20952H-Kn125 having heavy chain variable region 20952H (SEQ ID NO: 730) and heavy chain constant region Kn125 (SEQ ID NO: 735) in combination with light chain 20952L-k0MTC having light chain variable region 20952L (SEQ ID NO: 731) and light chain constant region k0MTC (SEQ ID NO: 746) in combination.

Genes encoding these antibodies were inserted to plasmids for expression in animals, and the antibodies were expressed and purified by the method described in (2-1). These antibodies exhibited enhanced binding to human Fc□RIIIa.

The structures of the prepared antibodies are shown in the table below.

**[Table 1]**

| Antibody name | Heavy chain sequence | | Light chain sequence | |
|---|---|---|---|---|
| | Variable region | Constant region | Variable region | Constant region |
| 20A10H-F760nN17/20A10L-k0MT | SEQ ID NO: 726 | SEQ ID NO: 727 | SEQ ID NO: 728 | SEQ ID NO: 729 |
| 20952H-F760nN17/20952L-k0MTC | SEQ ID NO: 730 | SEQ ID NO: 727 | SEQ ID NO: 731 | SEQ ID NO: 746 |
| TR01H113-F760nP17/L0011-k0 | SEQ ID NO: 732 | SEQ ID NO: 733 | SEQ ID NO: 734 | SEQ ID NO: 729 |
| 20A10H-Kn125/20A10L-k0MT | SEQ ID NO: 726 | SEQ ID NO: 735 | SEQ ID NO: 728 | SEQ ID NO: 729 |
| 20A10H-H1076/20A10L-k0MT | SEQ ID NO: 726 | SEQ ID NO: 736 | SEQ ID NO: 728 | SEQ ID NO: 729 |
| 20952H-H1076.G236E/20952L-k0MTC | SEQ ID NO: 730 | SEQ ID NO: 737 | SEQ ID NO: 731 | SEQ ID NO: 746 |
| 20952H - Kn125/20952L-k0MTC | SEQ ID NO: 730 | SEQ ID NO: 735 | SEQ ID NO: 731 | SEQ ID NO: 746 |

### [Example 3] Evaluation of in vitro cleavage of antibody that is cleavable by protease

### (3-1) Evaluation of cleavage of antibody containing linker that is cleavable by protease using recombinant protease

Anti-human GPC3 human IgG1 antibodies H0000-LK4.col2.LK4.G1T4/GL4-k0a and H0000-LK4.col2.LK4.G1T0/GL4-k0a harboring a linker (partial collagen II sequence) that is cleavable by MMP were prepared by coexpressing heavy chains H0000-LK4.col2.LK4.G1T4 and H0000-LK4.col2.LK4.G1T0 having heavy chain variable region H0000 (SEQ ID NO: 738) and heavy chain constant region LK4.col2.LK4.G1T4 (SEQ ID NO: 739) or LK4.col2.LK4.G1T0 (SEQ ID NO: 745) in combination with light chain GL4-k0a having light chain variable region GL4 (SEQ ID NO: 740) and light chain constant region k0a (SEQ ID NO: 729) in combination.

MMP-cleavable linker-free anti-human GPC3 human IgGI antibodies H0000-G1T4/GL4-k0a and H0000-G1T0/GL4-k0a were prepared by coexpressing heavy chains H0000-G1T4 and H0000-G1T0 having heavy chain variable region H0000 (SEQ ID NO: 738) and heavy chain constant region G1T4 (SEQ ID NO: 741) or heavy chain constant region G1T0 (SEQ ID NO: 742) in combination with light chain GL4-k0a having light chain variable region GL4 (SEQ ID NO: 730) and light chain constant region k0a (SEQ ID NO: 729) in combination.

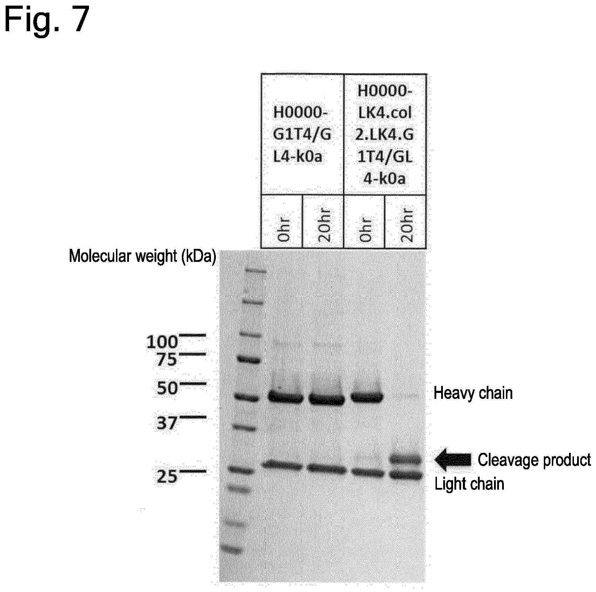

H0000-LK4.col2.LK4.G1T4/GL4-k0a and H0000-G1T4/GL4-k0a were expressed and purified by the method of Example 2-1. 0.27 mg/ml H0000-LK4.col2.LK4.G1T4/GL4-k0a or H0000-G1T4/GL4-k0a suspended in TCNB (50 mM Tris, 10 mM CaCl₂, 150 mM NaCl, 0.05% Brij-35 (w/v), pH 7.5) was treated at 37°C for 20 hours with recombinant MMP13 (R&D systems, Inc., 511-MM-010) (final concentration: 38 nM) which had been activated at 37□C for 2 hours in TCNB containing 1 mM APMA. The treated antibody was developed by reducing SDS-PAGE and analyzed for cleavage by MMP13. The appearance of a band associated with MMP13 cleavage was confirmed for H0000-LK4.col2.LK4.G1T4/GL4-k0a, whereas no cleavage was seen in H0000-G1T4/GL4-k0a (Figure 7). These results demonstrated that the protease cleavage of the antibody occurs in a manner dependent on the protease-cleavable linker.

Anti-human GPC3 human IgG1 antibody H0000-G1T3/GL4-k0a that is cleavable by IdeS was prepared by coexpressing heavy chain H0000-G1T3 having heavy chain variable region H0000 (SEQ ID NO: 738) and heavy chain constant region G1T3 (SEQ ID NO: 743) in combination with light chain GL4-k0a having light chain variable region GL4 (SEQ ID NO: 740) and light chain constant region k0a (SEQ ID NO: 729) in combination.

Anti-human GPC3 human IgG1 antibody H0000-dGG1T3/GL4-k0a that is not cleavable by IdeS was prepared by coexpressing heavy chain H0000-dGG1T3 having heavy chain variable region H0000 (SEQ ID NO: 738) and heavy chain constant region dGG1T3 (SEQ ID NO: 744) in combination with light chain GL4-k0a having light chain variable region GL4 (SEQ ID NO: 740) and light chain constant region k0a (SEQ ID NO: 729) in combination.

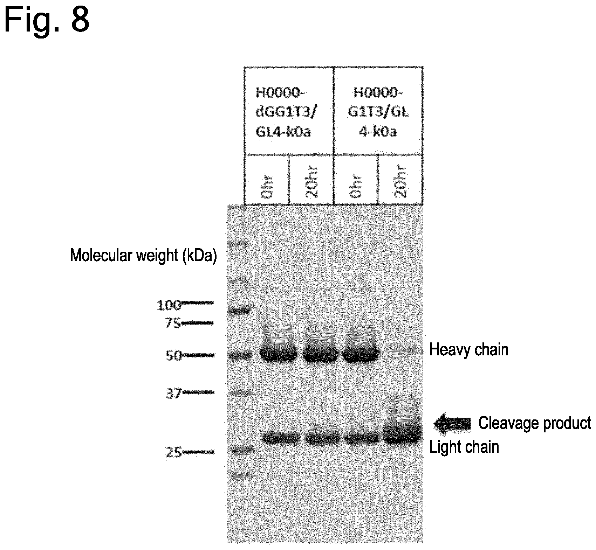

Recombinant IdeS (Promega Corp., V7511) (final concentration: 0.1 EU/µ1) was added to H0000-G1T3/GL4-k0a or H0000-dGG1T3/GL4-k0a suspended at 0.27 mg/ml in PBS, and treated at 37°C for 0 or 20 hours. The protease-treated antibody was developed by reducing SDS-PAGE and analyzed for cleavage by IdeS (Figure 8). A band resulting from IdeS cleavage was seen in H0000-G1T3/GL4-k0a. On the other hand, the band was not seen in H0000-dGG1T3/GL4-k0a containing no IdeS cleavage site. These results demonstrated that the protease cleavage of the antibody occurs in a manner dependent on the protease-cleavable linker.

The structures of the antibodies tested to confirm protease cleavage are shown in the table below.

**[Table 2]**

| Antibody name | Heavy chain sequence | | Light chain sequence | |
|---|---|---|---|---|
| | Variable region | Constant region | Variable region | Constant region |
| H0000-LK4.col2.LK4.G1T4/GL4-k0a | SEQ ID NO: 738 | SEQ ID NO:739 | SEQ ID NO: 740 | SEQ ID NO: 729 |
| H0000-LK4.col2.LK4.G1T0/GL4-k0a | SEQ ID NO: 738 | SEQ ID NO: 745 | SEQ ID NO: 740 | SEQ ID NO: 729 |
| H0000-G1T4/GL4-k0a | SEQ ID NO: 738 | SEQ ID NO: 741 | SEQ ID NO: 740 | SEQ ID NO: 729 |
| H0000-G1T0/GL4-k0a | SEQ ID NO: 738 | SEQ ID NO: 742 | SEQ ID NO: 740 | SEQ ID NO: 729 |
| H0000-G1T3/GL4-k0a | SEQ ID NO: 738 | SEQ ID NO: 743 | SEQ ID NO: 740 | SEQ ID NO: 729 |
| H0000-dGG1T3/GL4-k0a | SEQ ID NO: 738 | SEQ ID NO: 744 | SEQ ID NO: 740 | SEQ ID NO: 729 |

The light chain constant regions k0a and k0MT described in Examples have the same amino acid sequence, though differing in nucleotide sequence.

### [Example 4] Evaluation of binding specificity between antibody specifically binding to cleavable linker cleaved by protease and antibody cleaved by protease

### (4-1) Binding evaluation between MMP-treated antibody and bispecific antibody binding to peptide cleaved by MMP and human CD3

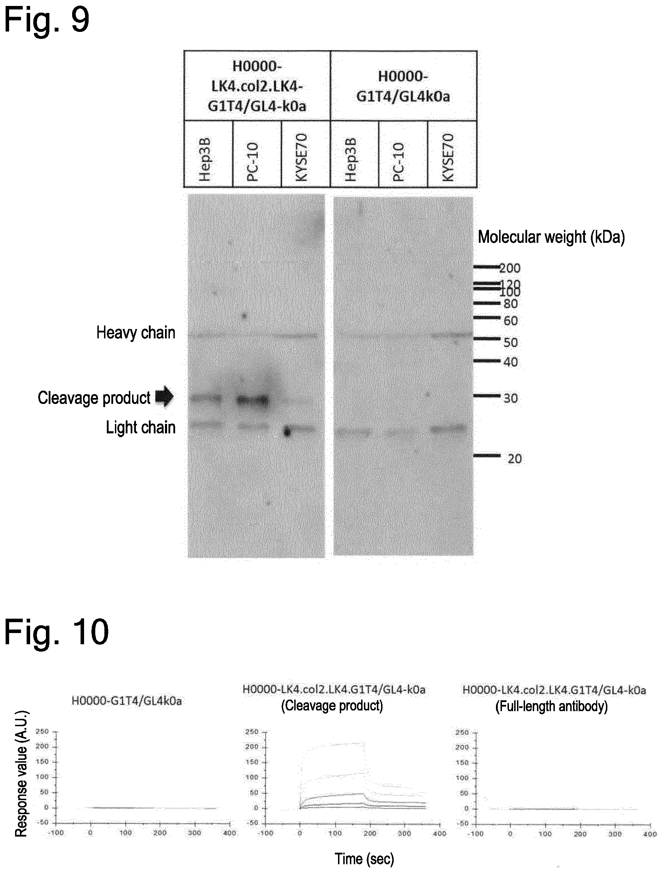

The binding of the bispecific antibody 20A10H-F760nN17/20A10L-k0MT//TR01H113-F760nP17/L0011-k0a prepared by the method described in Example 2 to the MMP-cleavable linker-containing antibody H0000-LK4.col2.LK4.G1T4/GL4-k0a or the MMP-cleavable linker-free antibody H0000-G1T4/GL4-k0a was evaluated using Biacore T200 (GE Healthcare Japan Corp.). The MMP13 cleavage of H0000-LK4.col2.LK4.G1T4/GL4-k0a was performed by the method described in Example 3. The full-length antibody H0000-LK4.col2.LK4.G1T4/GL4-k0a or its protease digestion product, or H0000-G1T4/GL4-k0aas a negative control was immobilized on sensor chip CM4 by amine coupling, and 20A10H-F760nN17/20A10L-k0MT//TR01H113-F760nP17/L0011-k0a as an analyte was interacted for 180 secondsand dissociated for 180 seconds at a flow rate of 20 µl/min. Change in the amount of the analyte bound was quantified. The running buffer used was HBS-EP+, and the measurement was performed at 37°C. The measurement results were analyzed using Biacore T200 Evaluation Software (GE Healthcare Japan Corp.). Association rate constant ka (1/Ms) and dissociation rate constant kd (1/s) were calculated by curve fitting using 1:1 binding model, and dissociation constant KD (M) was calculated based on the values. As a result, it was found that 20A10H-F760nN17/20A10L-k0MT//TR01H1 13-F760nP17/L0011-kOa binds to cleaved H0000-LK4.col2.LK4-G1T4/GL4-k0a, but bind to neither uncleaved H0000-LK4.col2.LK4-G1T4/GL4-k0a nor H0000-G1T4/GL4-k0a containing no cleavable linker (Figure 10, Table 3).

**[Table 3]**

| Antigen | Antigen binding of 20A10H-F760nN17/20A10L-k0MT//TR01H113-F760nP17/L0011-k0a | | |
|---|---|---|---|
| | KD(M) | ka(1/Ms) | kd(1/s) |
| H0000-G1T4/GL4-k0a (negative control) | ND | ND | ND |
| H0000-LK4.col2.LK4.G 1 T4/GL4-k0a (MMP treated) | 8.17.E - 08 | 4.94.E + 04 | 4.04.E - 03 |
| H0000-LK4.col2.LK4.G1T4/GL4-k0a (MMP untreated) | ND | ND | ND |

| | | | |
|---|---|---|---|
| ND: not quantifiable due to weak binding signal intensity | | | |

### (4-2) Binding evaluation between IdeS-treated antibody and bispecific antibody binding to peptide cleaved by IdeS and human CD3

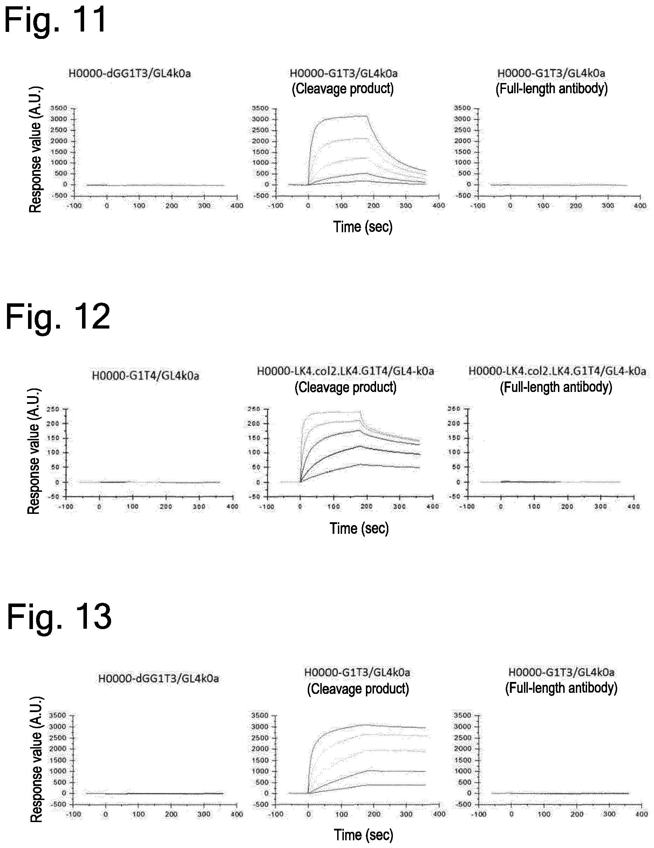

The binding of the bispecific antibody 20952H-F760nN17/20952L-k0MTC//TR01H1 13-F760nP17/L0011-k0a prepared by the method described in Example 2 to the IdeS-cleavable linker-containing H0000-G1T3/GL4-k0a or the IdeS-cleavable linker-free antibody H0000-dGG1T3/GL4-k0a was evaluated using Biacore T200 (GE Healthcare Japan Corp.). The IdeS cleavage of H0000-G1T3/GL4-k0a was performed by the method described in Example 3. The full-length antibody H0000-G1T3/GL4-k0a or its protease digestion product, or H0000-dGG1T3/GL4-k0a as a negative control was immobilized on sensor chip CM4 by amine coupling, and 20952H-F760nN17/20952L-k0MTC//TR01H113-F760nP17/L0011-k0a as an analyte was interacted for 180 seconds and dissociated for 180 seconds at a flow rate of 20 µl/min. Change in the amount of the analyte bound was quantified. The running buffer used was HBS-EP+ (GE Healthcare Japan Corp.), and the measurement was performed at 37°C. The measurement results were analyzed using Biacore T200 Evaluation Software (GE Healthcare Japan Corp.). Association rate constant ka (1/Ms) and dissociation rate constant kd (1/s) were calculated by curve fitting using 1:1 binding model, and dissociation constant KD (M) was calculated based on the values. As a result, it was found that 20952H-F760nN17/20952L-k0MTC//TR01H1 13-F760nP17/L0011-k0a binds to cleaved H0000-G1T3/GL4-k0a, but bind to neither uncleaved H0000-G1T3/GL4-k0 nor H0000-dGG1T3/GL4-k0a containing no cleavable linker (Figure 11, Table 4).

**[Table 4]**

| Antigen | Antigen binding of 20952H-F760nN17/20952L-k0MTC//TR01H113-F760nP17/L0011-k0a | | |
|---|---|---|---|
| | KD(M) | ka(1/Ms) | kd(1/s) |
| H0000-dGG1T3/GL4-k0a (negative control) | ND | ND | ND |
| H0000-GlT3/GL4-k0a (IdeS treated) | 7.26.E - 08 | 1.34.E + 05 | 9.72.E - 03 |
| H0000-GlT3/GL4-k0a (IdeS untreated) | ND | ND | ND |

| | | | |
|---|---|---|---|
| ND: not quantifiable due to weak binding signal intensity | | | |

### (4-3) Binding evaluation betweenMMP-treated antibody and antibody with enhanced ADCC specifically binding to peptide cleaved by MMP

The binding of the antibody 20A10H_Knl25/20A10L-k0MT//20A10H_H1076/20A10L-k0MT with enhanced ADCC prepared by the method described in Example 2(2-3) to the MMP-cleavable linker-containing antibody H0000-LK4.col2.LK4.G1T4/GL4-k0a or the MMP-cleavable linker-free antibody H0000-G1T4/GL4-k0a was evaluated using Biacore T200 (GE Healthcare Japan Corp.). The preparation of the already cleaved antibody serving as a ligand was performed by the method described in Example 3. The Biacore measurement was performed by the method described in Example 4(4-1). As a result, it was found that 20A10H_Knl25/20A10L-k0MT//20A10H_Hl076/20A10L-k0MT binds to cleaved H0000-LK4.col2.LK4.G1T4/GL4-k0a, but bind to neither uncleaved H0000-LK4.col2.LK4.G1T4/GL4-k0a nor H0000-G1T4/GL4-k0a containing no cleavable linker (Figure 12, Table 5).

**[Table 5]**

| Antigen | Antigen binding of 20A10H_Kn125/20A10L-k0MT//20A10H HI076/20A10L-k0MT | | |
|---|---|---|---|
| | KD(M) | ka(1/Ms) | kd(1/s) |
| H0000-G1T4/GL4-k0a(negative control) | ND | ND | ND |
| H0000-LK4.col2.LK4.G1 T4/GL4-k0a (MMP treated) | 3.01.E - 09 | 6.03.E + 05 | 1.81.E - 03 |
| H0000-LK4.col2.LK4.G1T4/GL4-k0a (MMP untreated) | ND | ND | ND |

| | | | |
|---|---|---|---|
| ND: not quantifiable due to weak binding signal intensity | | | |

### (4-4) Binding evaluation between IdeS-treated antibody and antibody with enhanced ADCC specifically binding to antigen binding molecule obtained through IdeS cleavage of linker

The binding of the antibody 20952H-H1076.G236E/20952L-k0MTC//20952H-Knl25/20952L-k0MTC with enhanced ADCC prepared by the method described in Example 2 to the IdeS-cleavable linker-containing H0000-G1T3/GL4-k0a or the IdeS-cleavable linker-free antibody H0000-dGG1T3/GL4-k0a was evaluated using Biacore T200 (GE Healthcare Japan Corp.). The preparation of the already cleaved antibody serving as a ligand was performed by the method described in Example 3. The Biacore measurement was performed by the method described in Example 4(4-2). As a result, it was found that 20952H-H1076.G236E/20952L-k0MTC//20952H-Kn125/20952L-k0MTC binds to cleaved H0000-G1T3/GL4-k0a, but bind to neither uncleaved H0000-G1T3/GL4-k0a nor antibody H0000-dGG1T3/GL4-k0a containing no cleavable linker (Figure 13, Table 6).

**[Table 6]**

| Antigen | Antigen binding of 20952H-H1076.G236E/20952L-k0MTC//20952H-Kn125/20952L-k0MTC | | |
|---|---|---|---|
| | KD(M) | ka(1/Ms) | kd(1/s) |
| H0000-dGG1T3/GL4-k0a (negative control) | ND | ND | ND |
| H0000-GlT3/GL4-k0a (IdeS treated) | 1.61.E - 09 | 1.51.E + 05 | 2.44.E - 04 |
| H0000-GlT3/GL4-k0a (IdeS untreated) | ND | ND | ND |

| | | | |
|---|---|---|---|
| ND: not quantifiable due to weak binding signal intensity | | | |

### [Example 5] Reporter gene assay using Jurkat cell expressing reporter gene depending on CD3 stimulation or FcyR stimulation

### (5-1) Preparation of culture solution of reporter cell

Jurkat cell line GloResponse(TM) NFAT-RE-luc2 Jurkat Cell Line (Promega Corp.) expressing luciferase in response to CD3 stimulation or Jurkat cell line ADCC Bioassay Effector Cells (Promega Corp.) expressing luciferase in response to FcyR stimulation were cultured and then suspended at a cell density of 3 × 10⁶ cells/ml in RPMI-1640 (Gibco, 22400) containing 10% FBS (HyClone Laboratories, Inc., SH30070.03), 1 mM sodium pyruvate (Invitrogen Corp., 11360), and MEM Non Essential Amino Acids (Invitrogen Corp., 11140) (hereinafter, referred to as an assay buffer). The cells were designated as NFAT-RE-Luc2-Jurkat or Jurkat-hFcgR-luc and subject to the subsequent experiments.

### (5-2) Preparation of target cell

Tumor cell lines PC-10 (Immuno-Biological Laboratories Co., Ltd.), Hep3B (ATCC) and KYSE70 (Health Science Research Resources Bank) expressing a tumor antigen human GPC3, or SK-pca-60 (see EP3296395) was cultured by a method known in the art. The cells were suspended in an assay buffer and subjected as target cells to the subsequent experiments.

### (5-3) Evaluation of cleavage of antibody harboring protease-cleavable linker by target cells

5 ×10⁶ cells/ml of PC-10, Hep3B and KYSE70 prepared in Example 5(5-2) were inoculated at 25 µl/well to 96-well plates (Corning Inc.) (1.25 × 10⁵ cells/well). Next, the anti-human GPC3 antibody H0000-LK4.col2.LK4.G1T4/GL4-k0a harboring an MMP-cleavable linker and the antibody H0000-G1T4/GL4-k0a harboring no MMP-cleavable linker were diluted (final concentration: 1 nM) with an assay buffer and added at 50 µL/well. The plates were incubated at 37°C for 0 hours or 24 hours in the presence of 5% CO₂. Then, a sample buffer was added thereto to prepare reducing SDS-PAGE specimens. After SDS-PAGE, H0000-LK4.col2.LK4.G1T4/GL4-k0a and H0000-G1T4/GL4-k0a in the specimens were detected by Western blotting using Goat anti-human IgG HRP (H+L) conjugate (Invitrogen Corp., cat no. 81-7120). As a result, the appearance of a band associated with the cleavage of the antibody in the cultured cells was seen for H0000-LK4.col2.LK4.G1T4/GL4-k0a harboring an MMP-cleavable linker. On the other hand, no cleavage was seen in the antibody H0000-G1T0/GL4-k0a harboring no cleavable linker (Figure 9). These results demonstrated that the cleavage of H0000-LK4.col2.LK4.G1T4/GL4-k0a occurs in a manner dependent on the protease-cleavable linker. Band shift associated with the cleavage of H0000-LK4.col2.LK4.G1T4/GL4-k0a was rarely seen in KYSE70, suggesting that H0000-LK4.col2.LK4.G1T4/GL4-k0a is rarely cleaved in KYSE70.

### (5-4) Reporter gene assay using MMP-cleavable linker-containing antibody and bispecific antibody recognizing cleavage product of the antibody and CD3

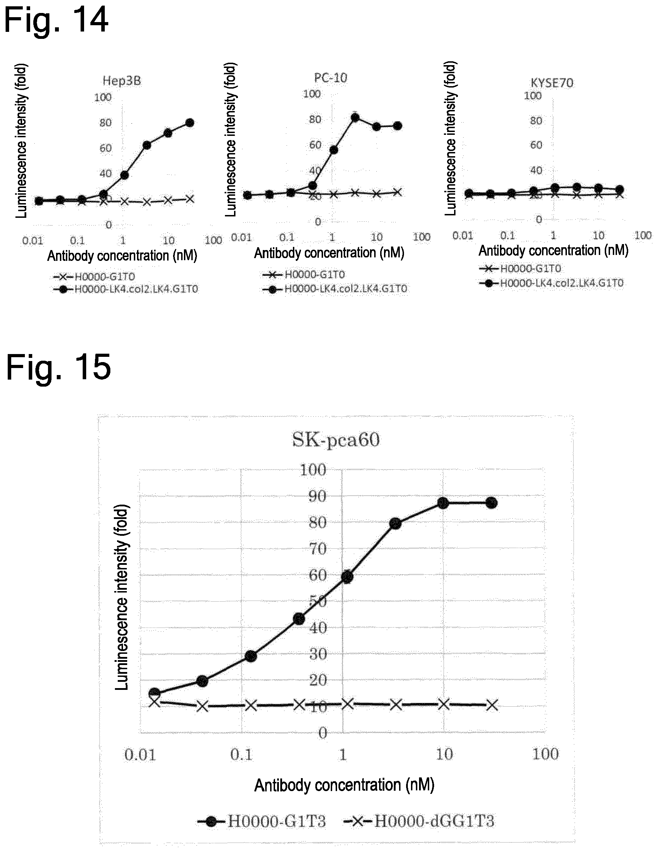

The ability of a test antibody to activate CD3 was evaluated by the expression of luciferase expressed under the control downstream of TCR signals in NFAT-RE-Luc2-Jurkat cells. First, 4 × 10⁶ cells/ml of PC-10, Hep3B and KYSE70 prepared in Example 5(5-2) were inoculated at 25 µl/well to 96-well flat-bottom plates (Coster, 3917) (1 × 10⁵ cells/well). Next, a solution of the anti-human GPC3 antibody H0000-LK4.col2.LK4.G1T0/GL4-k0 containing an MMP-cleavable linker or the anti-human GPC3 antibody H0000-G1T0/GL4-k0 containing no MMP-cleavable linker was prepared at each concentration using an assay buffer and added thereto. The anti-cleaved linker antibody//anti-human CD3 bispecific antibody 20A10H-F760nN17/20A10L-k0MT//TR01H113-F760nP17/L0011-k0a diluted (final concentration: 10 nM) with an assay buffer was further added thereto. The NFAT-RE-Luc2-Jurkat solution prepared in Example 5(5-1) was added at 25 µl/well (7.5 × 10⁴ cells/well). The plates were left standing overnight at 37°C in a 5% carbonic acid gas incubator. After reaction, Bio-Glo luciferase Assay System solution (Promega Corp.) was added at 75 µl/well. Luminescence from luciferase was quantified using a plate reader (EnVision, PerkinElmer, Inc.). The values quantified with the plate reader were normalized against measurement values without the addition of the test antibody. As a result, the activation of NFAT-RE-Luc2-Jurkat cells was confirmed by the addition of H0000-LK4.col2.LK4.G1T0/GL4-k0a and 20A10H-F760nN17/20A10L-k0MT//TR01H113-F760nP17/L0011-k0a to PC-10 and Hep3B having protease activity of cleaving H0000-LK4.col2.LK4.G1T0/GL4-k0, whereas the activation of NFAT-RE-Luc2-Jurkat cells was not confirmed by the addition of H0000-G1T0/GL4-k0a and 20A10H-F760nN17/20A10L-k0MT//TR01H113-F760nP17/L0011-k0a or in KYSE70 having no protease activity of cleaving H0000-LK4.col2.LK4.G1T0/GL4-k0a (Figure 14). These results suggested that TDCC activity is induced in a manner dependent on H0000-LK4.col2.LK4.G1T0/GL4-k0a cleaved by protease.

### (5-5) Reporter gene assay using IdeS-cleavable linker-containing antibody and bispecific antibody binding to antigen binding molecule obtained through cleavage of linker of the antibody and CD3

The ability of a test antibody to activate CD3 was evaluated by the expression of luciferase expressed under the control downstream of TCR signals in NFAT-RE-Luc2-Jurkat cells. IdeS is *Actinomycete-derived* protease, and none of mammalian cells express IdeS. Therefore, the protease was added to a reaction solution. First, 5 × 10⁵ cells/ml of SK-pca60 prepared in Example 5(5-2) were inoculated at 25 µl/well to 96-well flat-bottom plates (Coster, 3917) (1.25 × 10⁴ cells/well). Next, a solution of the anti-human GPC3 antibody H0000-G1T3/GL4-k0a containing an IdeS-cleavable linker or the anti-human GPC3 antibody H0000-dGG1T3/GL4-k0a containing no IdeS cleavable linker was prepared at each concentration using an assay buffer and added thereto at 12.5 µl/well. An assay buffer containing the anti-cleaved linker antibody//anti-human CD3 bispecific antibody 20952H-F760nN17/20952L-k0MTC//TR01H113-F760nP17/L001 1-k0a (final concentration: 10 nM) and IdeS (final concentration: 0.2 U/µl) was further added at 12.5 µl/well. The NFAT-RE-Luc2-Jurkat solution prepared in Example 5(5-1) was added at 25 µl/well (7.5 × 10⁴ cells/well). The plates were left standing overnight at 37°C in a 5% carbonic acid gas incubator. After the reaction, Bio-Glo luciferase Assay System solution (Promega Corp.) was added at 75 µl/well. Luminescence from luciferase was quantified using a plate reader (EnVision, PerkinElmer, Inc.). The values quantified with the plate reader were normalized against measurement values without the addition of the test antibody. As a result, the activation of Jurkat cells was confirmed in the cell suspension supplemented with the antibody H0000-G1T3/GL4-k0a that was cleaved by IdeS and 20952H-F760nN17/20952L-k0MTC//TR01H1 13-F760nP17/L001 1-kOa, whereas the response of NFAT-RE-Luc2-Jurkat cells was not seen by the addition of H0000-dGG1T3/GL4-k0a that was not cleaved by IdeS and 20952H-F760nN17/20952L-k0MTC//TR01H113-F760nP17/L0011-kOa (Figure 15). These results suggested that TDCC activity is induced in a manner dependent on H0000-G1T3/GL4-k0a cleaved by IdeS.

### (5-6) Reporter gene assay using MMP13-cleavable linker-containing antibody and antibody with enhanced ADCC activity binding to antigen binding molecule obtained through cleavage of linker of the antibody

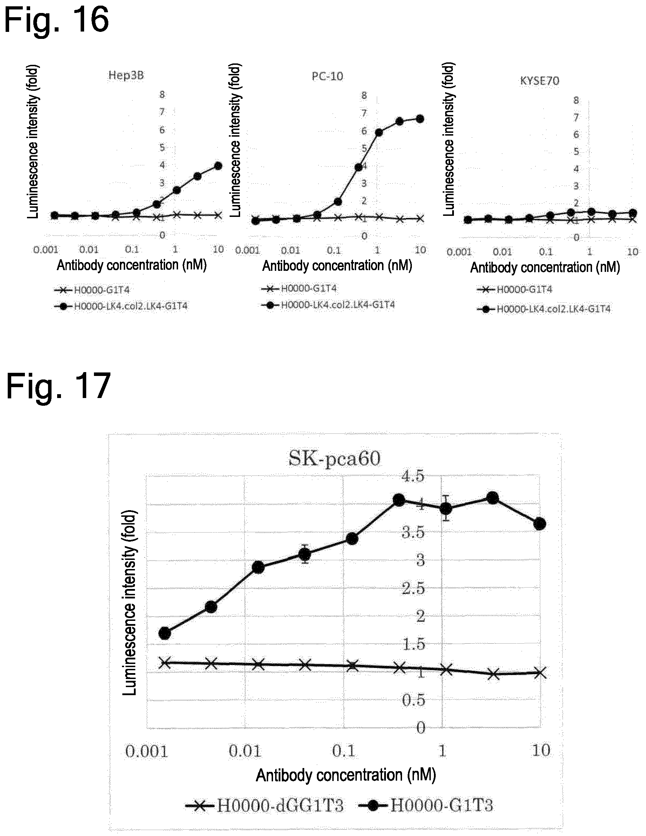

The ability of a test antibody to activate FcyR was evaluated by the expression of luciferase expressed under the control of FcyR signals in Jurkat-hFcgR-luc cells. First, 3 × 10⁶ cells/ml of the target cells PC-10,Hep3B,KYSE70 prepared in Example 5(5-2) were inoculated at 25 µl/well to 96-well flat-bottom plates (Coster, 3917) (7.5 × 10⁴ cells/well). Next, the anti-human GPC3 antibody H0000-LK4.col2.LK4.G1T4/GL4-k0a containing an MMP 13-cleavable linker or the anti-human GPC3 antibody H0000-G1T4/GL4-k0a containing no MMP13-cleavable linker was prepared at each concentration using an assay buffer and added at 12.5 µl/well. The antibody 20A10H_Kn125/20A10L-k0MT//20A10H_Hl076/20A10L-k0MT with enhanced ADCC binding to the antigen binding molecule obtained through the cleavage of the linker diluted (final concentration: 3 nM) with an assay buffer was further added at 12.5 µl/well. The Jurkat-hFcgR-luc solution prepared in Example 5(5-1) was added at 25 µl/well (7.5 × 10⁴ cells/well). The plates were left standing overnight at 37°C in a 5% carbonic acid gas incubator. After the reaction, Bio-Glo luciferase Assay System solution (Promega Corp.) was added at 75 µl/well. Luminescence from luciferase was quantified using a plate reader (EnVision, PerkinElmer, Inc.). The values quantified with the plate reader were normalized against measurement values without the addition of the test antibody. As a result, the activation of Jurkat cells was confirmed in the cell suspension supplemented with the antibody H0000-LK4.col2.LK4.G1T4/GL4-k0a that was cleaved by MMP13 and 20A10H_Kn125/20A10L-k0MT//20A10H_Hl076/20A10L-k0MT, whereas the response of Jurkat-hFcgR-luc cells was not seen by the addition of HOOOO-G1T4/GL4-kOa that was not cleaved by MMP and 20A10H_Kn125/20A10L-k0MT//20A10H_Hl076/20A10L-k0MT (Figure 16). These results suggested that ADCC activity is induced in a manner dependent on H0000-LK4.col2.LK4.G1T4/GL4-k0a cleaved by MMP13.

### (5-7) Reporter gene assay using IdeS-cleavable linker-containing antibody and antibody with enhanced ADCC activity binding to antigen binding molecule obtained through cleavage of linker of the antibody

The ability of a test antibody to activate FcyR was evaluated by the expression of luciferase expressed under the control of FcyR signals in Jurkat-hFcgR-luc cells. IdeS is *Actinomycete-derived* protease, and none of mammalian cells express IdeS. Therefore, the protease was added to a reaction solution. First, the target cells SK-pca60 prepared in Example 5(5-2) were suspended at a cell density of 2.5 × 10⁴ cells/ml and inoculated at 25 µl/well to 96-well flat-bottom plates (Coster, 3917) (2.5 × 10⁴ cells/well). Next, a solution of the anti-human GPC3 antibody H0000-G1T3/GL4-k0a containing an IdeS-cleavable linker or the anti-human GPC3 antibody H0000-dGG1T3/GL4-k0a containing no IdeS cleavable linker was prepared at each concentration using an assay buffer and added at 12.5 µl/well. An assay buffer containing the antibody 20952H-Hl076.G236E/20952L-k0MTC//20952H-Kn125/20952L-k0MT with enhanced ADCC diluted (final concentration: 0.5 nM) was further added at 12.5 µl/well. The Jurkat-hFcgR-luc solution prepared in Example 5(5-1) was added at 25 µl/well (7.5 × 10⁴ cells/well). IdeS was added at a final concentration of 0.033 U/µl. The plates were left standing overnight at 37°C in a 5% carbonic acid gas incubator. After reaction, Bio-Glo luciferase Assay System solution (Promega Corp.) was added at 75 µl/well. Luminescence from luciferase was quantified using a plate reader (EnVision, PerkinElmer, Inc.). The values quantified with the plate reader were normalized against measurement values without the addition of the test antibody. As a result, the activation of Jurkat-hFcgR-luc cells was confirmed in the cell suspension supplemented with the antibody H0000-G1T3/GL4-k0a that was cleaved by IdeS and 20952H-Hl076.G236E/20952L-k0MTC//20952H-Kn125/20952L-k0MTC, whereas the response of ADCC Bioassay Effector cells was not seen by the addition of H0000-dGG1T3/GL4-k0a that was not cleaved by IdeS and 20952H-Hl076.G236E/20952L-k0MTC//20952H-Kn125/20952L-k0MTC (Figure 17). These results suggested that ADCC activity is induced in a manner dependent on H0000-G1T3/GL4-k0a cleaved by IdeS.

### [Example 6] Confirmation of in vitro cytotoxic activity using MMP13-cleavable linker-containing antibody and bispecific antibody against the cleaved antibody and human CD3

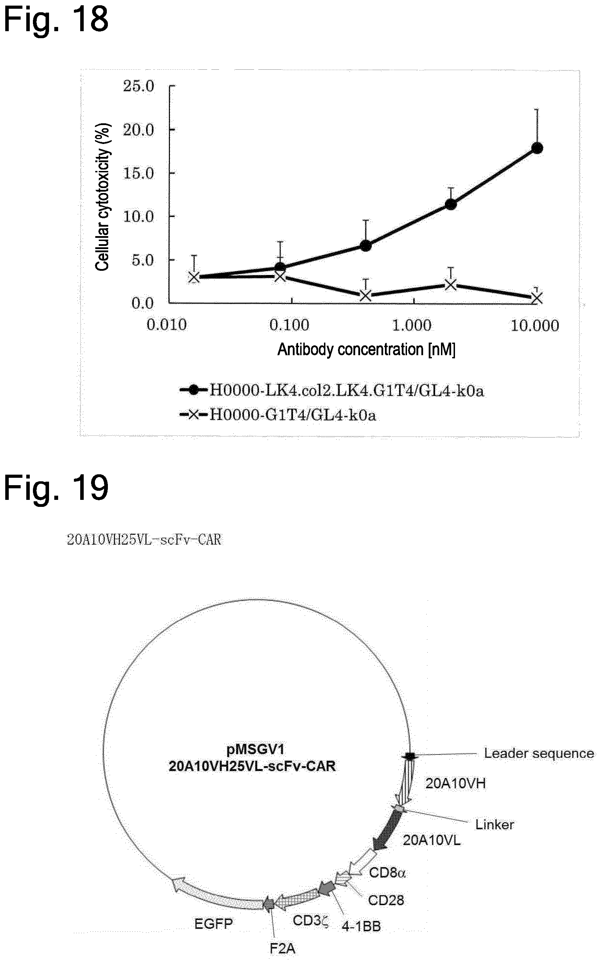

Blood was collected from a healthy human donor using heparin, diluted with PBS (Nacalai Tesque, Inc., #14249-95), and then layered on Ficoll-Paque Plus (GE Healthcare Japan Corp.). A peripheral blood mononuclear cell (PBMC) fraction was separated by centrifugation at 1000 g for 30 minutes. obtained PBMC was prepared with RPMI 1640 (Sigma-Aldrich Co., LLC, R8758) + 10% FBS (Sigma-Aldrich Co., LLC, 172012-500ML) (medium). A solution of the anti-human GPC3 antibody H0000-LK4.col2.LK4.G1T4/GL4-k0a containing an MMP-cleavable linker or the anti-human GPC3 antibody H0000-G1T4/GL4-k0a containing no cleavable linker was prepared at each concentration using a medium and added to 96 well round bottom plates (Corning Inc.). The bispecific antibody 20A10H-F760nN17/20A10L-k0MT//TR01H113-F760nP17/L0011-k0a diluted (final concentration: 10 nM) was further added thereto. PC-10 prepared according to the method described in Example 5(5-2) was inoculated as target cells at 1 × 10⁵ cells/well. The prepared PBMC was added at 2 × 10⁵ cells/well. The plates were incubated at 37°C in 5% CO₂. 24 hours later, the amount of LDH was quantified using Pierce LDH Cytotoxicity Assay Kit (Thermo Fisher Scientific Inc., 88954) according to the method designated by the manufacturer. As a result, the release of LDH was seen in a manner dependent on the added antibody concentration in the specimen supplemented with the antibody H0000-LK4.col2.LK4.G1T4/GL4-k0a harboring an MMP13-cleavable linker, whereas the release of LDH was not seen in the specimen supplemented with the MMP13-cleavable linker-free antibody H0000-G1T4/GL4-k0a. This study revealed that cytotoxic activity is specifically exhibited to human tumor cells expressing tumor-specific protease. From these results, it is expected that the bispecific antibody against the antigen binding molecule obtained through the cleavage of the linker and CD3 also exerts tumor-specific cytotoxic activity in humans *in vivo* (Figure 18).

### [Example 7] Preparation of antibody drug with protease-dependent release of VHH as antigen binding molecule obtained through cleavage of the linker

VHH that exhibits binding activity against a target antigen is obtained by a method known to those skilled in the art, such as the screening of a library prepared from *Camelidae* animal PBMC or the like. A gene of one of the protease-cleavable linkers of SEQ ID NOs: 1 to 725 is fused with a partial sequence of human IgG gene according to the method disclosed in WO/2018/097307 and WO/2018/097308. The prepared nucleotide sequence is inserted to expression vectors for mammalian cells. An antibody drug that releases VHH having target antigen binding activity in a protease-dependent manner is expressed and purified according to the method of Example 2-1.

### [Example 8] Obtainment of antibody specifically binding to antigen binding molecule obtained through cleavage of linker in antibody drug with protease-dependent release of VHH as the antigen binding molecule obtained through cleavage of linker

### (8-1) Obtainment of antibody specifically binding to cleavage site of protease-cleavable linker

Any of the protease-cleavable linkers (SEQ ID NOs: 1 to 725) disclosed in WO2018/097307 and WO2018/097308 (having the same sequence as that selected in Example 7) and an antigen binding molecule obtained through the cleavage of the linker of the peptide are prepared by a method known to those skilled in the art, such as peptide synthesis, and conjugated with KLH or the like. A rabbit is immunized with the conjugate by a method known to those skilled in the art. A suspension of cells collected from the blood or the spleen of the immunized rabbit is screened using autoMACS Pro Separator and FACSAria (Becton, Dickinson and Company) to select candidates of cells having binding activity against the antigen binding molecule obtained through the cleavage of the linker. Next, whether or not an antibody secreted into a culture supernatant of the cells has binding activity against the antigen binding molecule obtained through the cleavage of the linker is evaluated by ELISA. A specimen having a response value exceeding a control value in ELISA is selected as an antibody binding to the antigen binding molecule obtained through the cleavage of the linker (positive selection). On the other hand, no binding of the selected antibody to the uncleaved peptide sequence is confirmed from the absence of binding to a protease-untreated protease-cleavable linker (negative selection). A series of selection rounds yield an antibody that binds to the antigen binding molecule obtained through the cleavage of the linker and does not bind to an uncleaved protease-cleavable linker.

### (8-2) Obtainment of antibody specifically binding to antigen binding molecule VHH obtained through the cleavage of the linker by protease cleavage

A rabbit is immunized with the immunogen VHH having binding activity against a target antigen, obtained in Example 7, by a method known to those skilled in the art. A suspension of cells collected from the blood or the spleen of the immunized rabbit is screened using autoMACS Pro Separator and FACSAria (Becton, Dickinson and Company) to select candidates of cells having binding activity against VHH Next, whether or not an antibody secreted into a culture supernatant of the cells has binding activity against the antigen binding molecule VHH obtained through the cleavage of the linker is evaluated by ELISA. A specimen having a response value exceeding a control value in ELISA is selected as an antibody binding to VHH (positive selection). On the other hand, no recognition of the selected antibody to unreleased VHH is confirmed from the absence of binding to the protease-untreated target antigen VHH-protease-cleavable linker-human IgG fusion protein prepared in Example 7 (negative selection). A series of selection rounds yield an antibody that binds to the antigen binding molecule VHH obtained through the protease cleavage of the linker and does not bind to an unreleased VHH

### (8-3) Preparation of antibody specifically binding to antigen binding molecule obtained through cleavage of linker by protease cleavage

Heavy chain and light chain variable region genes are obtained by PCR from B cells expressing the antibody obtained in Example 8-1 or 8-2. The obtained variable region genes are expressed in combination with a human IgG1 heavy chain constant region and a human light chain constant region by a method known to those skilled in the art, such as PCR, and humanized, and the antibody is expressed and purified according to the method of Example 2-1. A bispecific antibody against human CD3 and the antigen binding molecule obtained through the cleavage of the linker and an antibody with enhanced ADCC recognizing the antigen binding molecule obtained through the cleavage of the linker are prepared according to the methods described in Examples 2-2 and 2-3, respectively.

### [Example 9] Reporter gene assay using antibody drug with protease-dependent release of VHH as antigen binding molecule obtained through cleavage of linker, and bispecific antibody recognizing the antigen binding molecule obtained through cleavage of linker of the molecule and human CD3

The bispecific antibody against human CD3 and the cleaved peptide sequence, prepared in Example 8, as a test specimen and the antibody drug with the protease-dependent release of VHH as the antigen binding molecule obtained through the cleavage of the linker, prepared in Example 7 are used to confirm the protease activity-dependent ability of the test specimen to activate CD3. The reporter gene assay of the test specimen is performed according to the method described in Example 5-4. The antibody drug with the protease-dependent release of VHH as the antigen binding molecule obtained through the cleavage of the linker is added to target cells expressing protease and a target antigen. The specimen molecule is cleaved by the protease so that VHH is released as the antigen binding molecule obtained through the cleavage of the linker and binds to a target cell. The bispecific antibody recognizing the antigen binding molecule obtained through the cleavage of the linker and human CD3 bridges the target cell to CD3 on effector cells via the antigen binding molecule obtained through the cleavage of the linker. This induces the luciferase expression of the effector cells. The expression of the luciferase is monitored to confirm the activation of the effector cells.

### [Example 10] Reporter gene assay using antibody drug with protease-dependent release of VHH as antigen binding molecule obtained through cleavage of the linker, and antibody with enhanced ADCC activity specifically recognizing the target antigen binding molecule released from the molecule

The antibody with enhanced ADCC against the cleaved peptide sequence, prepared in Example 8, as a test specimen and the antibody drug with the protease-dependent release of VHH as the antigen binding molecule obtained through the cleavage of the linker, prepared in Example 7 are used to confirm the protease activity-dependent ability of the test specimen to activate FcyR. The reporter gene assay of the antibody with enhanced ADCC is performed according to the method described in Example 5-6. The antibody drug with the protease-dependent release of VHH as the antigen binding molecule obtained through the cleavage of the linker is added to target cells expressing protease and a target antigen. The specimen molecule is cleaved by the protease so that VHH having target antigen binding activity is released and binds to a target cell. The antibody with enhanced ADCC specifically binding to the antigen binding molecule obtained through the cleavage of the linker bridges the target cell to FcyR on effector cells via the antigen binding molecule obtained through the cleavage of the linker. This induces the luciferase expression of the effector cells. The expression of the luciferase is monitored to confirm the activation of the effector cells.

### [Example 11] Confirmation of cytotoxic activity using bispecific antibody recognizing antigen binding molecule obtained through protease-dependent cleavage of the linker and human CD3 and antibody drug with protease-dependent release of VHH as antigen binding molecule obtained through the cleavage of the linker

The bispecific antibody against human CD3 and the antigen binding molecule obtained through the protease-dependent cleavage of the linker, prepared in Example 8, as a test specimen and the antibody drug with the protease-dependent release of VHH as the antigen binding molecule obtained through the cleavage of the linker, prepared in Example 7 are used to confirm the protease activity-dependent cytotoxic activity of the test specimen. Blood is collected from a healthy human donor using heparin, diluted with PBS (Nacalai Tesque, Inc., #14249-95), and then layered on Ficoll-Paque Plus (GE Healthcare Japan Corp.). A peripheral blood mononuclear cell (PBMC) fraction is separated by centrifugation at 1000 g for 10 minutes. The obtained PBMC is prepared with RPMI 1640 (Nacalai Tesque, Inc.) medium and inoculated at 2 × 10⁵ cells/well to 96 well round bottom plates (Corning Inc.). The antibody drug with the protease-dependent release of VHH as the antigen binding molecule obtained through the cleavage of the linker prepared at each concentration using RPMI is added thereto. The anti-VHH (antigen binding molecule obtained through the cleavage of the linker)//anti-human CD3 bispecific antibody is further added thereto. Cells expressing a target antigen and protease that can cleave the protease-cleavable linker of the test specimen are inoculated as target cells. The plates are incubated at 37°C in 5% CO₂. 24 hours later, the amount of LDH is quantified using Pierce LDH Cytotoxicity Assay Kit (Thermo Fisher Scientific Inc., 88954) according to the method designated by the manufacturer. This study reveals that cytotoxic activity is specifically exhibited to human tumor cells expressing tumor-specific protease.

### [Example 12] Preparation of universal CAR-T using antibody specifically binding to antigen binding molecule obtained through the cleavage of the linker

The antibody specifically binding to the antigen binding molecule obtained through the cleavage of the linker, prepared in Example 8-1 or 8-2 is used to prepare a chimeric receptor binding to the antigen binding molecule obtained through the cleavage of the linker. scFv of the antibody against the antigen binding molecule obtained through the cleavage of the linker, obtained in Example 8-1 or 8-2 is referred to as anti-antigen binding molecule obtained through the cleavage of the linker-scFv. A released molecule such as VHH prepared in Example 7 is used as the antigen binding molecule obtained through the cleavage of the linker. A gene of the anti-antigen binding molecule obtained through the cleavage of the linker-scFv is linked with nucleotide sequences encoding a transmembrane region and a cytoplasmic region by PCR or the like. The resulting sequence is inserted to, for example, virus vectors known in the art to produce virus vectors for the expression of CAR against the antigen binding molecule obtained through the cleavage of the linker (referred to as anti-antigen binding molecule obtained through the cleavage of the linker-CAR). CAR-T expressing scFv of the antibody against the antigen binding molecule obtained through the cleavage of the linker, binding to the antigen binding molecule obtained through the cleavage of the linker is prepared by a viral transduction method known in the art. The cells can bind to the antigen binding molecule obtained through the cleavage of the linker, irrespective of any tumor antigen as a target antigen, and are universal CAR-T for activation via the antigen binding molecule obtained through the cleavage of the linker.

### [Example 13] Evaluation of in vitro cytotoxic activity of universal CAR-T against antigen binding molecule obtained through the cleavage of the linker

The universal CAR-T cells against the antigen binding molecule obtained through the cleavage of the linker, such as VHH, prepared in Example 12 (anti-antigen binding molecule obtained through the cleavage of the linker-CAR) are evaluated for its cytotoxic activity by LDH release assay, assay using flow cytometry, or assay using xCELLigence (WO2016/047722), etc. SK-Hep1 is provided as a negative control, and SK-pca60 obtained by allowing SK-Hep1 to stably express human GPC3 is provided as target cells. The negative control and the target cells are inoculated to plates to start measurement with xCELLigence real-time cell analyzer. After overnight culture, the cells are mixed with cells expressing the anti-antigen binding molecule obtained through the cleavage of the linker-CAR as effector cells. Next, an anti-GPC3 antigen binding molecule obtained through the cleavage of the linker-protease-cleavable linker-human IgG fusion protein prepared according to the method of Example 7 is added thereto. The plates are loaded again in the xCELLigence real-time cell analyzer and incubated at 37°C for 72 hours in 5% CO₂. Then, a cell growth inhibition rate (%) is determined from an index value.

The cytotoxic activity is evaluated by the percentage of residual cancer cells. The percentage of residual cancer cells is calculated from the percentage of CD45⁻ fraction cells in live cells.

If the target antigen is an antigen other than GPC3, target tissue-specific cytotoxic activity can also be caused in the present universal CAR-T by using cells expressing the target antigen and a primary molecule having an antigen binding domain directed to the antigen.

### [Example 14] Preparation of universal CAR-T binding to antigen binding molecule obtained through the cleavage of MMP-cleavable linker

### (14-1) Construction of retrovirus vector for the expression of chimeric receptor binding to antigen binding molecule obtained through the cleavage of MMP-cleavable linker

A retrovirus vector for a chimeric receptor was prepared for the expression of scFv of the antibody recognizing the antigen binding molecule obtained through the cleavage of the MMP-cleavable linker. pMSGV1-CD8-28BBZ (Hughes M.S. et al., Hum Gene Ther 2005 Apr; 16 (4): 457-72; obtained from Dr. Richard Morgan (National Cancer Institute)) was used as a retrovirus vector sequence, and pMSGV (Tamada k et al., Clin Cancer Res 18: 6436-6445 (2002)) was used as a mouse stem cell virus-based splice-gag vector. Figure 19 is a schematic view showing the vector construct and the order of arrangement of components in frame units from the 5' end to the 3' end. Human scFv recognizing the antigen binding molecule obtained through the cleavage of the MMP-cleavable linker was referred to as 20A10VH25VL-scFv. This scFv consists of a heavy chain variable region followed by a 25-amino acid linker and a light chain variable region (SEQ ID NO: 747). 20A10VH25VL-scFv was linked to a hinge region and a transmembrane region of a human CD8 alpha chain (nucleotides 1271 to 1519, GenBank NM001768.6), and a cytoplasmic region of a human CD28 molecule (nucleotides 760 to 882, GenBank NM006139.2), a 4-1BB molecule (nucleotides 886 to 1026, GenBank NM001561.5), and a CD3 zeta molecule (nucleotides 299 to 634, GenBank NM000734.3), followed by 2A peptide (F2A) (derived from foot and mouth disease virus) and an eGFP molecule (nucleotides 5521 to 6237, GenBank KF957646.1). A gene encoding 20A10VH25VL-scFv-CAR (SEQ ID NO: 748) was synthesized by a method known to those skilled in the art. This sequence was ligated with pMSGV1 to produce a 20A10VH25VL-scFv-CD28-4-1BB-CD3 zeta-eGFP-CAR retrovirus vector (referred to as a 20A10VH25VL-scFv-CAR vector).

### (14-2) Preparation of universal CAR-T

The 20A10VH25VL-scFv-CAR vector constructed in (14-1) was used in the retroviral transduction method of human T cells to prepare universal CAR-T recognizing the antigen binding molecule obtained through the cleavage of the MMP-cleavable linker.

Specifically, first, GP2-293 packaging cell line (Takara Bio Inc.) was transfected with the 20A10VH25VL-scFv-CAR vector mentioned above and pAmpho plasmid (Takara Bio Inc.) using Lipofectamine 2000 or 3000 (Life Technologies Corp.) to prepare retrovirus harboring the 20A10VH25VL-scFv -CAR vector. 48 hours after the transfection, a supernatant containing the retrovirus was recovered and adsorbed onto two 24-well plates to prepare plates for transductions.

Subsequently, for the transduction of human T cells, 2 □ 10⁶ human peripheral blood mononuclear cells per well were cultured for 72 hours in the presence of IL-2 on a 6-well plate with an anti-CD3 monoclonal antibody and RetroNectin(R) (Takara Bio Inc.) immobilized thereon. The cells thus cultured were recovered, and cultured overnight on one of the plates for transductions with the 20A10VH25VL-scFv-CAR retrovirus adsorbed thereon, which were prepared as mentioned above, in the presence of IL-2. On the next day, the cells were transferred to the other plate for transduction and further cultured overnight to obtain human T cells harboring the 20A10VH25VL-scFv-CAR vector (20A10VH25VL-scFv-CAR-expressing T cells).

### (14-3) Confirmation of CAR protein expression rate

The cells thus transduced were maintained in the presence of human IL-2 and used in an experiment 7 to 8 days after the start of culture of the peripheral blood mononuclear cells. The surface expression of the 20A10VH25VL-scFv-CAR on the transduced human T cells was determined by the staining of the cells with CD8 and a biotin-labeled protease cleavage peptide (SEQ ID NO: 749), followed by flow cytometry. Approximately 60% of all T cells were confirmed to express 20A10VH25VL-scFv-CAR.

### [Example 15] Evaluation of in vitro cytotoxic activity of universal CAR-T against antigen binding molecule obtained through the cleavage of MMP-cleavable linker

The universal CAR-T cells against the antigen binding molecule obtained through the cleavage of the cleavable linker (20A10VH25VL-scFv-CAR-T) prepared in Example 14-2 were evaluated for cytotoxic activity using CytoFLEX S (Beckman Coulter Inc.). KYSE70 was provided as a negative control, and PC-10 was provided as target cells. Although both KYSE70 and PC-10 express GPC3, it was found from the results of Example 5(5-3) that KYSE70 can rarely cause cleavage of the MMP-cleavable linker, whereas PC-10 can cause cleavage of the MMP-cleavable linker. The negative control and the target cells were inoculated at 1 × 10⁵ cells and 1 × 10⁵ cells, respectively, to 24-well plates. 20A10VH25VL-scFv-CAR-T was used as effector cells and mixed at 1 × 10⁵ cells so as to attain an effector cell:target cell (E:T) ratio of 1:1. Subsequently, the anti-GPC3 antibody H0000-LK4.col2.LK4.G1T4/GL4-k0a containing a cleavable linker or H0000-G1T4/GL4-k0a containing no cleavable linker, prepared in Example 2 was added thereto. 48 hours after the addition, the CAR-T cells and the target cells were recovered. The recovered cells were applied to Zombie Yellow(TM) Fixable Viability Kit (BioLegend, 423104) to stain dead cells, and the CAR-T cells were stained with an anti-human CD45 antibody (BioLegend, Inc., 304039).

The cytotoxic activity was evaluated by the percentage of residual cancer cells.

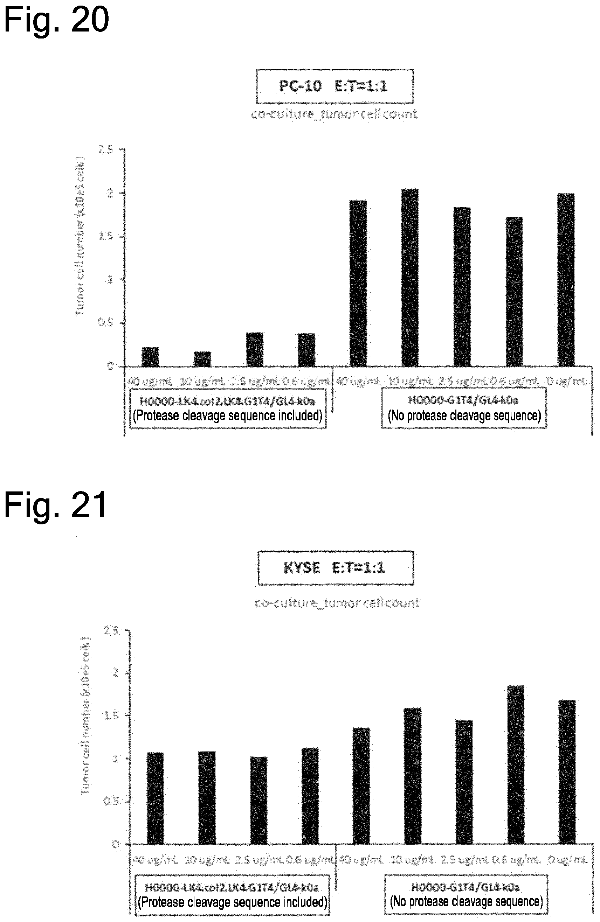

The percentage of residual cancer cells was calculated from the percentage of CD45⁻ fraction cells in live cells. The results are shown in Figures 20 and 21.

These results demonstrated the *in vitro* cytotoxic activity of the universal CAR-T cells binding to the antigen binding molecule obtained through the cleavage of the cleavable linker.

**[Table 7-1]**

| SEQ ID NO | Name | Amino acid sequence |
|---|---|---|
| SEQ ID NO: 726 | 20A10H | QIQLVQSGPELKEPGETVRISCKASGYTFTKYGINWVKQAPGKGLEW MAWINTYSGMTTYADDFKGRFAFSLETSANTAYLQINHLKNDDTAT YFCARSLGYDYGGFAYWGQGTLVTVSA |
| SEQ ID NO: 727 | F760nN17 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELRGGPKVFLFPPKPKDTLMISRTPEV TCVVVDVSHEDPEVKFNWYVDGVEVHNAKTKPREEQYASTYRVVS VLTVLHQDWLNGKEYKCKVSNKALPAPIEKTISKAKGQPREPQVYT LPPSRDELTKNQVSLTCLVKGFYPSDIAVEWESNGQPENNYKTTPPY LDSDGSFFLYSKLTVDKSRWQQGNVFSCSVMHEALHNHYTQESLSL SP |
| SEQ ID NO: 728 | 20A10L | DVLLTQTPLSLPVSLGDQAFISCRSGQTLVHDNENTYFHWYLQKPGQ SPKLLIYKISNRFSGVPDRFSGSGSGTDFTLKISRVEPEDLGIYFCSQNT HVPFTFGSGTKLEIK |
| SEQ ID NO: 729 | k0MT/k0a | |
| SEQ ID NO: 730 | 20952H | QSVEESGGRLVTPGTPLTLTCTVSGFSLSSYPMNWVRQAPGKGLEWI GGIGTSGNIWYASWAKGRFIISRASSTTVDLKVTSPTTEDTATYFCAR GLYNDYTVWGPGTLVTVSS |
| SEQ ID NO: 731 | 20952L | AQVLTQTPSSVSAAVGGTVTINCQASQSVYNKNYLAWYQQKPGQPP KRLIYSASTLASGVSSRFKGSGSGTQFTLTISDVQCDDVATYYCLGSY DCNRAECHAFGGGTKVVVEV |
| SEQ ID NO: 732 | TR01H11 3 | QVQLVESGGGVVQPGGSLRLSCAASGFTFSNAWMHWVRQAPGKGL EWVAQIKDKSQNYATYVAESVKGRFTISRADSKNSIYLQMNSLKTE DTAVYYCRYVHYAAGYGVDIWGQGTTVTVSS |
| SEQ ID NO: 733 | F760nP17 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELRGGPKVFLFPPKPKDTLMISRTPEV TCVVVDVSHEDPEVKFNWYVDGVEVHNAKTKPREEQYASTYRVVS VLTVLHQDWLNGKEYKCKVSNKALPAPIEKTISKAKGQPREPQVYT LPPSRKELTKNQVSLTCLVKGFYPSDIAVEWESNGQPENNYKTTPPY LDSDGSFFLYSKLTVDKSRWQQGNVFSCSVMHEALHNHYTQKSLSL SP |
| SEQ ID NO: 734 | L0011 | DIVMTQSPLSLPVTPGEPASISCRSSQPLVHSNRNTYLHWYQQKPGQ APRLLIYKVSNRFSGVPDRFSGSGSGTDFTLKISRVEAEDVGVYYCG QGTQVPYTFGQGTKLEIK |

**[Table 7-2]**

| | | |
|---|---|---|
| SEQ ID NO: 735 | Kn125 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPEYQWGPMVFLFPPKPKDTLMISRTPE VTCVVVDVSDEEPEVKFNWYVDGVEVHNAKTKPREEQYNATYRV VSVLTVLHQDWLNGKEYKCKVSNKALPAPIEKTISKAKGQPREPQV CTLPPSRDELTKNQVSLWCLVKGFYPSDIAVEWESNGQPENNYKTTP PVLDSDGSFFLYSKLTVDKSRWQQGNVFSCSVMHEALHNHYTQKSL SLSP |
| SEQ ID NO: 736 | Hl076 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEV TCVVVDVSHEEPEVKFNWYVDGVEVHNAKTKPREEQYNSTYRVVS VLTVLHQDWLNGKEYKCKVSNDALPMPIEETISKAKGQPREPQVYT LPPSRCELTKNQVSLSCAVKGFYPSDIAVEWESNGQPENNYKTTPPV LDSDGSFFLVSKLTVDKSRWQQGNVFSCSVMHEALHNHYTQKSLSL SP |
| SEQ ID NO: 737 | Hl076.G2 36E | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELLEGPSVFLFPPKPKDTLMISRTPEV TCVVVDVSHEEPEVKFNWYVDGVEVHNAKTKPREEQYNSTYRVVS VLTVLHQDWLNGKEYKCKVSNDALPMPIEETISKAKGQPREPQVYT LPPSRCELTKNQVSLSCAVKGFYPSDIAVEWESNGQPENNYKTTPPV LDSDGSFFLVSKLTVDKSRWQQGNVFSCSVMHEALHNHYTQKSLSL SP |
| SEQ ID NO: 738 | H0000 | QVQLVQSGAEVKKPGASVKVSCKASGYTFTDYEMHWIRQPPGQGL EWIGAIDPKTGDTAYSQKFKGRVTLTADKSTSTAYMELSSLTSEDTA VYYCTRFYSYTYWGQGTLVTVSS |
| SEQ ID NO: 739 | LK4.col2. LK4.G1T 4 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDGGSGGPPGPQGLAGQRGIVGLGSSGTHTCPPCPAPEL RRGPSVFLFPPKPKDTLMISRTPEVTCVVVDVSHEDPEVKFNWYVDG VEVHNAKTKPREEQYNSTYRVVSVLTVLHQDWLNGKEYKCKVSNK GLPSSIEKTISKAKGQPREPQVYTLPPSREEMTKNQVSLTCLVKGFYP SDIAVEWESNGQPENNYKTTPPVLDSDGSFFLYSKLTVDKSRWQEG NVFSCSVMHEALHNHYTQKSLSLSP |
| SEQ ID NO: 740 | GL4 | DIVMTQSPLSLPVTPGEPASISCRSSQSLVHSNRNTYLHWYQQKPGQ APRLLIYKVSNRFSGVPDRFSGSGSGTDFTLKISRVEAEDVGVYYCSQ NTHVPPTFGQGTKLEIK |

**[Table 7-3]**

| | | |
|---|---|---|
| SEQ ID NO: 741 | G1T4 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELRRGPSVFLFPPKPKDTLMISRTPEV TCVVVDVSHEDPEVKFNWYVDGVEVHNAKTKPREEQYNSTYRVVS VLTVLHQDWLNGKEYKCKVSNKGLPSSIEKTISKAKGQPREPQVYT LPPSREEMTKNQVSLTCLVKGFYPSDIAVEWESNGQPENNYKTTPPV LDSDGSFFLYSKLTVDKSRWQEGNVFSCSVMHEALHNHYTQKSLSL SP |
| SEQ ID NO: 742 | G1T0 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEV TCVVVDVSHEDPEVKFNWYVDGVEVHNAKTKPREEQYNSTYRVVS VLTVLHQDWLNGKEYKCKVSNKALPAPIEKTISKAKGQPREPQVYT LPPSREEMTKNQVSLTCLVKGFYPSDIAVEWESNGQPENNYKTTPPV LDSDGSFFLYSKLTVDKSRWQEGNVFSCSVMHEALHNHYTQKSLSL SP |
| SEQ ID NO: 743 | G1T3 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEV TCVVVDVSHEDPEVKFNWYVDGVEVHNAKTKPREEQYNSTYRVVS VLTVLHQDWLNGKEYKCKVSNKALPAPIEKTISKAKGQPREPQVYT LPPSREEMTKNQVSLTCLVKGFYPSDIAVEWESNGQPENNYKTTPPV LDSDGSFFLYSKLTVDKSRWQEGNVFSCSVMHEALHNHYTQKSLSL SP |
| SEQ ID NO: 744 | dGG1T3 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGPSVFLFPPKPKDTLMISRTPEVT CVVVDVSHEDPEVKFNWYVDGVEVHNAKTKPREEQYNSTYRVVSV LTVLHQDWLNGKEYKCKVSNKALPAPIEKTISKAKGQPREPQVYTL PPSREEMTKNQVSLTCLVKGFYPSDIAVEWESNGQPENNYKTTPPVL DSDGSFFLYSKLTVDKSRWQEGNVFSCSVMHEALHNHYTQKSLSLS P |
| SEQ ID NO: 745 | LK4.col2. LK4.G1T 0 | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDGGSGGPPGPQGLAGQRGIVGLGSSGTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPSREEMTKNQVSLTCLVKGFYP SDIAVEWESNGQPENNYKTTPPVLDSDGSFFLYSKLTVDKSRWQEG NVFSCSVMHEALHNHYTQKSLSLSP |
| SEQ ID NO: 746 | k0MTC | RTVAAPSVFIFPPSDEQLKSGTASVVCLLNNFYPREAKVQWKVDNA LQSGNSQESVTEQDSKDCTYSLSSTLTLSKADYEKHKVYACEVTHQ GLSSPVTKSFNRGEC |

**[Table 7-4]**

| | | |
|---|---|---|
| SEQ ID NO: 747 | 20A10VH 25VL-scFv | MGWSCIILFLVATATGVHS QIQLVQSGPELKEPGETVRISCKASGYTFTKYGINWVKQAPGKGLEW MAWINTYSGMTTYADDFKGRFAFSLETSANTAYLQINHLKNDDTAT YFCARSLGYDYGGFAYWGQGTLVTVSA SSADDAKKDAAKKDDAKKDDAKKDG DVLLTQTPLSLPVSLGDQAFISCRSGQTLVHDNENTYFHWYLQKPGQ SPKLLIYKISNRFSGVPDRFSGSGSGTDFTLKISRVEPEDLGIYFCSQNT HVPFTFGSGTKLEIK |
| SEQ ID NO: 748 | 20A10VH 25VL-scFv-CAR | MGWSCIILFLVATATGVHS QIQLVQSGPELKEPGETVRISCKASGYTFTKYGINWVKQAPGKGLEW MAWINTYSGMTTYADDFKGRFAFSLETSANTAYLQINHLKNDDTAT YFCARSLGYDYGGFAYWGQGTLVTVSA SSADDAKKDAAKKDDAKKDDAKKDG DVLLTQTPLSLPVSLGDQAFISCRSGQTLVHDNENTYFHWYLQKPGQ SPKLLIYKISNRFSGVPDRFSGSGSGTDFTLKISRVEPEDLGIYFCSQNT HVPFTFGSGTKLEIKAAAFVPVFLPAKPTTTPAPRPPTPAPTIASQPLS LRPEACRPAAGGAVHTRGLDFACDIYIWAPLAGTCGVLLLSLVITLY CNHRNRSKRSRLLHSDYMNMTPRRPGPTRKHYQPYAPPRDFAAYRS RFSVVKRGRKKLLYIFKQPFMRPVQTTQEEDGCSCRFPEEEEGGCEL RVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPREFGSGVKQTLNFDLLKLAGDVESN PGPCMVSKGEELFTGVVPILVELDGDVNGHKFSVSGEGEGDATYGK LTLKFICTTGKLPVPWPTLVTTLTYGVQCFSRYPDHMKQHDFFKSA MPEGYVQERTIFFKDDGNYKTRAEVKFEGDTLVNRIELKGIDFKEDG NILGHKLEYNYNSHNVYIMADKQKNGIKVNFKIRHNIEDGSVQLAD HYQQNTPIGDGPVLLPDNHYLSTQSALSKDPNEKRDHMVLLEFVTA AGITLGMDELYK |
| SEQ ID NO: 749 | | KSCDKTDGPSGAEGPPGPQG |

### INDUSTRIAL APPLICABILITY

The present invention provides a group of molecules that comprise an activated antibody that is produced by protease, have a short half-life in blood, and do not exert a pharmacological effect without cleavage by the protease, and antibody therapy having ADCC activity, bispecific antibody therapy and/or CAR-T therapy using the same.

Specifically, the group of molecules of the present invention specifically exert an effector function including ADCC or TDCC activity depending on both protease and a target antigen highly expressed at a lesion site. The antigen binding molecule obtained through the cleavage of the linker is rapidly metabolized when the cleavable linker is cleaved. Thus, there is low possibility that effector cells act on an antigen expressed in normal tissues. Desirable treatment with exceedingly high safety is attained even at the time of high-concentration administration.

## Claims

1. A pharmaceutical composition comprising a cell expressing a chimeric receptor, for use in combination with the administration of an antigen binding molecule, wherein
the antigen binding molecule comprises a linker that is cleavable by protease, and has the ability to bind to a target antigen after the cleavage of the linker, and
the chimeric receptor comprises an extracellular binding domain, a transmembrane domain and an intracellular signal transduction domain, and the extracellular binding domain has the ability to bind to the antigen binding molecule after cleavage of the linker, and is capable of binding to a cell expressing the target antigen via binding to the antigen binding molecule after the cleavage of the linker.

2. A pharmaceutical composition comprising an antigen binding molecule, for use in combination with the administration of a cell expressing a chimeric receptor, wherein
the antigen binding molecule comprises a linker that is cleavable by protease, and has the ability to bind to a target antigen after the cleavage of the linker,
the chimeric receptor comprises an extracellular binding domain, a transmembrane domain and an intracellular signal transduction domain, and the extracellular binding domain has the ability to bind to the antigen binding molecule after the cleavage of the linker, and is capable of binding to a cell expressing the target antigen via binding to the antigen binding molecule after the cleavage of the linker.

3. A pharmaceutical composition comprising a bispecific antibody, for use in combination with the administration of an antigen binding molecule, wherein
the antigen binding molecule comprises a linker that is cleavable by protease, and has the ability to bind to a target antigen after cleavage of the linker,
the bispecific antibody comprises antibody variable regions having binding activity against the antigen binding molecule after the cleavage of the linker by the protease, and antibody variable regions having binding activity against a molecule expressed on T cell surface, and
the bispecific antibody is capable of binding to a cell expressing the target antigen via binding to the antigen binding molecule after the cleavage of the linker.

4. A pharmaceutical composition comprising an antigen binding molecule, for use in combination with the administration of a bispecific antibody, wherein
the antigen binding molecule comprises a linker that is cleavable by protease, and has the ability to bind to a target antigen after the cleavage of the linker,
the bispecific antibody comprises antibody variable regions having binding activity against the antigen binding molecule after the cleavage of the linker by the protease, and antibody variable regions having binding activity against a molecule expressed on T cell surface, and
the bispecific antibody is capable of binding to a cell expressing the target antigen via binding to the antigen binding molecule after the cleavage of the linker.

5. A pharmaceutical composition comprising an IgG antibody having enhanced antibody-dependent cellular cytotoxicity, for use in combination with the administration of an antigen binding molecule, wherein
the antigen binding molecule comprises a linker that is cleavable by protease, and has binding activity against an antigen expressed on target cell surface after cleavage of the linker by the protease,
the IgG antibody comprises antibody variable regions having binding activity against the antigen binding molecule after cleavage of the linker by the protease, and
the IgG antibody is capable of binding to the target cell via binding to the antigen binding molecule after cleavage of the linker.

6. A pharmaceutical composition comprising an antigen binding molecule, for use in combination with the administration of an IgG antibody having enhanced antibody-dependent cellular cytotoxicity, wherein
the antigen binding molecule comprises a linker that is cleavable by protease, and has binding activity against an antigen expressed on target cell surface after cleavage of the linker by the protease, and
the IgG comprises antibody variable regions having binding activity against the antigen binding molecule after the cleavage of the linker by the protease, and
the IgG antibody is capable of binding to the target cell via binding to the antigen binding molecule after cleavage of the linker.

7. The pharmaceutical composition according to any one of claims 1 to 6, wherein a ratio of a K_{D} value of the antigen binding molecule after cleavage of the linker for the antigen to a K_{D} value of the antigen binding molecule before cleavage of the linker for the antigen (K_{D} (after cleavage)/K_{D} (before cleavage)) is 0.1 or less or 0.01 or less.

8. The pharmaceutical composition according to any one of claims 1 to 7, wherein the antigen binding molecule is an IgG antibody, an IgG antibody-like molecule, a heavy chain antibody or a single domain antibody.

9. The pharmaceutical composition according to any one of claims 1 to 8, wherein the antigen binding molecule comprises variable and constant regions of an antibody, and a linker that is cleaved by protease, wherein the antibody is selected from an IgG antibody, an IgG antibody-like molecule and a heavy chain antibody, and the antigen binding molecule obtained through the cleavage of the linker by the protease comprises an antigen binding domain and a portion of the cleaved linker.

10. The pharmaceutical composition according to any one of claims 1 to 9, wherein in the antigen binding molecule, the linker that is cleaved by protease is located near the boundary between the variable region and the constant region or near the boundary between CHI and CH2 in the constant region.

11. The pharmaceutical composition according to any one of claims 1 to 10, wherein the antigen binding molecule is an antibody or an IgG antibody-like molecule comprising a linker that is cleaved by protease, and the antigen binding molecule after cleavage of the linker is VL, VH, or VHH of the antibody or an antigen binding fragment thereof.

12. The pharmaceutical composition according to any one of claims 1 to 11, wherein the antigen binding molecule is a single domain antibody comprising a linker that is cleaved by protease, and the antigen binding molecule after cleavage of the linker is an antigen binding domain of the single domain antibody and a portion of the linker.

13. The pharmaceutical composition according to any one of claims 1 to 12, wherein the linker that is cleaved by protease comprises a protease cleavage sequence.

14. The pharmaceutical composition according to any one of claims 1 to 12, wherein the linker that is cleaved by protease comprises a peptide having any of the protease cleavage sequences of SEQ ID NOs: 1 to 725.

15. The pharmaceutical composition according to any one of claims 1 to 14 for use in the treatment or prevention of a cancer.
